(19) Europäisches Patentamt / European Patent Office / Office européen des brevets

(11) EP 4 678 646 A1

(12) EUROPEAN PATENT APPLICATION
published in accordance with Art. 153(4) EPC

(43) Date of publication:
**14.01.2026 Bulletin 2026/03**

(21) Application number: **24784331.1**

(22) Date of filing: **03.04.2024**

(51) International Patent Classification (IPC):
***C07H 21/04*** *(2006.01)* ***C07D 239/54*** *(2006.01)*
***C07F 9/141*** *(2006.01)* ***C07F 9/22*** *(2006.01)*
***C07F 9/32*** *(2006.01)* ***A61P 43/00*** *(2006.01)*

(52) Cooperative Patent Classification (CPC):
**A61P 43/00; C07D 239/54; C07F 9/141; C07F 9/22; C07F 9/32; C07H 21/04**

(86) International application number:
**PCT/CN2024/085752**

(87) International publication number:
**WO 2024/208249 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.04.2023 PCT/CN2023/086603**
**02.11.2023 PCT/CN2023/129195**

(71) Applicant: **Shanghai Argo Biopharmaceutical Co., Ltd.**
**Shanghai 201803 (CN)**

(72) Inventors:
- **SHAO, Pengcheng Patrick**
**Warrington Township Pennsylvania 18976 (US)**
- **SHU, Dongxu**
**Ningbo, Zhejiang 315100 (CN)**
- **GU, Kaichun**
**Shanghai 201210 (CN)**

(74) Representative: **Lavoix**
**Bayerstraße 83**
**80335 München (DE)**

Remarks:
The complete document including Reference Table(s) and the Sequence Listing(s) can be downloaded from the EPO website

# (54) NUCLEOSIDE ANALOG FOR 5'-PHOSPHONATE MODIFICATION AND OLIGONUCLEOTIDE PREPARED THEREFROM

(57) The present invention relates to a nucleoside analog for 5' modification and an oligonucleotide prepared therefrom, and more particularly to a modification nucleoside and an analog thereof that can be incorporated into the end of an oligonucleotide, and may be incorporated into a double-stranded oligonucleotide (short interfering RNA) or single-stranded oligonucleotide (e.g., an antisense oligonucleotide). The oligonucleotide provided is expected to hybridize with a portion of a target RNA, thereby resulting in loss of the normal function of the target RNA.

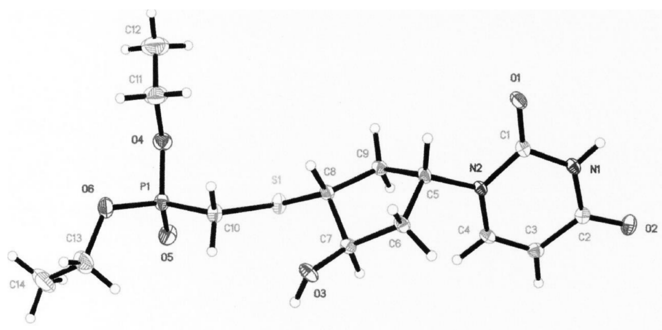

FIG. 1



Processed by Luminess, 75001 PARIS (FR)

## Description

### TECHNICAL FIELD

The present disclosure relates to a 5'-phosphonate-modified nucleoside analog and an oligonucleotide prepared therefrom, and more particularly to a modified nucleoside and an analog thereof that can be incorporated into an end of an oligonucleotide. Such an oligonucleotide may also be contained in a double-stranded composition.

### BACKGROUND

In recent years, oligonucleotides containing a nucleotide sequence that is at least partially complementary to the target RNA can alter the function and activity of the target both *in vitro* and *in vivo.* It has been demonstrated that when delivered into cells containing the target RNA (e.g., messenger RNA (mRNA)), such oligonucleotides function as antisense compounds to modulate the expression of the target, leading to changes in the transcription or translation of the target RNA. This technique is generally considered as an antisense technique. The antisense technique is based on the principle that antisense compounds hybridize with the target RNA and modulate gene expression activity or function, such as transcription or translation. The modulation of gene expression can be achieved through, for example, target degradation or occupancy-mediated inhibition. One example of modulating RNA target function via degradation is the RNase H-based degradation of the target RNA following hybridization with DNA antisense compounds. Another example of modulating gene expression via target degradation is RNA interference (RNAi). RNAi is a biological process of RNA or RNA-like molecules (e.g., chemically modified RNA molecules) silencing gene expression via degradation. The RNAi mechanism is initiated by the Dicer enzyme-mediated production of RNA molecules from longer non-coding RNAs. These RNA molecules are then loaded into the RNA-induced silencing complex (RISC), where the sense strand is discarded, and the antisense strand or guide strand hybridizes with a fully or partially complementary mRNA sequence, thereby inducing mRNA silencing through Ago2-mediated degradation or translational inhibition. Advances in RNAi techniques and delivery methods have yielded increasingly positive results for RNAi-based therapies, and such therapies represent a promising direction for disease treatment. However, the widespread application of this technique is hindered by the inherent metabolic issues of natural RNAs, such as the susceptibility of natural oligonucleotides to degradation by intracellular and extracellular nucleases, which compromises targeting property and *in vivo* stability. Thus, existing techniques focus on nucleotide modifications, particularly those that help improve nuclease resistance, binding affinity, targeting property, and *in vivo* stability.

Significant progress has been made by developing various chemical modifications for RNAi oligonucleotides, addressing the inherent metabolic issues of natural RNAs. These chemical modifications for RNAi oligonucleotides are crucial promoters in fully exploiting the potential of such therapeutic regimens, as they can improve pharmacokinetic and pharmacodynamic properties. For example, Choung et al. introduced 2'-OMe (2'-O-methyl), 2'-F (2'-fluoro), and phosphorothioate modifications, as well as their different combinations, to nucleotides, thereby conferring stability on the nucleotides in serum. Additionally, a class of 5'-modified phosphonate monomers and their use in the preparation of oligonucleotides have been described. A phosphonate-modified nucleotide at the 5'-end of an antisense strand can increase the likelihood of the 5'-end of the oligonucleotide being phosphorylated and maintaining the phosphorylation, which can increase the likelihood of loading a specific strand into RISC, thereby enabling an RNAi agent to be in the RNAi pathway and leading to improvements and enhancements to gene knockdown and gene silencing activity. For example, the incorporation of ethenyl phosphonate furanose monomers for the 5'-end of the antisense strand in WO2011139702 or the cycloalkyl phosphonate furanose monomers in WO2017214112 have also been a focus of research in recent years.

Although significant progress has been made in overcoming the inherent metabolic problems of natural oligonucleotides through the development of chemical modifications and improved delivery methods, there remains a need in the art for oligonucleotides that are suitable for therapeutic administration and exhibit enhanced expression inhibition duration and/or activity.

### SUMMARY

The present disclosure provides a 5'-phosphonate-modified nucleoside analog and an oligonucleotide prepared therefrom, and further provides a 5'-phosphonate-modified nucleoside analog that can be incorporated into an end of an oligonucleotide, particularly the 5'-end of an oligonucleotide, to obtain a double-stranded oligonucleotide (e.g., dsRNA) or a single-stranded oligonucleotide (e.g., antisense oligonucleotide), resulting in improvements and enhancements to gene silencing activity and/or duration. An oligonucleotide, such as an RNAi agent, comprising one or more 5'-phosphonate-modified nucleoside analogs may be further linked to a targeting ligand, such as N-acetylgalactosamine or a peptide, or may be further linked to a pharmacokinetic modulator, such as a polyethylene glycol (PEG) moiety or a lipid.

According to one aspect of the present disclosure, provided are compounds represented by formula (I), formula

(II), and formula (III), or stereoisomers thereof:

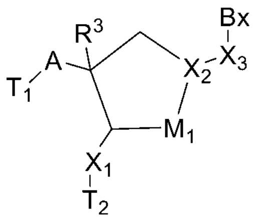 formula (I), 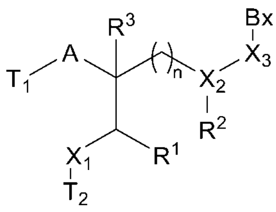 formula (II), 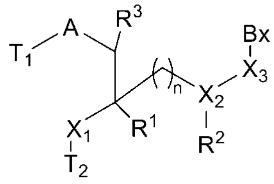

formula (III)

wherein:

each $T_1$ is independently an optionally protected phosphine moiety;
each $T_2$ is independently an active phosphorus group;
each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
each $X_2$ is independently $CR^{15}$ or N;
each $X_3$ is independently a chemical bond, optionally substituted $C_1$-$C_3$ alkylene, SO, $SO_2$, C(=O), or P(=O)R; R is OH, SH, $C_1$-$C_6$ alkyl, $NH_2$, or $NHSO_2CH_3$;
each Bx is independently a heterocyclic base moiety;
each $R^1$ and each $R^2$ are each independently H, halogen, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, sulfinyl, sulfonyl, or acetyl;
each $R^3$ and each $R^{15}$ are each independently H, halogen, optionally substituted $C_1$-$C_6$ alkyl, optionally substituted $C_1$-$C_6$ alkoxy, optionally substituted $C_2$-$C_6$ alkenyl, or optionally substituted $C_2$-$C_6$ alkynyl;
each A independently has one of the following formulas:

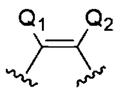 , 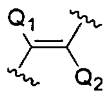 , 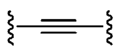 , 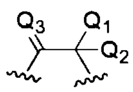 , 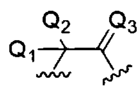 , 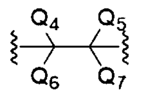 , 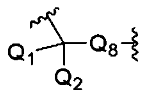 ,

and

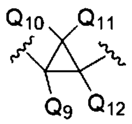 ,

wherein each $Q_1$ and each $Q_2$ are each independently H, halogen, -CN, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^4R^5$;
$Q_3$ is O, S, $NR^6$, or $CR^7R^8$;
$Q_4$, $Q_5$, $Q_6$, $Q_7$, $Q_9$, $Q_{10}$, $Q_{11}$, and $Q_{12}$ are each independently H, halogen, optionally protected hydroxyl, acetoxy, azido, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^9R^{10}$;
$Q_8$ is O, S, SO, $SO_2$, $PR^{16}R^{17}$, or $NR^{11}$;
$R^{16}$ and $R^{17}$ are independently (=O), (=S), OH, SH, $C_1$-$C_6$ alkyl, or $NR^{18}R^{19}$;
each $R^4$, each $R^5$, each $R^6$, each $R^7$, each $R^8$, each $R^9$, each $R^{10}$, each $R^{11}$, each $R^{18}$, and each $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$;
$M_1$ is C(Rd)(Re) or C(Rd)(Re)C(Rf)(Rg), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$;
each $J_3$, each $J_4$, each $J_5$, and each $J_6$ are independently H or $C_1$-$C_6$ alkyl;
each n is independently 0, 1, or 2.

In certain embodiments, in the compounds, each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, each $T_1$ is independently an optionally protected phosphine moiety having the following formula:

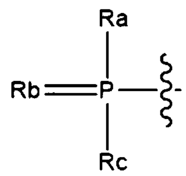

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

In certain embodiments, the sulfonyl is preferably methanesulfonyl.

In certain embodiments, each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

Examples of protecting groups commonly used for protecting phosphorus hydroxyl groups or phosphorus sulfhydryl groups include, but are not limited to, methyl, ethyl, benzyl (Bn), phenyl, isopropyl, tert-butyl, acetyl, chloroacetyl, trichloroacetyl, trifluoroacetyl, pivaloyl, tert-butoxymethyl, methoxymethyl, 1-ethoxyethyl, 1-(2-chloroethoxy) ethyl, 2-trimethylsilylethyl, allyl, cyclohexyl (cHex), 9-fluorenylmethoxycarbonyl, methanesulfonyl, p-toluenesulfonyl, trifluoromethanesulfonyl, benzoyl, benzoylformate, p-phenylbenzoyl, 4-methoxybenzyl, monomethoxytrityl, dimethoxytrityl, trimethoxytrityl, 4-chlorobenzyl, 4-nitrobenzyl, 2,4-dinitrophenyl, 4-acyloxybenzyl, 2-methylphenyl, 2,6-dimethylphenyl, 2-chlorophenyl, 2,6-dichlorobenzyl, diphenylmethyl, triphenylmethyl, 4-methylthio-1-butyl, 2-(S-acetylthio)ethyl (SATE), 2-cyanoethyl, 2-cyano-1,1-dimethylethyl (CDM), 4-cyano-2-butenyl, 2-(trimethylsilyl)ethyl (TSE), 2-(phenylthio) ethyl, 2-(triphenylsilyl)ethyl, 2-(benzylsulfonyl)ethyl, 2,2,2-trichloroethyl, 2,2,2-tribromoethyl, 2,3-dibromopropyl, 2,2,2-trifluoroethyl, phenylthio, 2-chloro-4-tritylphenyl, 2-bromophenyl, 2-[N-isopropyl-N-(4-methoxybenzoyl)amino]ethyl, 4-(N-trifluoroacetylamino)butyl, 4-oxopentyl, 4-tritylaminophenyl, 4-benzylaminophenyl, tetrahydropyranyl, morpholino, trimethylsilyl, triethylsilyl, tert-butyldimethylsilyl, tert-butyldiphenylsilyl, triphenylsilyl, triisopropylsilyl, pivaloyloxymethyl (POM), and 9-phenylxanthine-9-yl.

Examples commonly used for amino-protecting groups include, but are not limited to, 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), benzyl, formyl, acetyl, pivaloyl, trihaloacetyl, benzoyl, nitrophenyl, acetyl, 2-nitrobenzenesulfonyl, phthalimido (Pht), p-toluenesulfonyl (Tos), trityl (Trt), 2,4-dimethoxybenzyl (PMB), and dithiosuccinyl.

In certain embodiments, each $T_1$ is independently an optionally protected phosphine moiety having the following formula:

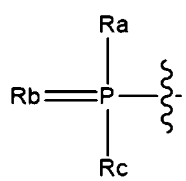

wherein:

Ra and Rc are each independently selected from protected hydroxyl and protected sulfhydryl, and $R_b$ is O or S.

In one embodiment, protecting groups for the hydroxyl are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, methanesulfonyl, p-toluenesulfonyl,

trifluoromethanesulfonyl, trityl, monomethoxytrityl, dimethoxytrityl, trimethoxytrityl, pivaloyloxymethyl (POM), and substituted 9-phenylxanthine-9-yl. In one embodiment, preferred protecting groups for the hydroxyl are each independently selected from acetyl, benzyl, tert-butyldimethylsilyl, pivaloyloxymethyl (POM), tert-butyldiphenylsilyl, and 4,4'-dimethoxytrityl.

In one embodiment, protecting groups for the sulfhydryl are each independently selected from methyl, ethyl, acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, methanesulfonyl, p-toluenesulfonyl, trifluoromethanesulfonyl, trityl, monomethoxytrityl, dimethoxytrityl, trimethoxytrityl, and substituted 9-phenylxanthine-9-yl. In one embodiment, preferred protecting groups for the sulfhydryl are each independently selected from benzyl, 4,4'-dimethoxytrityl, and trityl.

In certain embodiments, $R_b$ is O or S, and Ra and Rc are each independently selected from protected hydroxyl, $C_1$-$C_6$ alkyl, and $C_1$-$C_6$ alkoxy.

In certain embodiments, $R_b$ is O, and Ra and Rc are each independently selected from OH, SH, $OCH_3$, $OCH_2CH_3$, $OCH(CH_3)_2$, $OCH_2OC(=O)C(CH_3)_3$, $OCH_2CH_2CN$, and $NHSO_2CH_3$.

In certain embodiments, one of Ra and Rc is hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and the other is a natural or modified nucleoside; and $R_b$ is O, S, or $NR^{12}$, wherein $R^{12}$ is hydrogen, $C_1$-$C_6$ alkyl, or an amino-protecting group. More preferably, one of Ra and Rc is hydroxyl or protected hydroxyl, and the other is a natural nucleoside; more preferably, $R_b$ is O.

In certain embodiments, each $T_2$ is independently an active phosphorus group having the following structure:

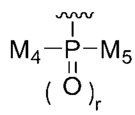

,

wherein $M_4$ is H, optionally substituted $C_1$-$C_6$ alkyl, OH, $OJ_7$, SH, $SJ_7$, or $NJ_7J_8$, and $M_5$ is optionally substituted $C_1$-$C_6$ alkyl, OH, $OJ_7$, SH, $SJ_7$, or $NJ_7J_8$, wherein each $J_7$ or $J_8$ is independently and optionally substituted $C_1$-$C_6$ alkyl or sulfonyl; r is 0 or 1.

In certain embodiments, each optionally substituted group of the active phosphorus group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, each $J_7$ or $J_8$ is independently substituted $C_1$-$C_6$ alkyl, and a substituent is selected from cyano and halogen.

In certain embodiments, $M_4$ is selected from methyl, ethyl, propyl, and isopropyl.

In certain embodiments, $M_4$ is selected from methanesulfonamido.

In certain embodiments, $M_4$ is $OJ_7$, wherein $J_7$ is substituted $C_1$-$C_6$ alkyl, and a substituent is selected from cyano and halogen.

In certain embodiments, $M_5$ is selected from $N(CH(CH_3)_2)_2$.

In certain embodiments, $M_4$ is $O(CH_2)_2CN$; $M_5$ is $N(CH(CH_3)_2)_2$; r is 0.

In certain embodiments, each $T_2$ active phosphorus group is independently a phosphoramidite.

In certain embodiments, each $T_2$ active phosphorus group is independently selected from diisopropylcyanoethoxy phosphoramidite, diisopropylethyl phosphoramidite, and H-phosphonate.

In certain embodiments, each $X_1$ is independently O.

In certain embodiments, each $B_X$ heterocyclic base moiety is independently selected from a natural nucleobase, a modified nucleobase, and a universal base.

In certain embodiments, each $B_X$ heterocyclic base moiety is independently pyrimidine, substituted pyrimidine, pseudouracil, substituted pseudouracil, purine, hypoxanthine, or substituted purine.

In certain embodiments, each $B_X$ heterocyclic base moiety is independently uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, each $R^{15}$ and each $R^3$ are each independently H.

In certain embodiments, each $R^1$ and each $R^2$ are each independently H, methanesulfonyl, or acetyl.

In certain embodiments, each $X_2$ is independently N, and each $R^1$ and each $R^2$ are each independently H, methanesulfonyl, or acetyl.

In certain embodiments, A in formula (I), formula (II), or formula (III) has one of the following formulas:

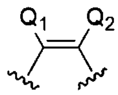, 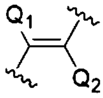, and 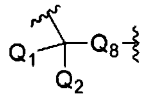,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$.

$R^{11}$, $R^{18}$, and $R^{19}$ are each independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, each $Q_1$ and each $Q_2$ are each independently H, F, -CN, or methyl, more preferably H.

In certain embodiments, $Q_8$ is S, SO, or $SO_2$.

In certain embodiments, a proviso is that when $M_1$ is C(Rd)(Re), $X_2$ is C, $X_3$ is a chemical bond, and A is

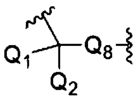,

$Q_8$ is S, SO, $SO_2$, $PR^{16}R^{17}$, or $NR^{11}$, and $Q_1$, $Q_2$, $R^{11}$, $R^{16}$, and $R^{17}$ are as defined in formula (I) herein.

In certain embodiments, $X_2$ is N, and $X_3$ is a chemical bond, $-CH_2-$, $-CH_2CH_2-$, SO, or $SO_2$.

In certain embodiments, $X_2$ is CH, and $X_3$ is a chemical bond.

In certain embodiments, in the compound of formula (II) or (III) provided herein, n is 0 or 1.

In certain embodiments, the compound provided herein has a compound represented by formula (I-1) or a stereoisomer thereof:

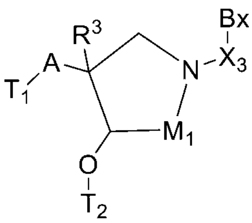

formula (I-1)

wherein $T_1$, $T_2$, A, $R^3$, and Bx are each as defined in formula (I) and the embodiments above; $X_3$ is a chemical bond, C(=O), P(=O)R, SO, or $SO_2$; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl; R is OH, SH, $C_1$-$C_6$ alkyl, $NH_2$, or $NHSO_2CH_3$.

In certain embodiments, in the compound of formula (I-1) provided herein, each Rd, each Re, each Rg, and each Rf are each independently selected from the following substituent groups: hydrogen, fluorine, hydroxyl, $C_1$-$C_6$ alkoxy, and $O(CH_2)_2$-$OCH_3$. More preferably, each Rd, each Re, each Rg, and each Rf are hydrogen.

In certain embodiments, in the compound of formula (I-1) provided herein, $R^3$ is hydrogen.

In certain embodiments, in the compound of formula (I-1) provided herein, $X_3$ is $SO_2$ or a chemical bond.

In certain embodiments, in the compound of formula (I-1) provided herein, A has one of the following formulas:

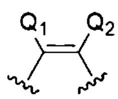, 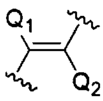, and 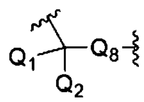,

wherein $Q_1$ and $Q_2$ are each independently H, halogen, or optionally substituted $C_1$-$C_6$ alkyl;
$Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$;
$R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, the compound provided herein has a compound represented by formula (I-2) or a stereoisomer thereof:

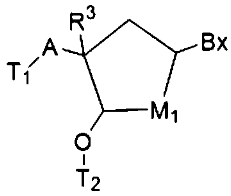

formula (I-2)

wherein $T_1$, $T_2$, A, $R^3$, and Bx are each as defined in formula (I) and the embodiments above; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl.

In certain embodiments, in the compound of formula (I-2) provided herein, each Rd, each Re, each Rg, and each Rf are each independently selected from the following substituent groups: hydrogen, fluorine, hydroxyl, $C_1$-$C_6$ alkoxy, and $O(CH_2)_2$-$OCH_3$.

In certain embodiments, in the compound of formula (I-2) provided herein, $M_1$ is $CH_2$ or $CH_2CH_2$.

In certain embodiments, in the compound of formula (I-2) provided herein, $R^3$ is hydrogen.

In certain embodiments, in the compound of formula (I-2) provided herein, A has one of the following formulas:

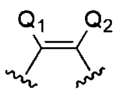 , 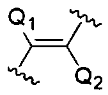 , and 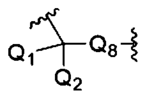 ,

wherein $Q_1$ and $Q_2$ are each independently H, halogen, or optionally substituted $C_1$-$C_6$ alkyl;
$Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, in the compound of formula (I-1) or formula (I-2) provided herein, $T_1$ is an optionally protected phosphine moiety having the following formula:

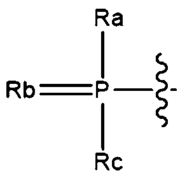

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group.

In certain embodiments, a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

In certain embodiments, the sulfonyl is preferably methanesulfonyl.

In certain embodiments, $R_b$ is O or S, and Ra and Rc are each independently selected from protected hydroxyl, $C_1$-$C_6$ alkyl, and $C_1$-$C_6$ alkoxy.

In certain embodiments, $R_b$ is O, and Ra and Rc are each independently selected from OH, $OCH_3$, $OCH_2CH_3$, $OCH(CH_3)_2$, $OCH_2OC(=O)C(CH_3)_3$, $OCH_2CH_2CN$, and $NHSO_2CH_3$.

In certain embodiments, in the compound of formula (I-1) or formula (I-2) provided herein, $T_2$ is an active phosphorus group, and the active phosphorus group is a phosphoramidite. In certain embodiments, the $T_2$ active

phosphorus group is selected from diisopropylcyanoethoxy phosphoramidite, diisopropylethyl phosphoramidite, and H-phosphonate.

In certain embodiments, in the compound of formula (I-1) or formula (I-2) provided herein, the $B_X$ heterocyclic base moiety is selected from a natural nucleobase, a modified nucleobase, and a universal base.

In certain embodiments, in the compound of formula (I-1) or formula (I-2) provided herein, the $B_X$ heterocyclic base moiety is pyrimidine, substituted pyrimidine, pseudouracil, substituted pseudouracil, purine, hypoxanthine, or substituted purine.

In certain embodiments, the $B_X$ heterocyclic base moiety is uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, the compound of formula (I-2) provided herein has a 1S,2S,4S or 1R,2R,4R stereoconfiguration.

In certain embodiments, the compound provided herein has a compound represented by formula (II-1) or a stereoisomer thereof:

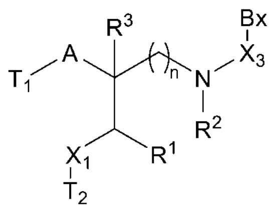

formula (II-1)

wherein $T_1$, $T_2$, $X_1$, $X_3$, A, $R^3$, Bx, and n are each as defined in formula (II) and the embodiments above; $R^1$ and $R^2$ are each independently H, methanesulfonyl, or acetyl.

In certain embodiments, in the compound of formula (II-1) provided herein, n is 0 or 1.

In certain embodiments, in the compound of formula (II-1) provided herein, $X_3$ is $CH_2$ or $CH_2CH_2$.

In certain embodiments, the compound provided herein has a compound represented by formula (II-2) or formula (II-3) or a stereoisomer thereof:

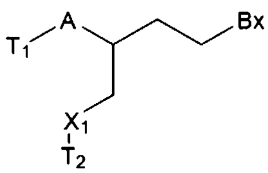

formula (II-2),

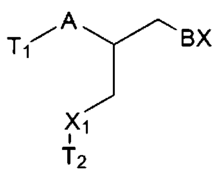

formula (II-3)

wherein $T_1$, $T_2$, $X_1$, A, and Bx are each as defined in formula (II) and the embodiments above.

In certain embodiments, the compound provided herein has a compound represented by formula (III-1) or formula (III-2) or a stereoisomer thereof:

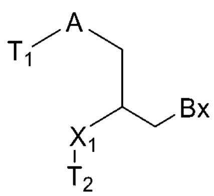

formula (III-1),

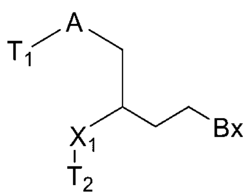

formula (III-2)

wherein $T_1$, $T_2$, $X_1$, A, and Bx are each as defined in formula (III) and the embodiments above.

In certain embodiments, A in formula (II-1), formula (II-2), formula (II-3), formula (III-1), or formula (III-2) has one of the following formulas:

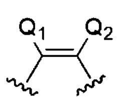

,

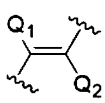

, and

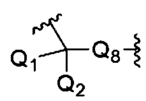

,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, the compound provided herein has formula (II-1), formula (II-2), formula (II-3), formula (III-1), or formula (III-2), wherein each $Q_1$ and each $Q_2$ are each independently H, F, -CN, or methyl, more preferably H.

In certain embodiments, the compound provided herein has formula (II-1), formula (II-2), formula (II-3), formula (III-I), or formula (III-2), wherein $T_1$ is an optionally protected phosphine moiety having the following formula:

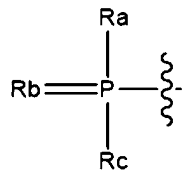

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group.

In certain embodiments, a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

In certain embodiments, the sulfonyl is preferably methanesulfonyl.

In certain embodiments, $R_b$ is O or S, and Ra and Rc are each independently selected from protected hydroxyl, $C_1$-$C_6$ alkyl, and $C_1$-$C_6$ alkoxy.

In certain embodiments, $R_b$ is O, and Ra and Rc are each independently selected from OH, $OCH_3$, $OCH_2CH_3$, $OCH(CH_3)_2$, $OCH_2OC(=O)C(CH_3)_3$, $OCH_2CH_2CN$, and $NHSO_2CH_3$.

In certain embodiments, in the compound of formula (II-1), formula (II-2), formula (II-3), formula (III-1), or formula (III-2) provided herein, $T_2$ is an active phosphorus group, and the active phosphorus group is a phosphoramidite. In certain embodiments, the $T_2$ active phosphorus group is selected from diisopropylcyanoethoxy phosphoramidite, diisopropylethyl phosphoramidite, and H-phosphonate.

In certain embodiments, in the compound of formula (II-1), formula (II-2), formula (II-3), formula (III-1), or formula (III-2) provided herein, the $B_X$ heterocyclic base moiety is selected from a natural nucleobase, a modified nucleobase, and a universal base.

In certain embodiments, in the compound of formula (II-1), formula (II-2), formula (II-3), formula (III-I), or formula (III-2) provided herein, the $B_X$ heterocyclic base moiety is pyrimidine, substituted pyrimidine, pseudouracil, substituted pseudouracil, purine, hypoxanthine, or substituted purine. In certain embodiments, the $B_X$ heterocyclic base moiety is uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In another aspect, the compound provided herein has a compound represented by formula (IV) or a stereoisomer thereof:

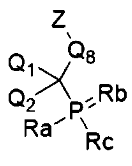

formula (IV)

wherein $Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{13}R^{19}$; Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, protected or optionally substituted amino, and a natural or modified nucleoside, and $R_b$ is O, S, or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;

$Q_1$ and $Q_2$ are each independently H, halogen, -CN, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^4R^5$;

each $R^4$, each $R^5$, each $R^{11}$, each $R^{18}$, and each $R^{19}$ are independently H, optionally substituted $C_1$-$C_6$ alkyl, optionally substituted $C_1$-$C_6$ alkoxy, methanesulfonyl, or sulfo;

Z is a nucleoside comprising a phosphoramidite or a sugar or sugar surrogate moiety.

In certain embodiments, in the compound of formula (IV) provided herein, each substituted group comprises one or more substituent groups optionally and independently selected from halogen, hydroxyl, $C_1$-$C_6$ alkyl, $C_1$-$C_6$ alkoxy, $C_1$-$C_6$ alkylsulfhydryl, and CN.

Hydroxyl-, sulfhydryl-, and amino-protecting groups may optionally be derived from those commonly used protecting groups described herein, such as, but not limited to, those described for formula (I).

In certain embodiments, in the compound of formula (IV) provided herein, $Q_8$ is SO or $SO_2$.

In certain embodiments, in the compound of formula (IV) provided herein, $Q_8$ is S.

In certain embodiments, in the compound of formula (IV) provided herein, $Q_1$ and $Q_2$ are each independently H.

In certain embodiments, in the compound of formula (IV) provided herein, in the nucleoside comprising a sugar or sugar surrogate moiety, the sugar or sugar surrogate moiety includes a 5-membered furanose ring, a non-furanose ring, or a 5- to 6-membered carbocyclic system, or an open system.

In certain embodiments, in the compound of formula (IV) provided herein, in the nucleoside comprising a sugar or sugar surrogate moiety, the sugar surrogate substitution moiety is morpholinyl, cyclohexenyl, cyclohexyl, cyclopentyl, pyranyl, or a cyclohexanehexol group. In certain embodiments, in the nucleoside comprising a sugar or sugar surrogate moiety, the sugar moiety is a furanose. In certain embodiments, the nucleoside comprising a sugar or sugar surrogate moiety comprises an unlocked nucleobase analog (UNA) or a glycerol nucleobase analog (GNA). In certain embodiments, the nucleoside comprising a sugar or sugar surrogate moiety comprises a locked nucleic acid (LNA) or a bridged nucleic acid (BNA).

In certain embodiments, in the compound of formula (IV) provided herein, $Q_8$ is bonded to the 4'-carbon or 5'-carbon of the sugar or sugar surrogate moiety.

In certain embodiments, in the compound of formula (IV) provided herein, the nucleoside comprising a sugar or sugar surrogate moiety has the following structural schematics:

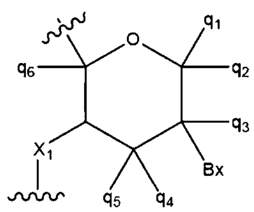 , 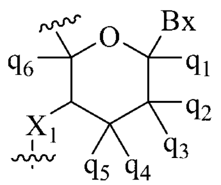 , 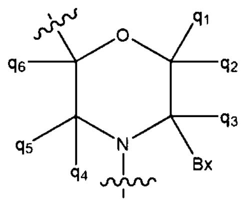 , 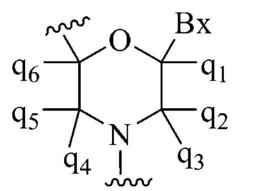 ,

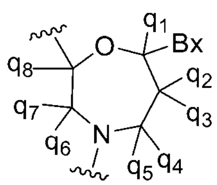 , 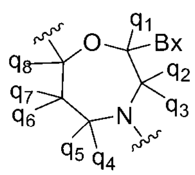 , 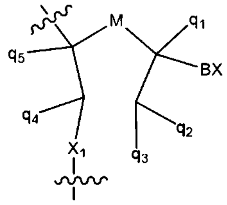 , 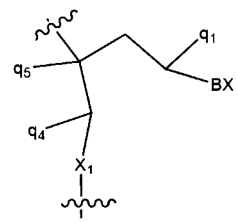 , 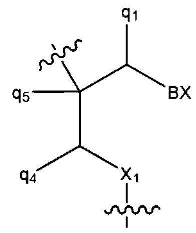 ,

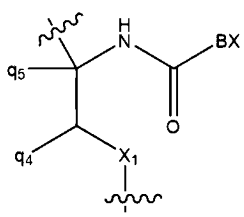 , 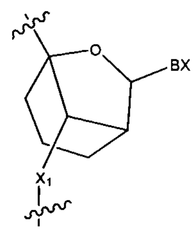 , 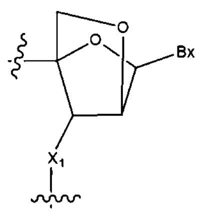 , 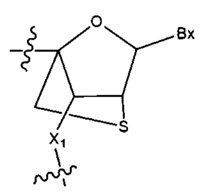 , 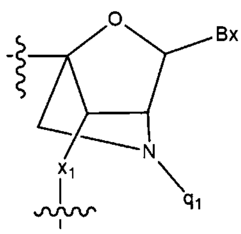 ,

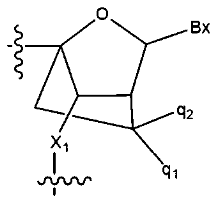, 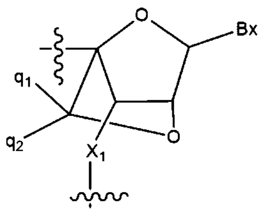, 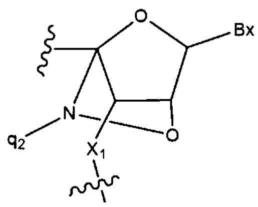, 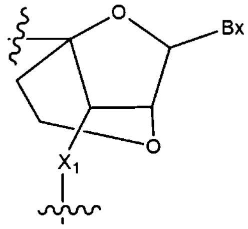,

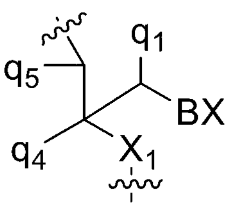, 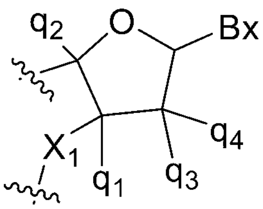, and 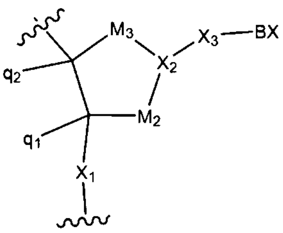,

wherein each $M_2$ is independently $C(q_3)(q_4)$ or $C(q_3)(q_4)C(q_5)(q_6)$;
each $M_3$ is independently O, S, $NR^{13}$, $C(q_7)(q_8)$, $C(q_7)(q_8)C(q_9)(q_{10})$, $C(q_7)=C(q_8)$, or $OC(q_7)(q_8)$;
each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
each $X_2$ is independently $CR^{15}$ or N;
each $X_3$ is independently a chemical bond, optionally substituted $C_1$-$C_3$ alkylene, SO, $SO_2$, C(=O), or P(=O)R; R is OH, SH, $C_1$-$C_6$ alkyl, $NH_2$, or $NHSO_2CH_3$;
each Bx is independently a heterocyclic base moiety;
$R^{15}$, $q_1$, $q_2$, $q_3$, $q_4$, $q_5$, $q_6$, $q_7$, $q_8$, $q_9$, and $q_{10}$ are each independently hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, CN, $OC(=O)J_5$, $OC(=O)N(J_5)(J_6)$, or $C(=O)N((J_5)(J_6)$, wherein $J_5$ and $J_6$ are independently H or $C_1$-$C_6$ alkyl;
each $R^{13}$ is independently hydrogen or $C_1$-$C_6$ alkyl.

In certain embodiments, each $R^{15}$, each $q_1$, each $q_2$, each $q_3$, each $q_4$, each $q_5$, each $q_6$, each $q_7$, each $q_8$, each $q_9$, and each $q_{10}$ are each independently selected from the following groups: hydrogen, fluorine, hydroxyl, $C_1$-$C_6$ alkyl, $C_1$-$C_6$ alkoxy, and $O(CH_2)_2$-$OCH_3$.

In certain embodiments, each $R^{15}$, each $q_1$, each $q_2$, each $q_3$, each $q_4$, each $q_5$, each $q_6$, each $q_7$, each $q_8$, each $q_9$, and each $q_{10}$ are each independently selected from the following group: hydrogen.

In certain embodiments, in the compound of formula (IV) provided herein, the nucleoside comprising a sugar or sugar surrogate moiety has the following structural schematics:

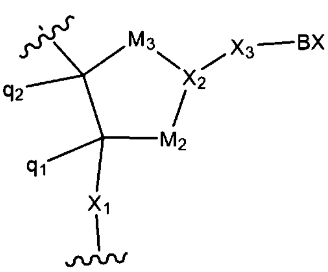

wherein $M_2$, $M_3$, $X_1$, $X_2$, $X_3$, $Q_1$, $q_2$, and Bx are each as defined in formula (IV) and the embodiments above.

In certain embodiments, in the compound of formula (IV) provided herein, the nucleoside comprising a sugar or sugar surrogate moiety has the following furanose structural schematic:

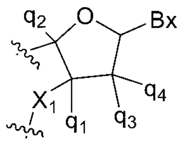

wherein $X_1$, $q_1$, $q_2$, $q_3$, $q_4$, and Bx are each as defined in formula (IV) and the embodiments above.

In certain embodiments, the compound provided herein has a compound represented by formula (IV-1) or a stereoisomer thereof:

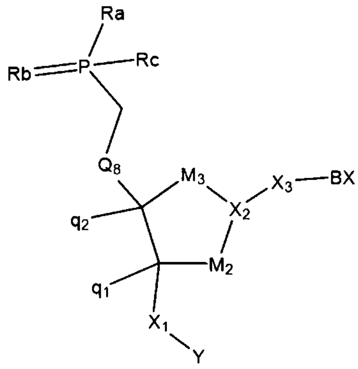

formula (IV-1)

wherein $Q_8$, Ra, Rc, $R_b$, $M_2$, $M_3$, $X_1$, $X_2$, $X_3$, $q_1$, $q_2$, and Bx are each as defined in formula (IV) and the embodiments above, and Y is a phosphoramidite.

In certain embodiments, in the compound of formula (IV-1) provided herein, $R^{15}$, $q_1$, $q_2$, $q_3$, $q_4$, $q_5$, $q_6$, $q_7$, $q_8$, $q_9$, and $q_{10}$ are each independently selected from hydrogen, fluorine, hydroxyl, methyl, methoxy, and $O(CH_2)_2$-$OCH_3$. In certain embodiments, in the compound of formula (IV-1) provided herein, $R^{15}$, $q_1$, $q_2$, $q_3$, $q_4$, $q_5$, $q_6$, $q_7$, $q_5$, $q_9$, and $q_{10}$ are each independently selected from hydrogen.

In certain embodiments, $M_3$ is O, S, $C(q_7)(q_8)$; $M_2$ is $C(q_3)(q_4)$; $X_2$ is $CR^{15}$ or N; $X_3$ is a chemical bond, SO, or $SO_2$; $R^{15}$, $q_1$, $q_2$, $q_3$, $q_4$, $q_7$, and $q_8$ are as defined herein and as shown in the following structural schematic:

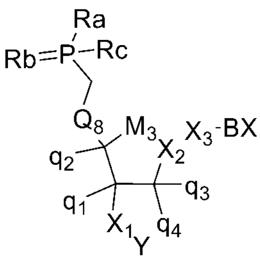

.

In certain preferred embodiments, $M_3$ is O, S, or $CH_2$; $M_2$ is $CH_2$; $X_2$ is CH; $X_3$ is a chemical bond. In certain preferred embodiments, $M_3$ is $CH_2$; $M_2$ is $CH_2$; $X_2$ is N; $X_3$ is a chemical bond, SO, or $SO_2$.

In certain embodiments, $M_3$ is $C(q_7)(q_8)$; $M_2$ is $C(q_3)(q_4)C(q_5)(q_6)$; $X_2$ is $CR^{15}$ or N; $X_3$ is a chemical bond, SO, or $SO_2$; $R^{15}$, $q_1$, $q_2$, $q_3$, $q_4$, $q_5$, $q_6$, $q_7$, and $q_8$ are as defined herein and as shown in the following structural schematic:

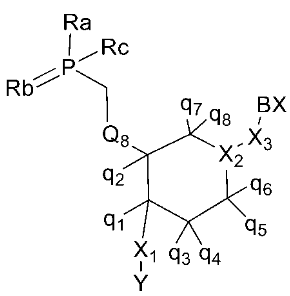

.

In certain preferred embodiments, $M_3$ is $CH_2$; $M_2$ is $CH_2CH_2$; $X_2$ is CH; $X_3$ is a chemical bond. In certain preferred embodiments, $M_3$ is $CH_2$; $M_2$ is $CH_2CH_2$; $X_2$ is N; $X_3$ is a chemical bond, SO, or $SO_2$.

In certain embodiments, in the compound of formula (IV-1) provided herein, $Q_8$ is SO or $SO_2$.

In certain embodiments, in the compound of formula (IV-1) provided herein, $Q_8$ is S.

In certain embodiments, in the compound of formula (IV-1) provided herein, $R_b$ is O or S, and Ra and Rc are each independently selected from protected hydroxyl, $C_1$-$C_6$ alkyl, and $C_1$-$C_6$ alkoxy.

In certain embodiments, in the compound of formula (IV-1) provided herein, Ra and Rc are each independently selected from hydroxyl, $OCH_3$, $OCH_2CH_3$, $OCH(CH_3)_2OCH_3$, $OCH_2CH_2CN$, and $NHSO_2CH_3$.

In certain embodiments, in the compound of formula (IV) or (IV-1) provided herein, the $B_X$ heterocyclic base moiety is selected from a natural nucleobase, a modified nucleobase, and a universal base.

In certain embodiments, in the compound of formula (IV) or (IV-1) provided herein, each $B_X$ heterocyclic base moiety is independently pyrimidine, substituted pyrimidine, pseudouracil, substituted pseudouracil, purine, hypoxanthine, or substituted purine. In certain embodiments, each $B_X$ heterocyclic base moiety is independently uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, in the compound of formula (IV) or (IV-1) provided herein, the phosphoramidite is selected from diisopropylcyanoethoxy phosphoramidite, diisopropylethyl phosphoramidite, and H-phosphonate.

In certain embodiments, the compounds provided herein have the following specific structures and stereoisomers thereof:

| | |
|---|---|
| 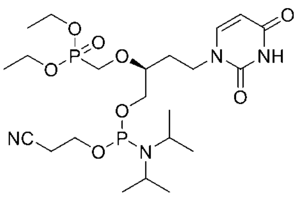 phosphoramidite-01 | 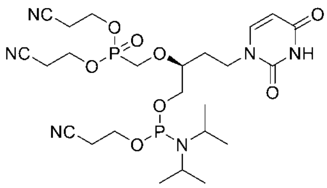 phosphoramidite-02 |
| 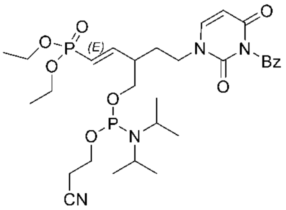 phosphoramidite-03 | 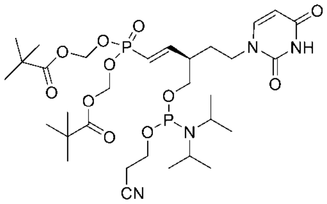 phosphoramidite-03-1 |
| 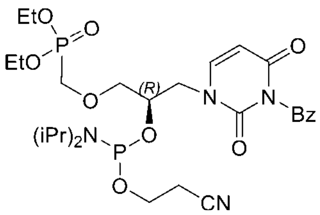 phosphoramidite-04 | 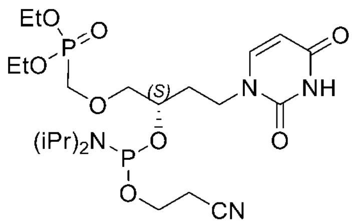 phosphoramidite-05 |
| 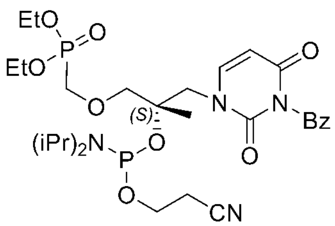 phosphoramidite-06 | 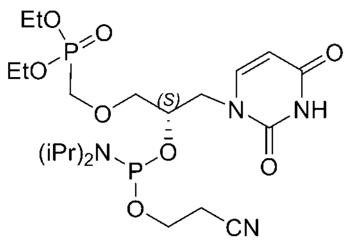 phosphoramidite-07 |
| 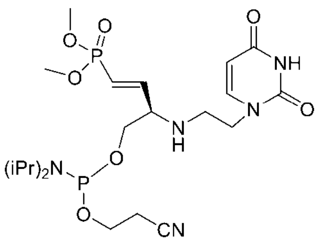 phosphoramidite-08 | 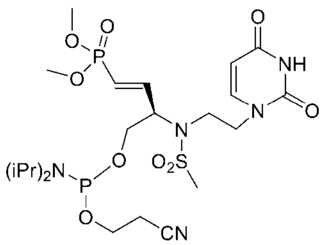 phosphoramidite-09 |
| 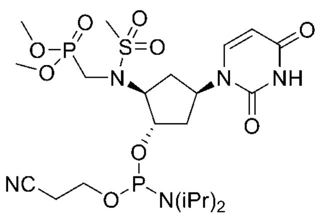 phosphoramidite-10 | 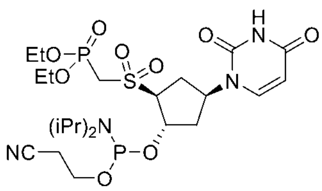 phosphoramidite-11-1 |

| | |
|---|---|
| 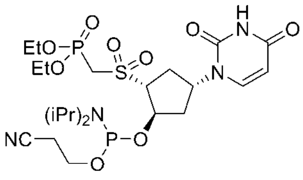 phosphoramidite-11-2 | 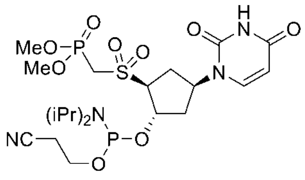 phosphoramidite-11-3 |
| 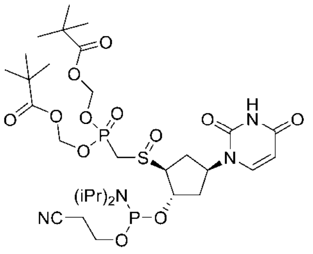 phosphoramidite-11-4 | 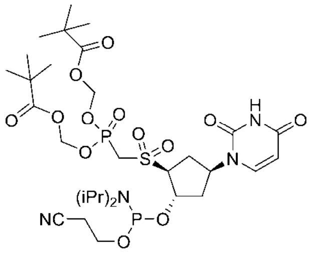 phosphoramidite-11-5 |
| 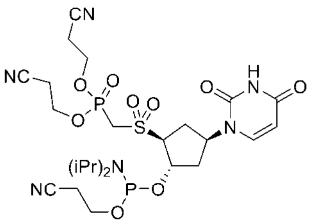 phosphoramidite-11-6 | 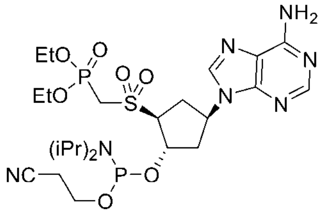 phosphoramidite-11-7 |
| 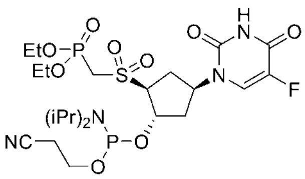 phosphoramidite-11-8 | 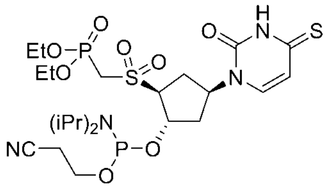 phosphoramidite-11-9 |
| 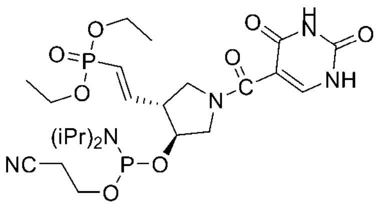 phosphoramidite-19-5 | 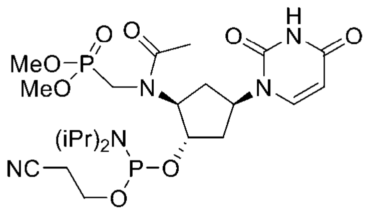 phosphoramidite-12-1 |
| 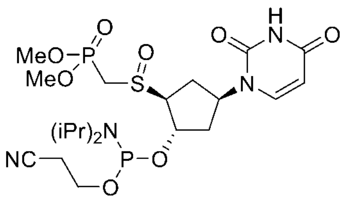 phosphoramidite-12 | 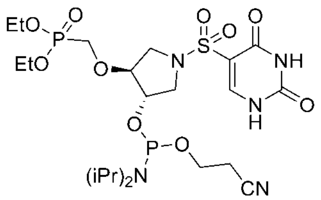 phosphoramidite-13 |
| 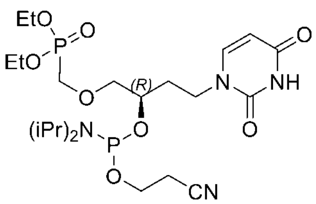 phosphoramidite-14 | 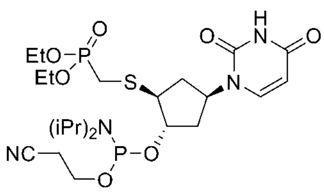 phosphoramidite-15-1 |

| | |
|---|---|
| 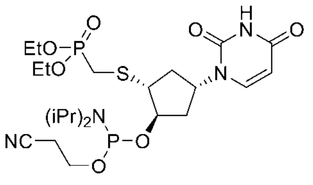 phosphoramidite-15-2 | 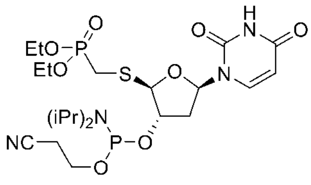 phosphoramidite-15-3 |
| 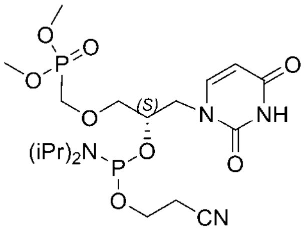 phosphoramidite-07-1 | 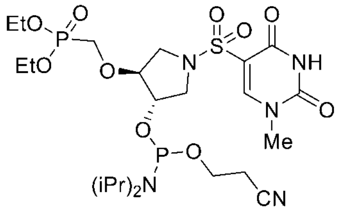 phosphoramidite-18 |
| 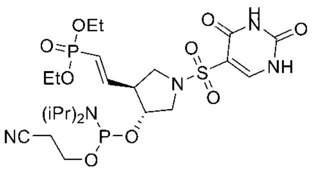 phosphoramidite-19-1 | 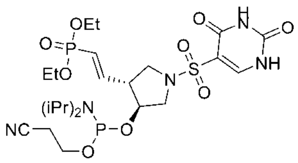 phosphoramidite-19-2 |
| 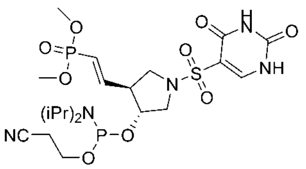 phosphoramidite-19-3 | 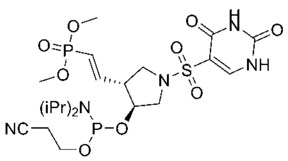 phosphoramidite-19-4 |
| 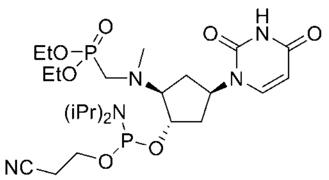 phosphoramidite-21 | 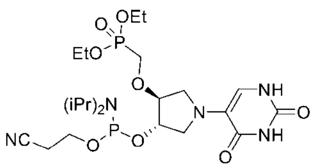 phosphoramidite-22 |
| 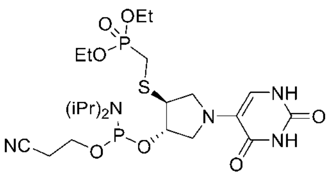 phosphoramidite-23 | 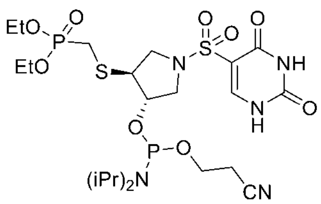 phosphoramidite-26 |
| 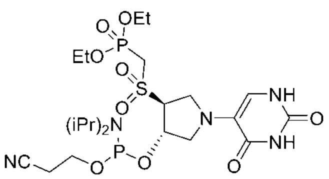 phosphoramidite-25 | 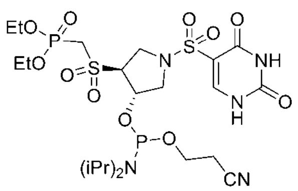 phosphoramidite-24 |
| 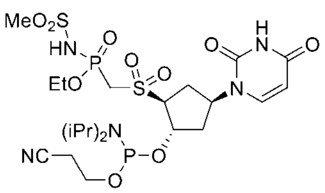 phosphoramidite-27 | 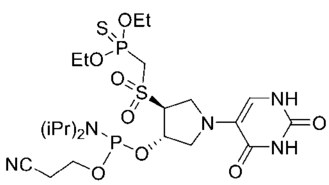 phosphoramidite-28 |

| | |
|---|---|
| 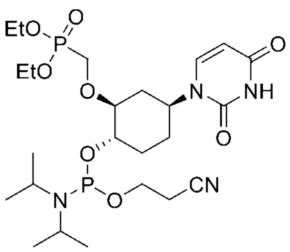 phosphoramidite-29 | 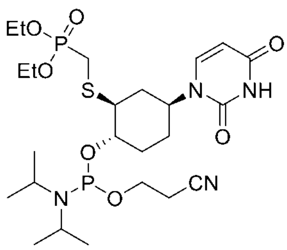 phosphoramidite-30 |
| 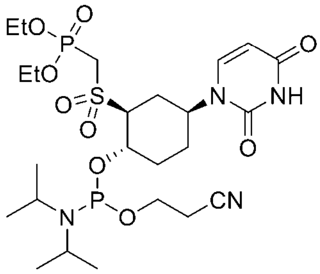 phosphoramidite-31 | 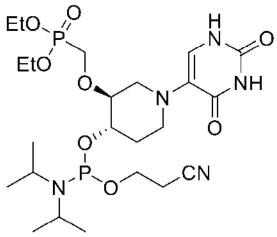 phosphoramidite-32 |
| 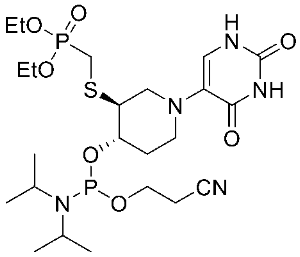 phosphoramidite-33 | 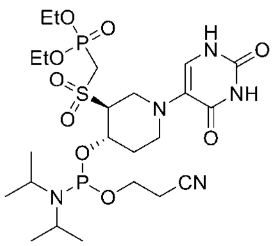 phosphoramidite-34 |
| 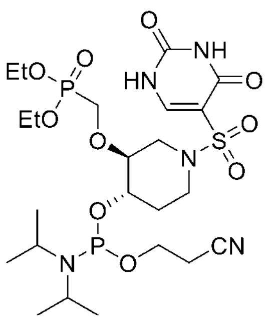 phosphoramidite-35 | 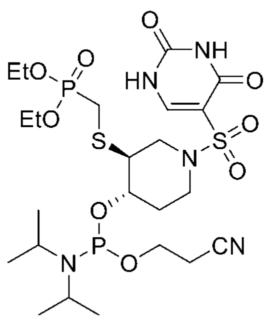 phosphoramidite-36-1 |
| 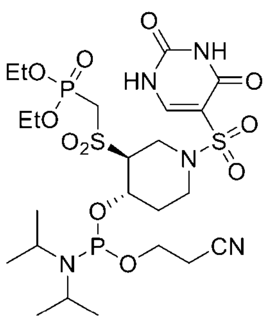 phosphoramidite-37 | 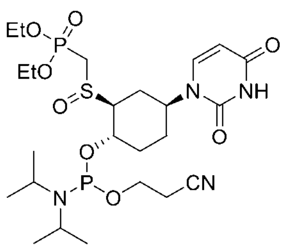 phosphoramidite-38 |

| | |
|---|---|
| 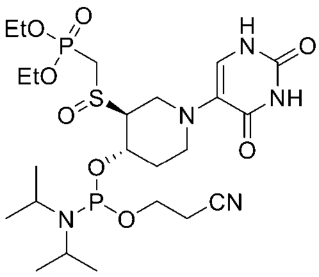 phosphoramidite-39 | 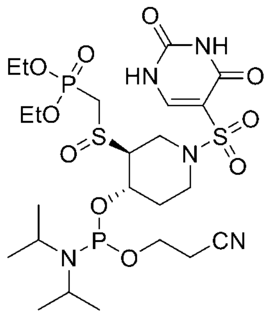 phosphoramidite-40 |
| 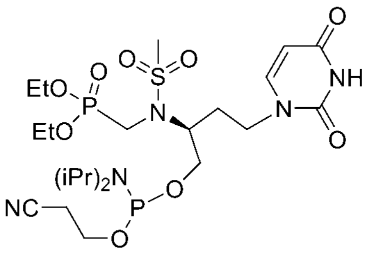 phosphoramidite-41 | 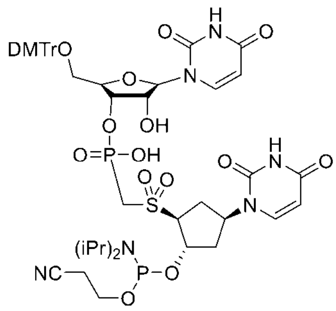 phosphoramidite-42-3 |
| 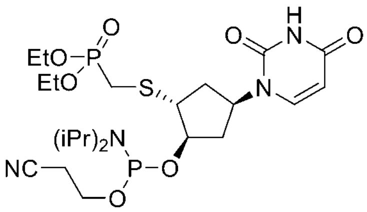 phosphoramidite-42-1 | 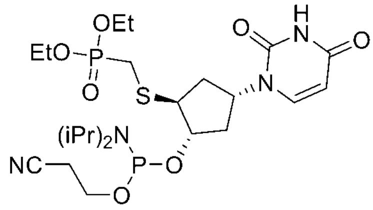 phosphoramidite-42-2 |
| 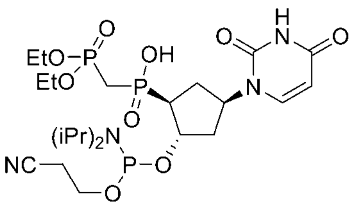 phosphoramidite-43-1 | 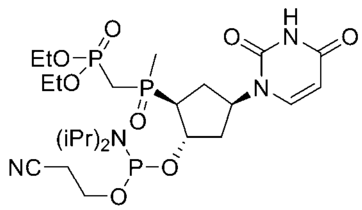 phosphoramidite-44-1 |
| 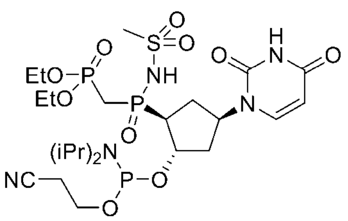 phosphoramidite-45-1 | 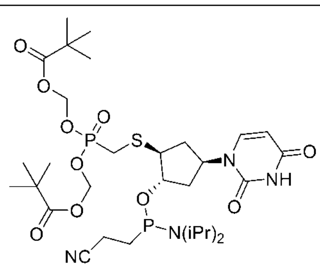 phosphoramidite-15-4 |

| | |
|---|---|
| 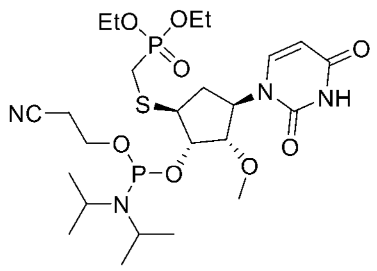<br>phosphoramidite-43 | 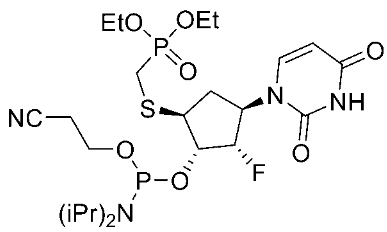<br>phosphoramidite-44 |
| 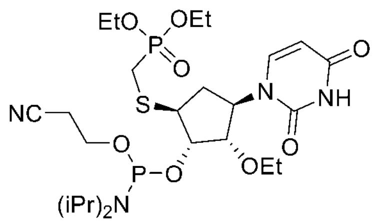<br>phosphoramidite-45 | 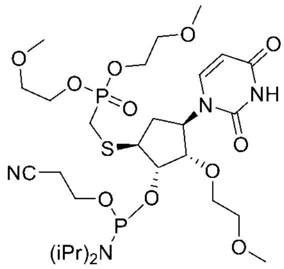<br>phosphoramidite-46 |
| 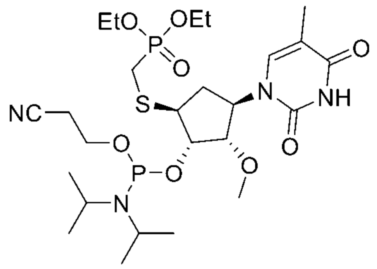<br>phosphoramidite-47 | 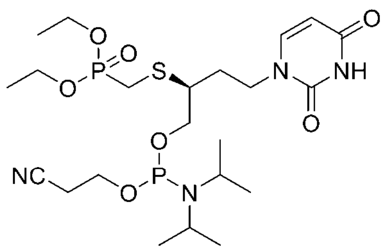<br>phosphoramidite-1-1 |
| 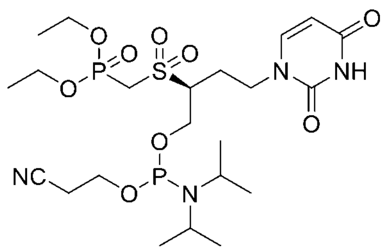<br>phosphoramidite-1-2 | 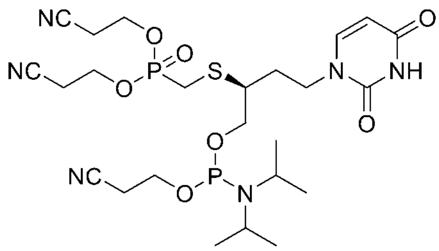<br>phosphoramidite-2-1 |
| 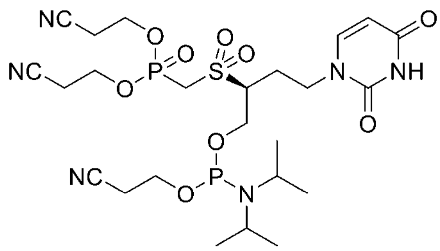<br>phosphoramidite-2-2 | 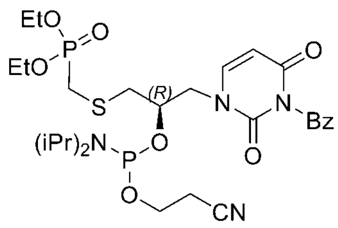<br>phosphoramidite-4-1 |

| | |
|---|---|
| 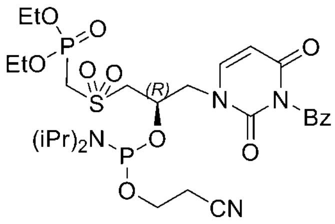 phosphoramidite-4-2 | 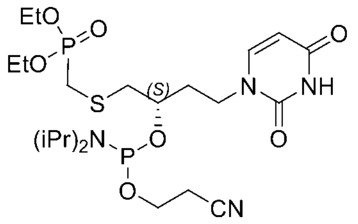 phosphoramidite-5-1 |
| 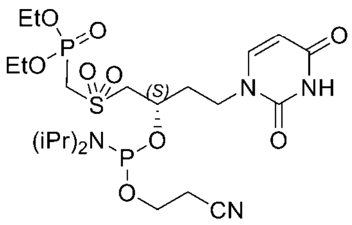 phosphoramidite-5-2 | 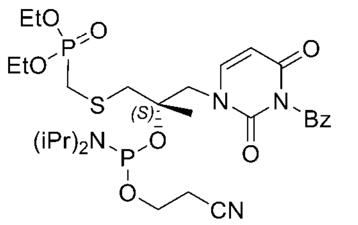 phosphoramidite-6-1 |
| 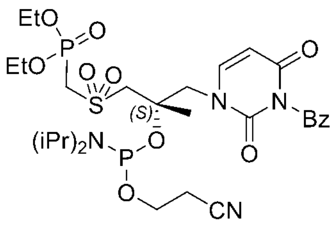 phosphoramidite-6-2 | 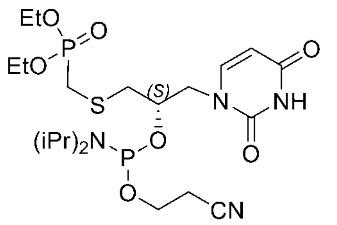 phosphoramidite-7-1 |
| 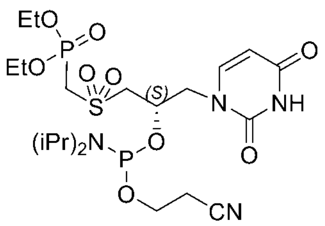 phosphoramidite-7-2 | 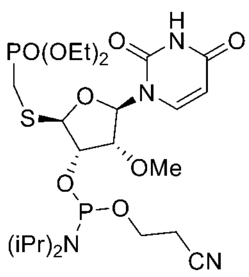 phosphoramidite-53 |
| 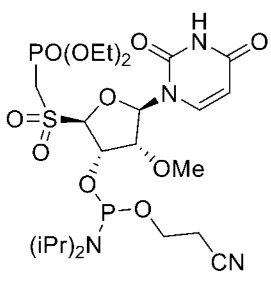 phosphoramidite-53-1. | |

In certain embodiments, the compounds provided herein have the following specific structures and stereoisomers thereof:

| | |
|---|---|
| 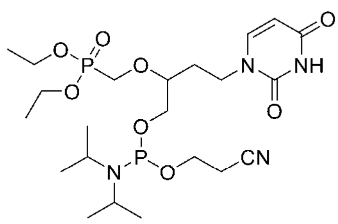 | 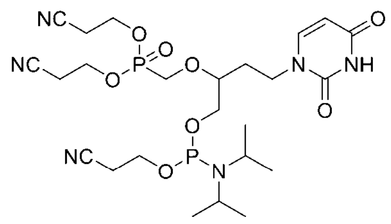 |
| 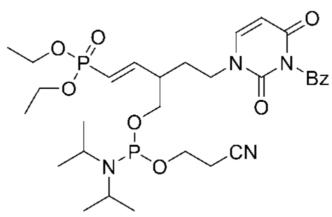 | 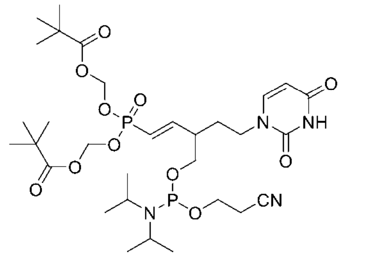 |
| 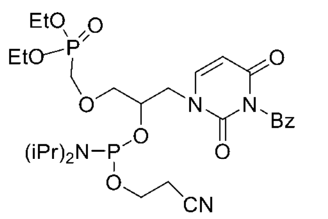 | 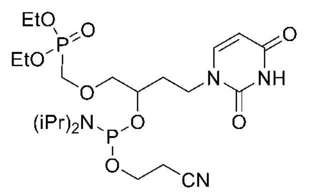 |
| 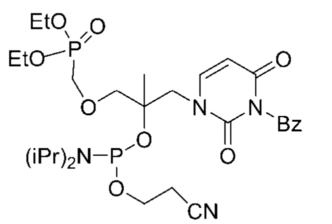 | 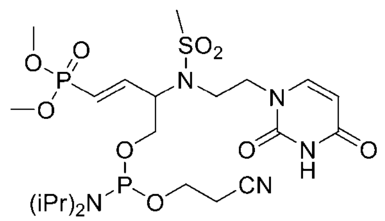 |
| 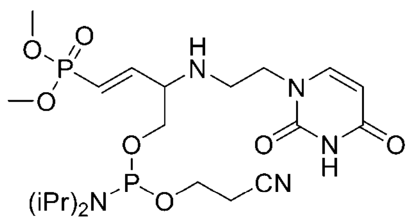 | 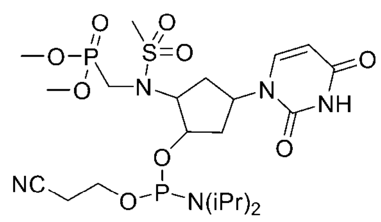 |
| 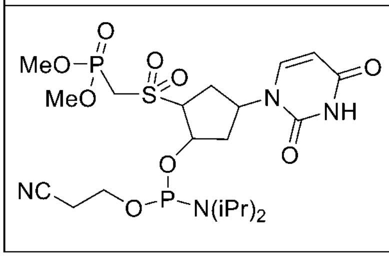 | 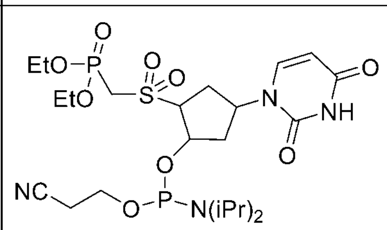 |

| | |
|---|---|
| 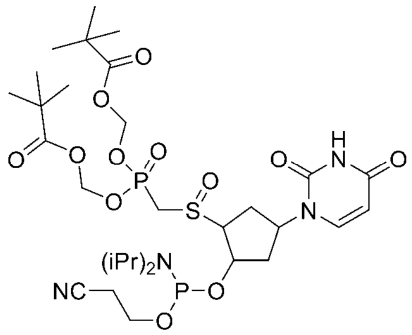 | 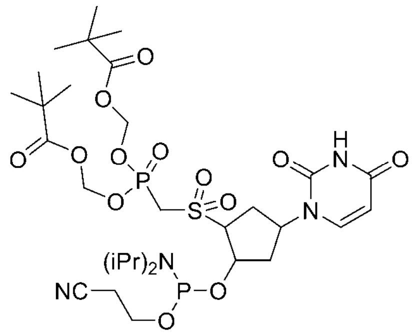 |
| 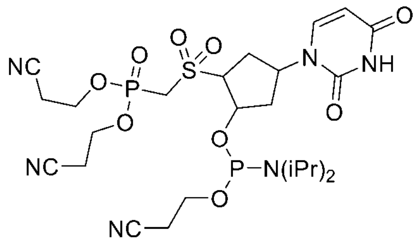 | 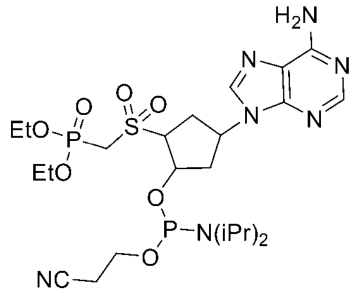 |
| 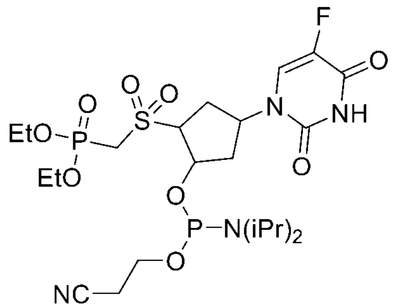 | 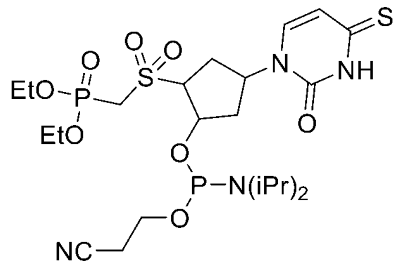 |
| 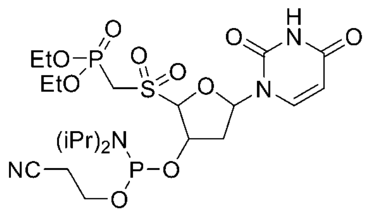 | 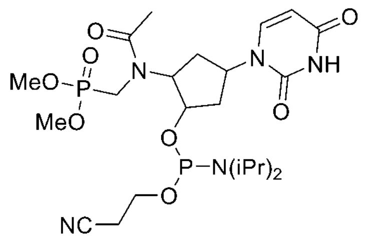 |
| 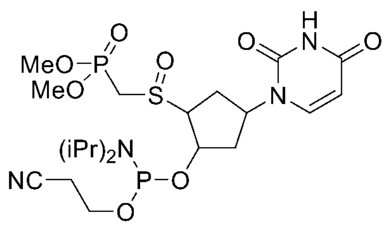 | 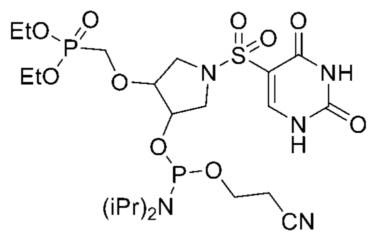 |
| 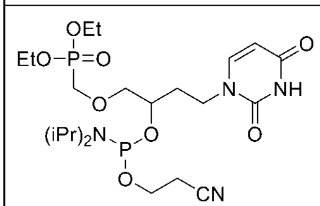 | 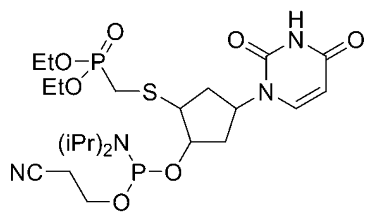 |

| | |
|---|---|
| 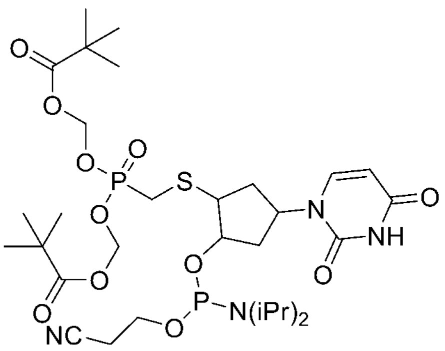 | 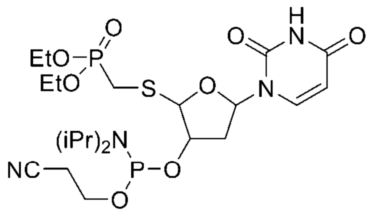 |
| 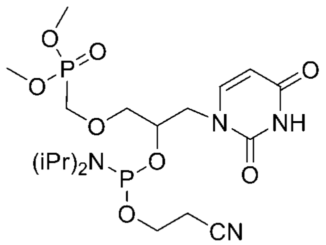 | 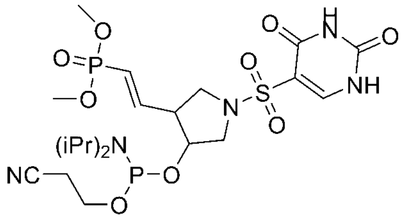 |
| 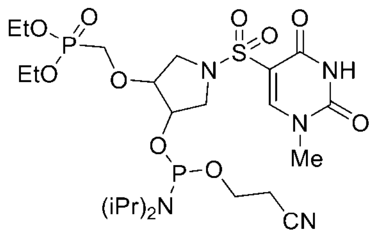 | 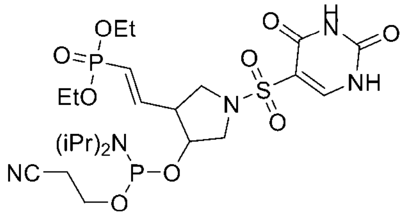 |
| 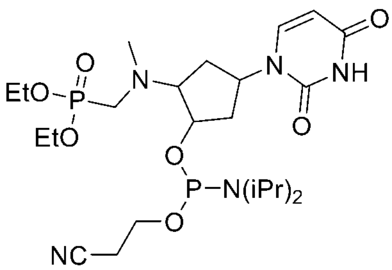 | 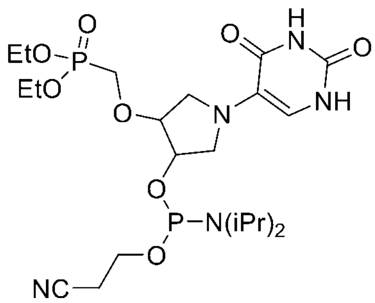 |
| 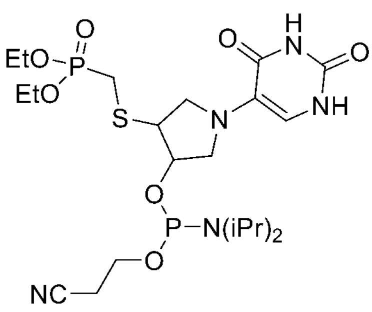 | 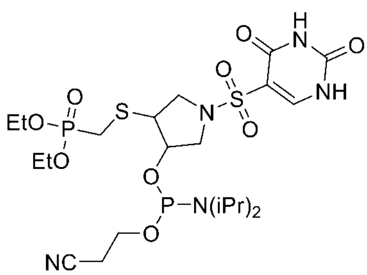 |

| | |
|---|---|
| 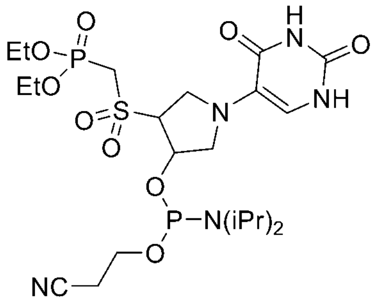 | 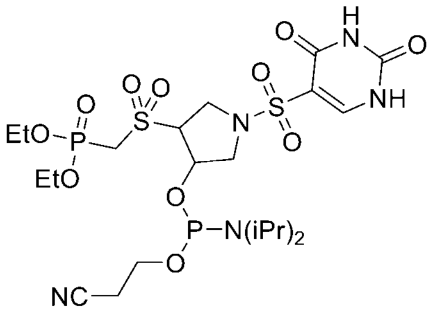 |
| 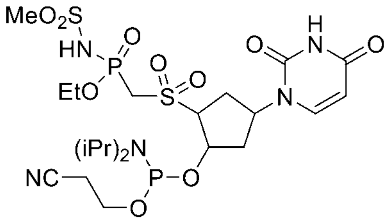 | 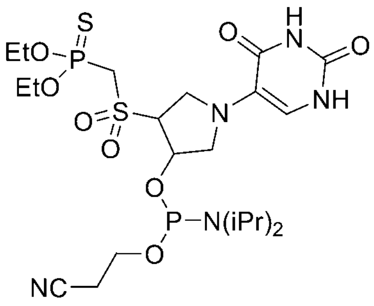 |
| 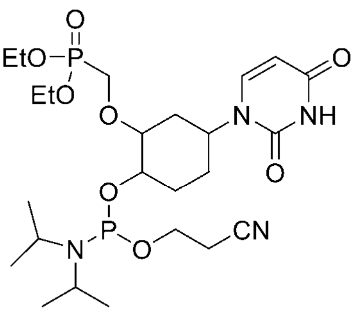 | 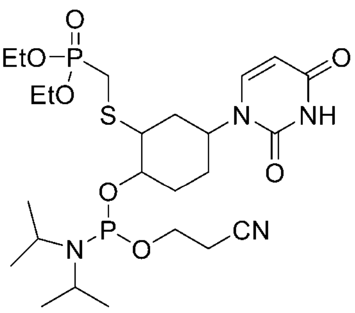 |
| 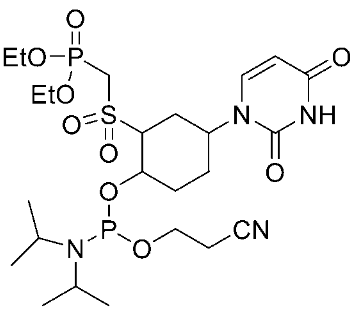 | 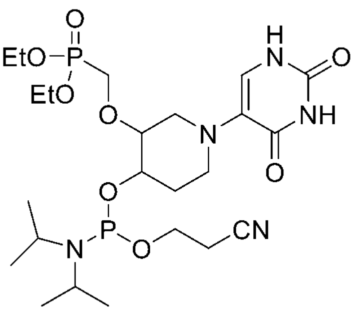 |
| 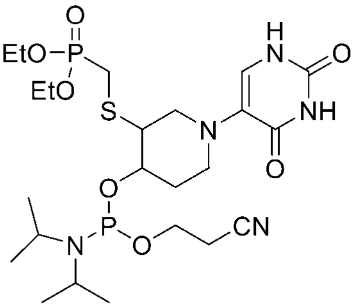 | 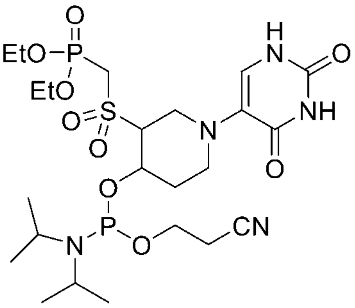 |

| | |
|---|---|
| 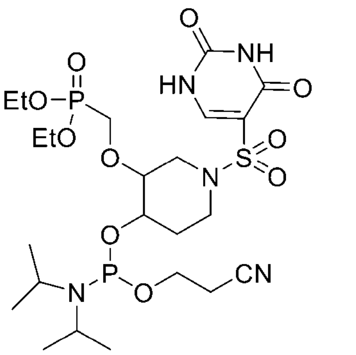 | 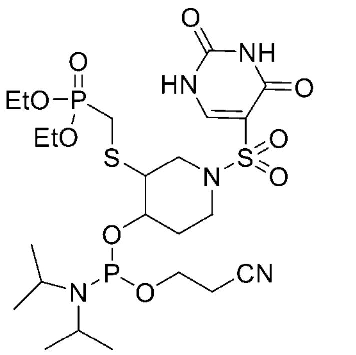 |
| 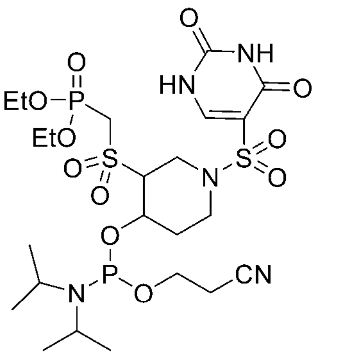 | 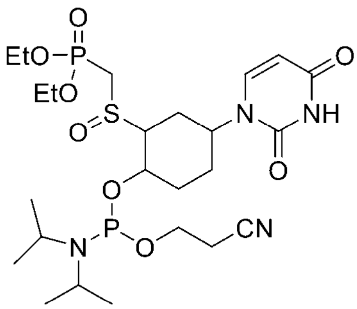 |
| 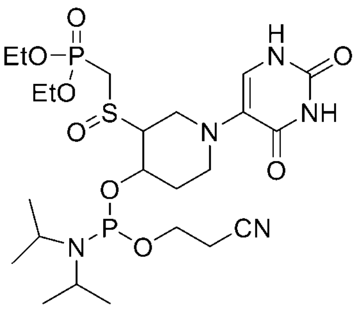 | 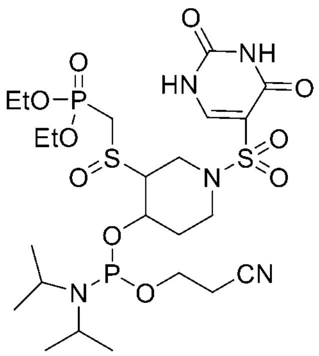 |
| 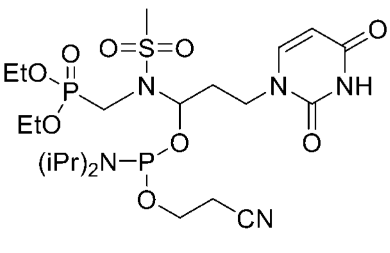 | 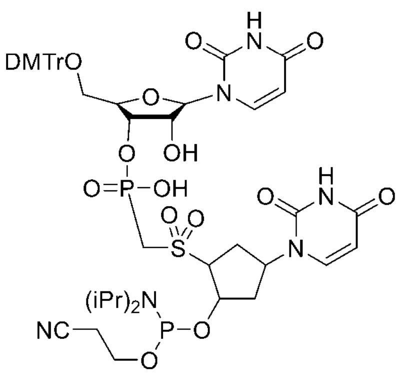 |

| | |
|---|---|
| 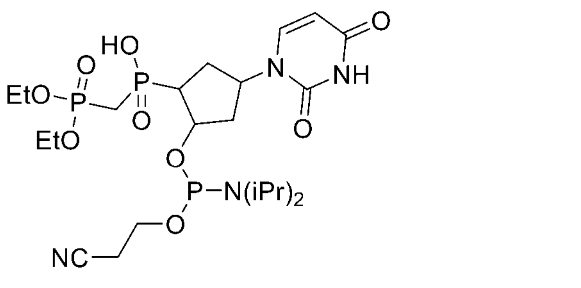 | 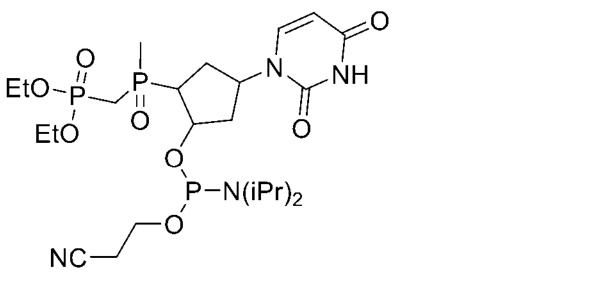 |
| 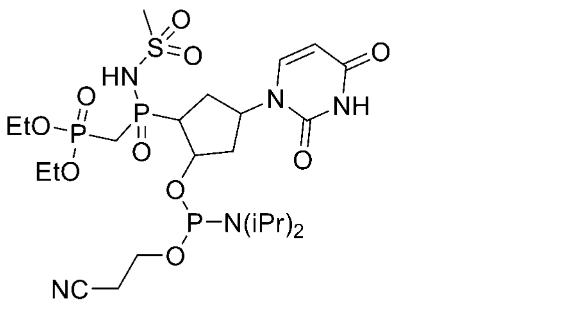 | 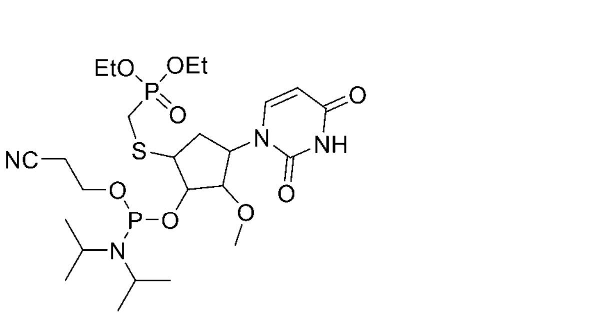 |
| 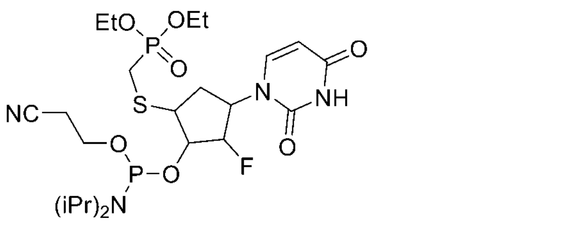 | 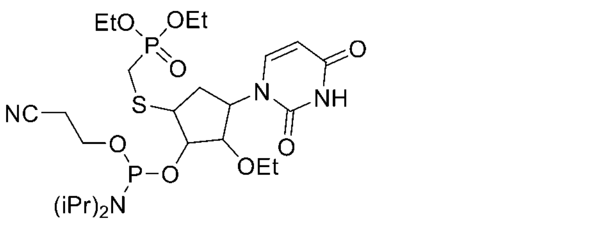 |
| 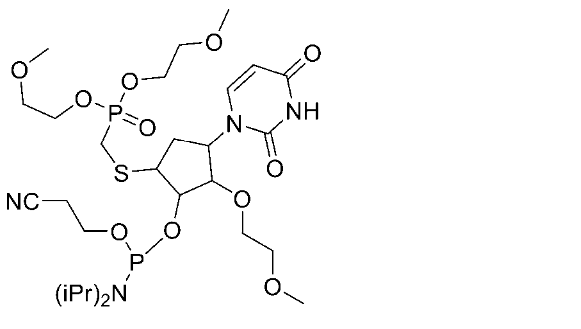 | 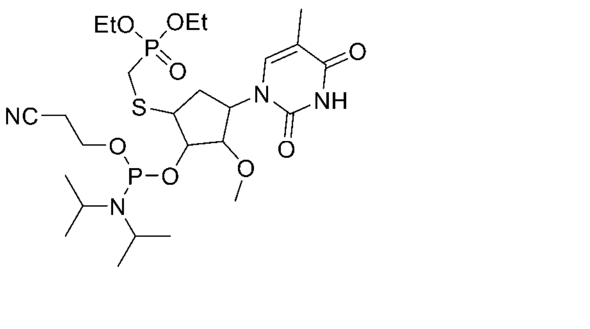 |
| 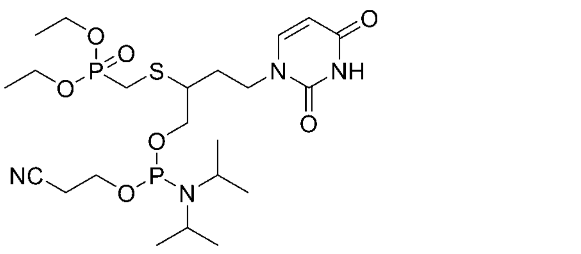 | 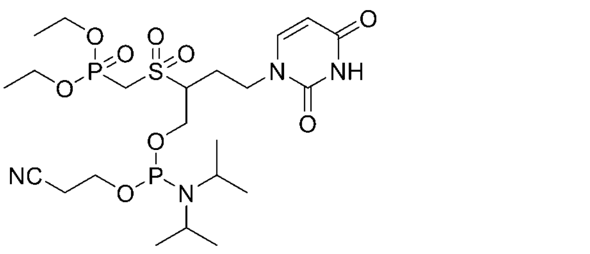 |
| 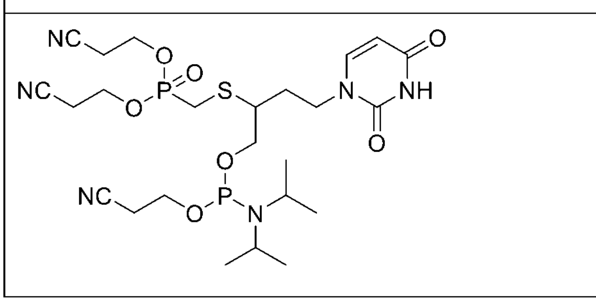 | 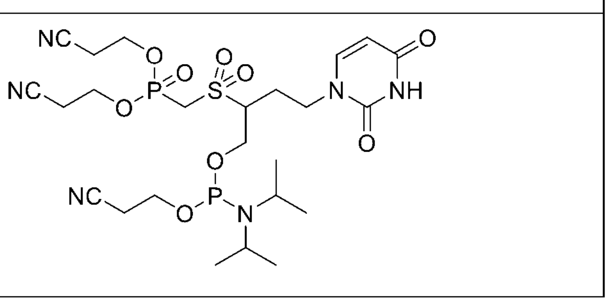 |

| | |
|---|---|
| 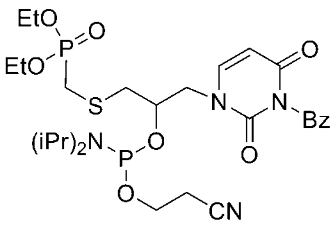 | 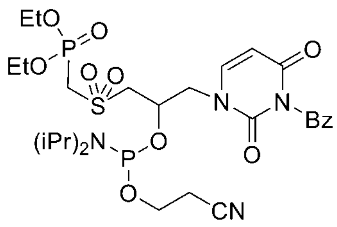 |
| 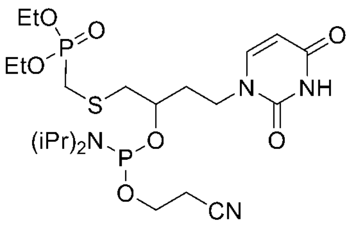 | 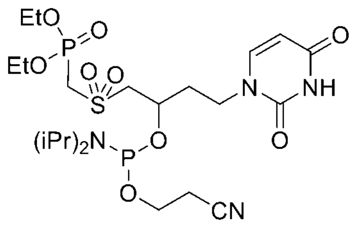 |
| 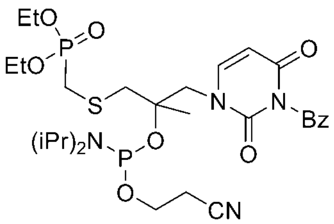 | 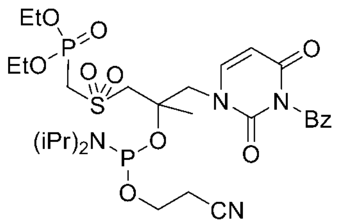 |
| 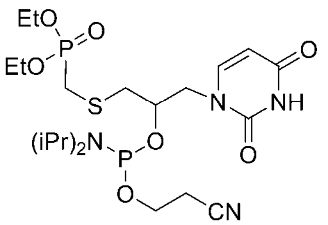 | 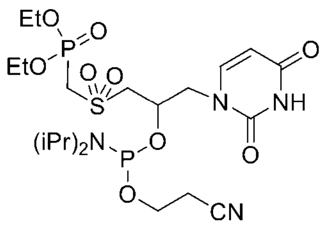 |
| 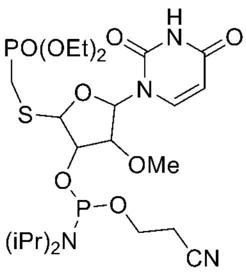 | 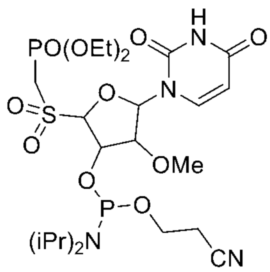 . |

The variables are defined separately in more detail in the present disclosure, and it should be understood that the compounds described in the present disclosure include all combinations of the embodiments and defined variables disclosed herein, e.g., formula (I), formula (I-1), formula (I-2), formula (II), formula (II-1), formula (II-2), formula (II-3), formula (III), formula (III-1), formula (III-2), formula (IV), formula (IV-1), or stereoisomers thereof. The technical features of more such compounds can be combined into any compound, and such compounds are applied to oligonucleotides, and further applied to the 5'-end to form oligonucleotides. The oligonucleotides include, but are not limited to, single-stranded antisense oligonucleotides (ASOs), miRNAs, and double-stranded ribonucleic acids (dsRNAs). In some examples, such a compound is applied to the 5'-end of an antisense strand in a double-stranded ribonucleic acid (dsRNA).

According to another aspect of the present disclosure, provided is an oligonucleotide comprising a 5'-terminal nucleotide represented by one of formula (V), formula (VI), or formula (VII), and a stereoisomer thereof:

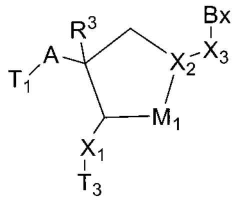 formula (V), 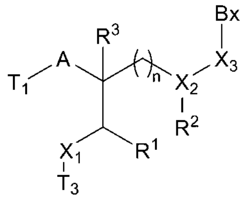 formula (VI), 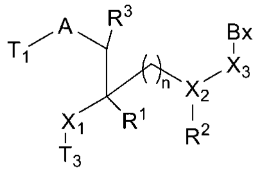 formula (VII)

wherein each $T_1$ is independently an optionally protected phosphine moiety;
each $T_3$ is independently an internucleoside linking group that links a 5'-terminal nucleotide to the oligonucleotide;
each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
each $X_2$ is independently $CR^{15}$ or N;
each $X_3$ is independently a chemical bond, optionally substituted $C_1$-$C_3$ alkylene, SO, $SO_2$, C(=O), or P(=O)R; R is OH, SH, $C_1$-$C_6$ alkyl, $NH_2$, or $NHSO_2CH_3$;
each Bx is independently a heterocyclic base moiety;
each $R^1$ and each $R^2$ are each independently H, halogen, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, sulfinyl, sulfonyl, or acetyl;
each $R^3$ and each $R^{15}$ are each independently H, halogen, optionally substituted $C_1$-$C_6$ alkyl, optionally substituted $C_1$-$C_6$ alkoxy, optionally substituted $C_2$-$C_6$ alkenyl, or optionally substituted $C_2$-$C_6$ alkynyl;
each A independently has one of the following formulas:

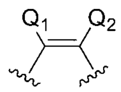, 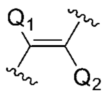, 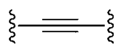, 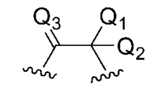, 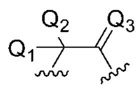, 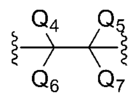, 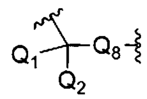, and 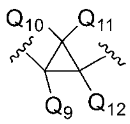,

$Q_1$ and $Q_2$ are each independently H, halogen, -CN, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^4R^5$;
each $Q_3$ is independently O, S, $NR^6$, or $CR^7R^8$;
each $Q_4$, each $Q_5$, each $Q_6$, each $Q_7$, each $Q_9$, each $Q_{10}$, each $Q_{11}$, and each $Q_{12}$ are each independently H, halogen, optionally protected hydroxyl, acetoxy, azido, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^9R^{10}$;
each $Q_8$ is independently O, S, SO, $SO_2$, $PR^{16}R^{17}$, or $NR^{11}$;
each $R^{16}$ and each $R^{17}$ are independently (=O), (=S), OH, SH, $C_1$-$C_6$ alkyl, or $NR^{18}R^{19}$;
each $R^4$, each $R^5$, each $R^6$, each $R^7$, each $R^8$, each $R^9$, each $R^{10}$, each $R^{11}$, each $R^{13}$, and each $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$;
$M_1$ is C(Rd)(Re) or C(Rd)(Re)C(Rf)(Rg), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$;
each $J_3$, each $J_4$, each $J_5$, and each $J_6$ are independently H or $C_1$-$C_6$ alkyl;
n is 0, 1, or 2.

In certain embodiments, in the oligonucleotide, each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, in the provided oligonucleotide, $T_1$ is an optionally protected phosphine moiety having the following formula:

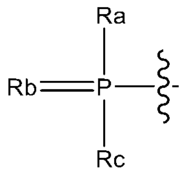

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;

each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

Examples of protecting groups commonly used for protecting phosphorus hydroxyl groups or phosphorus sulfhydryl groups include, but are not limited to, methyl, ethyl, benzyl (Bn), phenyl, isopropyl, tert-butyl, acetyl, chloroacetyl, trichloroacetyl, trifluoroacetyl, pivaloyl, tert-butoxymethyl, methoxymethyl, 1-ethoxyethyl, 1-(2-chloroethoxy) ethyl, 2-trimethylsilylethyl, allyl, cyclohexyl (cHex), 9-fluorenylmethoxycarbonyl, methanesulfonyl, p-toluenesulfonyl, trifluoromethanesulfonyl, benzoyl, benzoylformate, p-phenylbenzoyl, 4-methoxybenzyl, monomethoxytrityl, dimethoxytrityl, trimethoxytrityl, 4-chlorobenzyl, 4-nitrobenzyl, 2,4-dinitrophenyl, 4-acyloxybenzyl, 2-methylphenyl, 2,6-dimethylphenyl, 2-chlorophenyl, 2,6-dichlorobenzyl, diphenylmethyl, triphenylmethyl, 4-methylthio-1-butyl, 2-(S-acetylthio)ethyl (SATE), 2-cyanoethyl, 2-cyano-1,1-dimethylethyl (CDM), 4-cyano-2-butenyl, 2-(trimethylsilyl)ethyl (TSE), 2-(phenylthio) ethyl, 2-(triphenylsilyl)ethyl, 2-(benzylsulfonyl)ethyl, 2,2,2-trichloroethyl, 2,2,2-tribromoethyl, 2,3-dibromopropyl, 2,2,2-trifluoroethyl, phenylthio, 2-chloro-4-tritylphenyl, 2-bromophenyl, 2-[N-isopropyl-N-(4-methoxybenzoyl)amino]ethyl, 4-(N-trifluoroacetylamino)butyl, 4-oxopentyl, 4-tritylaminophenyl, 4-benzylaminophenyl, tetrahydropyranyl, morpholino, trimethylsilyl, triethylsilyl, tert-butyldimethylsilyl, tert-butyldiphenylsilyl, triphenylsilyl, triisopropylsilyl, pivaloyloxymethyl (POM), and 9-phenylxanthine-9-yl.

Examples commonly used for amino-protecting groups include, but are not limited to, 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), benzyl, formyl, acetyl, pivaloyl, trihaloacetyl, benzoyl, nitrophenyl, acetyl, 2-nitrobenzenesulfonyl, phthalimido (Pht), p-toluenesulfonyl (Tos), trityl (Trt), 2,4-dimethoxybenzyl (PMB), and dithiosuccinyl.

In certain embodiments, in the provided oligonucleotide, $R_b$ is O or S, and Ra and Rc are each independently selected from protected hydroxyl, $C_1$-$C_6$ alkyl, and $C_1$-$C_6$ alkoxy.

In certain embodiments, in the provided oligonucleotide, Ra and Rc are each OH, SH, $OCH_3$, $OCH_2CH_3$, $OCH(CH_3)_2$, $OCH_2OC(=O)C(CH_3)_3$, $NH_2$, $OCH_2CH_2CN$, or $NHSO_2CH_3$.

In certain embodiments, in the provided oligonucleotide, Ra and Rc are each OH, and $R_b$ is O.

In certain embodiments, one of Ra and Rc is OH, and the other is a natural nucleoside; $R_b$ is O.

In certain embodiments, in the provided oligonucleotide, $T_3$ is an internucleoside linking group that links a 5'-nucleotide represented by formula (V), formula (VI), or formula (VII), and a stereoisomer thereof to the 5'-end of the oligonucleotide, and the internucleoside linking group is selected from a phosphorus-containing linking group and a phosphorus-free linking group.

In certain embodiments, in the provided oligonucleotide, the phosphorus-containing internucleoside linking group is independently a phosphodiester linking group, a phosphotriester linking group, a phosphorothioate linking group, a phosphorodithioate linking group, an alkylphosphonate linking group, an aminophosphonate linking group, a phosphonate linking group, a phosphinate linking group, a phosphoramidothioate linking group, or a phosphoramidate linking group.

In certain embodiments, in the provided oligonucleotide, the internucleoside linking group is independently an alkylphosphonate linking group, a phosphodiester internucleoside linking group, or a phosphorothioate internucleoside linking group.

In certain embodiments, in the provided oligonucleotide, the $B_X$ heterocyclic base moiety is selected from a natural nucleobase, a modified nucleobase, and a universal base.

In certain embodiments, in the provided oligonucleotide, the $B_X$ heterocyclic base moiety is pyrimidine, substituted pyrimidine, pseudouracil, substituted pseudouracil, purine, hypoxanthine, or substituted purine.

In certain embodiments, in the provided oligonucleotide, the $B_X$ heterocyclic base moiety is uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, in the provided oligonucleotide, $R^{15}$ and $R^3$ are H.

In certain embodiments, in the provided oligonucleotide, $X_1$ is O.

In certain embodiments, in the provided oligonucleotide, each $R^1$ and each $R^2$ are each independently H, methanesulfonyl, or acetyl.

In certain embodiments, in the provided oligonucleotide, each $X_2$ is independently N, and each $R^1$ and each $R^2$ are each independently H, methanesulfonyl, or acetyl.

In certain embodiments, in the provided oligonucleotide, A in formula (V), (VI), or (VII) has one of the following formulas:

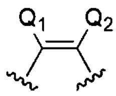
,
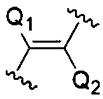
, and
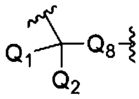
,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, in the provided oligonucleotide, $Q_1$ and $Q_2$ are each independently H, F, -CN, or methyl, more preferably H.

In certain embodiments, $Q_8$ is S, SO, or $SO_2$.

In certain embodiments, a proviso is that when $M_1$ is C(Rd)(Re), $X_2$ is C, $X_3$ is a chemical bond, and A is

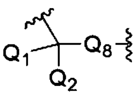
,

$Q_8$ is S, SO, $SO_2$, $PR^{16}R^{17}$, or $NR^{11}$, and $Q_1$, $Q_2$, $R^{11}$, $R^{16}$, and $R^{17}$ are as defined in formula (V) herein.

In certain embodiments, in the provided oligonucleotide, $X_2$ is N, and $X_3$ is a chemical bond, $-CH_2-$, $-CH_2CH_2-$, SO, or $SO_2$.

In certain embodiments, in the provided oligonucleotide, $X_2$ is CH, and $X_3$ is a chemical bond.

In certain embodiments, in the provided oligonucleotide, in the 5'-terminal nucleotide represented by formula (VI) or (VII) provided herein, n is 0 or 1.

In certain embodiments, the provided oligonucleotide comprises a 5'-terminal nucleotide represented by formula (V-1) or a stereoisomer thereof:

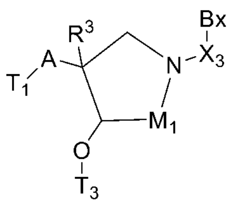

formula (V-1)

wherein $T_1$, $T_3$, A, $R^3$, and Bx are each as defined in formula (V) and the embodiments above; $X_3$ is a chemical bond, C(=O), P(=O)R, SO, or $SO_2$; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl; R is OH, SH, $C_1$-$C_6$ alkyl, $NH_2$, or $NHSO_2CH_3$.

In certain embodiments, the provided oligonucleotide comprises a 5'-terminal nucleotide having formula (V-1) or a stereoisomer thereof, wherein $X_3$ is $SO_2$ or a chemical bond, and $R^3$ is hydrogen.

In certain embodiments, the provided oligonucleotide comprises a 5'-terminal nucleotide having formula (V-1) or

a stereoisomer thereof, wherein Rd, Re, Rg, and Rf are each independently selected from the following substituent groups: hydrogen, fluorine, hydroxyl, $C_1$-$C_6$ alkoxy, and $O(CH_2)_2$-$OCH_3$. More preferably, Rd, Re, Rg, and Rf are hydrogen.

In certain embodiments, the provided oligonucleotide comprises a 5'-terminal nucleotide having formula (V-1) or a stereoisomer thereof, wherein A has one of the following formulas:

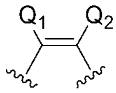 , 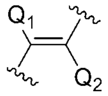 , and 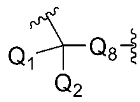 ,

wherein $Q_1$ and $Q_2$ are each independently H, halogen, or optionally substituted $C_1$-$C_6$ alkyl;
$Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$, $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, the provided oligonucleotide comprises a 5'-terminal nucleotide represented by formula (V-2) or a stereoisomer thereof:

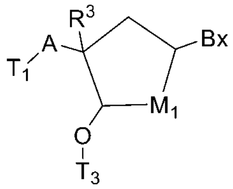

formula (V-2)

wherein $T_1$, $T_3$, A, $R^3$, and Bx are each as defined in formula (V) and the embodiments above; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl.

In certain embodiments, the oligonucleotide provided herein comprises a 5'-terminal nucleotide having formula (V-2) or a stereoisomer thereof, wherein each Rd, each Re, each Rg, and each Rf are each independently selected from the following substituent groups: hydrogen, fluorine, hydroxyl, $C_1$-$C_6$ alkoxy, and $O(CH_2)_2$-$OCH_3$. More preferably, each Rd, each Re, each Rg, and each Rf are hydrogen.

In certain embodiments, the oligonucleotide provided herein comprises a 5'-terminal nucleotide having formula (V-2) or a stereoisomer thereof, wherein $M_1$ is $CH_2$ or $CH_2CH_2$.

In certain embodiments, the oligonucleotide provided herein comprises a 5'-terminal nucleotide having formula (V-2) or a stereoisomer thereof, wherein $R^3$ is hydrogen.

In certain embodiments, the oligonucleotide provided herein comprises a 5'-terminal nucleotide having formula (V-2) or a stereoisomer thereof, wherein A has one of the following formulas:

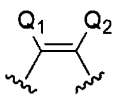 , 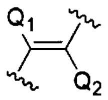 , and 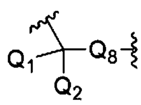 ,

wherein $Q_1$ and $Q_2$ are each independently H, halogen, or optionally substituted $C_1$-$C_6$ alkyl;
$Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In certain embodiments, the oligonucleotide provided herein comprises a 5'-terminal nucleotide having formula (V-1), formula (V-2), or a stereoisomer thereof, wherein each $Q_1$ and each $Q_2$ are each independently H, F, -CN, or methyl. More preferably, each $Q_1$ and each $Q_2$ are H.

In certain embodiments, the provided oligonucleotide comprises a 5'-terminal nucleotide represented by formula

(V-1) or formula (V-2), wherein $T_1$ is an optionally protected phosphine moiety having the following formula:

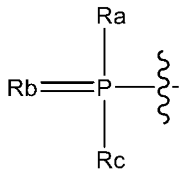

wherein:
Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group.

In certain embodiments, a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

In certain embodiments, the sulfonyl is preferably methanesulfonyl.

In certain embodiments, in the provided oligonucleotide, $R_b$ is O. In certain embodiments, in the provided oligonucleotide, $R_b$ is S.

In certain embodiments, in the provided oligonucleotide, Ra and Rc are each OH, SH, $NH_2$, or $NHSO_2CH_3$.

In certain embodiments, in the provided oligonucleotide, Ra and Rc are each OH, and $R_b$ is O.

In certain embodiments, the provided oligonucleotide comprises a 5'-terminal nucleotide represented by formula (V-1) or formula (V-2), wherein $T_3$ is an internucleoside linking group.

In certain embodiments, in the provided oligonucleotide, the internucleoside linking group is selected from an alkylphosphonate linking group, a phosphodiester internucleoside linking group, and a phosphorothioate internucleoside linking group.

In some preferred embodiments, the provided oligonucleotide comprises a 5'-terminal nucleotide represented by formula (V-1) or formula (V-2), wherein $Q_8$ is $SO_2$.

In some preferred embodiments, the provided oligonucleotide comprises a 5'-terminal nucleotide represented by formula (V-1) or formula (V-2), wherein $Q_8$ is S.

In certain embodiments, the provided oligonucleotide comprises a 5'-terminal nucleotide represented by formula (V-1) or formula (V-2), wherein each $B_X$ heterocyclic base moiety is selected from a natural nucleobase, a modified nucleobase, and a universal base.

In certain embodiments, the provided oligonucleotide comprises a 5'-terminal nucleotide represented by formula (V-1) or formula (V-2), wherein each $B_X$ heterocyclic base moiety is independently pyrimidine, substituted pyrimidine, pseudouracil, substituted pseudouracil, purine, hypoxanthine, or substituted purine.

In certain embodiments, the BX heterocyclic base moiety is uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, the 5'-terminal nucleotide of formula (V-2) provided herein has a 1S,2S,4S or 1R,2R,4R stereoconfiguration.

In certain embodiments, the provided oligonucleotide comprises a 5'-terminal nucleotide represented by formula (VI-1) or a stereoisomer thereof:

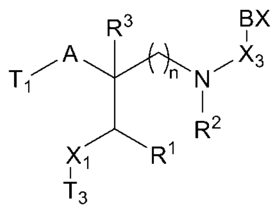

(VI-1)

wherein $T_1$, $T_3$, $X_1$, $X_3$, A, $R^3$, Bx, and n are each as defined in formula (VI) and the embodiments above; $R^1$ and $R^2$ are each independently H, methanesulfonyl, or acetyl.

In certain embodiments, in the provided oligonucleotide, n is 0 or 1.

In certain embodiments, in the provided oligonucleotide, $X_3$ is $CH_2$ or $CH_2CH_2$.

In certain embodiments, the provided oligonucleotide comprises a 5'-terminal nucleotide represented by formula (VI-2) or a stereoisomer thereof:

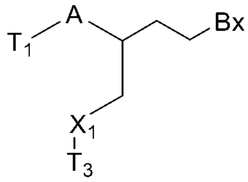

(VI-2)

wherein $T_1$, $T_3$, $X_1$, A, and Bx are each as defined in formula (VI) and the embodiments above.

In certain embodiments, the provided oligonucleotide comprises a 5'-terminal nucleotide represented by formula (VI-3) or a stereoisomer thereof:

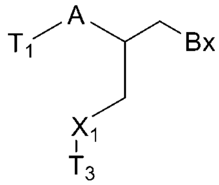

(VI-3)

wherein $T_1$, $T_3$, $X_1$, A, and Bx are each as defined in formula (VI) and the embodiments above.

In certain embodiments, the provided oligonucleotide comprises a 5'-terminal nucleotide represented by formula (VII-1) or a stereoisomer thereof:

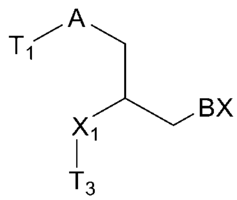

formula (VII-1)

wherein $T_1$, $T_3$, $X_1$, A, and Bx are each as defined in formula (VII) and the embodiments above.

In certain embodiments, the provided oligonucleotide comprises a 5'-terminal nucleotide represented by formula (VII-2) or a stereoisomer thereof:

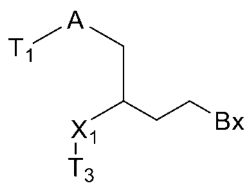

(VII-2)

wherein $T_1$, $T_3$, $X_1$, A, and Bx are each as defined in formula (VII) and the embodiments above.

In certain embodiments, the provided oligonucleotide comprises a 5'-terminal nucleotide represented by formula (VI-1), formula (VI-2), formula (VI-3), formula (VII-1), formula (VII-2), or a stereoisomer thereof, wherein A independently has one of the following formulas:

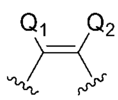 , 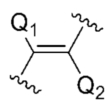 , and 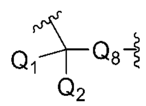 ,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl.

Each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

In some preferred embodiments, the provided oligonucleotide comprises a 5'-terminal nucleotide represented by

formula (VI-1), formula (VI-2), formula (VI-3), formula (VII-1), formula (VII-2), or a stereoisomer thereof, wherein each $Q_1$ and each $Q_2$ are each independently H, F, -CN, or methyl. More preferably, each $Q_1$ and each $Q_2$ are H.

In some preferred embodiments, the provided oligonucleotide comprises a 5'-terminal nucleotide represented by formula (VI-1), formula (VI-2), formula (VI-3), formula (VII-1), formula (VII-2), or a stereoisomer thereof, wherein $Q_8$ is independently $NR^{11}$, and $R^{11}$ is independently methyl or methanesulfonyl.

In some preferred embodiments, the provided oligonucleotide comprises a 5'-terminal nucleotide represented by formula (VI-1), formula (VI-2), formula (VI-3), formula (VII-1), formula (VII-2), or a stereoisomer thereof, wherein $Q_8$ is $SO_2$.

In some preferred embodiments, the provided oligonucleotide comprises a 5'-terminal nucleotide represented by formula (VI-1), formula (VI-2), formula (VI-3), formula (VII-1), formula (VII-2), or a stereoisomer thereof, wherein $Q_8$ is S.

In certain embodiments, the provided oligonucleotide comprises a 5'-terminal nucleotide represented by formula (VI-1), formula (VI-2), formula (VI-3), formula (VII-1), formula (VII-2), or a stereoisomer thereof, wherein each $B_X$ heterocyclic base moiety is selected from a natural nucleobase, a modified nucleobase, and a universal base.

In certain embodiments, the provided oligonucleotide comprises a 5'-terminal nucleotide represented by formula (VI-1), formula (VI-2), formula (VI-3), formula (VII-1), formula (VII-2), or a stereoisomer thereof, wherein each $B_X$ heterocyclic base moiety is independently pyrimidine, substituted pyrimidine, pseudouracil, substituted pseudouracil, purine, hypoxanthine, or substituted purine.

In certain embodiments, the $B_X$ heterocyclic base moiety is uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, the provided oligonucleotide comprises a 5'-terminal nucleotide represented by formula (VI-1), formula (VI-2), formula (VI-3), formula (VII-1), formula (VII-2), or a stereoisomer thereof, wherein $T_1$ is an optionally protected phosphine moiety having the following formula:

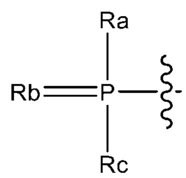

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group.

In certain embodiments, a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

In certain embodiments, the sulfonyl is preferably methanesulfonyl.

In certain embodiments, $R_b$ is O. In certain embodiments, $R_b$ is S.

In certain embodiments, in the provided oligonucleotide, Ra and Rc are each OH, SH, $NH_2$, or $NHSO_2CH_3$.

In certain embodiments, in the provided oligonucleotide, Ra and Rc are each OH, and $R_b$ is O.

In certain embodiments, the provided oligonucleotide comprises a 5'-terminal nucleotide represented by formula (VI-1), formula (VI-2), formula (VI-3), formula (VII-1), formula (VII-2), or a stereoisomer thereof, wherein $T_3$ is an internucleoside linking group.

In certain embodiments, the provided oligonucleotide comprises a 5'-terminal nucleotide represented by formula (VI-1), formula (VI-2), formula (VI-3), formula (VII-1), formula (VII-2), or a stereoisomer thereof, wherein the internucleoside linking group is selected from an alkylphosphonate linking group, a phosphodiester internucleoside linking group, and a phosphorothioate internucleoside linking group.

In another aspect, the provided oligonucleotide comprises a 5'-terminal nucleotide represented by formula (VIII) or a stereoisomer thereof:

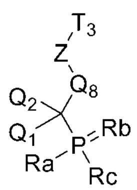 formula (VIII)

wherein $Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, protected or optionally substituted amino, and a natural or modified nucleoside, and $R_b$ is O or S or $NR^{12}$, wherein $R^{12}$ is hydrogen, $C_1$-$C_6$ alkyl, or an amino-protecting group;

$Q_1$ and $Q_2$ are each independently H, halogen, -CN, or optionally substituted $C_1$-$C_6$ alkyl;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;

$R^{11}$, $R^{18}$, and $R^{19}$ are independently H, optionally substituted $C_1$-$C_6$ alkyl, optionally substituted $C_1$-$C_6$ alkoxy, methanesulfonyl, or sulfo;

Z is a nucleoside comprising a sugar or sugar surrogate moiety;

$T_3$ is an internucleoside linking group that links the 5'-terminal nucleotide of formula (VIII) or the stereoisomer thereof to the oligonucleotide;

each substituted group comprises one or more substituent groups optionally and independently selected from halogen, hydroxyl, $C_1$-$C_6$ alkyl, $C_1$-$C_6$ alkoxy, $C_1$-$C_6$ alkylsulfhydryl, and CN.

In certain embodiments, in the provided oligonucleotide, in formula (VIII), $Q_8$ is SO or $SO_2$.

In certain embodiments, in the provided oligonucleotide, in formula (VIII), $Q_8$ is S.

In certain embodiments, in the provided oligonucleotide, in formula (VIII), $Q_1$ and $Q_2$ are each independently H.

In certain embodiments, in the provided oligonucleotide, in formula (VIII), in the nucleoside comprising a sugar or sugar surrogate moiety, the sugar or sugar surrogate moiety includes a 5-membered furanose ring, a non-furanose ring, or a 5- to 6-membered carbocyclic system, or an open system.

In certain embodiments, in the provided oligonucleotide, in formula (VIII), in the nucleoside comprising a sugar or sugar surrogate moiety, the sugar surrogate substitution moiety is morpholinyl, cyclohexenyl, cyclohexyl, cyclopentyl, pyranyl, or a cyclohexanehexol group. In certain embodiments, in the nucleoside comprising a sugar or sugar surrogate moiety, the sugar moiety is a furanose. In certain embodiments, the nucleoside comprising a sugar or sugar surrogate moiety comprises an unlocked nucleobase analog (UNA) or a glycerol nucleobase analog (GNA). In certain embodiments, the nucleoside comprising a sugar or sugar surrogate moiety comprises a locked nucleic acid (LNA) or a bridged nucleic acid (BNA).

In certain embodiments, in the provided oligonucleotide, in formula (VIII), $Q_8$ is bonded to the 4'-carbon or 5'-carbon of the sugar or sugar surrogate moiety.

In certain embodiments, in the provided oligonucleotide, in formula (VIII), the nucleoside comprising a sugar or sugar surrogate moiety has the following structural schematics:

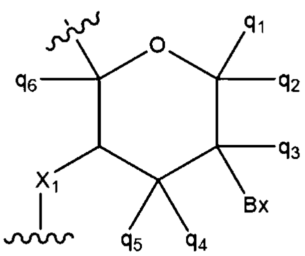 , 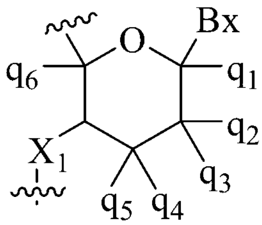 , 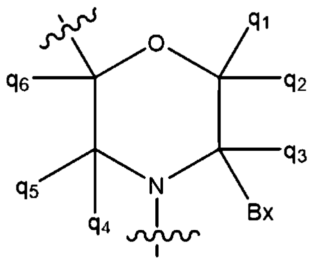 ,

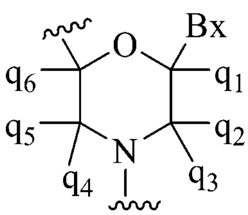 , 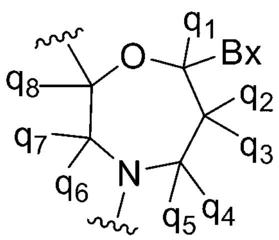 , 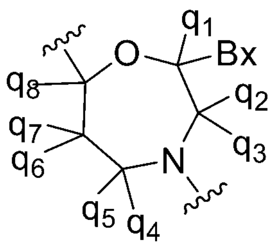 ,

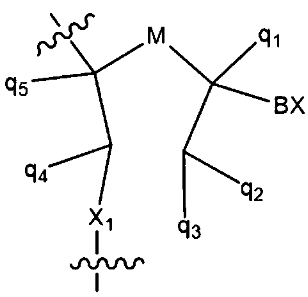 , 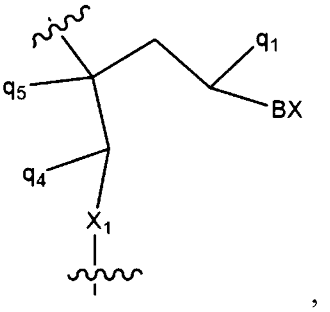 , 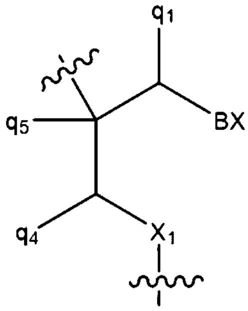 ,

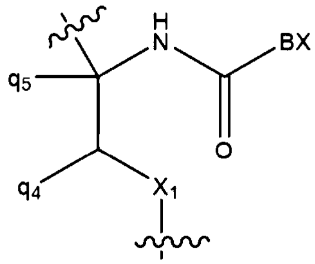 , 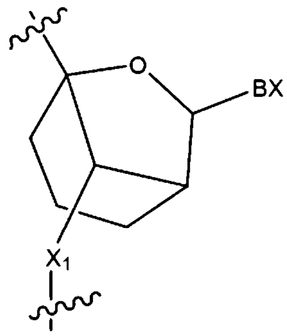 , 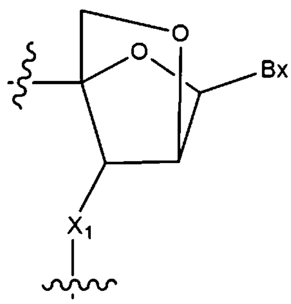 ,

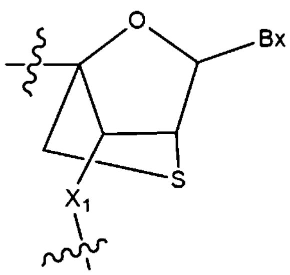 , 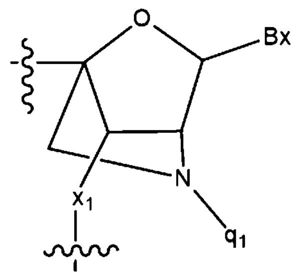 , 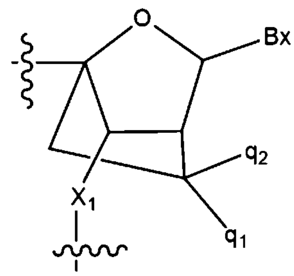 ,

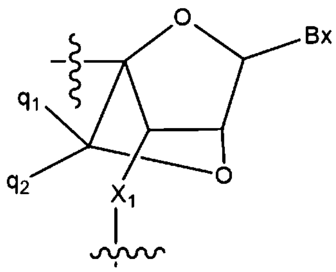 , 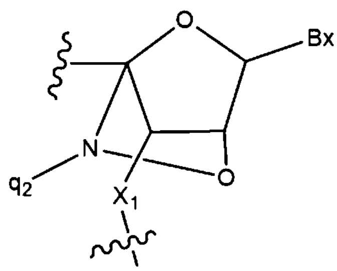 , 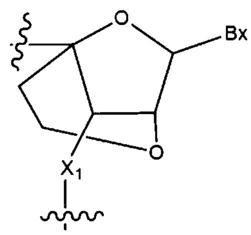 ,

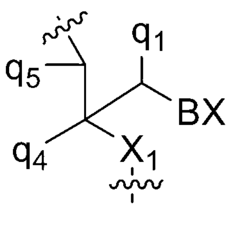 , 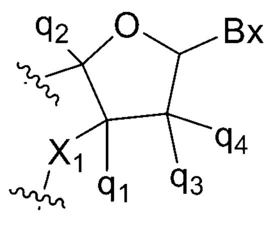 , and 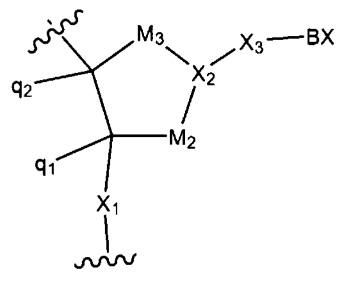 ,

wherein $M_2$ is $C(q_3)(q_4)$ or $C(Q_3)(Q_4)C(Q_5)(Q_6)$;
$M_3$ is O, S, $NR^{13}$, $C(q_7)(q_8)$, $C(q_7)(q_8)C(q_9)(q_{10})$, $C(q_7)=C(q_8)$, or $OC(q_7)(q_8)$;

each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
each $X_2$ is independently $CR^{15}$ or N;
each $X_3$ is independently a chemical bond, optionally substituted $C_1$-$C_3$ alkylene, SO, $SO_2$, C(=O), or P(=O)R; R is OH, SH, $C_1$-$C_6$ alkyl, $NH_2$, or $NHSO_2CH_3$;
each Bx is independently a heterocyclic base moiety;
each $R^{15}$, each $q_1$, each $q_2$, each $q_3$, each $q_4$, each $q_5$, each $q_6$, each $q_7$, each $q_8$, each $q_9$, and each $q_{10}$ are each independently hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, CN, $OC(=O)J_5$, $C(=O)N(J_5)(J_6)$, and $C(=O)N((J_5)(J_6)$, wherein $J_5$ and $J_6$ are independently H or $C_1$-$C_6$ alkyl;
each $R^{13}$ is independently hydrogen or $C_1$-$C_6$ alkyl.

In certain embodiments, in the provided oligonucleotide, each $R^{15}$, each $q_1$, each $q_2$, each $q_3$, each $q_4$, each $q_5$, each $q_6$, each $q_7$, each $q_8$, and each $q_9$ are each independently selected from the following groups: hydrogen, fluorine, hydroxyl, $C_1$-$C_6$ alkyl, $C_1$-$C_6$ alkoxy, and $O(CH_2)_2$-$OCH_3$. More preferably, each $R^{15}$, each $q_1$, each $q_2$, each $q_3$, each $q_4$, each $q_5$, each $q_6$, each $q_7$, each $q_8$, and each $q_9$ are hydrogen.

In certain embodiments, in the provided oligonucleotide, in formula (VIII), the nucleoside comprising a sugar or sugar surrogate moiety has the following structural schematics:

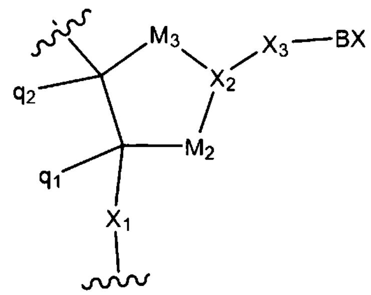

wherein $M_2$, $M_3$, $X_1$, $X_2$, $X_3$, $q_1$, $q_2$, and Bx are each as defined in formula (VIII) and the embodiments above.

In certain embodiments, $M_3$ is O, S, $C(q_7)(q_8)$, or $C(q_7)(q_8)C(q_9)(q_{10})$.

In certain embodiments, $q_1$, $q_2$, $q_7$, $q_8$, $q_9$, and $q_{10}$ are each independently selected from hydrogen, fluorine, hydroxyl, methyl, methoxy, and $O(CH_2)_2$-$OCH_3$.

In certain embodiments, in the provided oligonucleotide, in formula (VIII), the nucleoside comprising a sugar or sugar surrogate moiety has the following furanose structural schematic:

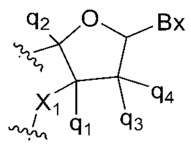

wherein $X_1$, $q_1$, $q_2$, $q_3$, $q_4$, and Bx are each as defined in formula (VIII) and the embodiments above.

In certain embodiments, the provided oligonucleotide comprises a 5'-terminal nucleotide represented by formula (VIII-1) or a stereoisomer thereof:

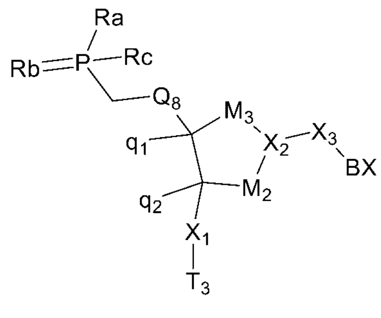

formula (VIII-1)

wherein $Q_8$, Ra, Rc, $R_b$, $M_2$, $M_3$, $X_1$, $X_2$, $X_3$, $q_1$, $q_2$, $T_3$, and Bx are each as defined in formula (VIII) and the embodiments above.

In certain embodiments, in the provided oligonucleotide, in formula (VIII-1), $R^{15}$, $q_1$, $q_2$, $q_3$, $q_4$, $q_5$, $q_6$, $q_7$, $q_8$, $q_9$, and $q_{10}$ are each independently selected from hydrogen, fluorine, hydroxyl, methyl, methoxy, and $O(CH_2)_2$-$OCH_3$. In certain embodiments, in the provided oligonucleotide, in formula (VIII-1), $R^{15}$, $q_1$, $q_2$, $q_3$, $q_4$, $q_5$, $q_6$, $q_7$, $q_8$, $q_9$, and $q_{10}$ are each independently selected from hydrogen.

In certain embodiments, in the provided oligonucleotide, $M_3$ is O, S, $C(q_7)(q_8)$; $M_2$ is $C(q_3)(q_4)$; $X_2$ is $R^{15}$ or N; $X_3$ is a chemical bond, SO, or $SO_2$; $R^{15}$, $q_1$, $q_2$, $q_3$, $q_4$, $q_7$, and $q_8$ are as defined herein and as shown in the following structural schematic:

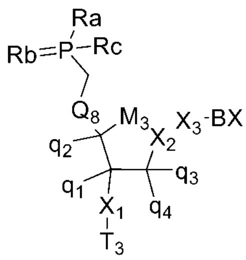

.

In certain preferred embodiments, $M_3$ is O, S, or $CH_2$; $M_2$ is $CH_2$; $X_2$ is CH; $X_3$ is a chemical bond. In certain preferred embodiments, $M_3$ is $CH_2$; $M_2$ is $CH_2$; $X_2$ is N; $X_3$ is a chemical bond, SO, or $SO_2$.

In certain embodiments, $M_3$ is $C(q_7)(q_8)$; $M_2$ is $C(q_3)(q_4)C(q_5)(q_6)$; $X_2$ is $CR^{15}$ or N; $X_3$ is a chemical bond, SO, or $SO_2$; $R^{15}$, $q_1$, $q_2$, $q_3$, $q_4$, $q_5$, $q_6$, $q_7$, and $q_8$ are as defined herein and as shown in the following structural schematic:

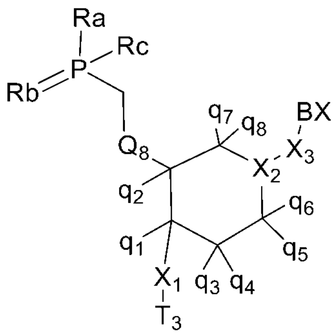

.

In certain preferred embodiments, $M_3$ is $CH_2$; $M_2$ is $CH_2CH_2$; $X_2$ is CH; $X_3$ is a chemical bond. In certain preferred embodiments, $M_3$ is $CH_2$; $M_2$ is $CH_2CH_2$; $X_2$ is N; $X_3$ is a chemical bond, SO, or $SO_2$.

In certain embodiments, in the provided oligonucleotide, in formula (VIII-1), $Q_8$ is SO or $SO_2$.

In certain embodiments, in the provided oligonucleotide, in formula (VIII-1), $Q_8$ is S.

In certain embodiments, in the provided oligonucleotide, in formula (VIII-1), $R_b$ is oxygen.

In certain embodiments, in the provided oligonucleotide, in formula (VIII-1), $Q_8$ is SO or $SO_2$, and Ra and Rc are each independently selected from OH, SH, $NH_2$, and $NHSO_2CH_3$.

In certain embodiments, in the provided oligonucleotide, in formula (VIII-1), $Q_8$ is SO or $SO_2$, $R_b$ is oxygen, and Ra and Rc are each independently selected from OH.

In certain embodiments, in the provided oligonucleotide, in formula (VIII-1), $Q_8$ is SO or $SO_2$, and $X_1$ is oxygen.

In certain embodiments, in the provided oligonucleotide, in formula (VIII-1), $Q_8$ is S, and $X_1$ is oxygen.

In certain embodiments, in the provided oligonucleotide, in formula (VIII-1), $Q_8$ is S, $R_b$ is oxygen, and Ra and Rc are each independently selected from OH.

In certain embodiments, in the provided oligonucleotide, in formula (VIII) or formula (VIII-1), the $B_X$ heterocyclic base moiety is selected from a natural nucleobase, a modified nucleobase, and a universal base.

In certain embodiments, in the provided oligonucleotide, in formula (VIII) or formula (VIII-1), the $B_X$ heterocyclic base moiety is pyrimidine, substituted pyrimidine, pseudouracil, substituted pseudouracil, purine, hypoxanthine, or substituted purine.

In certain embodiments, the $B_X$ heterocyclic base moiety is uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine. In certain embodiments, each $B_X$ heterocyclic base moiety is independently 2-thiouracil, 5-fluorouracil, dihydrouridine (D), or 7-methylguanosine (m7G).

In certain embodiments, in the provided oligonucleotide, in formula (VIII) or formula (VIII-1), provided is an oligonucleotide comprising linked monomer subunits, wherein each internucleoside linking group is independently an alkylphosphonate linking group, a phosphodiester internucleoside linking group, or a phosphorothioate nucleoside linking group.

In certain embodiments, the provided oligonucleotide for separate use has a compound fragment of the following formula at the 5'-terminal nucleotide:

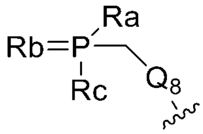

wherein $Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{16}$ and $R^{17}$ are independently (=O), (=S), OH, SH, $C_1$-$C_6$ alkyl, or $NR^{18}$;

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, protected or optionally substituted amino, and a natural or modified nucleoside, and $R_b$ is O or S or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;

$R^{11}$, $R^{18}$, and $R^{19}$ are independently H, optionally substituted $C_1$-$C_6$ alkyl, optionally substituted $C_1$-$C_6$ alkoxy, methanesulfonyl, or sulfo;

each substituted group comprises one or more substituent groups optionally and independently selected from halogen, hydroxyl, $C_1$-$C_6$ alkyl, $C_1$-$C_6$ alkoxy, $C_1$-$C_6$ alkylsulfhydryl, and CN; "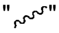" refers to the linkage to the remainder of the 5'-terminal nucleotide.

In certain embodiments, it can be understood that the 5'-terminal nucleotide has a compound fragment of the following formula:

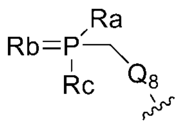,

wherein the definitions of $Q_8$, Ra, $R_b$, and Rc are each consistent with those in the 5'-terminal nucleotide of formula (V), formula (VI), or formula (VII) of the oligonucleotide described herein and the embodiments.

In certain embodiments, the provided oligonucleotide comprises a 5'-terminal nucleoside represented by one of the following specific structures or stereoisomers thereof:

| | |
|---|---|
| 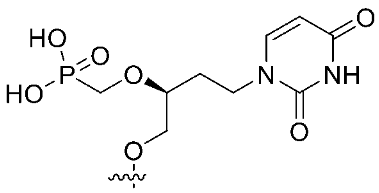 Phos-01 | 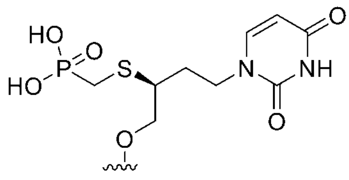 Phos-02 |
| 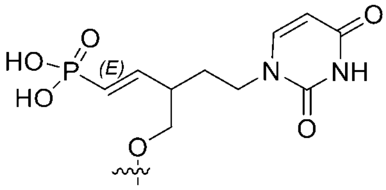 Phos-03 | 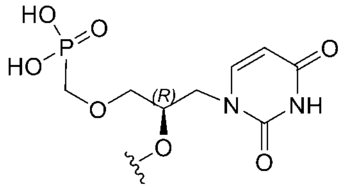 Phos-04 |
| 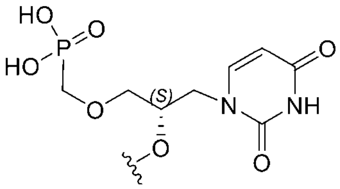 Phos-05 | 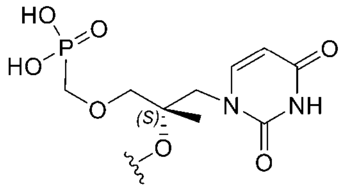 Phos-06 |
| 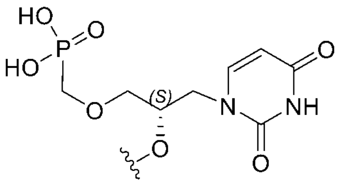 Phos-07 | 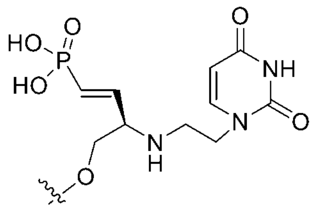 Phos-08 |
| 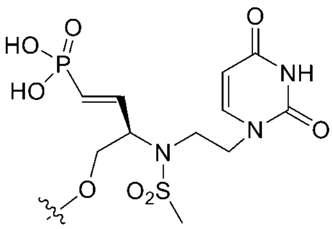 Phos-09 | 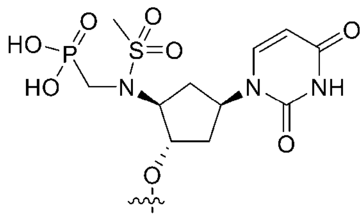 Phos-10 |

| | |
|---|---|
| 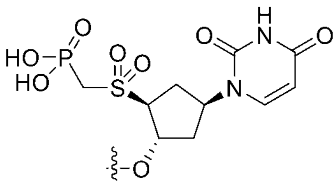 Phos-11-1 | 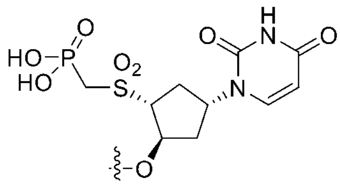 Phos-11-2 |
| 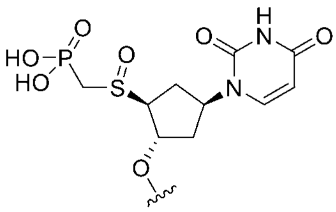 Phos-11-4 | 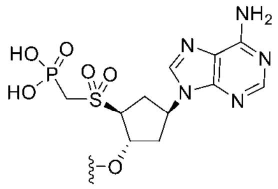 Phos-11-7 |
| 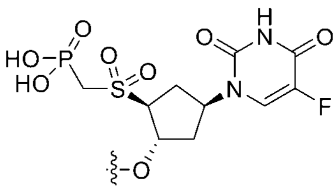 Phos-11-8 | 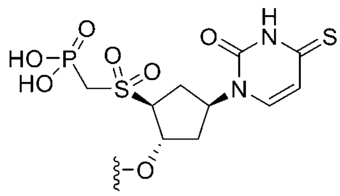 Phos-11-9 |
| 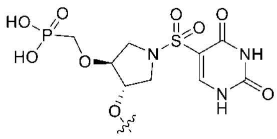 Phos-13 | 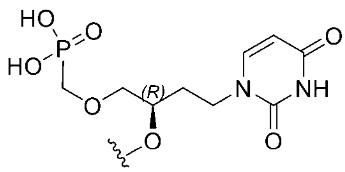 Phos-14 |
| 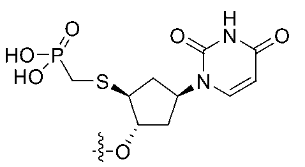 Phos-15-1 | 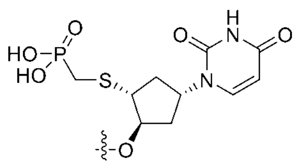 Phos-15-2 |
| 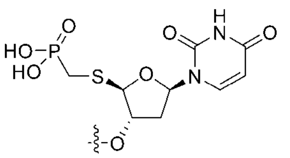 Phos-15-3 | 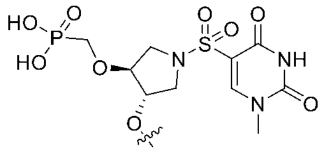 phos-18 |
| 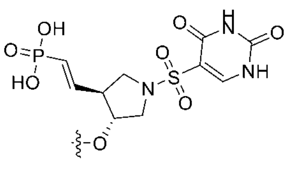 Phos-19-1 | 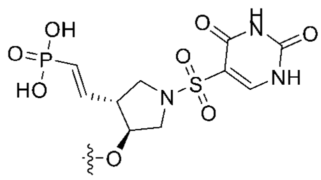 Phos-19-2 |
| 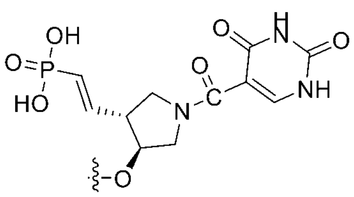 Phos-19-5 | 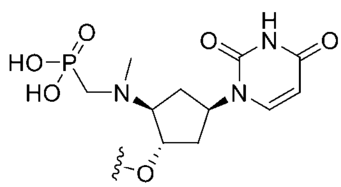 Phos-21 |

| | |
|---|---|
| 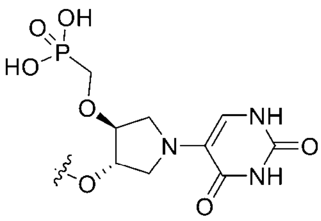 Phos-22 | 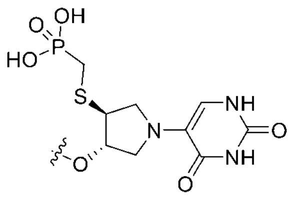 Phos-23 |
| 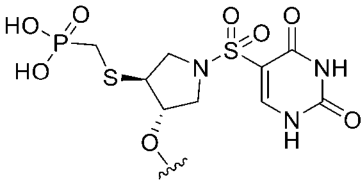 Phos-26 | 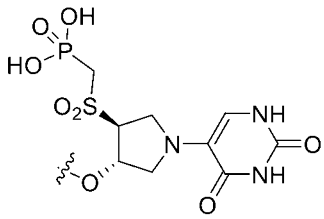 Phos-25 |
| 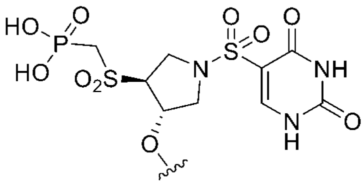 Phos-24 | 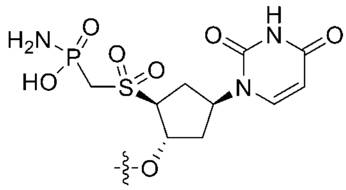 Phos-27 |
| 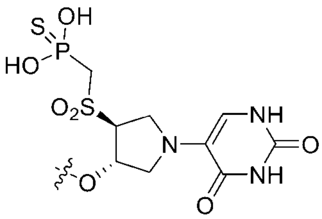 Phos-28 | 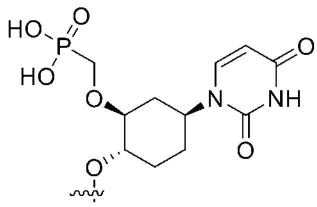 Phos-29 |
| 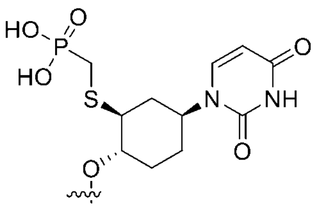 Phos-30 | 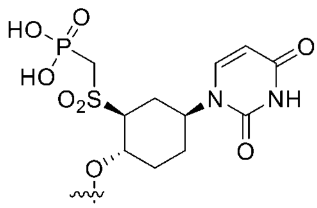 Phos-31 |
| 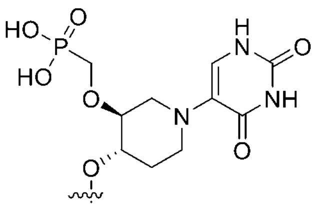 Phos-32 | 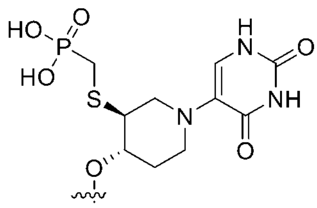 Phos-33 |
| 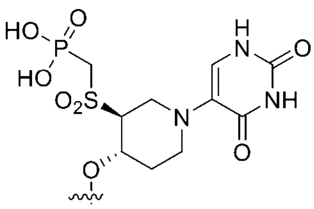 Phos-34 | 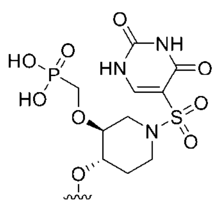 Phos-35 |

| | |
|---|---|
| 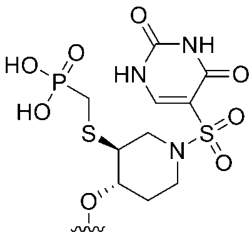 Phos-36-1 | 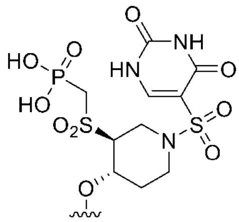 Phos-37 |
| 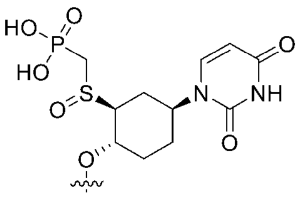 Phos-38 | 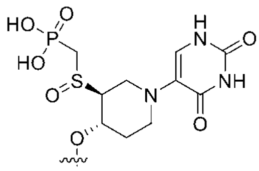 Phos-39 |
| 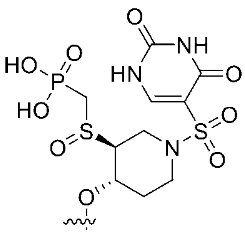 Phos-40 | 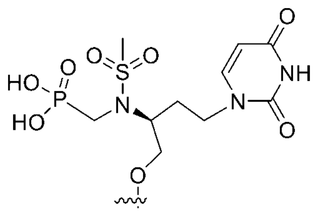 Phos-41 |
| 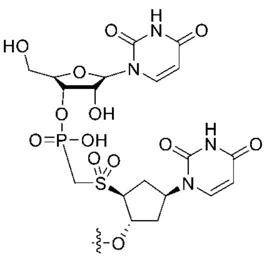 Phos-42-3 | 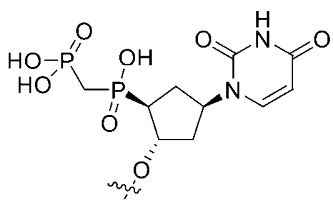 Phos-43-1 |
| 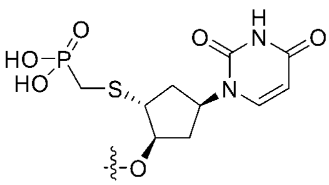 Phos-42-1 | 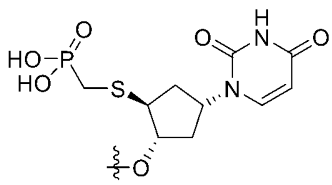 Phos-42-2 |
| 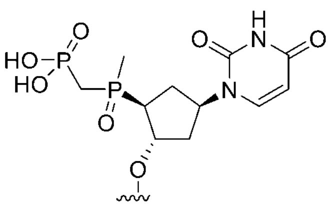 Phos-44-1 | 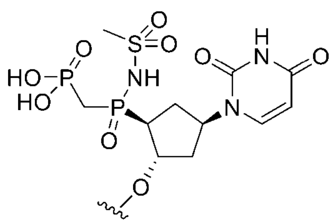 Phos-45-1 |
| 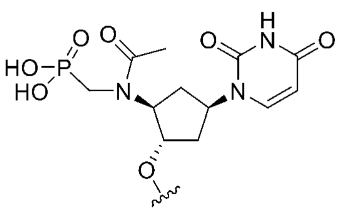 Phos-12-1 | 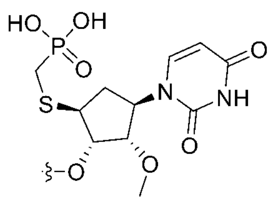 Phos-43 |

| | |
|---|---|
| 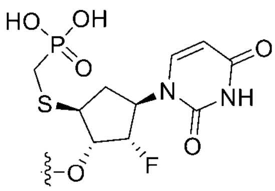 Phos-44 | 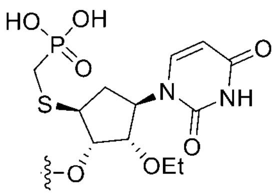 Phos-45 |
| 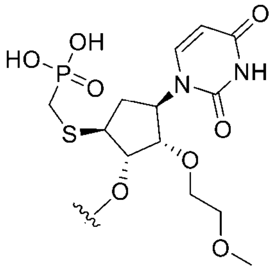 Phos-46 | 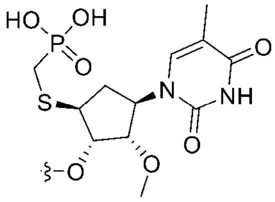 Phos-47 |
| 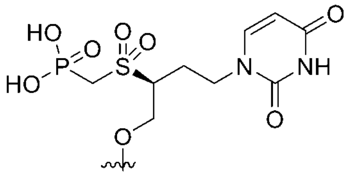 Phos-2-2 | 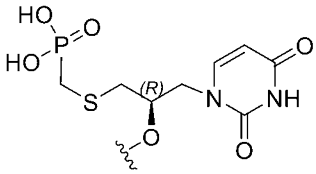 Phos-4-1 |
| 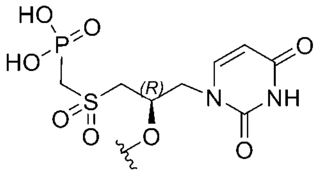 Phos-4-2 | 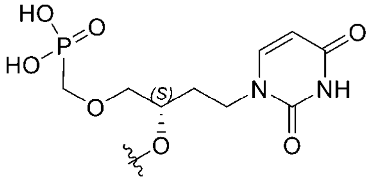 Phos-5-1 |
| 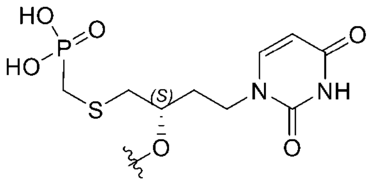 Phos-5-2 | 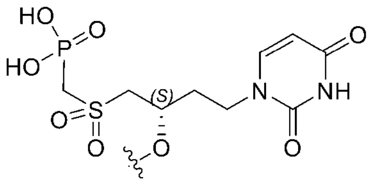 Phos-5-3 |
| 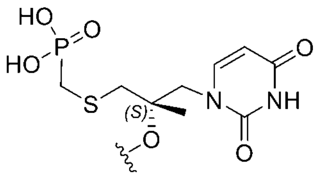 Phos-6-1 | 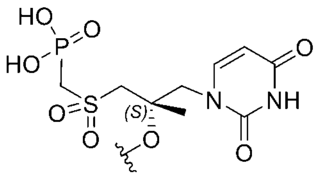 Phos-6-2 |
| 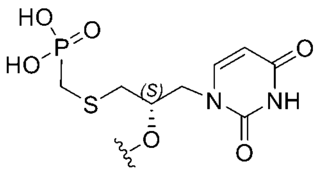 Phos-7-1 | 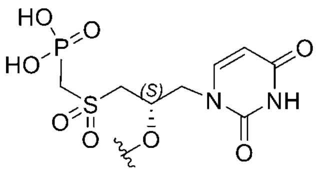 Phos-7-2 |

| | |
|---|---|
| 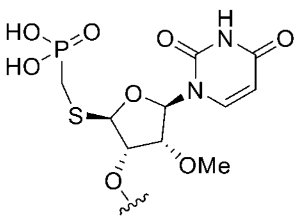 Phos-53 | . |

“ 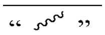 ”

indicates a linking moiety to an internucleoside linking group at the 5’-end of the oligonucleotide.

In certain embodiments, the provided oligonucleotide comprises a 5’-terminal nucleoside represented by one of the following specific structures or stereoisomers thereof:

| | |
|---|---|
| 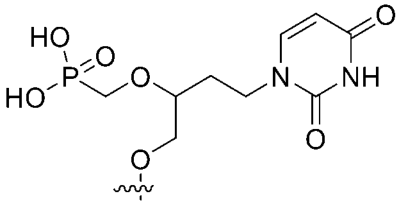 | 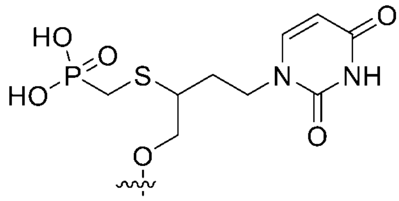 |
| 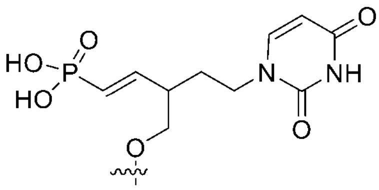 | 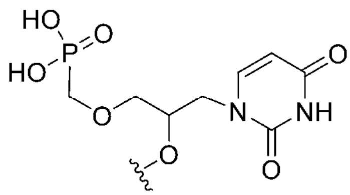 |
| 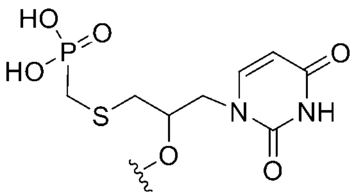 | 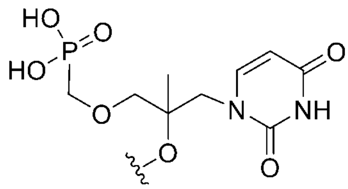 |
| 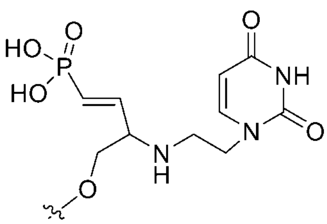 | 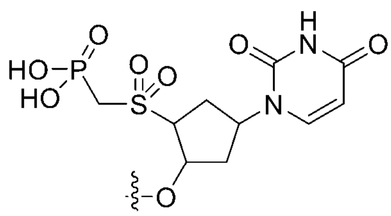 |
| 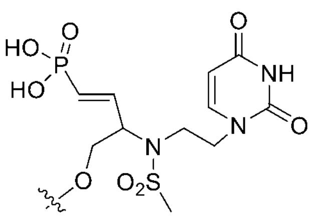 | 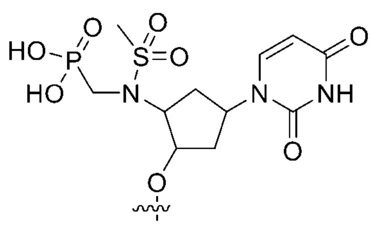 |

| | |
|---|---|
| 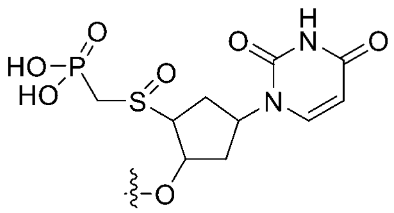 | 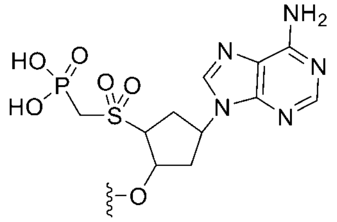 |
| 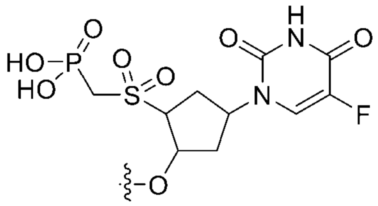 | 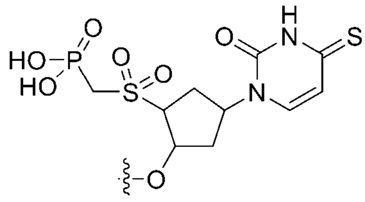 |
| 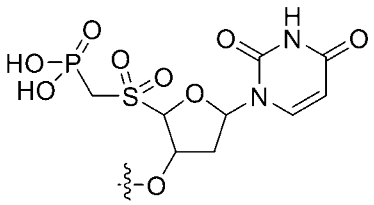 | 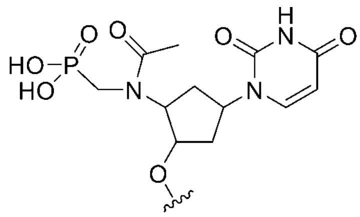 |
| 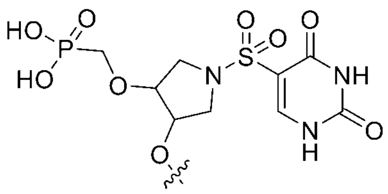 | 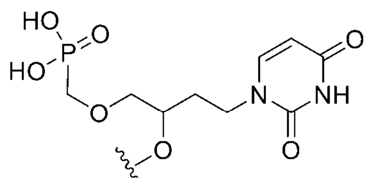 |
| 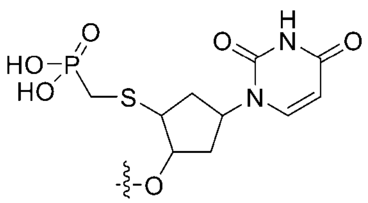 | 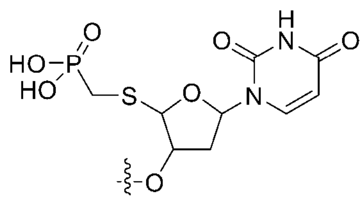 |
| 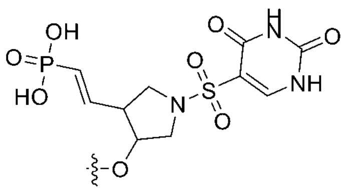 | 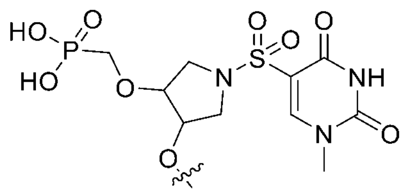 |
| 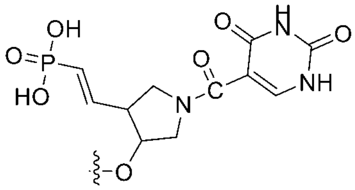 | 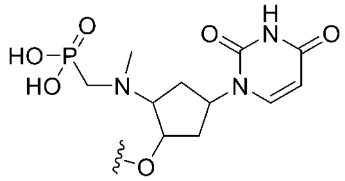 |

| | |
|---|---|
| 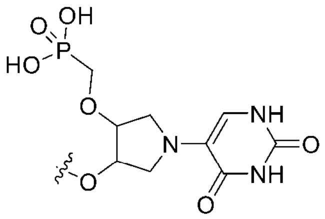 | 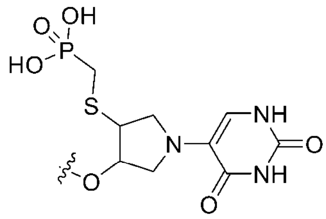 |
| 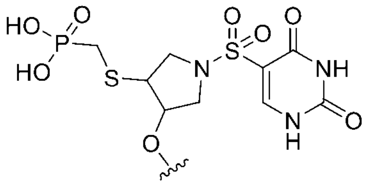 | 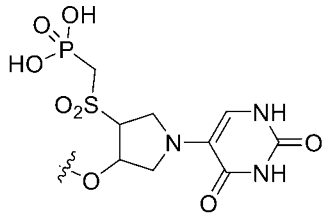 |
| 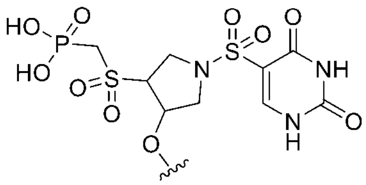 | 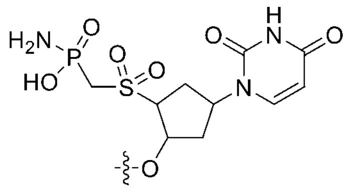 |
| 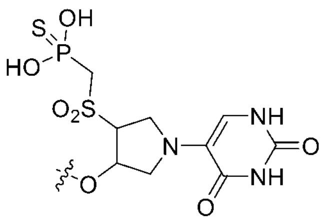 | 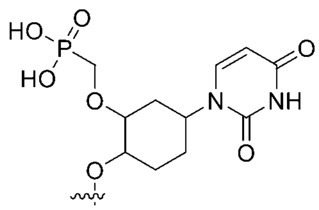 |
| 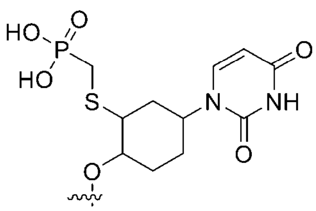 | 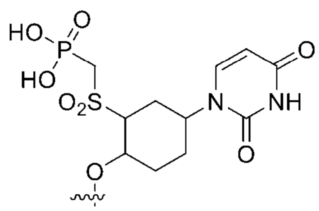 |
| 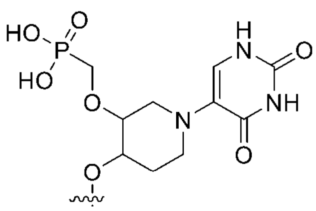 | 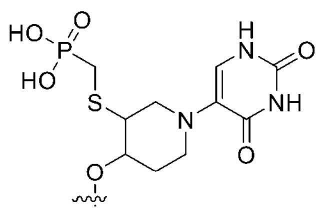 |
| 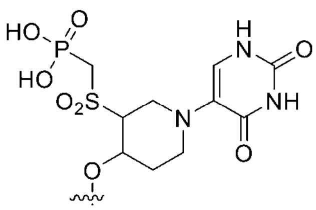 | 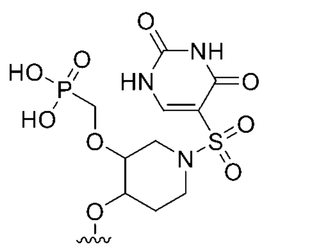 |

| | |
|---|---|
| 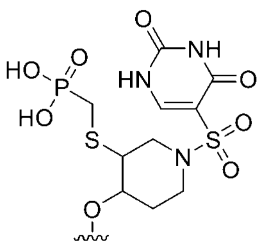 | 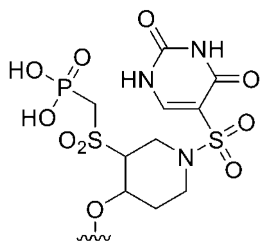 |
| 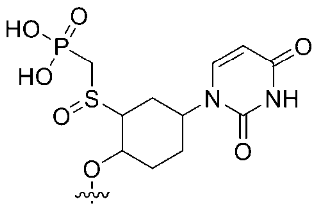 | 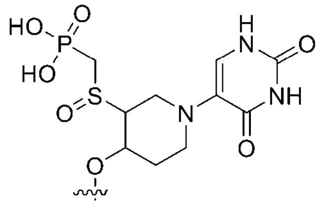 |
| 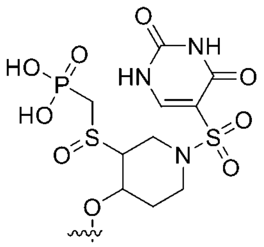 | 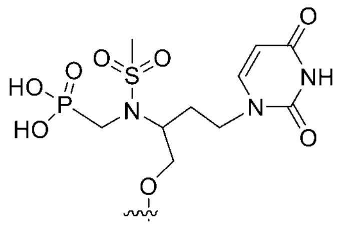 |
| 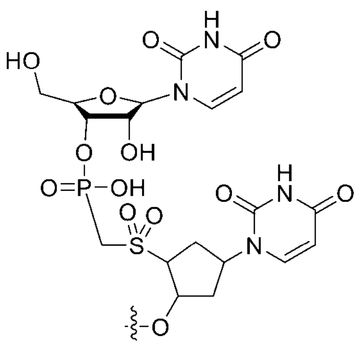 | 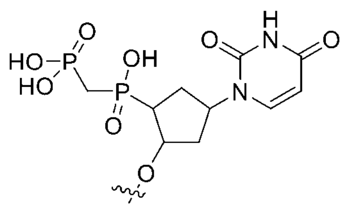 |
| 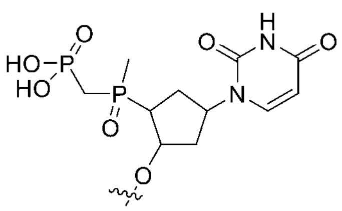 | 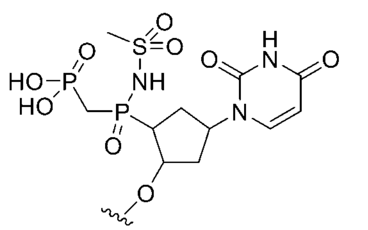 |
| 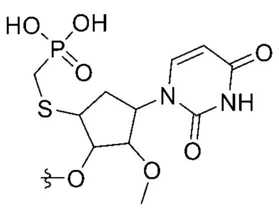 | 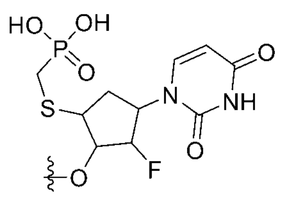 |

| | |
|---|---|
| 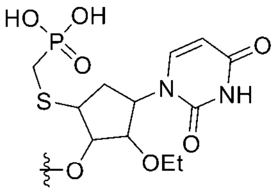 | 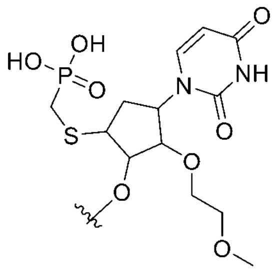 |
| 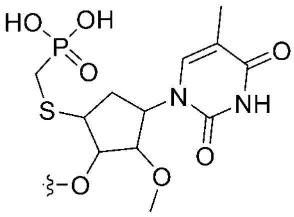 | 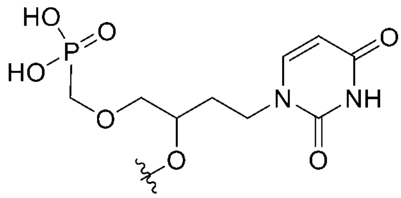 |
| 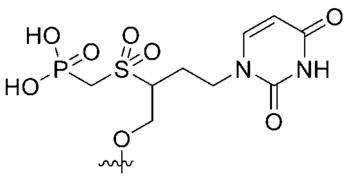 | 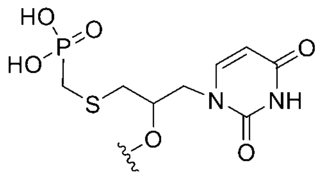 |
| 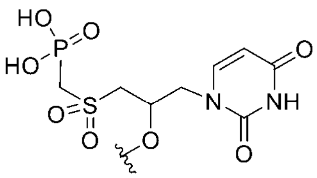 | 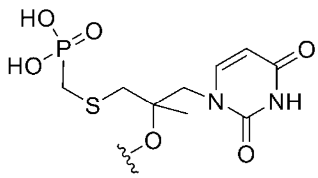 |
| 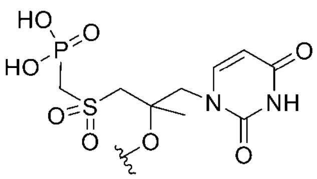 | 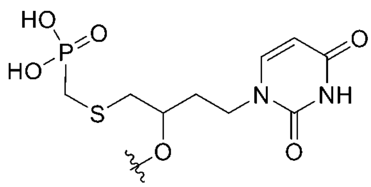 |
| 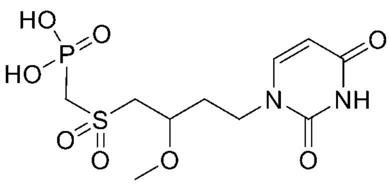 | 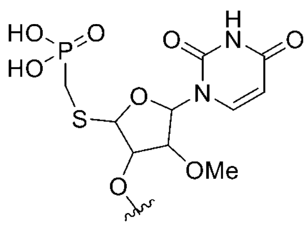. |

“ 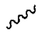 ”

indicates a linking moiety to an internucleoside linking group at the 5'-end of the oligonucleotide.

The oligonucleotide described herein includes, but is not limited to, single-stranded antisense oligonucleotides (ASOs), miRNAs, and double-stranded ribonucleic acids (dsRNAs). In certain embodiments, the provided oligonucleotide is a single-stranded oligonucleotide. In certain embodiments, the single-stranded oligonucleotide is a conventional antisense oligonucleotide (also known as ASO), a ribozyme, or an aptamer. In certain embodiments, the provided oligonucleotide is a double-stranded ribonucleic acid (dsRNA) agent, and such a compound is a double-stranded ribonucleic acid well known in the art, wherein one or both strands are oligonucleotides as disclosed herein.

In certain embodiments, the provided oligonucleotide is a double-stranded ribonucleic acid (dsRNA) agent, which comprises a sense strand and an antisense strand, wherein the sense strand and the antisense strand are fully or partially complementary, and the antisense strand is partially or fully complementary to a nucleic acid target gene; at least one of the sense strand and the antisense strand is an oligonucleotide as provided above, which comprises at least one 5'-terminal nucleotide represented by formula (V), formula (V-1), formula (V-2), formula (VI), formula (VI-1), formula (VI-2), formula (VI-3), formula (VII), formula (VII-1), formula (VII-2), formula (VIII), formula (VIII-1), or a stereoisomer thereof; and the double-stranded ribonucleic acid (dsRNA) agent optionally further comprises an independent targeting group.

In certain embodiments, the provided oligonucleotide is a double-stranded ribonucleic acid (dsRNA) agent, wherein the antisense strand comprises at least one 5'-terminal nucleotide represented by formula (V), formula (V-1), formula (V-2), formula (VI), formula (VI-1), formula (VI-2), formula (VI-3), formula (VII), formula (VII-1), formula (VII-2), formula (VIII), formula (VIII-1), or a stereoisomer thereof.

In certain embodiments, in the provided oligonucleotide for separate use or double-stranded ribonucleic acid (dsRNA), each strand comprises 8-40 nucleotides.

In certain embodiments, the provided double-stranded ribonucleic acid (dsRNA) agent optionally further comprises an independent targeting group, and the 5'-terminal and/or 3'-terminal nucleotides of either strand of the double-stranded ribonucleic acid (dsRNA) agent further comprise one or more targeting groups. The targeting group may be a ligand commonly used in the field of siRNA administration.

In some embodiments, in the provided double-stranded ribonucleic acid (dsRNA) agent, the targeting group may be selected from one or more of the following ligands formed from targeting molecules or derivatives thereof: polymers, sugars, ligands for receptors expressed on hepatocytes, antibodies, quantum dots, polypeptides, and small molecule ligands.

In some embodiments, in the provided double-stranded ribonucleic acid (dsRNA) agent, at least one of or each of the targeting groups is selected from ligands capable of binding to receptors on the surfaces of mammalian hepatocytes.

In some embodiments, in the provided double-stranded ribonucleic acid (dsRNA) agent, each of the targeting groups is independently a ligand that has an affinity for an asialoglycoprotein receptor (ASGPR) on the surface of a mammalian hepatocyte.

In some embodiments, in the provided double-stranded ribonucleic acid (dsRNA) agent, at least one of or each of the targeting groups is a ligand comprising galactose or N-acetylgalactosamine.

In some embodiments, in the provided double-stranded ribonucleic acid (dsRNA) agent, at least one of or each of the targeting groups is a fragment having the following structure:

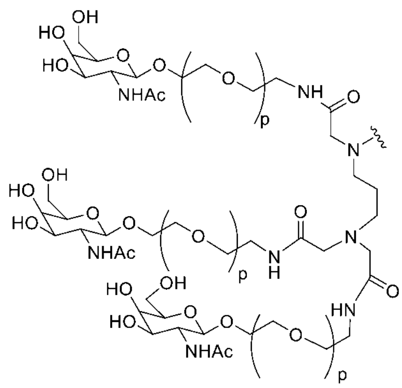

p is 1 or 2.

In certain embodiments, provided is use of the oligonucleotide or double-stranded ribonucleic acid (dsRNA) agent in the preparation of a medicament for inhibiting gene expression. In certain embodiments, the inhibition of gene expression comprises contacting one or more cells, tissues, or animals with the oligonucleotide or double-stranded ribonucleic acid (dsRNA).

In certain embodiments, provided is a method for inhibiting gene expression, and the method comprises contacting a cell with an agent comprising the oligonucleotide or double-stranded ribonucleic acid (dsRNA) described herein, wherein each strand of the oligonucleotide comprises 8-40 nucleotides, and the antisense strand of the oligonucleotide agent is complementary to a target RNA.

In certain embodiments, the cell is in an animal. In certain embodiments, the cell is in a human. In certain embodiments, the target RNA is selected from mRNA, pre-mRNA, and micro RNA. In certain embodiments, the target RNA is mRNA. In certain embodiments, the target RNA is human mRNA. In certain embodiments, the target RNA is cleaved, thereby inhibiting its function. In certain embodiments, the method further comprises detecting the level of the target RNA. In certain embodiments, provided is a method for inhibiting gene expression, and the method comprises

contacting one or more cells or tissues with the provided oligonucleotide or double-stranded ribonucleic acid (dsRNA) agent comprising a 5'-terminal nucleotide represented by formula (V), formula (V-1), formula (V-2), formula (VI), formula (VI-1), formula (VI-2), formula (VI-3), formula (VII), formula (VII-1), formula (VII-2), formula (VIII), formula (VIII-1), or a stereoisomer thereof.

## DETAILED DESCRIPTION OF THE PRESENT DISCLOSURE

In certain embodiments, provided is a 5'-phosphonate-modified nucleoside analog that can be incorporated, as a nucleotide monomer compound, into an end of an oligonucleotide, particularly the 5'-end of an antisense strand. Also provided herein are intermediates and methods for preparing these oligonucleotides. The 5'-phosphonate-modified nucleoside analog provided herein may be used to improve the expression inhibition duration and/or activity of an oligonucleotide. In certain embodiments, the oligonucleotide and the composition provided herein are expected to hybridize with a portion of a target RNA such that the target RNA loses normal function. The oligonucleotide is also expected to be used as a primer and a probe in diagnostic applications.

Unless specific definitions are provided, the nomenclature, as well as the procedures and techniques thereof, used in connection with analytical chemistry, organic synthetic chemistry, and medicinal chemistry as described herein are those well known and commonly used in the art. Standard techniques can be used for chemical synthesis and chemical analysis. They are hereby incorporated by reference for any purpose. Where permitted, all patents, patent applications, published patent applications, and other publications and data referred to throughout this disclosure are incorporated by reference in their entirety.

As used herein, the term "unsubstituted" or "substituted" means that in a parent compound, no hydrogen atom is substituted with other substituents, or one or more hydrogen atoms are substituted with substituents. As used herein, the terms "substituent" and "substituent group" are intended to include groups that are commonly added to other groups or parent compounds to enhance desired properties or to provide other desired effects. A substituent group may be protected or unprotected, and may be added to one available site or to many available sites in a parent compound. A substituent group may also be further substituted with other substituent groups and may be linked to a parent compound either directly or by a linking group such as an alkyl or hydrocarbyl group. Unless otherwise specified, the substituents herein may be generally suitable substituents well known in the art, and the suitable substituent groups herein include, but are not limited to: halogen, hydroxyl, alkyl, alkenyl, alkynyl, acyl ($-C(=O)R_{aa}$), carboxyl ($-C(=O)O-R_{aa}$), aliphatic groups, alicyclic groups, alkoxy, substituted oxygen ($-O-R_{aa}$), aryl, aralkyl, heterocyclyl, heteroaryl, heteroarylalkyl, amino ($-N(R_{bb})(R_{cc})$), imino ($=NR_{bb}$), amido ($-C(=O)N(R_{bb})(R_{cc})$ or $-N(R_{bb})C(=O)R_{aa}$), azido ($-N_3$), nitro ($-NO_2$), cyano (-CN), ureido ($-N(R_{bb})C(=O)N(R_{bb})(R_{cc})$), thioureido ($-N(R_{bb})C(S)N(R_{bb})(R_{cc})$), guanidino ($-N(R_{bb})C(=NR_{bb})N(R_{bb})(R_{cc})$), amidino ($-C(=NR_{bb})N(R_{bb})(R_{cc})$ or $-N(R_{bb})C(=NR_{bb})(R_{aa})$), thiol or sulfhydryl ($-SR_{bb}$), sulfinyl ($-S(=O)R_{bb}$), sulfonyl ($-S(=O)_2R_{bb}$), and sulfonamidyl ($-S(=O)_2N(R_{bb})(R_{cc})$ or $-N(R_{bb})S(=O)_2R_{bb}$). Each $R_{aa}$, each $R_{bb}$, and each $R_{cc}$ are independently H, an optionally linked chemical functional group, or an additional substituent group. The preferred list includes, but is not limited to, H, alkyl, alkenyl, alkynyl, aliphatic groups, alkoxy, acyl, aryl, aralkyl, heteroaryl, alicyclic groups, heterocyclyl, and heteroarylalkyl. In preferred embodiments of some substituent groups that are not specifically specified, the substituent groups are selected from halogen, hydroxyl, $C_1$-$C_6$ alkyl, $C_1$-$C_6$ alkoxy, $C_1$-$C_6$ alkylsulfhydryl, $O(CH_2)_2-OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl. As used herein, "substituents" are contemplated aspects of the present disclosure, and those of ordinary skill in the pharmaceutical and organic chemistry arts will understand the generality of such substituents. The "substituents" should satisfy the principle in the present disclosure that their total number will enable the chemical bonding formation with the atoms or groups to which they are attached. For example, when $Q_8$ is $PR^{16}R^{17}$, and $R^{16}$ and $R^{17}$ are independently (=O), (=S), OH, SH, $C_1$-$C_6$ alkyl, or $NR^{18}R^{19}$, to satisfy the chemical form of pentavalent phosphorus, at least one of $R^{16}$ and $R^{17}$ is (=O) or (=S), and the other is OH, SH, $C_1$-$C_6$ alkyl, or $NR^{18}R^{19}$.

As used herein, the terms "optionally protected" and "optionally substituted" mean that substitution or protection is optionally present; therefore, the terms encompass unsubstituted (unprotected) and substituted (protected) atoms and moieties.

As used herein, the term "alkyl" refers to a linear or branched saturated hydrocarbyl group having 1 to about 24 carbon atoms. When it appears herein, for example, as "$C_1$-$C_6$ alkyl", the number range is intended to refer generally to the number of carbon atoms in the alkyl group, and the alkyl group may contain only 1 carbon atom, 2 carbon atoms, 3 carbon atoms, etc., up to and including 6 carbon atoms. Unless otherwise indicated, the number of the carbon atoms added to the alkyl group by further substitution with other atoms or groups is generally not within the count range, and the same description also applies to the other counting methods herein. Examples of alkyl include, but are not limited to, methyl, ethyl, propyl, butyl, isopropyl, n-hexyl, octyl, decyl, dodecyl, and the like. The term "alkyl" further includes instances where no number range of carbon atoms is designated; for example, the term "alkyl" may refer to a sub-range of $C_1$-$C_{10}$ (e.g., $C_1$-$C_6$). "Substituted alkyl" refers to an alkyl moiety bearing a substituent. As used herein, "lower alkyl" refers to an alkyl moiety having 1 to about 6 carbon atoms. As used herein, the term "alkylene" refers to a divalent radical derived by

substitution of another carbon atom of an alkyl group, and it is either a standalone group or part of another substituent. Examples include, but are not limited to, $-CH_2CH_2-$.

As used herein, the term "alkenyl" refers to a linear or branched hydrocarbyl group having at least one carbon-carbon double bond. Examples of alkenyl include, but are not limited to, ethenyl, propenyl, butenyl, 1-methyl-2-buten-1-yl, dienes such as 1,3-butadiene, and the like. Alkenyl generally contains 2 to about 24 carbon atoms, more generally 2 to about 12 carbon atoms, and those containing 2 to about 6 carbon atoms are more preferred. As used herein, alkenyl may optionally contain one or more further substituent groups.

As used herein, the term "alkynyl" refers to a linear or branched hydrocarbyl group having at least one carbon-carbon triple bond. Examples of alkynyl include, but are not limited to, ethynyl, 1-propynyl, 1-butynyl, and the like. Alkynyl generally contains 2 to about 24 carbon atoms, more generally 2 to about 12 carbon atoms, and those containing 2 to about 6 carbon atoms are more preferred. As used herein, alkynyl may optionally contain one or more further substituent groups.

As used herein, the term "acyl" refers to a group formed by removing hydroxyl from an organic acid, and the group has the general formula -C(=O)-X, wherein X is generally aliphatic, alicyclic, or aromatic. Examples include aliphatic carbonyl, aromatic carbonyl, aliphatic sulfonyl, aromatic sulfinyl, aliphatic sulfinyl, aromatic phosphate, aliphatic phosphate, and the like. As used herein, acyl may optionally contain a further substituent group.

The term "cycloalkyl" refers to a ring system in which the ring is aliphatic. The ring system may contain one or more rings, wherein at least one ring is aliphatic. Preferred alicyclic compounds contain rings having about 5 to about 9 carbon atoms in the ring. As used herein, cycloalkyl may optionally contain a further substituent group.

As used herein, the term "alkoxy" refers to a group formed between an alkyl group and an oxygen atom, wherein the oxygen atom is used to link the alkoxy group to the parent molecule. Examples of alkoxy include, but are not limited to, methoxy, ethoxy, propoxy, isopropoxy, n-butoxy, sec-butoxy, tert-butoxy, n-pentoxy, neopentoxy, n-hexoxy, and the like. As used herein, alkoxy may optionally contain a further substituent group.

As used herein, the term "aminoalkyl" refers to amino-substituted $C_1$-$C_{12}$ alkyl. The alkyl moiety of the group forms a covalent bond with the parent molecule. The amino group may be located at any position, and the aminoalkyl group may be substituted at the alkyl group and/or the amino group with a further substituent group.

As used herein, the terms "aryl" and "aromatic" refer to a monocyclic or polycyclic carbocyclic group having one or more aromatic rings. As used herein, the term "aromatic group" refers to a planar ring having a delocalized $\pi$-electron system containing $4n + 2$ $\pi$ electrons, wherein n is an integer. The aromatic ring may be formed from 5, 6, 7, 8, 9, or more than 9 atoms. The term "aromatic" is intended to include carbocyclic aryl (e.g., phenyl) and heterocyclic aryl (or "heteroaryl" or "heteroaromatic") groups (e.g., pyridine). The term includes monocyclic or fused polycyclic rings, i.e., rings which share adjacent pairs of carbon atoms. "Substituted aromatic group" refers to an aromatic group further bearing one or more substituents. Examples of aryl include, but are not limited to, phenyl, naphthyl, tetrahydronaphthyl, indanyl, indenyl, and the like. Preferred aryl ring systems have about 5 to about 20 carbon atoms in one or more rings. As used herein, aryl may optionally contain a further substituent group.

As used herein, the terms "aralkyl" and "arylalkyl" refer to an aromatic group covalently linked to $C_{1-}C_{12}$ alkyl. The alkyl moiety of the resulting aralkyl (or arylalkyl) group forms a covalent bond with the parent molecule. Examples include, but are not limited to, benzyl, phenethyl, and the like. As used herein, aralkyl may optionally contain a further substituent group linked to the alkyl group, the aryl group, or the two that form the group.

As used herein, the terms "heteroaryl" and "heteroaromatic" refer to a group containing a monocyclic or polycyclic aromatic ring, ring system, or fused ring system, wherein at least one of these rings is aromatic and contains one or more heteroatoms. Heteroaryl is also intended to include fused ring systems, including systems in which one or more of the fused rings contain no heteroatom. Heteroaryl generally contains one ring atom selected from sulfur, nitrogen, and oxygen. Examples of heteroaryl include, but are not limited to, pyridyl, pyrazinyl, pyrimidinyl, pyrrolyl, pyrazolyl, imidazolyl, thiazolyl, oxazolyl, isoxazolyl, thiadiazolyl, oxadiazolyl, thiophenyl, furanyl, quinolinyl, isoquinolinyl, benzimidazolyl, benzoxazolyl, quinoxalinyl, and the like. Heteroaryl may be linked to the parent molecule directly or by a linking moiety such as an aliphatic group or a heteroatom. As used herein, heteroaryl may optionally contain a further substituent group.

As used herein, the term "heteroarylalkyl" refers to a heteroaryl group as previously defined that further contains covalently linked $C_1$-$C_{12}$ alkyl. The alkyl moiety of the resulting heteroarylalkyl group is capable of forming a covalent bond with the parent molecule. Examples include, but are not limited to, pyridylmethyl, pyridylethyl, napthyridinylpropyl, and the like. As used herein, heteroarylalkyl may optionally contain a further substituent group on one or both of the heteroaryl moiety or the alkyl moiety.

As used herein, the term "halo" or "halogen" refers to an atom selected from fluorine, chlorine, bromine, and iodine.

As used herein, the term "heterocyclic group" refers to an unsaturated, partially saturated, or fully saturated, monocyclic or polycyclic ring system containing at least one heteroatom, including heteroaryl groups. Heterocyclic groups are also intended to include fused ring systems, wherein one or more of the fused rings contain at least one heteroatom, and the other rings may contain one or more heteroatoms or optionally contain no heteroatoms. A heterocyclic group generally contains at least one atom selected from sulfur, nitrogen, and oxygen. Examples of heterocyclic groups include

[1,3]dioxolanyl, pyrrolidinyl, pyrazolinyl, pyrazolidinyl, imidazolinyl, imidazolidinyl, piperidinyl, pyrazinyl, oxazolidinyl, isoxazolidinyl, morpholinyl, thiazolidinyl, isothiazolidinyl, quinoxalinyl, pyridazinonyl, tetrahydrofuranyl, and the like. As used herein, an "n"-membered heterocyclic group refers to the sum of n atoms in the ring system, excluding atoms or groups outside the ring system. As used herein, a heterocyclic group may optionally contain a further substituent group.

As used herein, the term "monocyclic or polycyclic structure" includes all ring systems selected from monocyclic ring systems or polycyclic ring systems in which rings are fused or linked and is intended to include single and mixed ring systems individually selected from aliphatic groups, alicyclyl, aryl, heteroaryl, aralkyl, arylalkyl, heterocyclyl, heteroaryl, heteroaromatic groups, and heteroarylalkyl. Such monocyclic and polycyclic structures may contain rings that each have the same degree of saturation or that each independently have a different degree of saturation (including fully saturated, partially saturated, or fully unsaturated). Each ring may contain ring atoms selected from C, N, O, and S to give rise to heterocyclic rings and rings containing only C ring atoms. These rings may be present in a mixed motif, such as, for example, benzimidazole, wherein one ring has only carbon ring atoms, and the fused ring has two nitrogen atoms. The monocyclic or polycyclic structures may be further substituted with substituent groups such as, for example, phthalimide, which has two =O groups linked to one of the rings. The monocyclic or polycyclic structures may be linked to the parent molecules using a variety of strategies, such as directly by a ring atom, by a substituent group, or by a bifunctional linking moiety.

The term "oxo" refers to the group (=O).

As used herein, the term "sulfonyl" (alone or as part of another group) refers to a group of the formula $RSO_2$-, wherein R is hydrogen, alkyl, substituted alkyl, aryl, or substituted aryl, and $CH_3SO_2$- is referred to as methanesulfonyl. As used herein, the term "sulfinyl" (alone or as part of another group) refers to a group of the formula RSO-. As used herein, the term "sulfonamido" (alone or as part of another group) refers to a group of the formula $RSO_2$-NH-, wherein R is hydrogen, alkyl, substituted alkyl, aryl, or substituted aryl. Some non-limiting exemplary sulfonamido groups include $CH_3$-$SO_2$-N(H)-.

The term "alicyclic" or "alicyclyl" refers to a cyclic ring system, wherein the ring is aliphatic. The ring system may contain one or more rings, wherein at least one ring is an aliphatic ring. Preferred aliphatic rings include rings having about 5 to about 9 carbon atoms in the ring. As used herein, the aliphatic ring optionally contains other substituent groups. As used herein, the term "aliphatic" refers to a linear or branched hydrocarbyl group containing up to twenty-four carbon atoms, wherein the degree of saturation between any two carbon atoms is a single, double, or triple bond. An aliphatic group preferably contains 1 to about 24 carbon atoms, more generally 1 to about 12 carbon atoms, and more preferably 1 to about 6 carbon atoms. The linear or branched chain of an aliphatic group may be interrupted by one or more heteroatoms, including nitrogen, oxygen, sulfur, phosphorus, and the like. The aliphatic group interrupted by a heteroatom includes, but is not limited to, polyalkoxy groups such as polyalkylene glycols, polyamines, and polyimines. As used herein, an aliphatic group optionally contains other substituent groups. A spacer to which an oligonucleotide is tethered is used to position the oligonucleotide and the synthesis process away from the substrate or carrier. This method allows for greater flexibility and more space for synthesis, facilitating cleavage when synthesis is completed.

As used herein, the term "carboxyl" (alone or as part of another group) refers to a group of the formula -COOH.

Linking groups or bifunctional linking moieties, such as those known in the art, may be used to link chemical functional groups, conjugate groups, reporter groups, and other groups to selective sites in a parent compound such as, for example, an oligonucleotide. Generally, a bifunctional linking moiety contains a hydrocarbyl moiety having two functional groups. One of the functional groups is selected to bind to the parent molecule or the compound of interest, and the other is selected to bind to any essentially selected group such as a chemical functional group or a conjugate group. In some embodiments, the linking group comprises a polymer of chain structure or repeating units such as ethylene glycol or amino acid units. Examples of functional groups commonly used for bifunctional linking moieties include, but are not limited to, electrophiles for reaction with nucleophilic groups and nucleophiles for reaction with electrophilic groups. In some embodiments, the bifunctional linking moiety comprises amino, hydroxyl, a carboxylic acid, a thiol, a degree of unsaturation (e.g., a double bond or a triple bond), and the like. Some non-limiting examples of bifunctional linking moieties include 8-amino-3,6-dioxaoctanoic acid (ADO), succinimidyl 4-(N-maleimidomethyl)cyclohexane-1-carboxylate (SMCC), and 6-aminohexanoic acid (AHEX or AHA). Other linking groups include, but are not limited to, substituted $C_1$-$C_{10}$ alkyl, substituted or unsubstituted $C_2$-$C_{10}$ alkenyl, or substituted or unsubstituted $C_2$-$C_{10}$ alkynyl, wherein a non-limiting list of preferred substituent groups includes hydroxyl, amino, alkoxy, carboxyl, benzyl, phenyl, nitro, thiol, thioalkoxy, halogen, alkyl, aryl, alkenyl, and alkynyl.

As used herein, the term "ether" refers to a product in which the hydrogen in the hydroxyl group of an alcohol or phenol is substituted with a hydrocarbyl group, and the product has the general formula R-OR', wherein R and R' may be identical or different. Ethers in which the two are identical are referred to as symmetrical ethers, also known as simple ethers or monoethers. Ethers in which the two are different are referred to as asymmetrical ethers, also known as mixed ethers.

As used herein, the term

“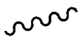” or “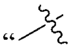”

generally refers to the linkage between adjacent groups or moieties. For example, in any structure of the formula A fragment herein,

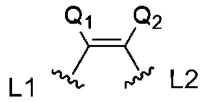,

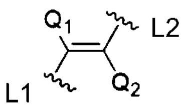, 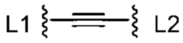, 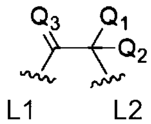, 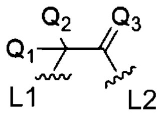, 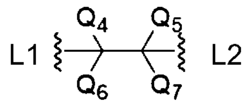,

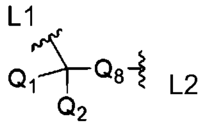, or 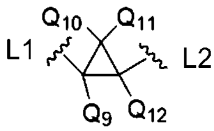, the “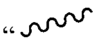”

at L1 represents the linkage to a phosphine moiety of $T_1$, and the one at L2 represents the linkage to a nucleoside sugar moiety or surrogate.

As used herein, the term "protecting group" refers to a labile chemical moiety known in the art that protects reactive groups (including but not limited to hydroxyl, amino, and thiol groups) from undesired reactions during synthetic procedures. Protecting groups are typically used selectively to protect sites during reactions at other reactive sites and can subsequently be removed to leave the unprotected group intact or available for further reactions.

Examples of protecting groups commonly used for hydroxyl or sulfhydryl include, but are not limited to, methyl, ethyl, benzyl (Bn), phenyl, isopropyl, tert-butyl, acetyl, chloroacetyl, trichloroacetyl, trifluoroacetyl, pivaloyl, tert-butoxymethyl, methoxymethyl, 1-ethoxyethyl, 1-(2-chloroethoxy)ethyl, 2-trimethylsilylethyl, allyl, cyclohexyl (cHex), 9-fluorenylmethoxycarbonyl, methanesulfonyl, p-toluenesulfonyl, trifluoromethanesulfonyl, benzoyl, benzoylformate, p-phenylbenzoyl, 4-methoxybenzyl, monomethoxytrityl, dimethoxytrityl, trimethoxytrityl, 4-chlorobenzyl, 4-nitrobenzyl, 2,4-dinitrophenyl, 4-acyloxybenzyl, 2-methylphenyl, 2,6-dimethylphenyl, 2-chlorophenyl, 2,6-dichlorobenzyl, diphenylmethyl, triphenylmethyl, 4-methylthio-1-butyl, 2-(S-acetylthio)ethyl (SATE), 2-cyanoethyl, 2-cyano-1,1-dimethylethyl (CDM), 4-cyano-2-butenyl, 2-(trimethylsilyl)ethyl (TSE), 2-(phenylthio)ethyl, 2-(triphenylsilyl)ethyl, 2-(benzylsulfonyl)ethyl, 2,2,2-trichloroethyl, 2,2,2-tribromoethyl, 2,3-dibromopropyl, 2,2,2-trifluoroethyl, phenylthio, 2-chloro-4-tritylphenyl, 2-bromophenyl, 2-[N-isopropyl-N-(4-methoxybenzoyl)amino]ethyl, 4-(N-trifluoroacetylamino)butyl, 4-oxopentyl, 4-tritylaminophenyl, 4-benzylaminophenyl, tetrahydropyranyl, morpholino, trimethylsilyl, triethylsilyl, tert-butyldimethylsilyl, tert-butyldiphenylsilyl, triphenylsilyl, triisopropylsilyl, pivaloyloxymethyl (POM), and 9-phenylxanthine-9-yl.

Examples commonly used for amino-protecting groups include, but are not limited to, 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), benzyl, formyl, acetyl, pivaloyl, trihaloacetyl, benzoyl, nitrophenyl, acetyl, 2-nitrobenzenesulfonyl, phthalimido (Pht), p-toluenesulfonyl (Tos), trityl (Trt), 2,4-dimethoxybenzyl (PMB), and dithiosuccinyl.

As used herein, the terms "phosphine moiety" and "phosphonate moiety" refer to the phosphorus-containing functional group or moiety in a phosphate (-O-P(=O)(OH)OH) molecule, and the phosphorus atom is directly bonded to a carbon atom. As used herein, the term "protected phosphine moiety" means that the hydroxyl, sulfhydryl, amino, and the like in a phosphonate moiety and a modified phosphonate moiety are protected by protecting groups.

As used herein, the term "phosphine moiety" refers to a monovalent $p^V$ phosphorus group (pentavalent phosphorus). In one embodiment, the phosphine moiety has the following formula:

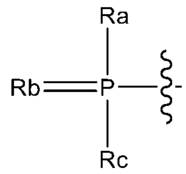

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

In certain embodiments, the sulfonyl is preferably methanesulfonyl.

In certain embodiments, each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

As used herein, the term "phosphine moiety" may be located at either end of an oligonucleotide, but is preferably located at the 5'-end nucleoside. In certain embodiments, the phosphine moiety has the following formulas:

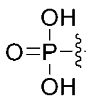, 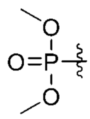, 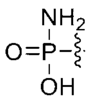, 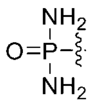, and 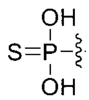.

In one embodiment, when one of Ra and Rc is a natural or modified nucleoside, the phosphine moiety has the following formula:

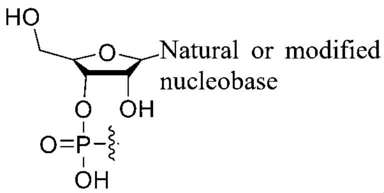,

wherein the natural base is selected from C, U, G, A, and T. Modified nucleobases may also be those as conventionally used herein or in the art, such as nucleosides formed by substituting the 2'-hydroxyl group of a ribosyl group with methoxy or fluorine. When one of Ra and Rc of the phosphine moiety is a natural or modified nucleoside at the 5'-end of a double-stranded oligonucleotide, e.g., the 5'-end of the antisense strand, an overhang is formed, and the base or modified base substituent group does not form a base pair with the sense strand; even if the sense strand comprises a 3'-terminal overhang, it does not form a base pair with the overhang. For its synthesis method, reference can be made to that described in the Chinese patent publication CN 115819484 A, which is also incorporated herein in its entirety.

As used herein, the term "active phosphorus" group can be used to form internucleoside linkages including, for example, phosphodiester and phosphorothioate internucleoside linkages. These active phosphorus groups are known in the art and comprise $P^{III}$ (trivalent phosphorus atom) or $p^V$ (pentavalent phosphorus atom). The active phosphorus groups include, but are not limited to, phosphoramidites, H-phosphonates, phosphotriesters, and phosphorus-containing chiral auxiliaries. In certain embodiments, reactive phosphorus groups that provide a 5'- or 3'-phosphonite internucleoside linkage upon reaction with a free hydroxyl group are included. These reactive phosphorus groups include, but are not limited to, - $(P{=}R_e)(OR_d)_2$, wherein each $R_d$ is independently a protecting group, substituted or unsubstituted $C_1$-$C_6$ alkyl, or substituted or unsubstituted aryl, and Re is O or S. In certain embodiments, the active phosphorus group is

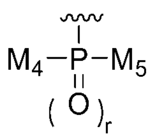,

wherein $M_4$ is H, optionally substituted $C_1$-$C_6$ alkyl, OH, $OJ_7$, SH, $SJ_7$, or $NJ_7J_8$, and $M_5$ is optionally substituted $C_1$-$C_6$ alkyl, OH, $OJ_7$, SH, $SJ_7$, or $NJ_7J_8$, wherein each $J_7$ or $J_8$ is independently and optionally substituted $C_1$-$C_6$ alkyl or sulfonyl; r is 0 or 1. Additional active phosphates and phosphites are disclosed in Beaucage and Iyer, Tetrahedron,1992, 48(12), 2223-2311.

As used herein, the term "phosphoramidite" refers to a nitrogen-containing trivalent phosphorus derivative, which is a conventional active phosphorus. Examples of suitable phosphoramidites are described herein.

As used herein, the term "internucleoside linkage" or "internucleoside linking group" is intended to include various types of internucleoside linking groups known in the art, and in such embodiments, any internucleoside linkage can be used to link nucleosides or analogs together. Two main classes of internucleoside linking groups are defined by the presence or absence of a phosphorus atom, including but not limited to: a phosphodiester linking group, a phosphotriester linking group, a phosphorothioate linking group, a phosphorodithioate linking group, an alkylphosphonate linking group, an aminophosphonate linking group, a phosphonate linking group, a phosphinate linking group, a phosphoramidothioate linking group, and a phosphoramidate linking group; as well as phosphorus-free internucleoside linking groups, such as thiodiester (-O-C(=O)-S-), thionocarbamate (-O-C(=O)(NH)-S-), siloxane (-O-Si(H)$_2$-O-), N,N'-dimethylhydrazine (-$CH_2$-N($CH_3$)-N($CH_3$)-), formyl, and methyleneimino (-$CH_2$-N($CH_3$)-O-$CH_2$-). Internucleoside linkages further include neutral nonionic internucleoside linkages. As used herein, the term "neutral internucleoside linkage" is intended to include nonionic internucleoside linkages. Neutral internucleoside linkages include, but are not limited to, phosphotriester, methyl phosphonate, MMI (3'-CH2-N($CH_3$)-O-5'), amido-3 (3'-$CH_2$-C(=O)-N(H)-5'), amido-4 (3'-$CH_2$-N(H)-C(=O)-5'), formacetal (3'-O-$CH_2$-O-5'), and thioformacetal (3'-S-$CH_2$-O-5'). Further neutral internucleoside linkages include nonionic linkages, including siloxanes (dialkylsiloxanes), carboxylates, carboxamides, sulfides, sulfonates, and amides (see, e.g., "Carbohydrate Modifications in Antisense Research"; Y. S. Sanghvi and P. D. Cook Eds. ACS Symposium Series 580; chapters 3 and 4, pages 40-65). Further neutral internucleoside linkages include nonionic linkages comprising mixed N, O, S, and $CH_2$ constituent parts, wherein the phosphorus atom is not always present. In some embodiments, the alkylphosphonate linking group is selected from a $C_1$-$C_6$ alkylphosphonate linking group.

In certain embodiments, in the provided oligonucleotide, the phosphorus-containing internucleoside linking group has a structural fragment as shown below:

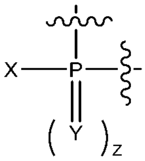
,

wherein X represents H, optionally substituted $C_1$-$C_6$ alkyl, $OR^{13}$, $SR^{13}$, OH, SH, or $NR^{13}R^{14}$; Y represents O or S; z may be 0 or 1; each $R^{13}$ or $R^{14}$ is independently hydrogen, optionally substituted $C_1$-$C_6$ alkyl, or sulfonyl; the "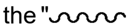" symbols independently represent a moiety linked to the 5'-terminal nucleoside herein and a moiety linked to an adjacent nucleotide, respectively.

Modified linkages can be used to alter (generally enhance) the nuclease resistance of oligonucleotides as compared to natural phosphodiester linkages. In certain embodiments, the modified linkages can be used as racemic mixtures or as separate enantiomers to prepare internucleoside linkages having a chiral atom. Representative chiral linkages include, but are not limited to, alkylphosphonates and phosphorothioates. Methods for preparing phosphorus-containing and phosphorus-free internucleoside linkages are well known to those skilled in the art.

As used herein, "nucleoside" refers to a natural or modified nucleoside compound comprising a heterocyclic base moiety and a sugar moiety. Nucleosides include, but are not limited to, naturally occurring nucleosides (e.g., present in DNA and RNA), abasic nucleosides, modified nucleosides, and nucleosides having mimetic bases and/or glycosyl groups. Nucleosides may be modified by any of a variety of substituents.

As used herein, "nucleotide" refers to a nucleoside further comprising a phosphate linking group. As used herein, "linked nucleoside" may or may not be linked by a phosphate linkage and thus includes "linked nucleotides".

As used herein, the term "nucleotide position" refers to the position of a nucleotide in an oligonucleotide or an oligonucleotide, counted starting from the nucleotide at the 5'-end. For example, nucleotide position 1 refers to the 5'-terminal nucleotide of the oligonucleotide.

As used herein, "sugar moiety" refers to a natural or modified sugar ring or sugar surrogate. "Sugar surrogate moiety" or "sugar surrogate" refers to a structure capable of taking the place of a 5-membered furanose ring of a naturally occurring nucleoside. In general, such nucleosides with sugar surrogate groups still possess a heterocyclic base moiety, retaining the hybridization capability. In certain embodiments, the sugar surrogate is a non-furanose (or 4'-substituted furanose) ring or ring system or open system. Without limitation, such a structure comprises simple changes relative to a natural furanose ring, such as a six-membered ring (e.g., morpholinyl, tetrahydropyran, and cyclohexane-1,2,3,4,5,6-hexol). The six-membered substitution is further modified (e.g., tetrahydropyran in place of the furanose of a naturally occurring nucleoside). Modified tetrahydropyran includes, but is not limited to, those structures known in the art as hexitol nucleic acids (HNAs), anitol nucleic acids (ANAs), mannitol nucleic acids (MNAs) (see Leumann, CJ., Bioorg. & Med. Chem. (2002) 10:841-854), and fluoro-HNA (F-HNA), as well as some nucleosides replaced by morpholinyl (see international application publications WO200836127 and WO2011150408). A carbocyclic nucleoside is a nucleoside analog in which the oxygen atom of the furanose ring of the sugar moiety is substituted with a carbon atom; thus, carbocycles are also common sugar surrogates. See, e.g., U.S. Patent No. 6,001,840. It has a cyclopentane ring in place of the tetrahydrofuran ring of the nucleoside. Examples may also be cyclohexyl, cyclopentyl, and cyclohexenyl (see PCT application WO 2010/036696; Robeyns et al., J. Am. Chem. Soc., 2008, 130(6), 1979-1984; Gu et al., Nucleosides, Nucleotides & Nucleic Acids, 2005, 24(5-7), 993-998; Nauwelaerts et al., Nucleic Acids Research, 2005, 33(8), 2452-2463; Gu et al., Oligonucleotides, 2003, 13(6), 479-489). "Sugar surrogate moiety" may be a bicyclic or tricyclic structure (see, e.g., those described in Leumann, J.c, Bioorganic & Medicinal Chemistry, 2002, 10, 841-854, US7399845, and international patent publications WO2009006478, WO2008150150729, and WO2011/139702), such as locked nucleic acids ("LNAs") (see, e.g., Koshkin et al. (1998), Tetrahedron, 54, 3607-3630) and bridged nucleic acids ("BNAs") (see, e.g., U.S. Patent No. 7,427,672 and Mitsuoka et al. (2009), Nucleic Acids Res., 37(4):1225-38). "Sugar surrogate moiety" may also be acyclic, such as unlocked nucleic acids ("UNAs") (see, e.g., U.S. Patent No. 8,314,227; Meghan A. et al., "Locked vs. unlocked nucleic acids (LNA vs. UNA): contrasting structures work towards common therapeutic goals". Chem. Soc. Rev., 2011, 40, 5680-5689), and glycerol nucleic acid ("GNA") structures as sugar surrogate substitution moieties (see, e.g., WO 2016/028649), or may be more complex, like acyclic systems used for peptide nucleic acids. More new "sugar surrogate moiety" nucleosides may be as described in international patent publication WO2011/139702. Each of them is incorporated herein by reference in its entirety.

Furanose: As used herein, the term "furanose" refers to a carbohydrate having a five-membered ring structure, wherein the ring structure has 4 carbon atoms and 1 oxygen atom, as shown in the following structure:

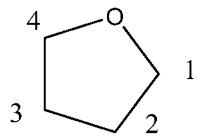

.

In the structure, the numbers indicate the positions of the 4 carbon atoms in the five-membered ring structure. Certain nucleosides in which the sugar moiety is replaced by tetrahydropyranyl have the following structural schematic:

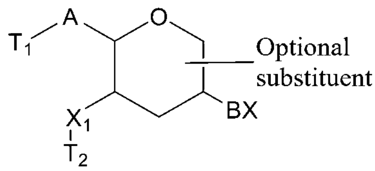

,

wherein the variables can be consistent with those in formula (I) herein, and it can be understood that the ring can be further substituted with any suitable group; some nucleosides in which the sugar moiety is replaced by morpholinyl have the following structural schematic:

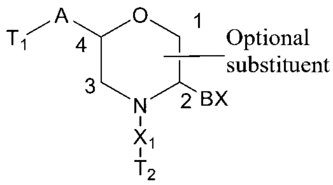

,

wherein the numbers indicate the positions of the 4 carbon atoms in the five-membered ring structure, the variables can be consistent with those in formula (I) herein, and it can be understood that the ring can be further substituted with any suitable group; some nucleosides in which the sugar moiety is replaced by cyclohexenyl have the following structural schematic:

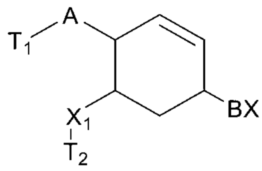

,

wherein the variables can be consistent with those in formula (I) herein, and it can be understood that the ring can be further substituted with any suitable group.

Certain nucleosides in which the sugar moiety is replaced by a bicyclic ring, including but not limited to those formed by bridging the 4' and 2' sugar ring atoms, have the following structural schematic:

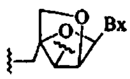
(A)
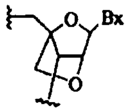
(B)
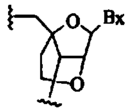
(C)

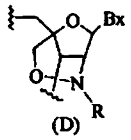
(D)
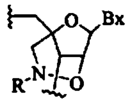
(E)
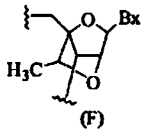
(F)

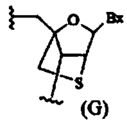
(G)
(H)
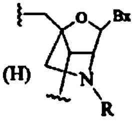
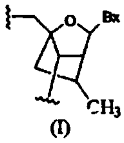
(I)

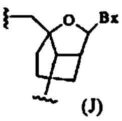
(J)

,

wherein Bx is a base moiety, and R is independently H, a protecting group, or $C_1$-$C_{12}$ alkyl.

Modified sugars (also referred to herein as "sugar surrogate moieties") include modified deoxyribose or ribose moieties, such as those with a modification at the 2'-, 3'-, 4'-, or 5'-carbon position of the sugar.

As used herein, "nucleobase" or "heterocyclic base moiety" refers to a heterocyclic base moiety of a nucleoside, a heterocyclic moiety located at the 1' position of the nucleotide sugar moiety in a modified nucleotide that can be incorporated into a nucleic acid double helix (or at the equivalent position in a nucleotide sugar moiety substitution that can be incorporated into a nucleic acid double helix). "Nucleobase" or "heterocyclic base moiety" may be a naturally occurring nucleobase, a nucleobase that can be modified, or a universal nucleobase. In certain embodiments, "nucleobase" or "heterocyclic base moiety" may comprise any atom or group of atoms capable of forming hydrogen bonds with abase of another nucleic acid, and the heterocyclic base moiety of each nucleoside may be modified by one or more substituent groups to enhance one or more properties, such as the affinity for a target strand, or to favorably influence some other properties. Modified nucleobases include, but are not limited to, universal bases, hydrophobic bases, promiscuous bases, size-expanded bases, and fluorinated bases as defined herein. These nucleobases can be used to enhance the binding affinity of the oligonucleotide provided herein. The two most common classes are purines and pyrimidines. For example, suitable natural nucleobases include purine bases and pyrimidine bases, such as adenine (A), thymine (T), cytosine (C), guanine (G), or uracil (U). Suitable modified nucleobases include diaminopurine and derivatives thereof, alkylated purines or pyrimidines, acylated purines or pyrimidines, thiolated purines or pyrimidines, and the like, such as 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-sulfhydrylcytosine, 5-halouracil and 5-halocytosine, 5-propynyl (-C≡C-CH3) uracil and 5-propynylcytosine and other alkynyl derivatives of pyrimidine bases, 6-azauracil, 6-azacytosine and 6-azathymine, 5-uracil (pseudouracil), N1-methyl-pseudouracil, 4-thiouracil, 8-halo, 8-amino, 8-thiol, 8-thioalkyl, 8-hydroxyl and other 8-substituted adenines and guanines, 5-halo (particularly 5-bromo), 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, and 3-deazaguanine and 3-deazaadenine. Further modified nucleobases include tricyclic pyrimidines such as phenoxazine cytidine ([5,4-b][1,4]benzoxazin-2(3H)-one), phenothiazine cytidine (1H-pyrimido[5,4-b][1,4]benzothiazin-2(3H)-one), G-clamps such as substituted phenoxazine cytidine (e.g., 9-(2-aminoethoxy)-H-pyrimido[5,4-b][1,4]benzoxazin-2(3H)-one), carbazole cytidine (2H-pyrimido[4,5-b]indol-2-one), and pyridoindole cytidine (H-pyrido[3',2':4,5]pyrrolo[2,3-d]pyrimidin-2-one). Modified nucleobases may also include those in which a purine or pyrimidine base is replaced by another heterocycle, such as 7-deazaadenine, 7-deazaguanosine, 2-aminopyridine, and 2-pyridone. Representative teachings on the preparation of the modified nucleobases and other modified nucleobases include, but are not limited to, those disclosed in international application publication WO2022007986 and U.S. Patent Nos. 3,687,808, 4,845,205, 5,130,302, 5,134,066, 5,175,273, 5,367,066, 5,432,272, 5,457,187, 5,459,255, 5,484,908, 5,502,177, 5,525,711, 5,552,540, 5,587,469, 5,594,121, 5,596,091, 5,614,617, 5,645,985, 5,681,941, 5,750,692, 5,763,588, 5,830,653, and 6,005,096. As used herein, "universal base" refers to a heterocyclic moiety that is located at the 1' position of the nucleotide sugar moiety in a modified nucleotide or at the equivalent position in a nucleotide sugar moiety substitution and has the same properties as natural bases; that is, when present in a nucleic acid double helix, it can be placed opposite to more than one type of base without altering the double helix structure (e.g., the phosphate backbone structure). Some universal bases are capable of base pairing by forming hydrogen bonds with all bases including guanine (G), cytosine (C), adenine (A), thymine (T), and uracil (U) under conditions for base pair formation. In the double helix, a universal base may form one hydrogen bond or more than one hydrogen bond with each of G, C, A, T, and U opposite thereto on the opposing strand of the double helix. In the double helix, the base pairing between universal bases occurs but does not alter the double helix structure of the phosphate backbone. A universal base may also interact with bases in adjacent nucleotides on the same nucleic acid strand through stacking interactions. Such stacking interactions stabilize the double helix, particularly when the universal base does not form any hydrogen bonds with bases placed opposite thereto on the opposing strand of the double helix. In some cases, 2,4-difluorophenyl is also a common substitute for heterocyclic bases. Limiting examples of universal binding nucleotides include inosine, 1-O-D-ribofuranosyl-5-nitroindole, and/or 1-β-D-ribofuranosyl-3-nitropyrrole (U.S. Patent Application Publication No. 20070254362 by Quay et al.; Van Aerschot et al., An acyclic 5-nitroindazole nucleoside analogue as ambiguous nucleoside, NUCLEIC ACIDS RES. Nov. 11, 1995; 23(21):4363-70; Loakes et al., 3-Nitropyrrole and 5-nitroindole as universal bases in primers for DNA sequencing and PCR, NUCLEIC ACIDS RES. Jul. 11, 1995; 23(13):2361-2366).

Some of them are co-owned with the present application, and each of them is incorporated herein by reference in its entirety. Some non-limiting structures are shown below:

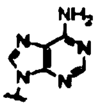 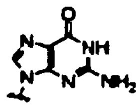 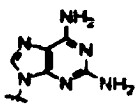 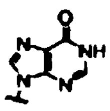 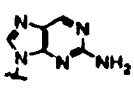 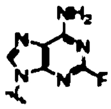 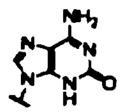

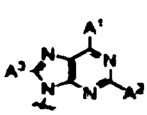 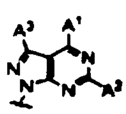 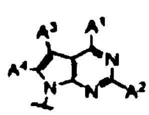 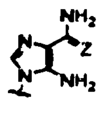 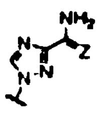 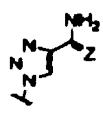 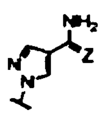

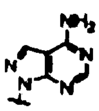 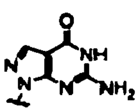 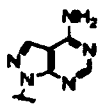 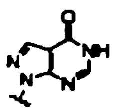 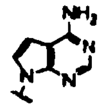 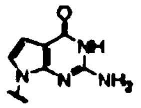 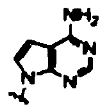 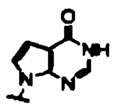

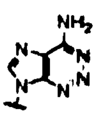 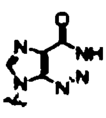 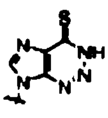 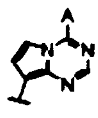 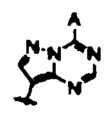 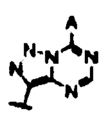 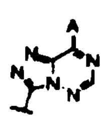 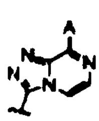

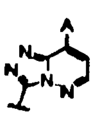 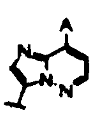 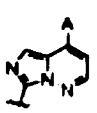 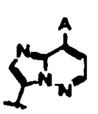 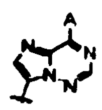 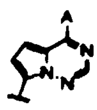 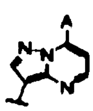 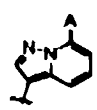

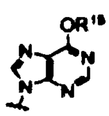 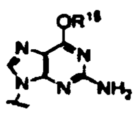 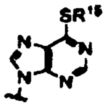 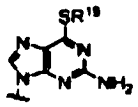 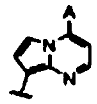 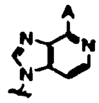 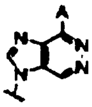 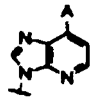

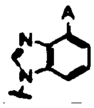 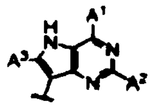 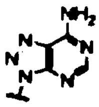 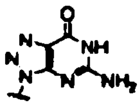 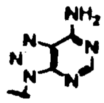 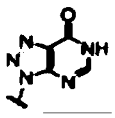

As used herein, "modified nucleoside" refers to a nucleoside comprising at least one modification as compared to a naturally occurring RNA or DNA nucleoside. Such modifications may be located at the sugar moiety and/or at the nucleobase. Modified nucleotides include those lacking a nucleobase (abasic).

As used herein, "2'-F" refers to a nucleoside comprising a sugar that comprises a fluoro group at the 2' position. As used herein, "2'-OMe" refers to a nucleoside comprising a sugar that comprises an $-OCH_3$ group at the 2' position of the sugar ring. As used herein, "2'-MOE", "2'-$OCH_2CH_2OCH_3$", or "2'-O-methoxyethyl" refers to a nucleoside comprising a sugar that comprises an $-OCH_2CH_2OCH_3$ group at the 2' position of the sugar ring.

As used herein, the term "5'-terminal nucleotide or 3'-terminal nucleotide" refers to a nucleotide or nucleotide derivative located at the 5'-end or the 3'-end of an oligonucleotide or an oligonucleotide.

As used herein, "oligonucleotide" refers to polymeric forms of nucleotides ranging in length from 2 to 2500 nucleotides. Oligonucleotides may be single-stranded or double-stranded. Typically, for example, the oligonucleotides are used in gene therapy; for example, the oligonucleotides are nucleic acid inhibitor molecules. In certain embodiments, one or more of these multiple nucleotides are modified. The term "oligonucleotide" in the context of the present disclosure refers to a polymer having at least one region capable of hybridizing with a nucleic acid molecule. Typically, an oligonucleotide comprises a backbone of linked monomer subunits, and the linkages linking monomer subunits, sugar moieties or surrogates, and heterocyclic base moieties can be independently modified. The linked sugar units, which may or may not comprise heterocyclic bases, may be replaced by mimetics such as peptide nucleic acid monomers. The ability to modify each monomer or substitute some or all of the monomers in an oligonucleotide can generate a large number of possible motifs. The term "oligonucleotide" includes oligonucleotide analogs and oligonucleotides, as well as nucleotide mimetics, and/or mixed polymers comprising nucleic acid and non-nucleic acid components. The term "oligonucleotide" further includes polymers comprising linked monomer subunits, wherein the monomer subunits include nucleosides, modified nucleosides, nucleoside analogs, nucleoside mimetics, and non-nucleic acid components such as coupling groups. In certain embodiments, mixtures of monomer subunits (such as but not limited to those listed) provide oligonucleotides having enhanced properties for uses such as therapy and diagnosis.

The oligonucleotides are conventionally prepared linearly, are also linked or otherwise prepared to be circular, and may also contain branches. The oligonucleotides may form double-stranded constructs, e.g., two strands hybridized to form double-stranded compositions. The double-stranded compositions may be linked or separated and may comprise overhangs at their ends.

As used herein, "expression" refers to the process by which a gene ultimately produces a protein. Expression includes, but is not limited to, transcription, splicing, post-transcriptional modification, and translation.

As used herein, "antisense oligonucleotide" refers to an antisense compound that is an oligonucleotide.

As used herein, "target nucleic acid" refers to any nucleic acid molecule whose expression level or activity can be modulated by an antisense compound. In certain embodiments, the target nucleic acid is DNA or RNA. In certain embodiments, the target RNA is mRNA, pre-mRNA, non-coding RNA, pri-microRNA, pre-microRNA, mature microRNA, promoter-regulated RNA, or a natural antisense transcript.

As used herein, "target mRNA" refers to a preselected protein-encoding RNA molecule.

As used herein, "hybridization" refers to the pairing of complementary oligonucleotides (e.g., an antisense compound and a target nucleic acid thereof). While not limited to a specific mechanism, the most common mechanism of pairing involves the formation of hydrogen bonds, which may be Watson-Crick, Hoogsteen, or reversed Hoogsteen hydrogen bonds, between complementary nucleoside or nucleotide bases (nucleobases). For example, the natural nucleobase adenine is a nucleobase complementary to the natural nucleobases thymidine and uracil, and they pair by forming hydrogen bonds. The natural base guanine is a nucleobase complementary to the natural bases cytosine and 5-methylcytosine.

In certain embodiments, the provided oligonucleotide is a single-stranded oligonucleotide. In certain embodiments, the single-stranded oligonucleotide is a conventional antisense oligonucleotide (also known as ASO), a ribozyme, or an aptamer. In certain embodiments, the provided oligonucleotide is a double-stranded ribonucleic acid (dsRNA) agent, and such a compound is a double-stranded ribonucleic acid well known in the art, wherein one or both strands are oligonucleotides as disclosed herein.

As used herein, "antisense compound" and "antisense strand" refer to an oligonucleotide of which at least a portion is at least partially complementary to a target nucleic acid that hybridizes with it. In certain embodiments, the antisense compound modulates (increases or decreases) the expression or amount of a target nucleic acid. In certain embodiments, the antisense compound alters the splicing of the target pre-mRNA, thereby resulting in different splice variants. In certain embodiments, the antisense compound modulates the expression of one or more different target proteins. The antisense mechanisms contemplated herein include, but are not limited to, RNase H mechanism, RNA mechanism, splicing modulation, translational arrest, altering RNA processing, inhibiting microRNA function, or mimicking microRNA function.

As used herein, the term "siRNA" refers to a short interfering RNA or silencing RNA. siRNAs are a class of double-stranded siRNA RNA molecules that may be 20 to 25 (or fewer) base pairs in length, and they are similar to microRNA (miRNA), which plays a role in the RNA interference (RNAi) pathway. siRNA interferes with the expression of specific genes with complementary nucleotide sequences to the siRNA by degrading mRNA after transcription, thereby preventing translation. siRNA acts in cells to silence gene expression by inducing the RNA-induced silencing complex (RISC) to cleave messenger RNA (mRNA). As used herein, the term "single-stranded oligonucleotide" refers to a single-stranded oligomeric compound having a sequence that is at least partially complementary to a target mRNA and capable of hybridizing with the target mRNA through hydrogen bonding under mammalian physiological conditions (or similar conditions *in vitro*). In some embodiments, the single-stranded oligonucleotide is a single-stranded antisense oligonucleotide.

As used herein, unless otherwise indicated or modified, the term "double-stranded" refers to two separate oligonucleotides that hybridize with each other. Such double-stranded compounds may have one or more non-hybridizing nucleosides (overhangs) at one or both ends of one or both strands and/or one or more internal non-hybridizing nucleosides (mismatches), provided there is sufficient complementarity to maintain hybridization under physiologically relevant conditions.

As used herein, the term "silence", "reduce", "inhibit", "down-regulate", or "knock down", when referring to the expression of a given gene, means that the expression of the gene, as measured by the level of the RNA transcribed from the gene or the level of the polypeptide, protein, or protein subunit translated from the mRNA in a cell, cell mass, tissue, organ, or subject in which the gene is transcribed, is reduced when the cell, cell mass, tissue, organ, or subject is treated with an oligomeric compound such as the RNAi agent described herein, as compared to a second cell, cell mass, tissue, organ, or subject that has not been so treated.

As used herein, the term "self-complementary" or "hairpin" refers to a single oligonucleotide comprising a duplex region formed by self-hybridization of the oligonucleotide.

As used herein, the term "single-stranded" refers to an oligonucleotide that is not hybridized with its complementary sequence and does not have sufficient self-complementarity to form a hairpin structure under physiologically relevant conditions. A single-stranded compound is capable of binding to its complementary sequence to become a double-stranded or partially double-stranded compound.

As used herein, "target mRNA" or "target nucleic acid" refers to any nucleic acid molecule whose expression, amount, or activity can be modulated by an antisense compound. In certain embodiments, the target nucleic acid is DNA or RNA. In certain embodiments, the target RNA is mRNA, pre-mRNA, non-coding RNA, pri-microRNA, pre-microRNA, mature microRNA, promoter-regulated RNA, or a natural antisense transcript. For example, the target nucleic acid may be a cellular gene whose expression is associated with a particular disease or disease state (or the mRNA transcribed from the gene), or a nucleic acid molecule from an infectious substance. In certain embodiments, the target nucleic acid is a viral or bacterial nucleic acid.

As used herein, "nucleobase complementarity", "complementary", or "complementarity", with respect to nucleobases, refers to a nucleobase capable of base pairing with another nucleobase. For example, in DNA, adenine (A) is complementary to thymine (T). For example, in RNA, adenine (A) is complementary to uracil (U). In certain embodiments, the complementary nucleobase refers to a nucleobase of an antisense compound that is capable of base pairing with a nucleobase of its target nucleic acid. As used herein, "non-complementary", with respect to nucleobases, refers to a pair of nucleobases that do not form a hydrogen bond with each other, or in other words, do not support hybridization. With respect to linked nucleosides, oligonucleotides, or nucleic acids, it refers to the ability of an oligonucleotide to hybridize with another oligonucleotide or nucleic acid through nucleobase complementarity. For example, when used to describe the correlation between the first nucleotide sequence (e.g., a sense strand or a target gene mRNA) and the second nucleotide sequence (e.g., a dsRNA agent antisense strand or a single-stranded antisense polynucleotide) of a dsRNA agent, it refers to the ability of an oligonucleotide or polynucleotide comprising the first nucleotide sequence to hybridize with an oligonucleotide or polynucleotide comprising the second nucleotide sequence [to form hydrogen bonds between base pairs under mammalian physiological conditions (or similar conditions *in vitro*)], and to form a double helix or double helix structure under certain conditions. Other conditions, such as physiologically relevant conditions that may be encountered in an organism, may also be applied. A skilled person will be able to determine the most appropriate set of conditions for testing the complementarity of two sequences according to the final application of the hybridized nucleotides. The

complementary sequences include Watson-Crick base pairs or non-Watson-Crick base pairs, and include natural or modified nucleotides or nucleotide mimetics, so long as at least to the extent that the hybridization requirements described above are achieved. Sequence identity or complementarity is not relevant to the modification.

In certain embodiments, at least a portion of the nucleobase sequence of the oligonucleotide is partially or fully complementary to the target nucleic acid. In certain embodiments, at least a portion of the nucleobase sequence of the oligonucleotide is 100% complementary to the target nucleic acid. In certain embodiments, at least a portion of the nucleobase sequence of the oligonucleotide is 90% complementary to the target nucleic acid. In certain embodiments, at least a portion of the nucleobase sequence of the oligonucleotide is 80% complementary to the target nucleic acid. In certain embodiments, at least a portion of the nucleobase sequence of the oligonucleotide is 90% complementary to the target nucleic acid. In certain embodiments, at least a portion of the nucleobase sequence of the oligonucleotide is 70% complementary to the target nucleic acid. In certain embodiments, at least a portion of the nucleobase sequence of the oligonucleotide is 90% complementary to the target nucleic acid. In certain embodiments, at least a portion of the nucleobase sequence of the oligonucleotide is 60% complementary to the target nucleic acid. For example, a complementary sequence within the target gene dsRNA as described herein comprises base pairing of an oligonucleotide or polynucleotide comprising a first nucleotide sequence with an oligonucleotide or polynucleotide comprising a second nucleotide sequence over the full length of one or two nucleotide sequences. Such sequences may be referred to herein as "fully complementary" to each other. It should be understood that in embodiments where two oligonucleotides are designed to form one or more single-stranded overhangs upon hybridization, such overhangs are not considered herein as mismatches determined on the basis of complementarity. For example, the target gene dsRNA agent comprises an oligonucleotide of 19 nucleotides in length and another oligonucleotide of 20 nucleotides in length, wherein the longer oligonucleotide comprises a sequence of 19 nucleotides that is fully complementary to the shorter oligonucleotide, which may be referred to as "fully complementary" for the purposes described herein. Thus, as used herein, "fully complementary" means that all (100%) bases in the contiguous sequence of the first polynucleotide will hybridize with the same number of bases in the contiguous sequence of the second polynucleotide. The contiguous sequence may comprise all or part of the first or second nucleotide sequence. As used herein, the term "substantially complementary" means that in a hybrid pair of nucleobase sequences, at least about 85% (but not all) of the bases in the contiguous sequence of the first polynucleotide will hybridize with the same number of bases in the contiguous sequence of the second polynucleotide. If two sequences comprise one or more mismatched base pairs, such as at least 1, 2, 3, 4, or 5 mismatched base pairs, during hybridization, the term "substantially complementary" may be used to refer to the first sequence forming a duplex of up to 15, 16, 17, 18, 19, 20, 21, 22, 23, 24, 25, 26, 27, 28, 29, or 30 base pairs (bp) in comparison with the second sequence, while retaining the ability to hybridize under conditions most relevant to its final application, e.g., inhibition of target gene expression by the RISC pathway. The term "partially complementary" may be used herein to mean that in a hybrid pair of nucleobase sequences, at least 75% (but not all) of the bases in the contiguous sequence of the first polynucleotide will hybridize with the same number of bases in the contiguous sequence of the second polynucleotide. In some embodiments, "partially complementary" means that at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the bases in the contiguous sequence of the first polynucleotide will hybridize with the same number of bases in the contiguous sequence of the second polynucleotide. The terms "complementary", "fully complementary", "substantially complementary", and "partially complementary" as used herein may be used to refer to a base match between a sense strand and an antisense strand of a dsRNA agent, a base match between an antisense strand of a dsRNA agent and a sequence of a target mRNA, or a base match between a single-stranded antisense oligonucleotide and a sequence of a target mRNA. It should be understood that the term "antisense strand of a dsRNA agent" may refer to the same sequence as "antisense polynucleotide agent".

As used herein, "mismatch" is known to those skilled in the art, and mismatches are tolerable for the efficacy of dsRNA, especially in the case where mismatches are within the terminal region of the dsRNA. Certain mismatches are better tolerated, such as mismatches with wobble base pairs G:U and A:C are better tolerated for efficacy (Du et el., A systematic analysis of the silencing effects of an active siRNA at all single-nucleotide mismatched target sites. Nucleic Acids Res. 2005 Mar 21; 33(5):1671-7. Doi: 10.1093/nar/gki312. Nucleic Acids Res. 2005; 33(11):3698).

As used herein, "motif" or "sequence" refers to a succession or order of nucleobases or nucleotides represented by a succession of letters using the standard nucleotide nomenclature.

In certain embodiments, the present disclosure provides an oligonucleotide comprising any of a variety of length ranges. In certain embodiments, in the oligonucleotide or double-stranded ribonucleic acid (dsRNA), any of the single strand, the sense strand, and/or the antisense strand has a length of 8-40 nucleotides. In certain embodiments, in the oligonucleotide or double-stranded ribonucleic acid (dsRNA), any of the single strand, the sense strand, and/or the antisense strand has a length of 15-30 nucleotides. For example, in certain embodiments, the nucleotide length of the oligonucleotide is selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40. For example, in certain embodiments, the present disclosure provides an oligonucleotide nucleotide length range comprising an oligonucleotide consisting of 8 to 9, 8 to 10, 8 to 11, 8 to 12, 8 to 13, 8 to 14, 8 to 15, 8 to 16, 8 to 17, 8 to 18, 8 to 9, 8 to 20, 8 to 21, 8 to 22, 8 to 23, 8 to 24, 8 to 25, 8 to 26, 8 to 27, 8 to 28, 8 to 29, 8 to 30, 9 to 10, 9 to

11, 9 to 12, 9 to 13, 9 to 14, 9 to 15, 9 to 16, 9 to 17, 9 to 18, 9 to 19, 9 to 20, 9 to 21, 9 to 22, 9 to 23, 9 to 24, 9 to 25, 9 to 26, 9 to 27, 9 to 28, 9 to 29, 9 to 30, 10 to 11, 10 to 12, 10 to 13, 10 to 14, 10 to 15, 10 to 16, 10 to 17, 10 to 18, 10 to 19, 10 to 20, 10 to 21, 10 to 22, 10 to 23, 10 to 24, 10 to 25, 10 to 26, 10 to 27, 10 to 28, 10 to 29, 10 to 30, 11 to 12, 11 to 13, 11 to 14, 11 to 15, 11 to 16, 11 to 17, 11 to 18, 11 to 19, 11 to 20, 11 to 21, 11 to 22, 11 to 23, 11 to 24, 11 to 25, 11 to 26, 11 to 27, 11 to 28, 11 to 29, 11 to 30, 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 29, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 9 to 30 linked nucleosides. In embodiments in which the number of nucleosides of an oligonucleotide or an oligonucleotide is limited, whether for a certain range or a specific numerical value, the oligonucleotide or the oligonucleotide may further comprise additional other substituents. For example, an oligonucleotide comprising 8-30 nucleosides excludes an oligonucleotide having 31 nucleosides, but, unless otherwise indicated, such an oligonucleotide may further comprise, for example, one or more conjugates, terminal groups, or other substituents; the monomers herein are generally considered to be terminal groups that the oligonucleotide further comprises. In certain embodiments, the terminal groups include, but are not limited to, terminal group nucleosides. In such embodiments, the terminal groups are modified differently from the terminal nucleosides of the oligonucleotide, thereby distinguishing such terminal groups from the nucleosides of the oligonucleotide.

In some embodiments, at least one or each of the targeting groups is selected from a ligand capable of binding to a receptor on the surface of a mammalian hepatocyte. In some embodiments, each of the targeting groups is independently a ligand having an affinity for an asialoglycoprotein receptor (ASGPR) on the surface of a mammalian hepatocyte. In some embodiments, each of the targeting groups is independently a ligand comprising an asialoglycoprotein or a sugar. In some embodiments, each of the targeting groups is independently a ligand comprising an asialoglycoprotein, such as asialoorosomucoid (ASOR) or asialofetuin (ASF). In some embodiments, each of the targeting groups independently comprises a ligand selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyl-36 acylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, and L-4-thioribose.

In some embodiments, at least one or each of the targeting groups is a ligand comprising galactose or N-acetylgalactosamine.

In some embodiments, the targeting group comprises one or more "GalNAc" (N-acetylgalactosamine) derivatives linked via a suitable tether by a divalent or trivalent branched linker:

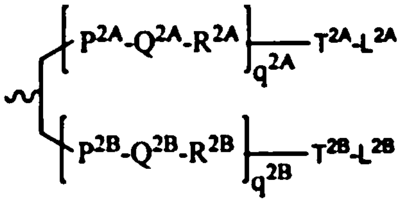

,

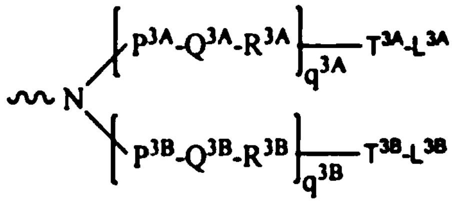

,

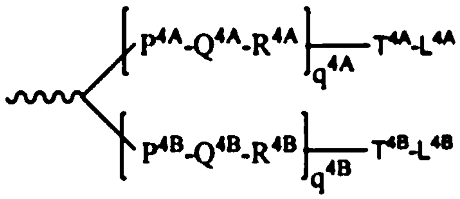 , 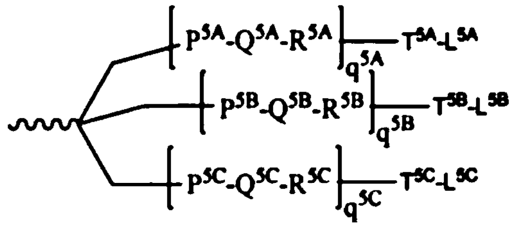 ,

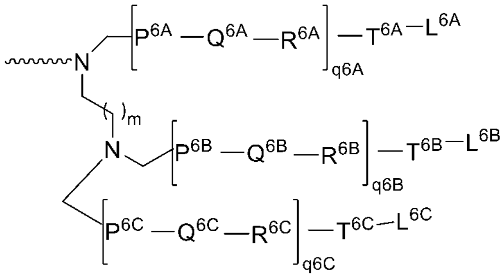

wherein:

$q^{2A}$, $q^{2B}$, $q^{3A}$, $q^{3B}$, $q^{4A}$, $q^{4B}$, $q^{5A}$, $q^{5B}$, $q^{5C}$, $q^{6A}$, $q^{6B}$, and $q^{6C}$ independently represent 0-20 at each occurrence, and the repeating units therein may be identical or different;

$P^{2A}$, $P^{2B}$, $P^{3A}$, $P^{3B}$, $P^{4A}$, $P^{4B}$, $P^{5A}$, $P^{5B}$, $P^{5C}$, $P^{6A}$, $P^{6B}$, $P^{6C}$, $T^{2A}$, $T^{2B}$, $T^{3A}$, $T^{3B}$, $T^{4A}$, $T^{4B}$, $T^{4A}$, $T^{5B}$, $T^{5C}$, $T^{6A}$, $T^{6B}$, and $T^{6C}$ each independently represent absent, CO, NH, O, S, OC(=O), NHC(=O), $CH_2$, $CH_2NH$, or $CH_2O$ at each occurrence;

$Q^{2A}$, $Q^{2B}$, $Q^{3A}$, $Q^{3B}$, $Q^{4A}$, $Q^{4B}$, $Q^{5A}$, $Q^{5B}$, $Q^{5C}$, $Q^{6A}$, $Q^{6B}$, and $Q^{6C}$ independently represent absent, alkylene, and substituted alkylene at each occurrence, wherein one or more methylene groups may be interrupted or terminated by one or more of the following groups: O, S, S(=O), $SO_2$, NH, C(R')=C(R"), C≡C, or C(=O);

$R^{2A}$, $R^{2B}$, $R^{3A}$, $R^{3B}$, $R^{4A}$, $R^{4B}$, $R^{5A}$, $R^{5B}$, $R^{5C}$, $R^{6A}$, $R^{6B}$, and $R^{6C}$ each independently represent absent, NH, O, S, $CH_2$, C(O)O, C(O)NH, NHCH($R^a$)C(O), -C(O)-CH($R^a$)-NH-, CO, CH=N-O, or heterocyclyl at each occurrence;

$L^{2A}$, $L^{2B}$, $L^{3A}$, $L^{3B}$, $L^{4A}$, $L^{4B}$, $L^{5A}$, $L^{5B}$, $L^{5C}$, $L^{6A}$, $L^{6B}$, and $L^{6C}$ each independently represent monosaccharides (such as GalNAc), disaccharides, trisaccharides, tetrasaccharides, oligosaccharides, or polysaccharides at each occurrence.

In some non-limiting examples, provided is the oligonucleotide described herein, wherein the targeting group is selected from one of the following compound fragments:

| | |
|---|---|
| 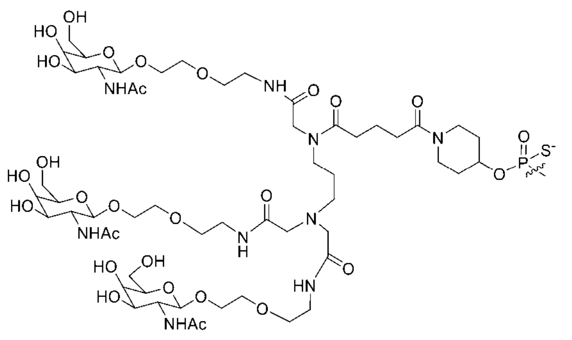 GLS-15 | 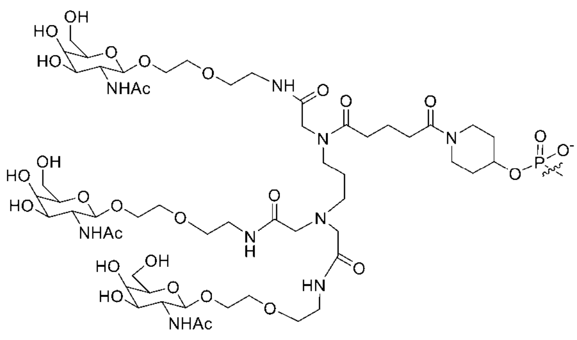 GLO-15 |

| | |
|---|---|
| 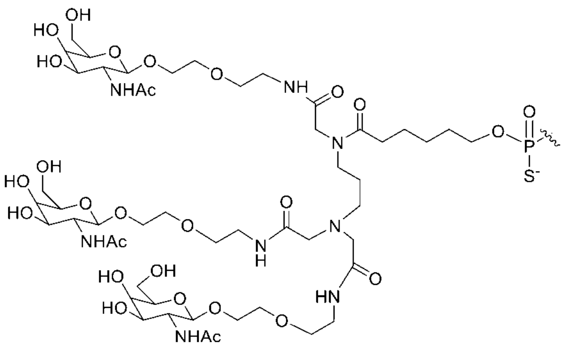 GLS-5. | |

In some non-limiting examples, provided is the oligonucleotide described herein, wherein the antisense strand in a double-stranded RNA (dsRNA) comprises a 5'-terminal compound (or a terminal nucleotide) represented by formula (IX-1) or (IX-2):

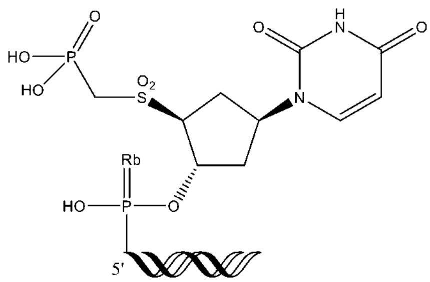

(IX-1),

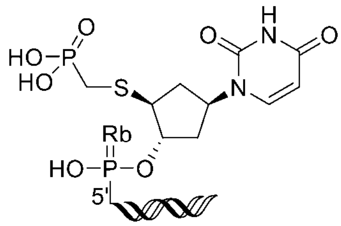

(IX-2),

and

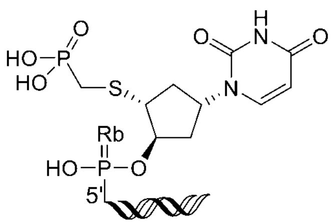

(IX-3);

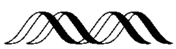

represents the rest of the double-stranded RNA (dsRNA), and Rb is as described in the foregoing.

When a 5'-phosphonate-modified nucleoside analog is in the form of the phosphoramidite compound described herein, it can be used for linking a 5'-phosphonate-modified nucleoside analog using a phosphoramidite synthesis method for nucleotides well known in the art. A 5'-phosphonate-modified nucleoside analog can be prepared into a phosphoramidite compound by: linking a phosphorus atom of a phosphoramidite forming agent by a coupling (e.g., phosphorylation) reaction to form the phosphoramidite compound. In some embodiments, the 5'-phosphonate-modified nucleoside analog-phosphoramidite compound is used for linking a 5'-phosphonate-modified nucleoside analog to the 5'-end of the antisense strand of a double-stranded RNA.

The oligonucleotide described herein comprises one or more asymmetric centers and thus gives rise to enantiomers, diastereomers, and other stereoisomeric configurations, and these configurations can be defined in terms of absolute stereochemistry as (R) or (S), $\alpha$ or $\beta$, e.g., for sugar anomers, or as (D) or (L), e.g., for amino acids, etc. All such possible isomers, as well as their racemic forms and optionally pure forms, are included in the oligonucleotide provided herein.

In certain embodiments, provided is a method for inhibiting gene expression, and the method comprises contacting a cell with an agent comprising the oligonucleotide or double-stranded ribonucleic acid (dsRNA) provided

above, wherein the sense strand and the antisense strand in the oligonucleotide and the double-stranded ribonucleic acid (dsRNA) agent each have a length of 8-40 nucleotides, and the antisense strand in the oligonucleotide or the double-stranded ribonucleic acid (dsRNA) agent is complementary to a target RNA. In certain embodiments, the cell is in an animal. In certain embodiments, the cell is in a human. In certain embodiments, the target RNA is selected from mRNA, pre-mRNA, and micro RNA. In certain embodiments, the target RNA is mRNA. In certain embodiments, the target RNA is human mRNA. In certain embodiments, the target RNA is cleaved, thereby inhibiting its function. In certain embodiments, the method further comprises detecting the level of the target RNA. In certain embodiments, provided is a method for inhibiting gene expression, and the method comprises contacting one or more cells or tissues with an oligonucleotide or double-stranded ribonucleic acid (dsRNA) comprising a 5'-terminal nucleotide represented by formula (V), formula (V-1), formula (V-2), formula (VI), formula (VI-1), formula (VI-2), formula (VI-3), formula (VII), formula (VII-1), formula (VII-2), formula (VIII), formula (VIII-1), or a stereoisomer thereof.

A therapeutic formulation of a dsRNA agent or a target gene antisense polynucleotide agent can be prepared for storage by mixing a molecule or compound with the desired purity with an optional pharmaceutically acceptable carrier, excipient, or stabilizer (Remington's Pharmaceutical Sciences, 21st edition, (2006)) in the form of a lyophilized formulation or an aqueous solution. Acceptable carriers, excipients, or stabilizers are nontoxic to recipients at dosages and concentrations employed, and include buffers, such as phosphate, citrate, and other organic acids; antioxidants, including ascorbic acid and methionine; preservatives (such as octadecyl dimethyl benzyl ammonium chloride; hexamethonium chloride; benzalkonium chloride and benzethonium chloride; phenol, butanol, or benzyl alcohol; parabens, such as methyl or propyl paraben; catechol; resorcinol; cyclohexanol; 3-pentanol; and m-cresol); low-molecular-weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, histidine, arginine, or lysine; monosaccharides, disaccharides, and other carbohydrates including glucose, mannose, or dextrins; chelating agents, such as EDTA; sugars such as sucrose, mannitol, trehalose, or sorbitol; salt-forming counterions, such as sodium; metal complexes (e.g., zinc-protein complexes); and/or nonionic surfactants, such as TWEEN®, PLURONICS®, or polyethylene glycol (PEG).

## Mode of Administration

In some embodiments, the tissue to which a compound is administered is a tissue in which a target gene-associated disease or condition is present or is likely to occur. Non-limiting examples of the tissue are the liver or kidneys. Direct tissue administration may be achieved by direct injection or other modes. Many orally delivered compounds naturally enter and pass through the liver and kidneys, and some embodiments of the treatment method of the present disclosure comprise oral administration of one or more target gene dsRNA agents to a subject. The dsRNA agent or the target gene antisense polynucleotide agent, either alone or in combination with other therapeutic agents, may be administered once, or they may be administered multiple times. If administered multiple times, the target gene dsRNA agent or the target gene antisense polynucleotide agent may be administered by different routes. For example, although not intended to be limiting, the first administration (or first few administrations) may be performed subcutaneously, and one or more additional administrations may be oral and/or systemic administrations.

For embodiments of the present disclosure in which the systemic administration of the target gene dsRNA agent or the target gene antisense polynucleotide agent is desired, the target gene dsRNA agent or the target gene antisense polynucleotide agent may be formulated for parenteral administration by injection, e.g., by bolus injection or continuous infusion. Formulations for injection may be present in unit dosage form, e.g., in ampoules or in multi-dose containers, with or without the addition of a preservative. Target gene dsRNA agent formulations (also referred to as pharmaceutical compositions) may take such forms as suspensions, solutions, or emulsions in oily or aqueous carriers, and may contain formulation agents such as suspending agents, stabilizers, and/or dispersants.

Formulations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffer media. Parenteral carriers include sodium chloride solution, Ringer's dextrose solution, dextrose and sodium chloride solution, lactated Ringer's solution, or fixed oils. Intravenous excipients include fluid and nutritional supplements, electrolyte supplements (such as those based on Ringer's dextrose solution), and the like. Preservatives and other additives may also be present, for example, antimicrobials, antioxidants, chelating agents, inert gases, and the like. Lower doses will result from other forms of administration, such as intravenous administration. If the response of a subject is insufficient at the initial dose, a higher dose (or effectively increased dose by a different, more localized delivery route) may be used within the tolerance of the patient. Multiple doses per day may be used as needed to achieve appropriate systemic or local levels of one or more target gene dsRNA agents or target gene antisense polynucleotide agents, and to achieve appropriate reduction in target gene activity.

In other embodiments, the method of the present disclosure comprises the use of a delivery carrier, such as a biocompatible microparticle, nanoparticle, or implant suitable for implantation into a recipient, such as a subject.

Exemplary biodegradable implants that can be used in accordance with this method are described in PCT publication No. WO 95/24929 (incorporated herein by reference), which describes a biocompatible, biodegradable polymer matrix used for containing a biological macromolecule.

Both non-biodegradable and biodegradable polymer matrices may be used in the methods of the present disclosure to deliver one or more target gene dsRNA agents or target gene antisense polynucleotide agents to a subject. In some embodiments, the matrix may be biodegradable. The matrix polymer may be a natural or synthetic polymer. The polymer may be selected based on the period of time over which release is desired, generally on the order of a few hours to a year or longer. Generally, release over a period of time ranging from a few hours to three to twelve months may be used. The polymer is optionally in the form of a hydrogel, which can absorb up to about 90% of its weight in water, and is also optionally cross-linked with multivalent ions or other polymers.

Generally, in some embodiments of the present disclosure, the target gene dsRNA agent or the target gene antisense polynucleotide agent may be delivered using a biodegradable implant by diffusion or by degradation of a polymer matrix. Exemplary synthetic polymers for such use are well known in the art. Biodegradable and non-biodegradable polymers may be used to deliver the target gene dsRNA agent or the target gene antisense polynucleotide agent using methods known in the art. Bioadhesive polymers such as bioerodible hydrogels (H. S. Sawhney, C. P. Pathak and J. A. Hubell in Macromolecules, 1993, 26, 581-587) may also be used to deliver the target gene sRNA agent or the target gene antisense polynucleotide agent for the treatment of target gene-associated diseases or conditions. Other suitable delivery systems may include timed release, delayed release, or sustained release delivery systems. Such systems can avoid repeated administration of the target gene dsRNA agent or the target gene antisense polynucleotide agent, thereby improving the convenience of subjects and healthcare professionals. Many types of release delivery systems are available and known to those of ordinary skill in the art. See, e.g., U.S. Pat. Nos. 5,075,109, 4,452,775, 4,675,189, 5,736,152, 3,854,480, 5,133,974, and 5,407,686. In addition, pump-based hardware delivery systems may be used, some of which are also suitable for implantation.

The use of a long-term sustained release implant may be suitable for prophylactic treatment of subjects and for subjects at risk of developing a recurrent target gene)-associated disease or condition. As used herein, long-term release means that the implant is constructed and arranged to deliver a therapeutic level of a target gene dsRNA agent or a target gene antisense polynucleotide agent for at least up to 10 days, 20 days, 30 days, 60 days, 90 days, six months, a year, or longer. Long-term sustained release implants are well known to those of ordinary skill in the art and include some of the release systems described above.

**Effective Amount**

In some aspects, the method of the present disclosure comprises contacting a cell with an effective amount of a dsRNA agent or an antisense polynucleotide agent to reduce gene expression in the contacted cell. Certain embodiments of the method of the present disclosure comprise administering to a subject a dsRNA agent or an antisense polynucleotide agent in an amount effective to reduce gene expression and treat an associated disease or condition in the subject. The "effective amount" used in terms of reducing expression and/or for treating the associated disease or condition is an amount necessary or sufficient to achieve a desired biological effect. For example, the effective amount of the dsRNA agent or the antisense polynucleotide agent for treating the associated disease or condition may (i) be an amount necessary to slow or stop the progression of the disease or condition; (ii) reverse, reduce, or eliminate one or more symptoms of the disease or condition. In some aspects of the present disclosure, the effective amount is an amount of the dsRNA agent or the antisense polynucleotide agent that, when administered to a subject in need of treatment of an associated disease or condition, results in a therapeutic response that prevents and/or treats the disease or condition. According to some aspects of the present disclosure, the effective amount is an amount of the dsRNA agent or the antisense polynucleotide agent of the present disclosure that, when combined or co-administered with another therapeutic treatment for the associated disease or condition, results in a therapeutic response that prevents and/or treats the disease or condition. In some embodiments of the present disclosure, the biological effect of treating a subject with the dsRNA agent or the antisense polynucleotide agent of the present disclosure may be the amelioration and/or complete elimination of symptoms caused by the associated disease or condition. In some embodiments of the present disclosure, the biological effect is the complete elimination of the associated disease or condition, for example, as evidenced by a diagnostic test indicating that the subject does not have the associated disease or condition. In some embodiments, the effective amount is an amount that results in a desired response, such as an amount that reduces the associated disease or condition in cells, tissues, and/or subjects with the disease or condition. Some embodiments of the present disclosure comprise a method for determining the efficacy of the target gene dsRNA agent or the target gene antisense polynucleotide agent of the present disclosure administered to a subject to treat a target gene-associated disease or condition, which is performed by assessing and/or monitoring one or more "physiological characteristics" of the target gene-associated disease or condition in the subject. Non-limiting examples of physiological characteristics of the target gene-associated disease or condition are many patients

It should be understood that gene silencing may be performed constitutively or by genomic engineering in any expressing cell and determined by any suitable assay. In some embodiments of the present disclosure, the expression of the target gene is reduced by at least 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% by administration of the dsRNA agent of the present disclosure. In some embodiments of the present disclosure, the expression of the target gene is reduced by 5% to 10%, 5% to 25%, 10% to 50%, 10% to 75%, 25% to 75%, 25% to 100%, or 50% to 100% by administration of the dsRNA agent of the present disclosure.

The dsRNA agent and the antisense polynucleotide agent in the present disclosure are delivered in a pharmaceutical composition at a dose sufficient for the expression of the target gene. In certain embodiments of the present disclosure, the dose of the dsRNA agent or the antisense polynucleotide agent is 0.01 to 200.0 mg per kilogram of body weight of a recipient per day, generally 1 to 50 mg/kg body weight, 5 to 40 mg/kg body weight, 10 to 30 mg/kg body weight, 1 to 20 mg/kg body weight, 1 to 10 mg/kg body weight, or 4 to 15 mg/kg body weight per day (inclusive).

A variety of factors may be considered in determining the delivery dose and time of the dsRNA agent of the present disclosure. The absolute amount of the dsRNA agent or the antisense polynucleotide agent delivered will depend on a variety of factors, including co-treatment, the number of doses, and individual subject parameters, including age, physical condition, physical size, and body weight. These factors are well known to those of ordinary skill in the art and can be solved by routine experimentation. In some embodiments, a maximum dose, i.e., the highest safe dose based on sound medical judgment, may be used.

In some embodiments, the method of the present disclosure may comprise administering to the subject 1, 2, 3, 4, 5, 6, 7, 8, 9, 10, or more doses of the agent or the antisense polynucleotide agent. In some cases, a dose of a pharmaceutical compound may be administered to a subject at least daily, every other day, weekly, every other week, monthly, etc., and may be administered once daily or more than once daily, e.g., 2, 3, 4, 5, or more times over a 24-hour period. The pharmaceutical composition of the present disclosure may be administered once daily; or the dsRNA agent or the antisense polynucleotide agent may be administered in two, three, or more sub-doses at appropriate intervals within a day, or even delivered using continuous infusions or by controlled-release formulations. In some embodiments of the method of the present disclosure, the pharmaceutical composition of the present disclosure is administered to the subject one or more times per day, one or more times per week, one or more times per month, or one or more times per year.

Certain embodiments of the present disclosure comprise use of a pharmaceutical composition comprising a dsRNA agent or an antisense polynucleotide agent and a pharmaceutically acceptable carrier. The pharmaceutical composition comprising the dsRNA agent or the antisense polynucleotide agent may be used in the method of the present disclosure to reduce gene expression and activity in a cell, and may be used for treating an associated disease or condition. Such a pharmaceutical composition may be formulated based on the mode of delivery. Non-limiting examples of formulations for the modes of delivery are compositions formulated for subcutaneous delivery, compositions formulated for systemic administration by parenteral delivery, compositions formulated for intravenous (IV) delivery, compositions formulated for intrathecal delivery, compositions formulated for direct delivery into the brain, and the like. The pharmaceutical composition of the present disclosure may be administered using one or more modes to deliver the dsRNA agent or the antisense polynucleotide agent into a cell, for example, via surface (e.g., by a transdermal patch); lung, e.g., by inhalation or insufflation of powders or aerosols, including by a nebulizer; intra-airway, intranasal, epidermal and transdermal, oral, or parenteral. Parenteral administration includes intravenous, intraarterial, subcutaneous, intraperitoneal, or intramuscular injection or infusion; subcuticular administration, e.g., by an implantation device; intracranial administration, e.g., by intraparenchymal administration; or intrathecal or intraventricular administration. The dsRNA agent or the antisense polynucleotide agent may also be delivered directly to a target tissue, e.g., directly to the liver, directly to the kidney, etc. It will be understood that "delivering the dsRNA agent" or "delivering the antisense polynucleotide agent" into a cell includes separately delivering the dsRNA agent or the antisense polynucleotide agent, directly expressing the dsRNA agent in a cell and expressing the dsRNA agent from a coding vector delivered into a cell, or any suitable manner of making the dsRNA or antisense polynucleotide agent appear in a cell. Preparation and use of formulations and means for delivering inhibitory RNA are well known and routinely used in the art.

In some embodiments, the composition further comprises one or more additional therapeutic agents. The composition of the present disclosure may comprise one or more dsRNA agents and optionally one or more pharmaceutically acceptable carriers, delivery agents, targeting agents, detectable labels, etc. Non-limiting examples of targeting agents that may be used according to some embodiments of the method of the present disclosure are agents that direct the dsRNA agent of the present disclosure to and/or into a cell to be treated. The choice of targeting agents will depend on the following factors: the nature of the associated disease or condition, and the type of the target cell. In one non-limiting example, in some embodiments of the present disclosure, it may be necessary to target the dsRNA agent to and/or into hepatocytes. It should be understood that in some embodiments of the method of the present disclosure, the therapeutic agent comprises a dsRNA agent having only a delivery agent, e.g., a delivery agent comprising N-acetylgalactosamine (GalNAc), without any additional linking elements. For example, in some aspects of the present disclosure, the dsRNA agent may be linked to a delivery compound comprising GalNAc and contained in a composition comprising a

pharmaceutically acceptable carrier and administered to a cell or subject in the absence of any detectable label or targeting agent or the like linked to the dsRNA agent.

In the case where the dsRNA agent of the present disclosure is administered together with and/or linked to one or more delivery agents, targeting agents, labeling agents, and the like, those skilled in the art are able to understand and to select and use suitable agents for use in the method of the present disclosure. Labeling agents may be used in certain methods of the present disclosure to determine the location of the dsRNA agent in cells and tissues, and may be used to determine the location of therapeutic compositions comprising dsRNA agents that have been administered in the methods of the present disclosure in cells, tissues, or organs. Means for attaching and using labeling agents such as enzyme labels, dyes, radioactive labels, and the like are well known in the art. It should be understood that in some embodiments of the composition and method of the present disclosure, the labeling agent is linked to one or both of the sense polynucleotide and the antisense polynucleotide contained in the dsRNA agent.

In some embodiments, the composition is packaged in a kit, container, package, dispenser, pre-filled syringe, or vial. A kit comprising one or more target gene dsRNA agents and/or target gene antisense polynucleotide agents and instructions for their use in the method of the present disclosure is also within the scope of the present disclosure. The kit of the present disclosure may comprise one or more of a target gene dsRNA agent, a target gene sense polynucleotide agent, and a target gene antisense polynucleotide agent that may be used to treat a target gene-associated disease or condition. A kit comprising one or more target gene dsRNA agents, target gene sense polynucleotide agents, and target gene antisense polynucleotide agents may be prepared for use in the treatment method of the present disclosure. The components of the kit of the present disclosure may be packaged in an aqueous medium or lyophilized form. The kit of the present disclosure may comprise a support that is partitioned to hermetically receive one or more container devices or a series of container devices (e.g., test tubes, vials, flasks, bottles, syringes, etc.) therein. The first container device or series of container devices may comprise one or more compounds, e.g., a target gene) dsRNA agent and/or a target gene sense or antisense polynucleotide agent. The second container device or series of container devices may comprise a targeting agent, a labeling agent, a delivery agent, etc., which may be included as part of the target gene) dsRNA agent and/or the target gene antisense polynucleotide administered in an embodiment of the treatment method of the present disclosure.

The kit of the present disclosure may further comprise instructions. The instructions are usually in writing and will provide guidance for performing the treatment embodied by the kit and making decisions based on that treatment.

**Cell, Subject, and Control**

The method of the present disclosure may be used in conjunction with cells, tissues, organs, and/or subjects. In some aspects of the present disclosure, the subject is a human or a vertebrate mammal, including but not limited to, dogs, cats, horses, cows, goats, mice, rats, and primates, such as monkeys. Thus, the present disclosure may be used to treat target gene-associated diseases or conditions in both human and non-human subjects.

In some aspects of the present disclosure, the subject may be a farm animal, a zoo animal, a domesticated animal, or a non-domesticated animal, and the method of the present disclosure may be used in veterinary prophylactic and therapeutic regimens. In some embodiments of the present disclosure, the subject is a human, and the method of the present disclosure may be used in prophylactic and therapeutic regimens for humans.

Non-limiting examples of subjects to which the present disclosure may be applied are subjects diagnosed with, suspected of having, or at risk of having a disease or condition associated with target gene expression and/or activity higher than the desired expression and/or activity, also referred to as "increased target gene expression level". Non-limiting examples of diseases and conditions associated with target gene expression and/or activity higher than the desired level are described elsewhere herein. The method of the present disclosure may be applied to subjects who have been diagnosed with the disease or condition at the time of treatment, subjects associated with target gene expression and/or activity higher than the desired expression and/or activity, or subjects considered to be at risk of having or developing a disease or condition associated with target gene expression and/or activity higher than the desired expression and/or activity. In some aspects of the present disclosure, the disease or condition associated with target gene expression and/or activity higher than the desired level is an acute disease or condition; in certain aspects of the present disclosure, the disease or condition associated with target gene expression and/or activity higher than the desired level is a chronic disease or condition.

In another non-limiting example, the target gene dsRNA agent of the present disclosure is administered to treat a disease or disorder that is caused by or associated with activation of the target gene, or a disease or disorder whose symptoms or progression are responsive to inactivation of the target gene. The term "target gene-associated disease" includes diseases, disorders, or conditions that benefit from decreased expression of the target gene.

Cells to which the method of the present disclosure may be applied include *in vitro, in vivo,* and *ex vivo* cells. The cells may be in a subject, in a culture and/or suspension, or in any other suitable state or condition. The cells to which the method of the present disclosure may be applied may be liver cells, hepatocytes, heart cells, pancreatic cells, cardiovascular cells, kidney cells, or other types of vertebrate cells, including human and non-human mammalian cells.

In certain aspects of the present disclosure, the cells to which the method of the present disclosure may be applied are healthy normal cells, which are not known to be diseased cells. In certain embodiments of the present disclosure, the method and composition of the present disclosure are applied to cells such as liver cells, hepatocytes, heart cells, pancreatic cells, cardiovascular cells, and/or kidney cells. In certain aspects of the present disclosure, the control cell is a normal cell, but it should be understood that cells with a disease or condition may also be used as control cells in particular cases, e.g., in the case of comparing the results of treated cells with a disease or condition to the results of untreated cells with the disease or condition, and the like.

According to the method of the present disclosure, the level of target gene polypeptide activity may be determined and compared to the control level of the target gene polypeptide activity. The control may be a predetermined value, which may take a variety of forms. It may be a single cutoff value, such as a median or mean. It can be established based on comparison groups, e.g., in a group with normal levels of target gene polypeptide and/or target gene polypeptide activity and a group with increased levels of target gene polypeptide and/or target gene polypeptide activity. Another non-limiting example of the comparison group may be a population with one or more symptoms or diagnoses of a target gene-associated disease or condition versus a population without one or more symptoms or diagnoses of the disease or condition; a group of subjects to whom the siRNA treatment of the present disclosure has been administered versus a group of subjects to whom the siRNA treatment of the present disclosure has not been administered. Generally, the control may be based on apparently healthy normal individuals or apparently healthy cells in an appropriate age group. It should be understood that, in addition to the predetermined value, the control according to the present disclosure may be a material sample tested in parallel with the experimental material. Examples include samples from control populations or control samples produced by manufacturing for parallel testing with experimental samples. In some embodiments of the present disclosure, the control may include a cell or subject not contacted by or treated with the target gene dsRNA agent of the present disclosure, in which case the control level of the target gene polypeptide and/or target gene polypeptide activity may be compared to the level of the target gene polypeptide and/or target gene polypeptide activity in a cell or subject contacted with the target gene dsRNA agent or the target gene antisense polynucleotide agent of the present disclosure.

In some embodiments of the present disclosure, the control level may be a target gene polypeptide level determined for a subject, wherein the target gene polypeptide levels determined for the same subject at different times are compared to the control level. In one non-limiting example, the level of the target gene is determined in a biological sample obtained from a subject who has never received the target gene treatment of the present disclosure. In some embodiments, the biological sample is a serum sample. The target gene polypeptide level determined in the sample obtained from the subject may be used as a baseline or control value for the subject. After one or more target gene dsRNA agents are administered to the subject in the treatment method of the present disclosure, one or more additional serum samples may be obtained from the subject, and the target gene polypeptide levels in the subsequent one or more samples may be compared to the control/baseline levels of the subject. Such comparisons may be used to assess the onset, progression, or regression of the target gene)-associated disease or condition in the subject. For example, the level of the target gene) polypeptide in the baseline sample obtained from the subject is higher than the level obtained from the same subject after the target gene dsRNA agent or the target gene) antisense polynucleotide agent of the present disclosure is administered to the subject, indicating regression of the target gene-associated disease or condition and the efficacy of the administered target gene dsRNA agent of the present disclosure in treating the target gene-associated disease or condition.

In certain aspects of the present disclosure, one or more values in the level of the target gene polypeptide and/or target gene polypeptide activity determined for a subject may serve as a control value and be used to later compare the level of the target gene polypeptide and/or target gene activity in the same subject, thereby allowing for the assessment of changes from the "baseline" target gene polypeptide activity in the subject. Thus, in the case where the initial level is used as a control level for the subject, the initial target gene polypeptide level and/or the initial target gene polypeptide activity level may be used to demonstrate and/or determine the level of the target gene polypeptide and/or target gene polypeptide activity in the subject that the method and compound of the present disclosure are able to reduce in the subject.

The target gene dsRNA agent and/or the target gene antisense polynucleotide agent of the present disclosure may be administered to a subject using the method of the present disclosure. Such dsRNAi agents include those whose efficacy of administration and treatment in the present disclosure may be assessed as follows: after administration and treatment, the level of the target gene polypeptide in the serum sample obtained from the subject is reduced by at least 0.5%, 1%, 5%, 10%, 20%, 30%, 40%, 50%, 60%, 70%, 80%, 90%, 95%, or more compared to the level of the target gene) polypeptide before administration in the serum sample obtained from the subject at a previous time point, or compared to the level of a non-contact control (e.g., the level of the target gene polypeptide in the control serum sample). It should be understood that both the level of the target gene polypeptide and the level of the target gene polypeptide activity are associated with the level of target gene expression. Certain embodiments of the method of the present disclosure comprise administering to the subject the target gene dsRNA and/or the target gene antisense agent of the present disclosure in an amount effective to inhibit the expression of the target gene, thereby reducing the level of the target gene polypeptide and reducing the level of the target gene polypeptide activity in the subject. In some embodiments of the method of the present disclosure, contacting (also referred to herein as treating) the cell with the siRNA agent of the

present disclosure results in inhibition of the target gene expression in the cell by at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or about 100%, for example to a level below the detection level of the assay.

Some embodiments of the present disclosure comprise determining the presence, absence, and/or amount (also referred to herein as level) of the target gene polypeptide in one or more biological samples obtained from one or more subjects. This determination may be used to assess the efficacy of the treatment method of the present disclosure. For example, the method and composition of the present disclosure may be used to determine the level of the target gene polypeptide in a biological sample obtained from a subject previously treated by administering the target gene dsNA agent and/or the target gene antisense agent of the present disclosure. After administration and treatment, the level of the target gene polypeptide in the serum sample obtained from the subject is reduced by at least 0.5%, 1%, 5%, 10%, 20%, 30%, 40%, 50%, 60%, 70%, 80%, 90%, 95%, or more compared to the level of the target gene polypeptide before administration in the serum sample obtained from the subject at a previous time point, or compared to the level of a non-contact control (e.g., the level of the target gene polypeptide in the control serum sample), and thus the efficacy level of the treatment administered to the subject is indicated.

In some embodiments of the present disclosure, the physiological characteristics of the target gene-associated disease or condition determined for the subject may serve as control results, and the determination results of the physiological characteristics of the same subject at different times are compared to the control results. In one non-limiting example, the pathological characteristic hemolysis is measured from a subject that has never received the target gene treatment of the present disclosure, which may be used as a baseline or control value for the subject. After one or more target gene dsRNA agents are administered to the subject in the treatment method of the present disclosure, the blood cells are compared to the control/baseline levels of the subject, respectively. Such comparisons may be used to assess the onset, progression, or regression of the target gene-associated disease or condition in the subject. For example, the baseline blood cell obtained from the subject is higher than the thrombus determined from the same subject after the target gene dsRNA agent or the target gene antisense polynucleotide agent of the present disclosure is administered to the subject, indicating regression of the target gene-associated disease or condition and the efficacy of the administered target gene dsRNA agent of the present disclosure in treating the target gene-associated disease or condition.

Some embodiments of the present disclosure comprise determining the presence, absence, and/or change of physiological characteristics of the target gene-associated disease or condition using methods, for example, but not limited to, (1) measuring blood cells of the subject (2) assessing physiological characteristics of one or more biological samples obtained from one or more subjects; (3) or performing a physical examination on the subject. This determination may be used to assess the efficacy of the treatment method of the present disclosure.

## Regarding Modification

In some embodiments of the present disclosure, the RNA of the gene RNAi agent is chemically modified to obtain enhanced stability and/or one or more other beneficial properties. Nucleic acids in certain embodiments of the present disclosure may be synthesized and/or modified by methods well known in the art, see, e.g., "Current protocols in Nucleic Acid Chemistry," Beaucage, S. L. et al. (Eds.), John Wiley & Sons, Inc., New York, N.Y., USA, which is incorporated herein by reference. Modifications that may be present in certain embodiments of the dsRNA agent of the present disclosure include, for example: (a) terminal modifications, such as a modification at the 5'-end (phosphorylation, conjugation, reverse linkage, etc.), and a modification at the 3'-end (conjugation, DNA nucleotides, reverse linkage, etc.); (b) base modifications, such as a base substitution with a stabilized base, a destabilized base, or an extended partner pool for base pairing, a base deletion (an abasic nucleotide), or a base conjugation; (c) sugar modifications (e.g., at the 2' position or 4' position) or sugar substitutions; and (d) backbone modifications, including modifications or substitutions to phosphodiester bonds. Specific examples of RNA compounds that may be used in certain embodiments of the dsRNA agent, antisense polynucleotide, and sense polynucleotide of the present disclosure include, but are not limited to, RNAs comprising a modified backbone or lacking natural internucleoside linkages. As a non-limiting example, an RNA with a backbone modification may not have a phosphorus atom in the backbone. The RNA that does not have a phosphorus atom in its internucleoside backbone may be referred to as an oligonucleoside. In certain embodiments of the present disclosure, the modified RNA has a phosphorus atom in its internucleoside backbone.

It should be understood that the term "RNA molecule" or "RNA" or "ribonucleic acid molecule" includes not only RNA molecules expressed or found in nature, but also analogs and derivatives of RNA, which comprise one or more ribonucleotide/ribonucleoside analogs or derivatives as described herein or known in the art. The terms "ribonucleoside" and "ribonucleotide" are used interchangeably herein. RNA molecules may be modified in the nucleobase structure or the ribose-phosphate backbone structure (e.g., as described below), and molecules comprising ribonucleoside analogs or

derivatives must retain the ability to form duplexes.

The following examples are provided to illustrate the specific examples of the practice of the present disclosure and are not intended to limit the scope of the present disclosure. It is apparent to those of ordinary skill in the art that the present disclosure can be applied to a variety of compositions and methods.

About: As used herein, the term "about" or "approximately", as applied to one or more values of interest, refers to a value that is similar to the stated reference value. In certain embodiments, unless otherwise specified or clearly indicated from the context, the term "about" or "approximately" refers to a range of values falling within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value (unless such a numerical value would exceed 100% of a possible value).

Some abbreviations used herein are as follows: "Ac" refers to acetyl; the abbreviation "Bn" refers to benzyl; the abbreviation "Bz" refers to benzoyl; the abbreviation "DMTrCl" refers to 4,4-dimethoxytrityl chloride; the abbreviation "DMTr" refers to 4,4-dimethoxytrityl; the abbreviation "TIIP" refers to tetrahydropyranyl; the abbreviation "TBDMS" refers to tert-butyldimethylsilyl; the abbreviation "TIPDS" refers to tetraisopropyldisilyl; and the abbreviation "DTBS" refers to di(tert-butyl)silyl.

## DESCRIPTION OF DRAWINGS

FIG. 1 shows a single crystal X-ray diffraction pattern of Phos-15-1E-2 (diethyl ((((1R,2R,4R)-4-(2,4-dioxo-3,4-dihydropyrimidin-1(2H)-yl)-2-hydroxycyclopentyl)thio)methyl)phosphonate).

## DETAILED DESCRIPTION

The following examples are provided to illustrate the specific examples of implementing the present disclosure and are not intended to limit the scope of the present disclosure. It is apparent to those of ordinary skill in the art that the present disclosure will be applied in a variety of compositions and methods.

### Example 1

### Preparation of phosphoramidite-01

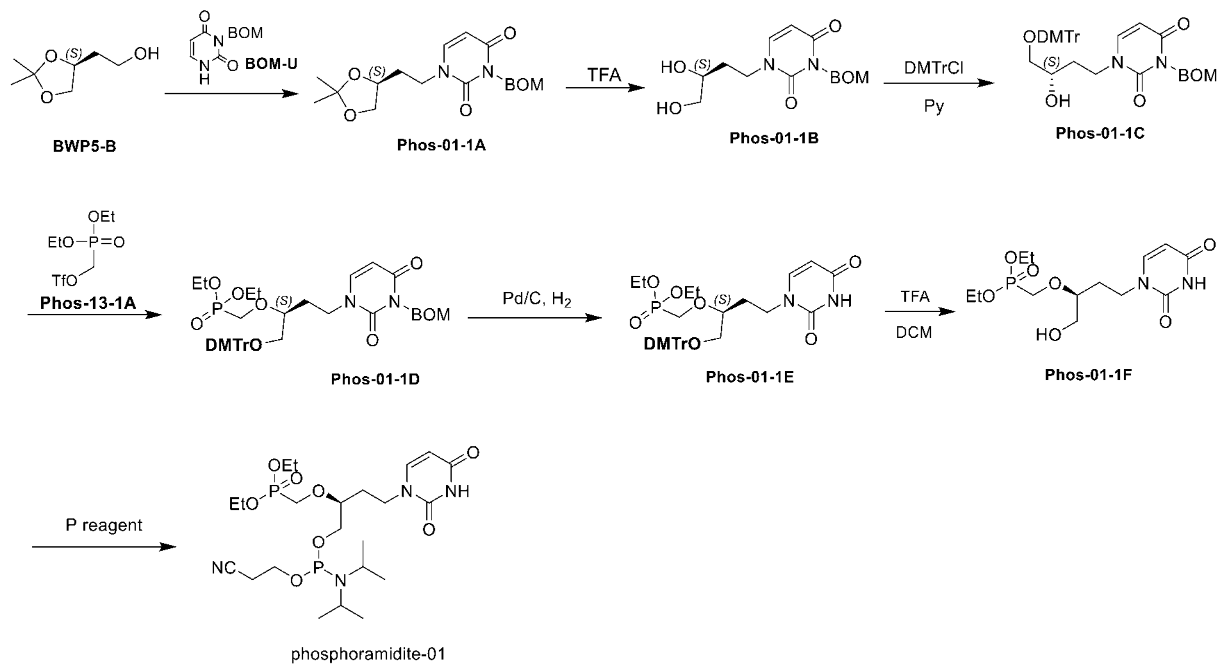


BWP5-B

Phos-01-1A

Phos-01-1B

Phos-01-1C

Phos-01-1D

Phos-01-1E

Phos-01-1F

phosphoramidite-01

### Synthesis of Phos-01-1A

$PPh_3$ (8.87 g, 2.0 eq) was added to a solution of BWP5-B (3.71 g, 1.5 eq) in anhydrous tetrahydrofuran, and

DEAD (6.19 g, 2.1 eq) was then added to the above mixture. After 30 min of stirring at 0 °C in a nitrogen atmosphere, BOM-U (10.0 g, 1.0 eq) was added to the mixture, and the mixture was stirred for 16 h. TLC (MeOH:DCM = 1:20, Rf = 0.6) showed that BOM-U was completely consumed. $CaBr_2$ (13.6 g, 4.0 eq.) was added to the above mixture, and the mixture was stirred at room temperature for 16 h and then filtered. The filtrate was concentrated under reduced pressure, and the crude product was purified by silica gel column chromatography (eluent: ethyl acetate:petroleum ether = 0:100 to 50:50) to give Phos-01-1A as an off-white solid (2.3 g, 38% yield).

MS (M+H) = 361.

**Synthesis of Phos-01-1B**

Trifluoroacetic acid (10 mL) was added to an aqueous solution (10 mL) of Phos-01-1A (1.9 g, 1.0 eq). The reaction was stirred at room temperature for 3 h, and TLC analysis (MeOH:DCM = 1:20, Rf = 0.3) showed that Phos-01-1A was completely consumed. The mixture was concentrated under reduced pressure, and the crude product was purified by silica gel column chromatography (eluent: MeOH:DCM = 0:100 to 1:10) to give Phos-01-1B as a colorless oil (0.97 g, 57% yield).

MS (M+H) = 321.

**Synthesis of Phos-01-1C**

DMTr-Cl (1.13 g, 1.1 eq.) was added to a solution of Phos-01-1B (0.97 g, 1.0 eq.) in pyridine at 0 °C in a nitrogen atmosphere, and the mixture was stirred at room temperature for 16 h. TLC (MeOH:DCM = 1:20, Rf = 0.9) showed that Phos-01-1B was completely consumed. The mixture was concentrated to dryness under reduced pressure, and the crude product was purified by silica gel column chromatography (eluent: MeOH:DCM = 0:100 to 1:20) to give Phos-01-1C as a brown solid (0.75 g, 40% yield).

MS (M+H) = 623.

**Synthesis of Phos-01-1D**

NaH (139 mg, 1.1 eq) was added to a solution of Phos-01-1C (0.75 g, 1.0 eq) in dry tetrahydrofuran at 0 °C in a nitrogen atmosphere, and the mixture was stirred at 0 °C for 30 min. Phos-13-1A (1.3 g, 3.0 eq.) was added to the above mixture at 0 °C, and the mixture was stirred at room temperature for 5 h. TLC (MeOH:DCM = 1:20, Rf = 0.7) showed that Phos-01-1C was completely consumed. The reaction solution was poured into a saturated aqueous sodium bicarbonate solution and extracted with ethyl acetate. The ethyl acetate phase was washed with brine and dried over anhydrous sodium sulfate. After the drying agent was removed by filtration, the filtrate was concentrated to dryness under reduced pressure. The crude product was purified by silica gel column chromatography (eluent: MeOH:DCM = 0:100 to 1:25) to give Phos-01-1D as a yellow oil (0.75 g, 81% yield).

MS (M+H) = 773.

**Synthesis of Phos-01-1F**

Pd/C (300 mg) was added to a solution of Phos-01-1D (0.75 g, 1.0 eq.) in isopropanol-water (10:1, 0.5% formic acid) in a nitrogen atmosphere, and the mixture was stirred overnight in a hydrogen atmosphere under atmospheric pressure. TLC (MeOH:DCM = 1:20, Rf = 0.3) showed that Phos-01-1D was completely consumed. The mixture was filtered using a Buchner funnel with a fritted disc, and the filtrate was concentrated to dryness under reduced pressure. The crude product was purified by silica gel column chromatography (eluent: MeOH:DCM = 0:100 to 1:20) to give Phos-01-1F as a brown solid (200 mg, 59% yield).

MS (M+H) = 351.

**phosphoramidite-01**

Dichloromethane (20 mL) and Phos-01-1F (400 mg, 1.0 eq.) were added to a 100-mL flask at room temperature in a nitrogen atmosphere. Tetrazole (132 mg, 1.65 eq., 0.45 M in MeCN) was added dropwise at room temperature, and a solution of bis(diisopropylamino)(2-cyanoethoxy)phosphine (775 mg, 2.25 eq.) in dichloromethane (5 mL) was then added

dropwise to the above reaction mixture. The resulting mixture was then stirred at room temperature for 3 h. TLC (MeOH:DCM = 1:20, Rf = 0.8) showed that Phos-01-1D was completely consumed. The reaction solution was cooled to 0 °C, poured into a saturated aqueous sodium bicarbonate solution (100 mL), and extracted with dichloromethane (100 mL × 3), and the combined organic layers were washed with saturated brine (100 mL), dried over anhydrous sodium sulfate, and concentrated under reduced pressure. The crude product was purified by silica gel column chromatography (eluent: MeOH:DCM = 0:100 to 1:100; 1% $Et_3N$) to give phosphoramidite-01 as a colorless oil (190 mg, 30% yield).

$^1H$ NMR (400 MHz, $CD_3CN$) δ 7.48 (d, *J* = 8.0 Hz, 1H), 5.54 (d, *J* = 8.0 Hz, 1H), 4.14-4.08 (m, 4H), 3.89-3.67 (m, 7H), 3.66-3.53 (m, 4H), 2.66-2.63 (m, 2H), 1.90-1.73 (m, 2H), 1.20-1.38 (m, 6H), 1.23-1.15 (m, 12H).

MS (M+H) = 551.

**Preparation of phosphoramidite-03**

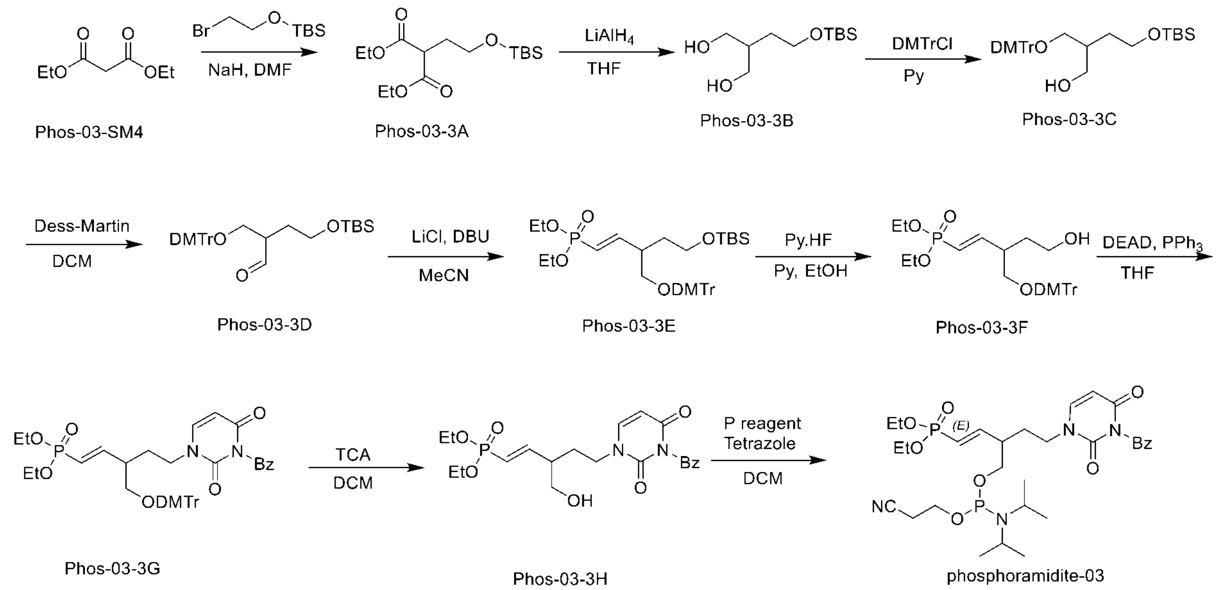


Phos-03-SM4, Phos-03-3A, Phos-03-3B, Phos-03-3C, Phos-03-3D, Phos-03-3E, Phos-03-3F, Phos-03-3G, Phos-03-3H, phosphoramidite-03

Diethyl malonate (32 g, 0.2 mol, 1.0 eq.) and DMF (200 mL) were added to a 500-mL flask in a nitrogen atmosphere, and the solution was cooled to 0-5 °C with stirring. Sodium hydride (60%, 8.4 g, 0.21 mol, 1.05 eq.) was then added in batches. During the addition of sodium hydride, the temperature of the reaction solution gradually increased from 5 °C to 15 °C. The reaction solution was cooled to 5 °C and stirred for another 20 min. Then, potassium iodide (1.66 g, 0.01 mol, 0.05 eq.) was added to the mixture, and (2-bromoethoxy)(tert-butyl)dimethylsilane (57.41 g, 0.24 mol, 1.2 eq.) was added. The reaction solution was heated to 50 °C and stirred overnight in a nitrogen atmosphere. TLC showed that the starting material was converted into a new main product. The reaction mixture was quenched with a saturated ammonium chloride solution (250 mL) and stirred at 0-25 °C for 0.5 h. The organic substance was extracted with ethyl acetate:petroleum ether (50:50, 500 mL × 2), and the extract was concentrated under vacuum to give a crude product. The crude product was purified by flash column chromatography (eluent: ethyl acetate:petroleum ether = 0%-20%) to elute the product. The product was concentrated under vacuum to give phos-03-3A as a colorless oil (50.84 g, 79.8% yield).

$^1H$ NMR: (400 MHz, DMSO-d6), δppm 4.13-4.07 (q, 4 H), 3.60 (t,2 H), 3.50 (t, 1 H), 1.96-1.94 (m, 2 H), 1.20(t, 6 H), 0.85 (s, 9 H), 0.00 (s, 6 H).

Tetrahydrofuran (100 mL) was added to a 500-mL flask and cooled to 0 °C in a nitrogen atmosphere, and lithium aluminum hydride (0.077 mol, 1.5 eq.) was then added. A solution of Phos-03-3A (16.4 g, 0.0515 mol, 1.0 eq.) in tetrahydrofuran (30 mL) was added to the reaction solution at 0-5 °C, and the reaction solution was stirred at 5 °C for 1 h. TLC showed that the starting material was converted into a new main product. The reaction mixture was slowly quenched with 100 mL of ethyl acetate and stirred at 5-25 °C for 0.5 h. The mixture was filtered using a diatomaceous earth thin layer silica gel layer. The filtrate was concentrated to give a crude product, and the crude product was further purified by silica gel column chromatography (eluent: 0%-10% MeOH in DCM) to elute the product. The product was concentrated under vacuum to give Phos-03-3A as a yellow oil (4.8 g, 39.8% yield).

$^1H$ NMR:(400 MHz, DMSO-d6), δppm 4.27 (s, 2H), 3.62 (t,2H), 3.35-3.32 (m, 4H), 1.56-1.53 (m,1H), 1.43-1.38 (m,2H), 0.84 (s,9H), 0.00 (s,6H).

Pyridine (50 mL) and Phos-03-3B (4.5 g, 0.019 mol, 1.0 eq.) were added to a 250-mL flask, and the mixture was stirred in a nitrogen atmosphere and cooled to 0 °C. DMTrCl (7.2 g, 0.021 mol, 1.15 eq.) was then added, and the mixture was stirred overnight at room temperature. TLC showed that the starting material was converted into a new main product. The volatiles were removed by distillation under reduced pressure, and the residue was purified by silica gel column chromatography (eluent: EA:PE:Py = 1:8:0.2%) to elute the product. The product was concentrated under vacuum to give Phos-03-3C as a yellow oil (4.6 g, 44.6% yield).

$^1$H NMR: (400 MHz, DMSO-d6), δppm 7.45-7.39 (m, 5H), 7.359-7.24 (m, 8H), 4.48 (s, 1H), 3.78 (s,6H),3.57-3.40 (m, 4H), 3.04 (d, 2H), 1.89-1.83 (m,1H), 1.56-1.52 (m, 2H), 0.86 (s, 9H), 0.00 (s, 6H).

Dichloromethane (40 mL) and Phos-03-3C (4.6 g, 8.6 mmol, 1.0 eq.) were added to a 250-mL flask, and Dess-Martin periodinane (18.3 g, 0.043 mol, 5.0 eq.) was then added. The reaction was stirred at room temperature for 2 h in a nitrogen atmosphere. TLC showed that the starting material was converted into a new main product. The solvent was filtered, and the filtrate was purified by silica gel column chromatography (eluent: EA:PE:Py = 3:100:0.2%) to elute the product. The product was concentrated under vacuum to give Phos-03-3D as a yellow oil (4.0 g, 87.0% yield).

Acetonitrile (60 mL) and lithium chloride (523 mg, 1.2 mmol, 1.2 eq.) were added to a 250-mL flask at room temperature, and tetraethyl methylenediphosphonate (3.56 g, 1.2 mmol, 1.2 eq.), DBU (1.56 g, 1.0 mmol, 1.0 eq.), and Phos-03-3D (5.5 g, 1.0 mmol, 1.0 eq.) were added in a nitrogen atmosphere. The mixture was then stirred at room temperature for 2 h. TLC showed that the starting material was converted into a new main product. The volatiles were removed by rotary evaporation, and the residue was purified by silica gel column chromatography (eluent: EA:PE:Py = 1:4:0.2%) to elute the product. The product was concentrated under vacuum to give Phos-03-3E as a pale yellow oil (4.0 g, 59.8% yield).

$^1$H NMR (400 MHz, DMSO-d6) δ 7.42 (dd, 2H), 7.33 (t, 2H), 7.29-7.24 (m, 5H), 6.91 (d, 4H), 6.65 (ddd, 1H), 5.87 (s, 1H), 5.82 (d, 1H), 5.78 (s, 1H), 4.07 (q, 4H), 3.77 (s, 6H), 3.51 (dd, 2H), 3.12 (dd, 1H), 3.05-2.98 (m, 1H), 2.67 (d, 1H), 1.71-1.53 (m, 2H), 1.27-1.23 (m, 6H), 0.86 (s, 9H), 0.00 (d, 6H).

Ethanol (15 mL), pyridine (4.0 g, 51 mmol, 8.43 eq.), and Phos-03-3E (4.0 g, 6 mmol, 1.0 eq.) were added to a 250-mL flask. The reaction solution was stirred in a nitrogen atmosphere and cooled to 0 °C, and pyridine hydrofluoride (3.0 g, 0.03 mol, 5.0 eq.) was added to the flask. The reaction was stirred at room temperature for 2 h. TLC showed that the starting material was completely consumed. The volatiles were removed by rotary evaporation, and the residue was purified by silica gel column chromatography (eluent: EA:PE = 0%-80%, + 0.2% pyridine) to elute the byproducts. Subsequently, a 5%-10% solution of MeOH in DCM was used to elute the product. The product was concentrated under vacuum to give Phos-03-3F as a colorless oil (2.44 g, 71.6% yield).

$^1$H NMR: (400 MHz, DMSO-d6), δ ppm 7.37-7.35 (m, 2 H), 7.32-7.28 (m, 2 H), 7.24-7.20 (m, 5H), 6.89-6.87 (m, 4H),6.64-6.52 (m, 1H), 5.85-5.75 (m, 1H),4.42 (s, 1 H), 3.96-3.92 (m, 4 H), 3.74 (s,6 H),3.33-3.29 (m, 2 H), 3.06-2.93(m,2 H), 2.64 (s, 1 H), 1.58-1.48 (m, 2 H), 1.23-1.18 (m, 6 H).

Tetrahydrofuran (15 mL), triphenylphosphine (1.36 g, 5.2 mmol, 1.2 eq.), 3-benzoyluracil (1.12 g, 5.2 mmol, 1.2 eq.), diethyl azodicarboxylate (0.91 g, 5.2 mmol, 1.2 eq.), and Phos-03-3F (2.4 g, 4.3 mmol, 1.0 eq.) were sequentially added to a 100-mL flask in a nitrogen atmosphere, and the reaction was stirred overnight at room temperature. LC-MS showed that the main product was the desired product. The volatiles were removed by rotary evaporation, and the residue was purified by silica gel column chromatography (eluent: EA:PE = 0-80%, + 0.2% Py) to elute the byproducts. A 5%-10% solution of MeOH in dichloromethane was used to elute the product. The product was concentrated under vacuum to give Phos-03-3G as a yellow solid (3.08 g, 95.1% yield).

$^1$H NMR: (400 MHz, DMSO-d6), δ ppm 8.59-8.57 (m, 1 H), 7.96 (dd, 2 H), 7.86 (d, 1 H), 7.79-7.75 (m,1 H), 7. 60 (t, 2 H), 7.35 (d, 2 H), 7.31 (t, 2 H), 7.24 (d, 4 H), 6.89 (d, 4 H), 6.64-6.52 (m, 1 H), 5.94-5.82 (m, 2 H), 3.96-3.88 (m, 4 H), 3.74 (s,6 H), 3.68-3.63 (m, 2 H), 3.07-2.97 (m, 2 H), 2.56-2.53 (m,1 H), 1.81-1.77 (m, 2 H), 1.19 (t, 6 H).

Dichloromethane (50 mL) and Phos-03-3G (3.0 g, 4 mmol, 1.0 eq.) were added to a 250-mL flask. The reaction was stirred in a nitrogen atmosphere. Trichloroacetic acid (1.5 g, 13.2 mmol, 3.3 eq.) was then added, and the reaction was stirred at room temperature for 2 h. TLC showed that there was no residue of the starting material. The reaction mixture was then distilled, and the residue was purified by silica gel column chromatography (eluent: 0%-7% MeOH in DCM) to elute the product. The product was concentrated under vacuum to give Phos-03-3H as a colorless oil (1.3 g, 72.2% yield). HNMR showed that there was a residue of triethylamine. The residue was removed by reversed-phase flash column chromatography purification (C18, 40 g, 40-63 μm, eluent, $H_2O$/MeOH, 0-30% to elute the product).

$^1$H NMR: (400 MHz, DMSO-d6), δ ppm 7.96-7.94 (m, 2 H), 7.91 (d, 1 H), 7.81-7.76 (m, 1 H), 7.62-7.58 (m, 2 H), 6.60-6.48 (m, 1 H), 5.89-5.75 (m, 2 H),4.79 (t, 1 H), 3.97-3.89 (m, 4 H), 3.74-3.70 (m, 2 H),3.43 (t, 2 H), 2.40-2.35 (m,1 H), 1.88-1.69 (m, 2 H), 1.22-1.17 (m, 6 H).

Dichloromethane (6 mL), tetrazole (0.061 g, 0.88 mmol, 1.1 eq.), bis(diisopropylamino)(2-cyanoethoxy)phosphine (0.31 g, 1.04 mmol, 1.3 eq.), and Phos-03-3H (0.36 g, 0.8 mmol, 1.0 eq.) were added to a 100-mL flask in a nitrogen atmosphere, and the mixture was stirred at room temperature for 1.5 h. LC-MS showed that the main peak was the desired product. The reaction mixture was quenched with a saturated aqueous sodium bicarbonate solution (50 mL) and extracted twice with dichloromethane (50 mL). The organic phases were combined, dried, and concentrated to dryness, and the

residue was washed twice with petroleum ether (20 mL). The residue was further purified by silica gel column chromatography (eluent: MeOH:DCM = 0%-2%, + 0.2% TEA) to elute the product. The product was concentrated under vacuum at 35 °C to give phosphoramidite-03 as a colorless oil (0.32 g, 61.5% yield).

$^1$H NMR: (400 MHz, CD3CN), δ ppm 8.00-7.97 (m, 2 H), 7.80 -7.75 (m, 1 H), 7.62 -7.58 (m, 2 H), 7.53 -7.51 (m, 1 H), 6.66-6.55(m, 1 H),5.91-5.77 (m, 2 H), 4.02-3.97 (m, 4 H), 3.85-3.74 (m, 4 H), 3.73-3.59 (m, 3 H), 2.67-2.64 (m, 2 H), 2.63-2.57(m,1 H), 2.01-1.94 (m, 2 H), 1.87-1.82 (m, 1 H), 1.31-1.24 (m, 6 H), 1.20 (dd, 12 H).

**Preparation of phosphoramidite-13**

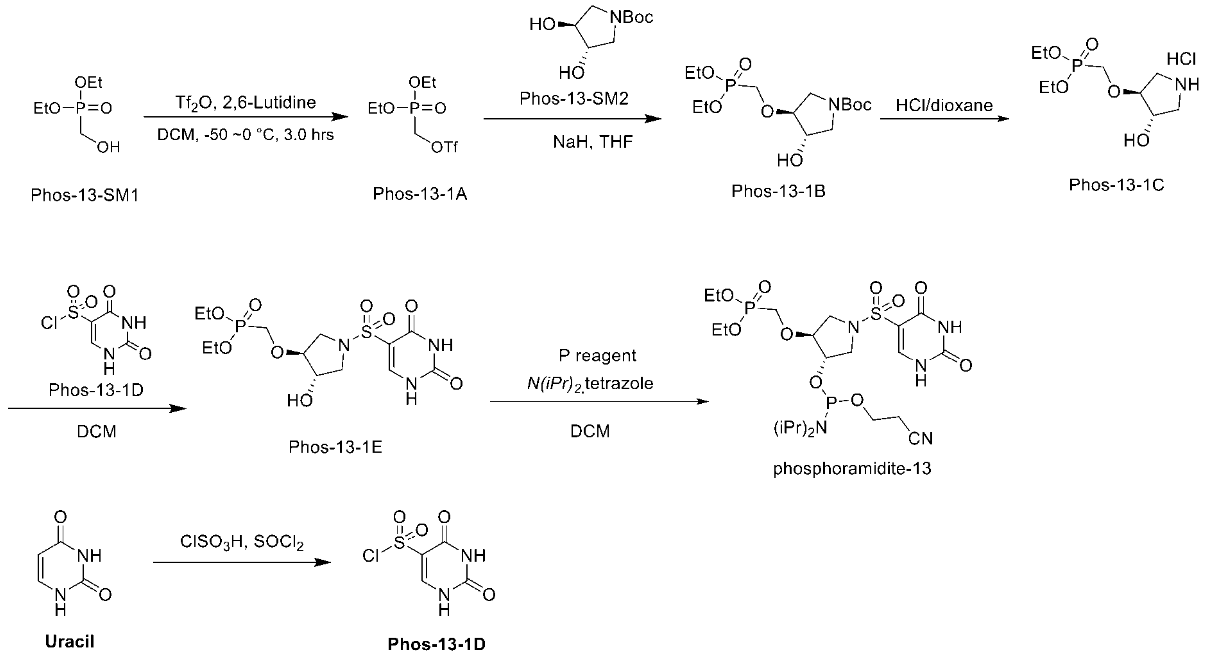


Trifluoroacetic anhydride (83.9 g, 297 mmol, 49.0 mL) was added dropwise to a solution of compound Phos-13-SM1 (50 g, 297 mmol) and 2,6-dimethylpyridine (48.4 g, 452 mmol, 52.6 mL) in dichloromethane (500 mL) at -50 °C. After 10 min of stirring, the reaction solution was heated to 40 °C and stirred for 3.0 h. TLC showed that the starting material was completely consumed and a new spot formed. The reaction mixture was triturated with isopropyl ether, and a precipitate formed. The precipitate was removed by filtration using diatomaceous earth, and the filtrate was washed with 1.0 M HCl and brine and then concentrated under vacuum to give Phos-13-1A as a brown oil (61 g, 203 mmol, 68.3% yield).

$^1$H NMR: EC10615-62-P1N (400 MHz, DMSO-$d_6$) δ ppm 4.78 (d, *J*=7.63 Hz, 2H), 4.07 - 4.17 (m, 4 H), 1.25 - 1.30 (m, 6 H).

Sodium hydride (60% purity, 787 mg, 19.7 mmol) was added in batches to stirred tetrahydrofuran (50 mL) at 0 °C in a nitrogen atmosphere, and compound Phos-13-SM2 (5.0 g, 24.6 mmol) was added to the solution. The mixture was stirred at 0 °C for 45 min. Then, Phos-13-1A (3.54 g, 11.8 mmol) was added to the above solution at 0 °C, and the mixture was stirred at that temperature for another 2.0 h. LC-MS showed that the reaction was complete. The reaction mixture was cooled to 0-5 °C, carefully added dropwise to a stirred saturated ammonium chloride solution (80 mL) (the temperature was kept below 0-5 °C), and then extracted with ethyl acetate (80 mL × 2). The organic phases were combined, washed with brine (80 mL), dried over anhydrous sodium bisulfate, and concentrated under reduced pressure to give a residue. The residue was purified by silica gel column chromatography (100-200 mesh silica gel; eluent: methanol:dichloromethane (1:100:1:30)) to give Phos-13-1B as a brown oil (6.0 g, 76.7% yield).

HCl/dioxane (4 M, 30.0 mL) was added to a solution of compound Phos-13-1B (6.0 g, 17.0 mmol) in dioxane (30 mL) at 0-5 °C, and the mixture was then stirred at 0-5 °C for 1.0 h. LC-MS showed that the reaction was complete. The mixture was triturated with isopropyl ether (300 mL × 2) and filtered to give a solid, and the solid was further purified by preparative HPLC to give compound Phos-13-1C as a brown oil (1.0 g, 23.2% yield).

$^1$H NMR: EC10615-84-all3 (400MHz,CHLOROFORM-*d*) δ ppm4.45 (d,*J*=3.25 Hz, 1 H), 4.11 - 4.24 (m, 5H), 3.98 (d, *J*=9.13 Hz, 2H), 3.48 -3.56(m, 1H), 3.37 - 3.43 (m, 2H), 3.23 - 3.28 (m, 1H), 1.30-1.40 (m, 6H).

Uracil (3.0 g, 26.8 mmol) and $SOCl_2$ (5.0 mL, 44.6 mmol) were added to a 100-mL reaction flask at 25 °C, and $ClSO_3H$ (8.0 g, 69.0 mmol) was added to the flask. The mixture was stirred at room temperature for 1.5 h, heated to 60 °C,

left to react for another 4.0 h, and then stirred at 75 °C for 7.0 h. TLC showed that the compound uracil had been completely consumed and a new spot formed. The reaction mixture was added to a mixture of ice and acetic acid (1:1). The mixture was filtered to give a precipitate, and the resulting filter cake was washed repeatedly with purified water and dried under vacuum to give Phos-13-1D as a white powder (1.0 g, 4.75 mmol, 17.7% yield).

$^{1}$H NMR: EC4783-422-P1B1 (400 MHz, DMSO-$d_6$) $\delta$ ppm 11.02 (br s, 1 H) 10.88 (br d, *J*=5.75 Hz, 1 H) 7.64 (d, *J*=6.00 Hz, 1 H).

A solution of compound Phos-13-1D (1.25 g, 5.92 mmol) in dichloromethane (5.0 mL) was added to a solution of compound Phos-13-1C (1.0 g, 3.95 mmol) and triethylamine (1.20 g, 11.9 mmol, 1.65 mL) in dichloromethane (10 mL) at 0-5 °C, and the mixture was then stirred at 25 °C for 1.0 h. LC-MS showed that compound Phos-13-1C had been completely consumed, and about 50.9% of the desired product was produced. The resulting reaction mixture was diluted with water (20 mL) and extracted with 30% $CF_3CH_2OH$/DCM (30 mL × 5). The combined organic layers were dried over anhydrous sodium sulfate and concentrated under vacuum to give a residue. The residue was purified by silica gel column chromatography (100-200 mesh silica gel; eluent: methanol:dichloromethane (1:30 to 1:8)) to give Phos-13-1E as a white solid (570 mg, 33.77% yield).

$^{1}$H NMR: EC12346-4-P1B1-C (400 MHz, CHLOROFORM-d) $\delta$ ppm 8.09 (s, 1 H), 4.21 - 4.25 (m, 1 H), 4.10 - 4.19 (m, 4 H), 3.82 - 3.98 (m, 3 H), 3.66 - 3.70 (m, 2 H), 3.58 (dd, *J*=10.88, 4.25 Hz, 1 H), 3.36 (d, *J*=11.01 Hz, 1 H), 1.33 - 1.35 (m, 6 H).

A solution of bis(diisopropylamino)(2-cyanoethoxy)phosphine (P reagent, 804 mg, 2.67 mmol, 0.85 mL) in dichloromethane (0.5 mL) was added to a solution of compound Phos-13-1E (380 mg, 0.89 mmol) and diisopropylamine tetrazolide (167 mg, 0.98 mmol) in dichloromethane (1.0 mL) in a nitrogen atmosphere, and the mixture was stirred at 40 °C for 1.0 h. LC-MS showed that compound Phos-13-1E was completely consumed, and detected the desired product. The resulting reaction mixture was cooled to -20 °C, poured into a cold (0-5 °C) saturated aqueous sodium bicarbonate solution (10 mL), and extracted with dichloromethane (10 mL × 2). The combined organic layers were washed with a cold (0-5 °C) saturated aqueous sodium bicarbonate solution/brine (5 mL/5 mL), dried over $Na_2SO_4$, and concentrated under vacuum to give a residue (~2.0 mL). The residue was triturated with isopropyl ether (20 mL × 2) to give a crude product, and the crude product was then purified by column chromatography (alkaline $Al_2O_3$, methanol:dichloromethane = 0/50 to 1/50, 0.1% TEA) to give phosphoramidite-13 as a white solid (95 mg, 17.0% yield).

$^{1}$H NMR: EC12346-6-P1Nre (400 MHz, DMSO-$d_6$) $\delta$ ppm 9.75 (br d, *J*=8.13 Hz, 1 H), 7.97 (d, *J*=4.88 Hz, 1 H), 4.16 - 4.25 (m, 1 H), 4.01 (quin, *J*=7.29 Hz, 5 H), 3.82 (br dd, *J*=7.25, 2.00 Hz, 2 H), 3.63 - 3.75 (m, 2 H), 3.44 - 3.58 (m, 4 H), 3.42 (br s, 1 H), 3.39 (br d, *J*=2.00 Hz, 1 H), 3.24 (d, *J*=11.38 Hz, 1 H), 2.76 (q, J=5.46 Hz, 2 H), 1.21 (t, J=7.07 Hz, 6 H), 1.07 - 1.16 (m, 12 H).

**Preparation of enantiomer phosphoramidite-15-1 and enantiomer phosphoramidite-15-2**

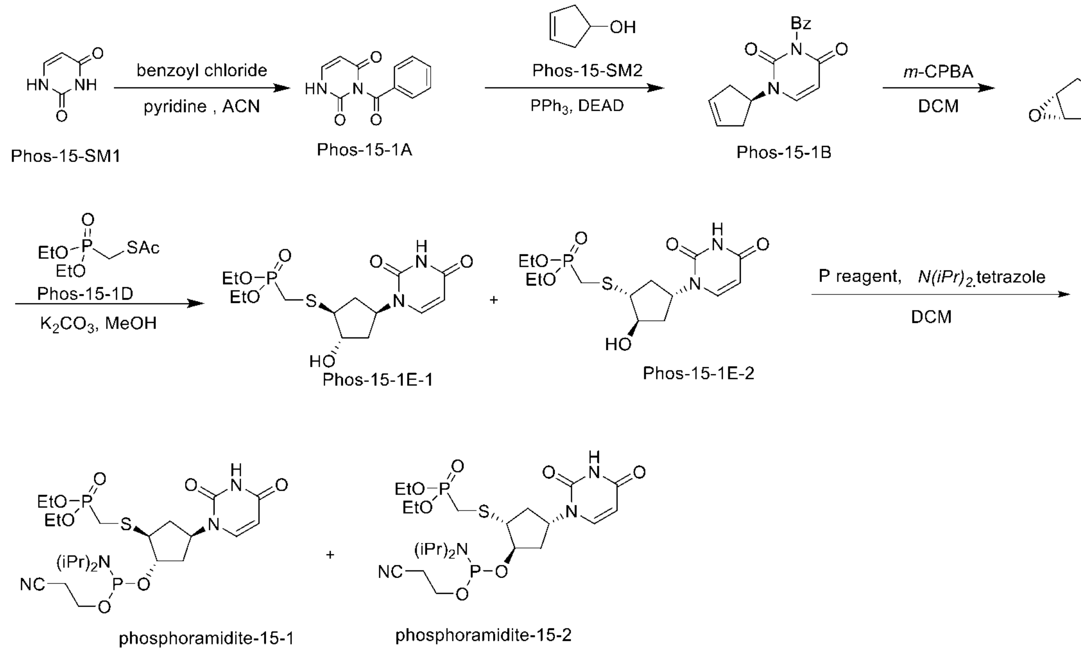


Phos-15-SM1 Phos-15-1A Phos-15-1B Phos-15-1C

Phos-15-1E-1 + Phos-15-1E-2

phosphoramidite-15-1 + phosphoramidite-15-2

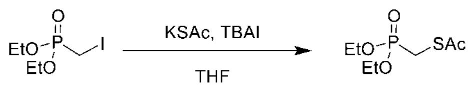


**Phos-15-SM3** **Phos-15-1D**

Benzoyl chloride (126 g, 893 mmol, 104 mL) was added to a solution of uracil (50.0 g, 446 mmol) in pyridine (735 g, 9.29 mol, 750 mL) and acetonitrile (1.50 L) at 0 °C, and the reaction solution was stirred at 20-25 °C for 12.0 h. TLC showed that the compound uracil had been completely consumed. The reaction mixture was concentrated under vacuum to give a residue, and the residue was diluted with cold water (1.0 L) and extracted with ethyl acetate (1.0 L × 3). The combined organic layers were washed with brine (500 mL) and dried over anhydrous sodium sulfate to give a residue, and the residue was purified by column chromatography ($SiO_2$, ethyl acetate/petroleum ether = 1/10 to 1/1) to give Phos-15-1A as a white solid (63 g, 65.3% yield).

$^1H$ NMR: EC4783-420-P1N (400 MHz, DMSO-$d_6$) $\delta$ ppm 7.96 (dd, *J*=8.4, 1.2 Hz, 2 H), 7.76 - 7.81 (m, 1 H), 7.67 (dd, *J*=7.6, 5.6 Hz, 1 H), 7.58 - 7.64 (m, 2 H), 5.75 (dd, *J*=7.6, 1.2 Hz, 1 H).

Triphenylphosphine (11.5 g, 43.9 mmol) and diethyl azodicarboxylate (7.64 g, 43.9 mmol, 7.98 mL) were added to a solution of Phos-15-SM2 (4.0 g, 47.6 mmol) and compound Phos-15-1A (7.91 g, 36.6 mmol) in tetrahydrofuran (80 mL), and the mixture was stirred at 20-25 °C for 16 h. LC-MS showed that compound Phos-15-1A was completely consumed. The reaction mixture was concentrated under reduced pressure to remove tetrahydrofuran. The residue was diluted with water (80 mL) and then extracted with ethyl acetate (80 mL × 3). The combined organic phases were washed with brine (80 mL), dried over anhydrous sodium sulfate, and concentrated under reduced pressure. The residue was purified by column chromatography ($SiO_2$, MeOH/DCM = 0/10 to 1/10) to give compound Phos-15-1B as a white solid (14 g, crude product).

A mixture of compound Phos-15-1B (7.0 g, 9.30 mmol) and m-chloroperoxybenzoic acid (2.27 g, 11.1 mmol, 85% purity) in dichloromethane (70 mL) was allowed to react at 0-5 °C for 16 h in a nitrogen atmosphere. TLC showed that compound Phos-15-1B had been completely consumed, and detected one main new spot with relatively low polarity. The pH of the reaction mixture was slowly adjusted to 7-8 with a saturated $NaHSO_3$ and $NaHCO_3$ solution (1:1), and the mixture was then extracted with ethyl acetate (70 mL × 3). The combined organic phases were washed with brine (700 mL), dried over anhydrous sodium sulfate, and concentrated under reduced pressure. The residue was purified by silica gel column chromatography (100-200 mesh silica gel; eluent: ethyl acetate:petroleum ether (1:30 to 1:1)) to give compound Phos-15-1C as a white solid (1.2 g, crude product).

KSAc (1.81 g, 15.8 mmol) and tetrabutylammonium iodide (TBAI, 531.4 mg, 1.44 mmol) were added to a solution of compound Phos-15-SM3 (4.0 g, 14.4 mmol) in tetrahydrofuran (24.0 mL), and the mixture was stirred at 70 °C for 4.0 h. LC-MS showed that the starting material Phos-15-SM3 was completely consumed, and detected one main peak with the molecular weight of the desired target molecule. The reaction mixture was cooled and concentrated under reduced pressure. The solid residue was removed by filtration using a short silica gel pad and rinsed with ethyl acetate. The filtrate

was concentrated under vacuum to give compound Phos-15-1D as a brown oil (3.50 g, 98.5% yield). Compound Phos-15-1D was used in the next step without further purification.

$^1H$ NMR: EC11950-13-P1B (400 MHz, DMSO-$d_6$) $\delta$ ppm 3.96 - 4.07 (m, 4H) 3.27 (d, $J$=14.0 Hz, 2H) 2.40 (s, 3H) 1.22 (t, $J$=7.2 Hz, 6H).

Potassium carbonate (1.11 g, 8.05 mmol) and compound Phos-15-1D (1.91 g, 8.45 mmol) were added to a solution of compound Phos-15-1C (1.20 g, 4.02 mmol) in ethanol (15.0 mL), and the mixture was stirred at 20-25 °C for 3.0 h. TLC showed that compound Phos-15-1C had been completely consumed, and detected one main new spot with relatively large polarity. The resulting reaction mixture was filtered, diluted with water (20 mL), and extracted with dichloromethane (20 mL × 3). The combined organic layers were washed with brine, dried over anhydrous sodium sulfate, and concentrated under vacuum to give a residue. The residue was purified by column chromatography ($SiO_2$, MeOH/DCM = 1/100 to 10/100) to give compound Phos-15-1E as a brown oil (1.00 g, 65.7% yield, a 1:1 mixture of enantiomers compound-1E-1 and compound-1E-2).

$^1H$ NMR: EC10615-82-P1N1 (400 MHz, DMSO-$d_6$) $\delta$ ppm 11.23 (br s, 1 H), 7.69 (d, $J$=8.0 Hz, 1 H), 5.58 (dd, $J$=8.0, 1.6 Hz, 1 H), 4.93 (q, $J$=8.8 Hz, 1 H), 3.96 - 4.17 (m, 5 H), 3.08 - 3.17 (m, 1 H), 3.03 (dd, $J$=14.0, 2.0 Hz, 2 H), 2.38 - 2.47 (m, 1 H), 2.04-2.07 (m, 1 H), 1.82 - 1.90 (m, 1 H), 1.56-1.59 (m, 1 H), 1.25 (t, $J$=6.8 Hz, 6 H).

Compound Phos-15-1E can be resolved into enantiomers Phos-15-1E-1 and Phos-15-1E-2 by chiral resolution (resolution conditions: DAICELCHIRALPAK AD 40 mm column, 140 mL/min, ethanol:carbon dioxide = 35:75). It will be appreciated that when enantiomer phosphoramidite-15-1 or enantiomer phosphoramidite-15-2 is desired, it can be obtained by using the corresponding enantiomer, Phos-15-1E-1 or Phos-15-1E-2, as the starting material and reacting it with P reagent.

A solution of bis(diisopropylamino)(2-cyanoethoxy)phosphine (P reagent, 956 mg, 3.17 mmol, 1.01 mL) in dichloromethane (0.5 mL) was added to a solution of compound Phos-15-1E (400 mg, 1.06 mmol) and diisopropylamine tetrazolide (199 mg, 1.16 mmol) in dichloromethane (4.0 mL) at room temperature in a nitrogen atmosphere, and the mixture was then stirred at 40 °C for 1.0 h. LC-MS showed that compound Phos-15-1E had been completely consumed. LC-MS showed several new peaks and detected about 80% of the desired compound. The resulting reaction mixture was cooled to -20 °C, poured into a cold (0-5 °C) saturated aqueous sodium bicarbonate solution (10 mL), and extracted with dichloromethane (10 mL × 2). The combined organic layers were washed with a cold (0-5 °C) saturated aqueous sodium bicarbonate solution/brine (5 mL/5 mL), dried over anhydrous sodium sulfate, and concentrated under vacuum to give a residue (~2.0 mL). The residue was purified by column chromatography (alkaline $Al_2O_3$, MeOH/DCM = 1/80 to 1/40, 0.1% $Et_3N$) to give phosphoramidite-15 as a colorless oil (350 mg, 0.6 mmol, 57.2% yield, a 1:1 mixture of enantiomer phosphoramidite-15-1 and enantiomer phosphoramidite-15-2).

Enantiomer phosphoramidite-15-1 or enantiomer phosphoramidite-15-2 can be obtained by using the corresponding Phos-15-1E-1 or Phos-15-1E-2, obtained by SFC separation and purification, as the starting material and subjecting it to the same process as above.

$\delta$ ppm 11.23 (br s, 1 H), 7.70 (d, $J$=8.0 Hz, 1 H), 5.55 - 5.60 (m, 1 H), 4.89 (q, $J$=8.4 Hz, 1H), 4.29 - 4.42 (m, 1H), 3.99 - 4.09 (m, 4H), 3.65 - 3.84 (m, 2H), 3.53 - 3.62 (m, 2H), 3.35 - 3.41 (m, 1H), 3.02 (dd, $J$=14.0, 8.0 Hz, 2H), 2.76-2.79 (m, 2H), 2.40 - 2.49 (m, 1H), 2.15 - 2.25 (m, 1H), 1.95 - 2.07 (m, 1H), 1.65 - 1.75 (m, 1H), 1.23-1.26 (m, 6H) 1.12 - 1.21 (m, 12H).

The specific chiral preparation method for Phos-15-1E-1 or Phos-15-1E-2 was as follows:

System: Waters SFC 150
Column name: DAICELCHIRALCEL®AD
Column model: 250 × 50 mm 10 μm
Mobile phase A: Supercritical $CO_2$
Mobile phase B: EtOH
Wavelength: 214 nm
Flow rate: 140 mL/min
Column temperature: RT
Injection volume: 7.0 mL; cycle time: 10.0 min
Solvents: supercritical $CO_2$ : food grade EtOH : redistilled grade;

The single crystal X-ray diffraction pattern of Phos-15-1E-2 (diethyl ((((1R,2R,4R)-4-(2,4-dioxo-3,4-dihydropyrimidin-1(2H)-yl)-2-hydroxycyclopentyl)thio)methyl)phosphonate) is shown in FIG. 1.

**Preparation of enantiomer phosphoramidite-42-1 and enantiomer phosphoramidite-42-2**

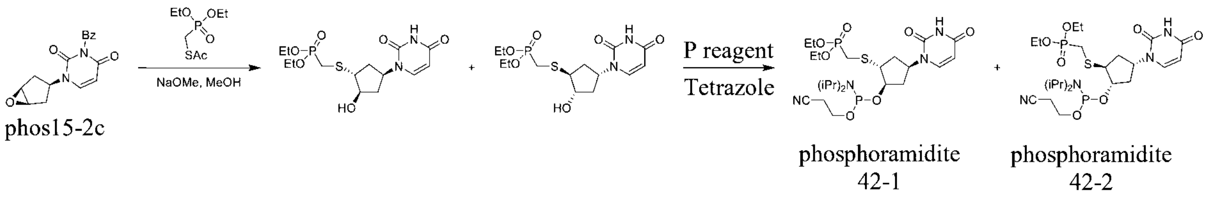


phos15-2c

phosphoramidite 42-1

phosphoramidite 42-2

The preparation method for enantiomer phosphoramidite-42-1 and enantiomer phosphoramidite-42-2 was the same as that for enantiomer phosphoramidite-15-1 and enantiomer phosphoramidite-15-2, with the difference being that a different stereoisomeric intermediate phos12-2C was used.

Preparation of enantiomer phosphoramidite-11-1 and enantiomer phosphoramidite-11-2

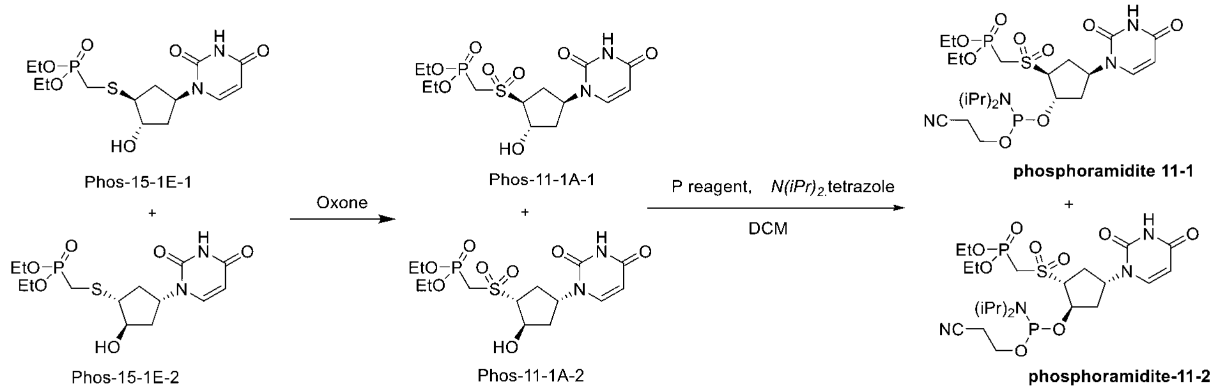


Phos-15-1E-1 + Phos-15-1E-2

Phos-11-1A-1 + Phos-11-1A-2

phosphoramidite 11-1 + phosphoramidite-11-2

Ethanol (15 mL) and the above compound Phos-15-1E (0.53 g, 1.0 eq, a 1:1 mixture of enantiomers Phos-15-1E-1 and Phos-15-1E-2) were added to a 100-mL flask at room temperature, and an aqueous solution (10 mL) of potassium peroxymonosulfate (1.72 g, 2.0 eq.) was added in batches. The mixture was then stirred overnight at room temperature. TLC analysis (MeOH:DCM = 1:10, Rf = 0.4) showed that a new spot formed and compound Phos-15-1E was completely consumed. The mixture was poured into a saturated aqueous sodium bicarbonate solution (20 mL) and extracted with DCM:$CF_3CH_2OH$ (30 mL × 5). The combined organic layers were washed with saturated brine (50 mL × 2), dried over anhydrous sodium sulfate, and concentrated under reduced pressure to give Phos-11-1A as a yellow oil (0.5 g, 87% yield, a 1:1 mixture of enantiomers Phos-11-1A-1 and Phos-11-1A-22).

Dichloromethane (5 mL), compound Phos-11-1A (0.3 g, 1.0 eq.), and tetrazole (56 mg, 1.1 eq.) were added to a 100-mL flask at room temperature in a nitrogen atmosphere, and a solution of bis(diisopropylamino)(2-cyanoethoxy) phosphine (0.33 g, 1.5 eq.) in dichloromethane (1 mL) was then added dropwise. The reaction solution was stirred at room temperature for 1 h. TLC analysis (MeOH:DCM = 1:10, Rf = 0.6) showed that a new spot formed and compound Phos-11-1A was completely consumed. The mixture was poured into a saturated aqueous sodium bicarbonate solution (20 mL) and extracted with dichloromethane (30 mL × 3). The combined organic layers were washed with saturated brine (50 mL), dried over anhydrous sodium sulfate, and concentrated under reduced pressure. The crude product of phosphoramidite-11 was purified by silica gel column chromatography (eluent: MeOH:DCM = 0:100 to 2:100; 1% $Et_3N$) to give phosphoramidite-11 as a yellow oil (0.2 g, 45% yield, a 1:1 mixture of enantiomers phosphoramidite-11-1 and phosphoramidite-11-2).

$^1H$ NMR (400 MHz, $CD_3CN$) $\delta$ 7.40 (d, $J$ = 8.0, 1H), 5.58 (d, $J$ = 8.0, 1H), 5.01-5.04 (m, 1H), 4.84-4.87 (m, 1H), 4.14-4.18 (m, 4H), 3.76-3.86 (m, 4H), 3.59-3.65 (m, 2H), 2.73-2.77 (m, 2H), 2.66-2.69 (m, 2H), 2.18-2.26 (m, 3H), 1.28-1.32 (m, 6H), 1.17-1.21 (m, 12H).

$^{31}P$ NMR (400 MHz, $CD_3CN$) $\delta$ 148.45, 149.21.

Enantiomer phosphoramidite-11-1 or enantiomer phosphoramidite-11-2 can be obtained by using the corresponding Phos-15-1E-1 or Phos-15-1E-2, obtained by SFC separation and purification, as the starting material and subjecting it to the above 2 steps.

**Preparation of phosphoramidite-18**

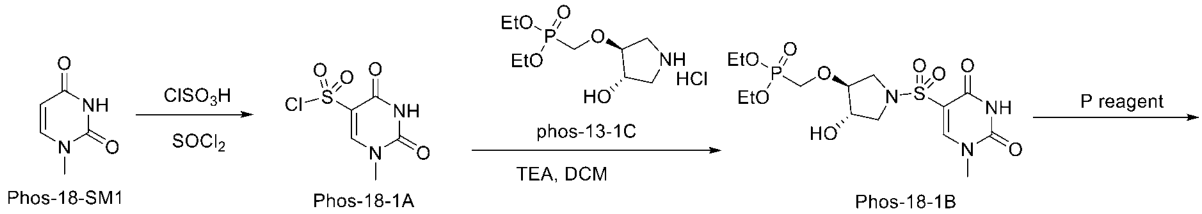


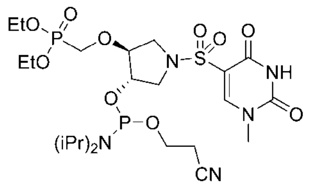

phosphoramidite-18

1-Methyluracil (1.0 g, 7.9 mmol) and $SOCl_2$ (2.36 g, 19.8 mmol) were added to a 100-mL reaction flask, and the mixture was stirred at 25 °C for 1.5 h. After $ClSO_3H$ (3.67 g, 31.7 mmol) was added, the reaction solution was stirred at 75 °C for 16 h. TLC showed that 1-methyluracil had been completely consumed and a new spot formed. The reaction mixture was added to a mixture of ice and glacial acetic acid (1:1), and the mixture was filtered. The filter cake was repeatedly washed with deionized water and dried under vacuum to give intermediate Phos-18-1A as a gray powder (1.0 g, 90% yield).

$^1H$ NMR (400 MHz, DMSO): δ 14.25 (s, 1H), 11.26 (s, 1H), 7.99 (s, 1H), 3.27 (s, 3H).

A solution of Phos-18-1A (340 mg, 1.52 mmol) in tetrahydrofuran (10 mL) was added to a solution of Phos-13-1C (384 mg, 1.52 mmol) and triethylamine (460 mg, 4.56 mmol) in dichloromethane (30 mL) at 0-5 °C, and the mixture was left to react at 25 °C for 1.0 h. LC-MS showed that Phos-13-1C was completely consumed. The resulting reaction mixture was diluted with water (20 mL) and extracted with 30% $CF_3CH_2OH$/DCM (30 mL × 5). The combined organic layers were dried over anhydrous sodium sulfate and concentrated under vacuum to give a residue. The residue was purified by silica gel column chromatography (eluent: 0% to 5% MeOH/DCM) to give Phos-18-1B as a colorless paste (400 mg, 60% yield). LC-MS: $[M+H^+]$ = 442

Tetrazole (51 mg, 0.726 mmol) and bis(diisopropylamino)(2-cyanoethoxy)phosphine (P reagent, 327.8 mg, 1.09 mmol) were added to a solution of Phos-18-1B (400 mg, 0.907 mmol) in dry dichloromethane (10 mL) in a nitrogen atmosphere, and the reaction was stirred at 25 °C for 1.0 h. LC-MS showed that the starting material was completely consumed. The resulting reaction mixture was washed with a saturated aqueous sodium bicarbonate solution (10 mL × 2) and brine (5 mL). The organic phase was dried and concentrated to give a crude product, and the crude product was purified by flash silica gel column chromatography (eluent: 0% to 5% MeOH/DCM, containing 1% TEA) to give phosphoramidite-18 as a white solid (400 mg, 68.8% yield).

$^1H$ NMR (400 MHz, $CH_3CN$) δ 9.29 (s, 1H), 8.15 (d, *J*= 3.6 Hz, 1H), 4.44 - 4.31 (m, 1H), 4.09 (dd, *J*= 15.0, 7.4 Hz, 4H), 3.90 - 3.73 (m, 4H), 3.72 - 3.56 (m, 5H), 3.49 (dd, *J* = 31.5, 11.2 Hz, 1H), 3.36 (d, *J*= 2.3 Hz, 3H), 2.75 - 2.62 (m, 2H), 1.36 - 1.25 (m, 6H), 1.19 (dd, *J*= 9.1, 5.3 Hz, 12H).

$^{31}P$-NMR (162 MHz, DMSO-$d_6$) 6 ppm 146.99 - 147.13 (d, 1 P), 20.58 - 20.76 (s, 1 P).

Preparation of phosphoramidite-19

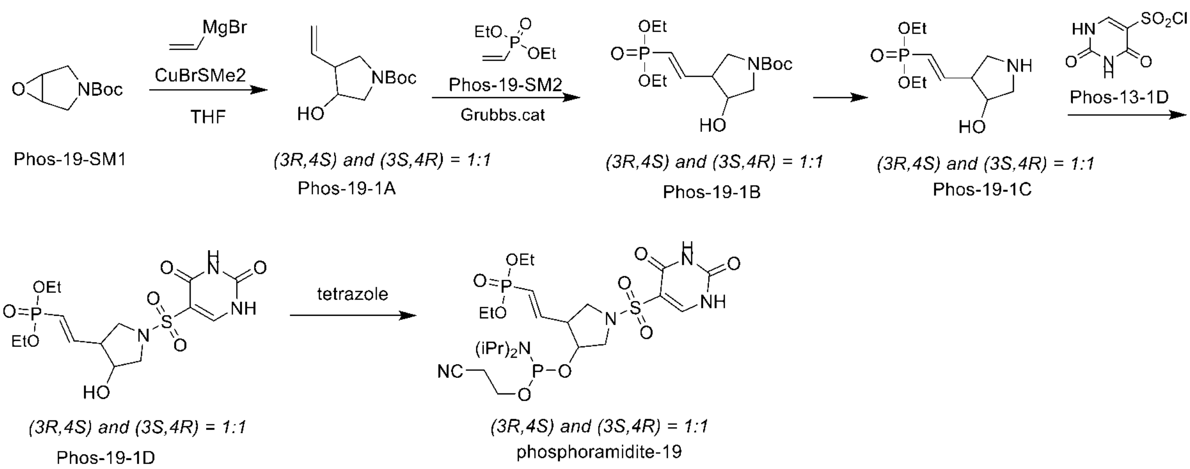


Tetrahydrofuran (60 mL), Phos-19-SM1 (10 g, 1.0 eq.), and $CuBrSMe_2$ were added to a 250-mL flask at room temperature. After the mixture was cooled to -70 °C, vinylmagnesium bromide (216 mL, 4.0 eq.) was added dropwise, and the mixture was then slowly warmed to -10 °C over 2 h. TLC analysis (petroleum ether:ethyl acetate = 2:1, Rf = 0.4) showed that a new spot formed and Phos-19-SM1 was completely consumed. Citric acid (150 mL, 10% in water) was added dropwise to the reaction mixture at - 10 °C, and extraction was performed with ethyl acetate (100 mL × 3). The combined organic layers were washed with saturated brine (100 mL), dried over anhydrous sodium sulfate, and concentrated under reduced pressure to give Phos-19-1A as a brown oil (11 g, 95% yield).

Phos-19-1A contained a pair of enantiomers (3R,4S):(3S,4R) = 1:1, including

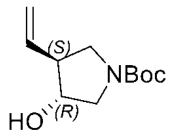

and

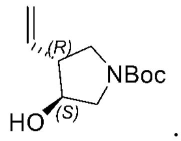

.

It will be appreciated that the corresponding chirally pure compounds of the following intermediates and phosphoramidite-19 can be obtained as needed by using the Phos-19-1A racemate or a corresponding chirally pure enantiomer as the starting material.

Dichloromethane (50 mL), Phos-19-1A (5 g, 1.0 eq.), and Phos-19-SM2 (10.8 g, 3.0 eq.) were added to a 100-mL flask at room temperature in a nitrogen atmosphere, and a Grubbs catalyst (1 g, 0.05 eq.) was then added under $N_2$. The mixture was heated overnight at reflux. TLC analysis of the mixture (ethyl acetate:petroleum ether = 1:1, Rf = 0.25) showed that a new spot formed and Phos-19-2 was completely consumed. The solvent was removed, and the residue was purified by silica gel column chromatography (eluent: MeOH:DCM = 0:100 to 5:95) to give Phos-19-1B as a brown oil (4.5 g, 55% yield).

Ethyl acetate (15 mL) and Phos-19-1B (1.5 g, 1.0 eq.) were added to a 100-mL flask at room temperature in a nitrogen atmosphere, and HCl/EtOAc (15 mL, 4 M) was then added dropwise. The mixture was stirred overnight at room temperature. LC-MS analysis showed that Phos-19-1B was completely consumed. The solvent was removed to give Phos-19-1C as a brown solid (1.5 g, crude product).

MS (M+H) = 250.1.

Tetrahydrofuran (15 mL) and Phos-19-1C (1.5 g, 1.0 eq.) were added to a 100-mL flask at room temperature in a nitrogen atmosphere, and triethylamine (1.52 g, 3.5 eq.) and compound Phos-13-1D (0.72 g, 0.8 eq.) were then added. The mixture was stirred overnight at room temperature. LC-MS showed that Phos-19-1C was completely consumed. The reaction solution was poured into citric acid (15 mL, 10% in water) and extracted with dichloromethane (30 mL × 5). The extract was dried over anhydrous sodium sulfate and concentrated under reduced pressure. The residue was purified by silica gel column chromatography (eluent: MeOH:DCM = 0:100 to 10:90) to give Phos-19-1D as a brown oil (1 g, 39%

yield).

MS (M+H) = 424.1.

Dichloromethane (5 mL), Phos-19-1D (0.5 g, 1.0 eq.), and tetrazole (83 mg, 1.1 eq.) were added to a 100-mL flask at room temperature in a nitrogen atmosphere, and a solution of bis(diisopropylamino)(2-cyanoethoxy)phosphine (0.46 g, 1.5 eq.) in dichloromethane (1 mL) was then added dropwise. The reaction mixture was stirred at room temperature for 1 h. TLC (MeOH:DCM = 1:10, Rf = 0.65) showed that a new spot formed and Phos-19-1D was completely consumed. The mixture was poured into a saturated aqueous sodium bicarbonate solution (20 mL) and extracted with dichloromethane (20 mL × 3). The combined organic layers were washed with saturated brine (50 mL), dried over anhydrous sodium sulfate, and concentrated under reduced pressure. The crude product was purified by silica gel column chromatography (eluent: MeOH:DCM = 0:100 to 5:95; 1% $Et_3N$) to give phosphoramidite-19 as a brown oil (0.27 g, 36% yield).

$^1H$ NMR (400 MHz, DMSO) δ 11.21 (s,1H), 7.97 (s,1H), 6.45-6.57 (m, 1H), 5.88-5.98 (m, 1H), 4.18-4.23 (m, 1H), 3.91-3.96 (m, 4H), 3.71-3.74 (m, 3H), 3.57-3.62 (m, 1H), 3.49-3.53 (m, 2H), 3.15-3.24 (m, 1H), 2.91-3.01 (m, 1H), 2.83-2.85 (m, 2H), 2.74-2.78 (m, 2H), 1.18-1.23 (m, 6H), 1.06-1.12 (m, 12H).

$^{31}P$ NMR (400 MHz, DMSO) δ 147.51, 17.01, 16.88.

Preparation of phosphoramidite-43

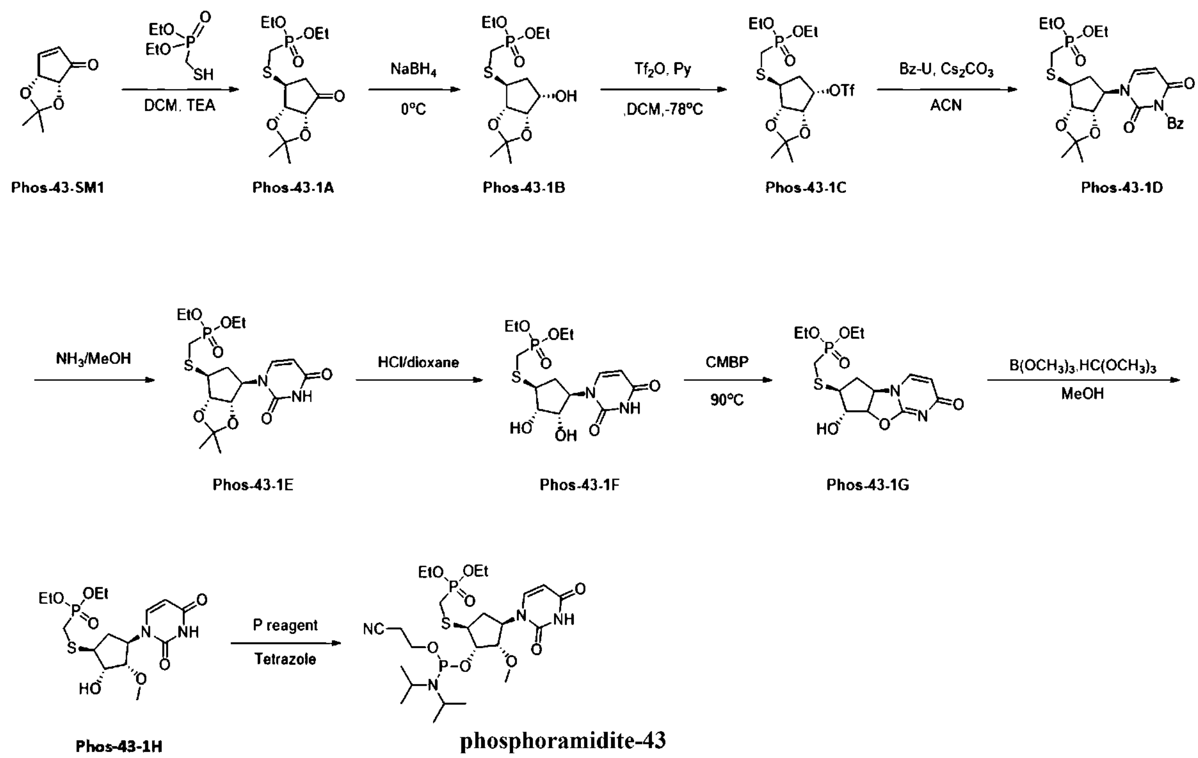


Phos-43-SM1 Phos-43-1A Phos-43-1B Phos-43-1C Phos-43-1D

Phos-43-1E Phos-43-1F Phos-43-1G

Phos-43-1H phosphoramidite-43

(3aR,6aR)-2,2-Dimethyltetrahydro-3aH-cyclopenta[d][1,3]dioxol-4(6aH)-one (16.2 g, 105 mmol, 1.0 eq), diethyl (mercaptomethyl)phosphonate (19.3 g, 105 mmol, 1.0 eq), and dichloromethane (200 mL) were added to a 500-mL flask. The mixture in the flask was stirred in a nitrogen atmosphere and cooled to 0-5 °C, and triethylamine (1.06 g, 10.5 mmol, 0.1 eq) was then added dropwise. After the dropwise addition, the mixture was warmed to 25 °C and stirred overnight in a nitrogen atmosphere. LCMS analysis showed that the reaction was complete. The reaction solution was concentrated under vacuum to give a crude product. The crude product was purified by flash column chromatography (eluent: EA:DCM = 0%-15%) to elute the product. The product was concentrated under vacuum to give Phos-43-1A as a pale yellow oil (25 g, 70.3% yield).

LCMS: M+H = 339.5

Phos-43-1A (25 g, 73.9 mmol, 1.0 eq) and ethanol (250 mL) were added to a 500-mL flask. The mixture in the flask was stirred in a nitrogen atmosphere and cooled to 0-5 °C, and sodium borohydride (3.1 g, 81.3 mmol, 1.1 eq) was then added in batches. After the addition, the mixture was stirred at 0-5 °C for 0.5 h. LCMS analysis showed that the reaction was complete. Ice water (200 mL) was added dropwise to the reaction solution, and the mixture was stirred for 10 min and then extracted twice with dichloromethane (500 mL). The combined organic phases were dried over anhydrous sodium sulfate

and concentrated under vacuum to give a crude product. The crude product was purified by flash column chromatography (eluent: MeOH:DCM = 0%-5%) to elute the product. The product was concentrated under vacuum to give Phos-43-1B as a pale yellow oil (24.5 g, 97.4% yield).

LCMS: M+H = 341.5.

$^1H$ NMR: (400 MHz, CD3CN), δ ppm 4.67-4.65 (d, *J*=8.0, 1 H), 4.47-4.42 (m, 2 H), 4.08-4.01 (m, 5 H), 3.27-3.25 (m, 1 H), 2.97-2.93 (d, *J*=16.0, 2 H), 2.03-1.99 (m, 1 H), 1.73-1.71 (m, 1 H), 1.38 (s, 3 H), 1.26-1.22 (m, 9 H).

Phos-43-1B (10 g, 29.4 mmol, 1.0 eq), pyridine (7 g, 88.1 mmol, 3.0 eq), and dichloromethane (100 mL) were added to a 250-mL flask. The mixture in the flask was stirred in a nitrogen atmosphere and cooled to -78 °C, and trifluoromethanesulfonic anhydride (12.4 g, 44.1 mmol, 1.5 eq) was then added dropwise. After the dropwise addition, the mixture was stirred at -78 °C for 3 h in a nitrogen atmosphere. LCMS analysis showed that the reaction was complete. The reaction solution was poured into 50 mL of ice water and then extracted twice with dichloromethane (100 mL). The combined organic phases were dried over anhydrous sodium sulfate and concentrated under vacuum to give Phos-43-1C (crude product), which was directly used in the next step.
LCMS: M+H = 473.4

Phos-43-1C (16 g, 33.8 mmol, 1.0 eq), 3-benzoyluracil (8.8 g, 40.6 mmol, 1.2 eq), cesium carbonate (22 g, 67.7 mmol), and acetonitrile (200 mL) were added to a 500-mL flask. The mixture in the flask was stirred overnight at 25 °C in a nitrogen atmosphere. LCMS analysis showed that the reaction was complete. The reaction solution was filtered, and the filtrate was concentrated under vacuum to give a crude product. The crude product was purified by flash column chromatography (eluent: MeOH:DCM = 0%-5%) to elute the product. The product was concentrated under vacuum to give Phos-43-1D as a brown oil (18 g, 98.7% yield).
LCMS: M+H = 539.4

Phos-43-1D (18 g, 33.4 mmol, 1.0 eq) and methanol (180 mL) were added to a 500-mL flask. A solution of ammonia in methanol (180 mL) was added dropwise to the flask in a nitrogen atmosphere. After the dropwise addition, the mixture was stirred at 25 °C for 5 h in a nitrogen atmosphere. LCMS analysis showed that the reaction was complete. The reaction solution was concentrated under vacuum to give Phos-43-1E (crude product), which was directly used in the next step.
LCMS: M+H = 435.4

Phos-43-1E (14.5 g, 33.4 mmol, 1.0 eq) and dioxane (180 mL) were added to a 500-mL flask. A solution of hydrochloric acid in dioxane (4 M, 180 mL) was added dropwise, and the mixture in the flask was stirred overnight at 25 °C in a nitrogen atmosphere. LCMS analysis showed that the reaction was complete. The reaction solution was concentrated under vacuum to give a crude product. The crude product was purified by flash column chromatography (eluent: MeOH:DCM = 0%-10%) to elute the product. The product was concentrated under vacuum to give Phos-43-1F as a white solid (5.2 g, 39.5% yield).
LCMS: M+H = 395.4

Phos-43-1F (5.2 g, 13.2 mmol, 1.0 eq), toluene (100 mL), and acetonitrile (20 mL) were added to a 250-mL flask, and cyanomethylenetri(n-butyl)phosphorane (6.4 g, 26.5 mmol, 2.0 eq) was then added. The mixture in the flask was stirred at 90 °C for 48 h in a nitrogen atmosphere. LCMS analysis showed that the reaction was complete. The reaction solution was concentrated under vacuum to give a crude product. The crude product was purified by flash column chromatography (eluent: MeOH:DCM = 0%-10%) to elute the product. The product was concentrated under vacuum to give Phos-43-1G as a white solid (3.5 g, 70.5% yield).
LCMS: M+H = 377.3

Phos-43-1G (2.0 g, 5.3 mmol, 1.0 eq), absolute methanol (20 mL), trimethyl borate (1.1 g, 10.6 mmol, 2.0 eq), methyl orthoformate (0.56 g, 5.3 mmol, 1.0 eq), and sodium bicarbonate (44.5 mg, 0.52 mmol, 0.2 eq) were added to a 250-mL sealed reactor. The above mixture was heated to 120 °C and stirred for 48 h. The sealed reactor was cooled to room temperature. LCMS analysis showed that the reaction was complete. The reaction solution was concentrated under vacuum to give a crude product. The crude product was purified by flash column chromatography (eluent: MeOH:DCM = 0%-10%) to elute the product. The product was concentrated under vacuum to give Phos-43-1H as a white solid (1.3 g, 60% yield).
LCMS: M+H = 409.4

Phos-43-1H (0.6 g, 1.47 mmol, 1.0 eq) and anhydrous dichloromethane (10 mL) were added to a 50-mL flask, and tetrazole (0.13 g, 1.76 mmol, 1.2 eq) and bis(diisopropylamino)(2-cyanoethoxy)phosphine (0.66 g, 2.2 mmol, 1.5 eq) were sequentially added. The above mixture was stirred at 25 °C for 1 h in a nitrogen atmosphere. LCMS analysis showed that the reaction was complete. The reaction solution was poured into an aqueous sodium bicarbonate solution and extracted twice with dichloromethane (20 mL). The organic phases were combined, dried over anhydrous sodium sulfate, and then concentrated under vacuum to give a crude product. The crude product was purified by flash silica gel column chromatography (eluent: DCM:MeOH:TEA = 0%-5% + 0.2% TEA) to elute the product. The product was concentrated under vacuum at 35 °C to give phosphoramidite-43 as a colorless oil (0.89 g, 100% yield).
LCMS: M+H = 609.6

$^1H$ NMR: (400 MHz, CD3CN), δ ppm 8.97 (s, 1 H), 7.43-7.41 (d, J=8.0, 1 H), 5.61-5.59 (d, J=8.0, 1 H), 4.76-4.69 (m, 1 H), 4.44-4.34(m, 1 H), 4.14-4.04 (m, 5 H), 3.90-3.81 (m, 2 H), 3.69-3.62 (m, 2 H), 3.46-3.44 (m, 1 H), 3.36-3.33 (d, J=12.0, 3 H), 2.97-2.87 (m, 2 H), 2.77-2.65(m,3 H), 1.67-1.56 (m, 1 H), 1.32-1.27 (m, 6 H), 1.24-1.18 (m, 12 H).

31P NMR: (400 MHz, CD3CN), δ ppm 150.03, 148.62; 23.44, 23.24.

Phosphoramidite-47 was prepared using the same method as phosphoramidite-43, with the difference being that the starting material 5-methyluracil was used as the nucleobase.

Preparation of phosphoramidite-45

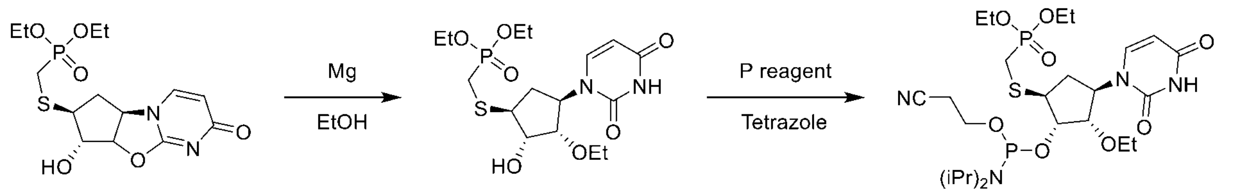


Phos-43-1G Phos-45-1A phosphoramidite-45

Magnesium turnings (0.26 g, 10.6 mmol, 10.0 eq) and absolute ethanol (40 mL) were added to a 100-mL sealed reactor, and the above mixture was heated to 90 °C and stirred for 18 h. The sealed reactor was cooled to room temperature, and Phos-43-1G (0.4 g, 1.06 mmol, 1.0 eq) was added. The mixture was heated to 90 °C and stirred for 18 h. The sealed reactor was cooled to room temperature. LCMS analysis showed that the reaction was not complete-about 50% was converted to Phos-45-1A. The reaction solution was concentrated under vacuum to give a crude product. The crude product was purified by flash column chromatography (eluent: MeOH:DCM = 0%-8%) to elute the product. The product was concentrated under vacuum to give Phos-45-1A as a pale yellow oil (0.13 g, 29% yield).
LCMS: M+H = 423.4

Phos-45-1A (0.11 g, 0.26 mmol, 1.0 eq) and anhydrous dichloromethane (3 mL) were added to a 50-mL flask, and tetrazole (22 mg, 0.31 mmol, 1.2 eq) and bis(diisopropylamino)(2-cyanoethoxy)phosphine (0.12 g, 0.4 mmol, 1.5 eq) were sequentially added. The above mixture was stirred at 25 °C for 1 h in a nitrogen atmosphere. LCMS analysis showed that the reaction was complete. The reaction solution was poured into an aqueous sodium bicarbonate solution and extracted twice with dichloromethane (10 mL). The organic phases were combined, dried over anhydrous sodium sulfate, and then concentrated under vacuum to give a crude product. The crude product was purified by flash silica gel column chromatography (eluent: DCM:MeOH:TEA = 0%-3% + 0.2% TEA) to elute the product. The product was concentrated under vacuum at 35 °C to give phosphoramidite Phos-45 as a yellow oil (0.1 g, 61.7% yield).

LCMS: M+H = 623.5,
$^1H$ NMR: (400 MHz, CD3CN), δ ppm 7.42-7.40 (d, J=8.0, 1 H), 5.61-5.59 (d, J=8.0, 1 H), 4.73-4.68 (m, 1 H), 4.34-4.30(m, 1 H), 4.12-4.06 (m, 5 H), 3.88-3.83 (m, 2 H), 3.68-3.65 (m, 2 H), 3.58-3.42 (m, 2 H), 2.97-2.86 (m, 3 H), 2.72-2.62(m,3 H), 1.65-1.53 (m, 1 H), 1.32-1.17 (m, 18 H), 1.13-1.10 (t, J=6.8, 3 H).
$^{31}P$ NMR: (400 MHz, CD3CN), δ ppm 149.85, 148.50; 23.46, 23.25.

**Preparation of phosphoramidite-46**

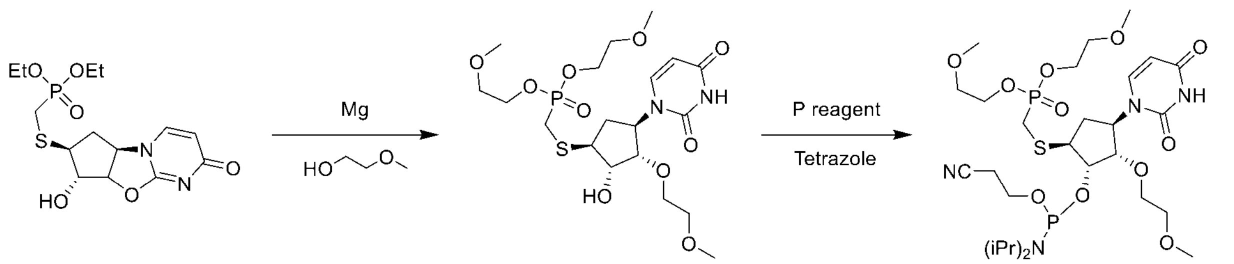


Phos-43-1G Phos-46-1A phosphoramidite-46

Magnesium turnings (0.48 g, 20.0 mmol, 15.0 eq) and anhydrous ethylene glycol monomethyl ether (50 mL) were

added to a 100-mL sealed reactor, and the above mixture was heated to 90 °C and stirred for 1 h. The sealed reactor was cooled to room temperature, and Phos-43-1G (0.5 g, 1.33 mmol, 1.0 eq) was added. The mixture was heated to 90 °C and stirred for 18 h. The sealed reactor was cooled to room temperature. LCMS analysis showed that the starting material of the reaction disappeared completely. The reaction solution was transferred to a flask, and the pH was adjusted to 6 by adding 0.5 N dilute hydrochloric acid dropwise at 0 °C. The mixture was extracted 5 times with dichloromethane (50 mL). The organic phases were combined, dried over anhydrous sodium sulfate, and then concentrated under vacuum to give a crude product. The crude product was purified by flash column chromatography (eluent: MeOH:DCM = 0%-10%) to elute the product. The product was concentrated under vacuum to give Phos-46-1A as a pale yellow oil (0.13 g, 20% yield).

LCMS: M+H = 513.4.

Phos-46-1A (0.1 g, 0.19 mmol, 1.0 eq) and anhydrous dichloromethane (3 mL) were added to a 50-mL flask, and tetrazole (16 mg, 0.23 mmol, 1.2 eq) and bis(diisopropylamino)(2-cyanoethoxy)phosphine (0.09 g, 0.3 mmol, 1.5 eq) were sequentially added. The above mixture was stirred at 25 °C for 1 h in a nitrogen atmosphere. LCMS analysis showed that the reaction was complete. The reaction solution was poured into an aqueous sodium bicarbonate solution and extracted twice with dichloromethane (10 mL). The organic phases were combined, dried over anhydrous sodium sulfate, and then concentrated under vacuum to give a crude product. The crude product was purified by flash silica gel column chromatography (eluent: DCM:MeOH:TEA = 0%-5% + 0.2% TEA) to elute the product. The product was concentrated under vacuum at 35 °C to give phosphoramidite Phos-46 as a yellow oil (93 mg, 66.9% yield).

LCMS: M+H = 713.6.

$^1$H NMR: (400 MHz, $CD_3CN$), δ ppm 8.92 (s, 1 H), 7.47-7.44 (dd, J=8.0, J=2.8, 1 H), 5.64-5.62 (d, J=8.0, 1 H), 4.78-4.68 (m, 1 H), 4.42-4.22 (m, 2H), 4.18-4.15 (m, 4 H), 3.92-3.85 (m, 2 H), 3.78-3.66 (m, 3 H), 3.60-3.52 (m, 5 H), 3.48-3.45 (m, 3 H), 3.36-3.35 (dd, J=2.4, J=0.8, 6 H), 3.28-3.27 (d, J=5.6, 3 H), 3.03-2.94 (m, 2 H), 2.75-2.63(m,3 H), 1.65-1.53 (m, 1 H), 1.22-1.17 (m, 12 H).

$^{31}$P NMR: (400 MHz, $CD_3CN$), δ ppm 149.85, 148.42; 24.43, 24.22.

The other 5'-phosphonate-modified nucleoside analogs herein can be prepared using similar methods or synthetic schemes well known in the art.

**Example 2.** Synthesis of Oligonucleotides

The oligonucleotides used according to the present disclosure can be conveniently and routinely prepared by well-known solid-phase synthesis techniques. Devices used for this synthesis were commercially available from multiple suppliers, including, for example, Mermade 12 (LGC). Additionally or alternatively, any other apparatus known in the art for such synthesis could be employed. It is well known that similar techniques were used to prepare oligonucleotides, such as alkylated derivatives and those with phosphorothioate linkages.

Oligonucleotides: Unsubstituted and substituted phosphodiester (O) oligonucleotides (including but not limited to oligonucleotides) could be synthesized on an automated DNA synthesizer, Mermade 12 (LGC), using standard phosphoramidite chemistry with iodine oxidation.

In certain embodiments, phosphorothioate internucleoside linkages (S) were synthesized in a manner similar to that in which phosphodiester internucleoside linkages were synthesized, except that thiation was achieved by oxidation of the phosphite linkage using a 10% w/v solution of 3,H-1,2-benzodithiol-3-one-1,1-dioxide in acetonitrile. The time for the thiation reaction step was increased to 180 s, and the process was continued through normal capping steps. After cleavage and deprotection from the CPG column by treatment with concentrated ammonia water at 55 °C(for 12-16 h), the oligonucleotides were recovered by precipitation from a 1 M $NH_4OAc$ solution with more than 3 volumes of ethanol.

Phosphinate internucleoside linkages could be prepared as described in US5,508,270. Alkyl phosphate internucleoside linkages could be prepared as described in US4,469,863 or by the phosphoramidite method described herein. 3'-Deoxy-3'-methylenephosphonate internucleoside linkages could be prepared as described in US5,610,289 or 5,625,050. Phosphoramidite internucleoside linkages could be prepared as described in US5,256,775 or US5,366,878. Alkyl phosphorothioate internucleoside linkages could be prepared as described in the published WO 94/17093 and WO94/02499 publications. 3'-Deoxy-3'-aminophosphoramidate internucleoside linkages could be prepared as described in US5,476,925. Phosphotriester internucleoside linkages were prepared as described in US5,023,243. Some methods for preparing phosphate internucleoside linkages are described in Beilstein J Org Chem. 2017; 13: 1368-1387.

Oligonucleotides having one or more phosphorus-free internucleoside linkages, including but not limited to methylene methylimino-linked oligonucleosides, methylene dimethylhydrazino-linked oligonucleosides, methylene carbonylamino-linked oligonucleosides, and methylene aminocarbonyl-linked oligonucleosides, as well as mixed-backbone oligonucleotides with, for example, alternating O or S linkages, could be prepared as described in US5,378,825, US5,386,023, US5,489,677, US5,602,240, and US5,610,289.

Double-stranded ribonucleic acid (dsRNA) agents were obtained by mixing each of the two complementary strands (sense strand and antisense strand) in a 1:1 molar ratio to form a duplex.

The duplexes listed in Table 2 were generally synthesized using mature solid-phase synthesis methods based on

phosphoramidite chemistry on an oligonucleotide synthesizer for the sense and antisense strand sequences of dsRNA. The elongation of the oligonucleotide chains was achieved through a 4-step cycle: deprotection, condensation, capping, and an oxidation or sulfurization step for the addition of each nucleotide. The synthesis was performed on a solid support made of controlled pore glass (CPG, 1000 Å). General monomeric phosphoramidites were purchased from commercial sources, while the 5'-phosphonate-modified nucleoside analog compound herein can be used as an alternative monomeric phosphoramidite and incorporated into the oligonucleotide chain. Where the 5'-phosphonate-modified nucleoside analog compound herein can be linked as an alternative monomeric phosphoramidite to the 5'-end, it will be used for the final coupling reaction. A 3% solution of trichloroacetic acid (TCA) in dichloromethane was used for the deprotection of the 4,4'-dimethoxytrityl (DMT) protecting group. 5-Ethylthio-1H-tetrazole was used as an activator. $I_2$ in THF/Py/$H_2O$ and phenylacetyl disulfide (PADS) in pyridine/MeCN were used for oxidation and sulfurization reactions, respectively. After the final solid-phase synthesis step, the solid carrier-bound oligomers were cleaved and the protecting groups were removed by treatment with a 1:1 volume mixture of 20 wt% aqueous methylamine solution and 28% ammonium hydroxide solution. In order to synthesize oligonucleotides for *in vitro* screening, the crude mixture was concentrated. The remaining solid was dissolved in 1.0 M NaOAc, and cold EtOH was added to precipitate a single-stranded product as a sodium salt, which could be used for annealing without further purification. In order to synthesize oligonucleotides for *in vivo* testing, the crude single-stranded product was further purified by ion-pair reversed-phase HPLC(IP-RP-HPLC). The purified single-stranded oligonucleotide product from IP-RP-HPLC was dissolved in 1.0 M NaOAc and converted to a sodium salt by precipitation with the addition of cold EtOH. The sense and antisense strand oligonucleotides were annealed in water by equimolar complementarity to form a double-stranded oligonucleotide product.

The phosphoramidite compounds described in Example 1 were coupled to the 5'-ends of oligonucleotides to generate 5'-terminal nucleotides by methods described in CN110072530A and CN103154014A. For atoms of the respective hydroxyl-protecting groups of the phosphonate group, such as containing two methyl- or ethyl-protected oxygen atoms, one or two of the methyl or ethyl were removed according to the deprotection step used. In some examples, the ethyl protection was removed using a carbonitrile:trimethylsilyl iodide:pyridine e = 50:2:2 (v/v/v) ethyl-removing solution.

The oligonucleotides comprising the 5'-terminal nucleotides of the present disclosure were obtained using the methods described herein or methods well known to those skilled in the art. The sequence structures of the oligonucleotides, which are dsRNA duplexes targeting FXII, are shown in Table 2.

The 5'-terminal nucleoside structures of the duplexes in Table 2 are shown below:

| | |
|---|---|
| 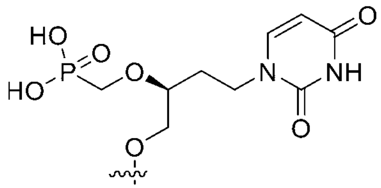 Phos-01 | 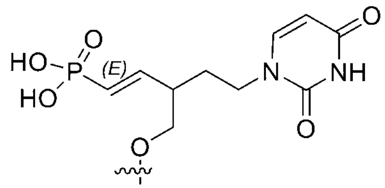 Phos-03 |
| 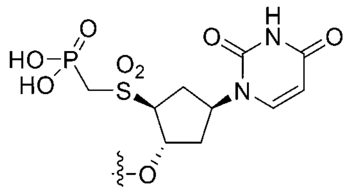 and 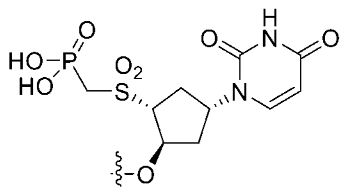 , Phos-11 | mixed in a 1:1 ratio |
| 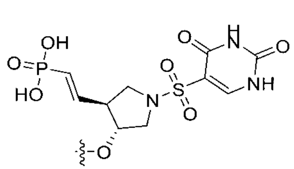 and 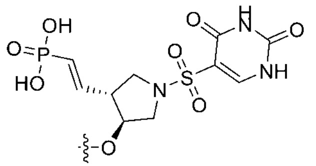 , Phos-19 | mixed in a 1:1 ratio |
| 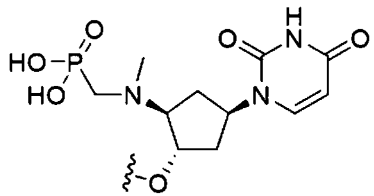 Phos-21 | Phos-22 |

(continued)

| | |
|---|---|
| 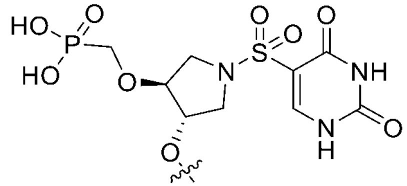 Phos-13 | 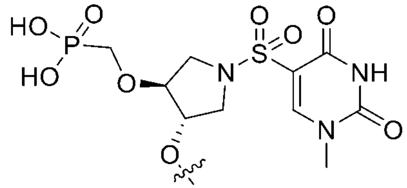 Phos-18 |
| 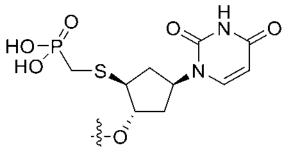 (Phos-15-1, (1S,2S,4S)) and 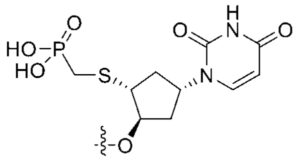 (Phos-15-2, (1R,2R,4R)), mixed in a 1:1 ratio Phos-15 | |
| Phos-42 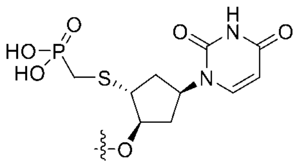 Phos-42-1 and 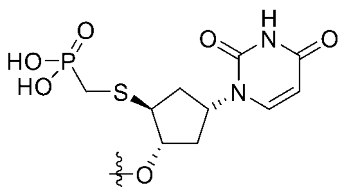 Phos-42-2, mixed in a 1:1 ratio | |
| 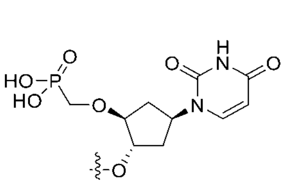 and 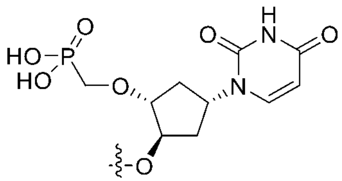 , mixed in a 1:1 ratio, Phos-16 | |

(continued)

| | |
|---|---|
| 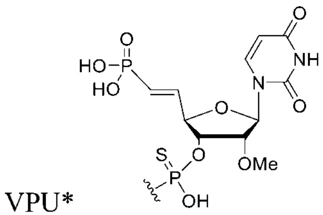 VPU* | 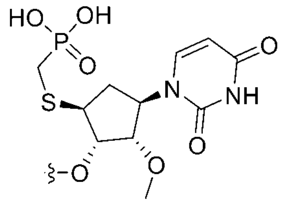 Phos-43 |
| 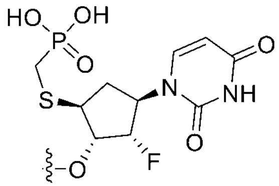 Phos-44 | 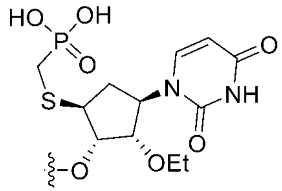 Phos-45 |
| 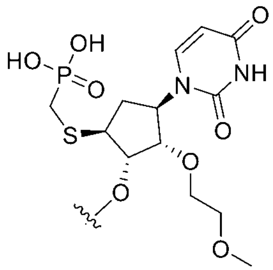 Phos-46 | 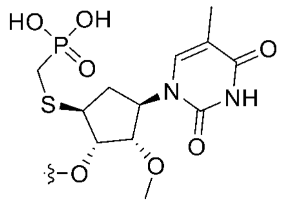 Phos-47 |
| 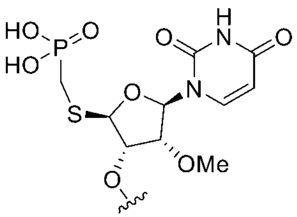 Phos-53 | 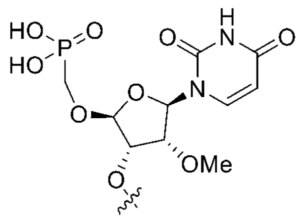 Phos-54 |
| 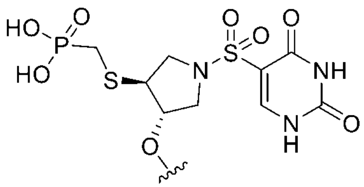 Phos-26 | . |

As shown in Tables 2-3, each of the duplexes contains a sense strand and an antisense strand; chemical modifications are indicated as follows: uppercase letters: 2'-fluoro-modified nucleotides; lowercase letters: 2'-methoxy-modified nucleotides; the superscript "*" between two nucleosides indicates a phosphorothioate internuclear linkage, and the absence of a superscript between two nucleosides indicates a phosphodiester internuclear linkage; Invab: inverted abasic; GLS-15 is the targeting group described herein.

Table 2. FXII-targeting duplexes

| Duplex # | Sequence of antisense strand 5'->3' | Sequence No. | Sequence of sense strand 5'->3" | Sequence No. |
|---|---|---|---|---|
| AD00699 | u*U*caaaGcacuUuAuU gag* u*u | 1 | GLS-15*(Invab)*aacucaauAaAgUgcuuu ga*a*(Invab) | 2 |
| AD00909 | (Phos-13)*U*caaaGcacuUuAuUgag *u*u | 3 | GLS-15*(Invab)*aacucaauAaAgUgcuuu ga*a*(Invab) | 4 |
| AD00910 | (Phos-11)*U*caaaGcacuUuAuUgag *u*u | 5 | GLS-15*(Invab)*aacucaauAaAgUgcuuu ga*a*(Invab) | 6 |
| AD01176 | (Phos-01)*U*caaaGcacuUuAuUgag *u*u | 7 | GLS-15*(Invab)*aacucaauAaAgUgcuuu ga*a*(Invab) | 8 |
| AD01177 | (Phos-15)*U*caaaGcacuUuAuUgag *u*u | 9 | GLS-15*(Invab)*aacucaauAaAgUgcuuu ga*a*(Invab) | 10 |
| AD01204 | (Phos-19)*U*caaaGcacuUuAuUgag *u*u | 11 | GLS-15*(Invab)*aacucaauAaAgUgcuuu ga*a*(Invab) | 12 |
| AD01205 | (Phos-03)*U*caaaGcacuUuAuUgag *u*u | 13 | GLS-15*(Invab)*aacucaauAaAgUgcuuu ga*a*(Invab) | 14 |
| AD01210 | (Phos-18)*U*caaaGcacuUuAuUgag *u*u | 15 | GLS-15*(Invab)*aacucaauAaAgUgcuuu ga*a*(Invab) | 16 |

Table 3. FXII-targeting duplexes

| Duplex # | Sequence of antisense strand 5'->3' | Sequence No. | Sequence of sense strand 5'->3" | No. |
|---|---|---|---|---|
| AD01558 | (Phos-16)*U*caaaGcacuUuAuUgag*u*u | 17 | GLS-15*(Invab)* aacucaauAaA-gUgcuuuga*a *(Invab) | 32 |
| AD00911 | (VPu)*U*caaaGcacuUuAuUgag*u*u | 18 | | |
| AD01534 | (Phos-15-1)*U*caaaGcacuUuAuUgag*u*u | 19 | | |
| AD01535 | (Phos-15-2)*U*caaaGcacuUuAuUgag*u*u | 20 | | |
| AD01531 | (Phos-26)*U*caaaGcacuUuAuUgag*u*u | 21 | | |
| AD01475 | (Phos-21)*U*caaaGcacuUuAuUgag*u*u | 22 | | |
| AD01476 | (Phos-22)*U*caaaGcacuUuAuUgag*u*u | 23 | | |
| AD02405 | (Phos-43)*U*caaaGcacuUuAuUgag*u*u | 24 | | |
| AD02430 | (Phos-45)*U*caaaGcacuUuAuUgag*u*u | 25 | | |
| AD02431 | (Phos-46)*U*caaaGcacuUuAuUgag*u*u | 26 | | |
| AD02429 | (Phos-44)*U*caaaGcacuUuAuUgag*u*u | 27 | | |
| AD02569 | (Phos-47)*U*caaaGcacuUuAuUgag*u*u | 28 | | |
| AD02570 | (Phos-53)*U*caaaGcacuUuAuUgag*u*u | 29 | | |
| AD02571 | (Phos-54)*U*caaaGcacuUuAuUgag*u*u | 30 | | |
| AD02123 | (Phos-42)*U*caaaGcacuUuAuUgag*u*u | 31 | | |

As shown in Tables 2-3 above, the duplexes have identical sense strands, and the duplexes differ only in that they contain different uracil nucleoside derivatives at the 5'-ends of their antisense strands.

Example 3. *In Vivo* Evaluation of Oligonucleotides Containing 5'-Terminal Nucleotides of Present Disclosure

To evaluate the *in vivo* activity of FXII double-stranded ribonucleic acid (also known as dsRNA), specific-pathogen-free 6-week-old female C57BL/6 mice (purchased from Shanghai SLAC Laboratory Animal Co., Ltd.) were used.

**a) Experimental protocol**

In the animal experiment, each group consisted of 4 female C57BL/6 mice; administration was performed at a dose of 1 or 2 (mg/kg); SC administration was performed once on day 1; blood samples were collected on days 8, 22, and 29, and the FXII protein level in plasma was determined.
b) Definition of experimental days: The day of the first administration to the mice was defined as day 1, the day before that day was defined as day -1, the day after that day was defined as day 2, and so on.
c) All test compounds were prepared into 5 mg/mL stock solutions with PBS before administration. A small amount of a 5 mg/mL stock solution was diluted 20-fold, and the OD value was determined using a Nanodrop instrument. Based on the OD value, the actual concentration of the stock solution was calculated, and an appropriate amount of the solution was diluted to a working concentration (0.2 mg/mL) for administration.
d) After a 6-day acclimation period, all mice (C57BL/6, female, 6 weeks old) were given subcutaneous injections as described above on day 1.
e) On days 8, 22, and 29, blood was collected from the submandibular vein of all mice to obtain plasma for FXII protein level determination.
f) Sample detection and analysis
An ELISA kit was used to determine the FXII protein level in mouse plasma.
An ELISA kit (Molecular Innovations, IMSFXIIKTT) was used to determine the FXII protein level in mouse plasma. Briefly, plasma samples collected with EDTA-K2 were diluted 30,000-fold and incubated in a detection plate coated with a capture antibody. A detection antibody and an HRP-conjugated secondary antibody were then sequentially added, and color development was finally performed with TMB. The absorbance at 450 nm was measured. A four-parameter method was used to fit a standard curve, and the OD values of the test samples were used to calculate the FXII protein content in each sample. The FXII protein concentration in the original plasma was obtained by multiplying the FXII protein content by the dilution factor. The data results are shown in Table 4.
Table 4. The relative FXII protein expression levels, with oligomeric compounds corresponding to the sequences, chemical modifications, and delivery shown in Table 2 used.

Table 4. The remaining percentage of FXII protein expression in mouse plasma

| Compound ID | Dose (mg/kg) | Day 8 | Day 22 | Day 29 |
|---|---|---|---|---|
| | | Remaining percentage ± SD | Remaining percentage ± SD | Remaining percentage ± SD |
| AD00699 | 1 | 0.20±0.03 | 0.14±0.01 | 0.16±0.03 |
| AD00909 | 1 | 0.22±0.01 | 0.18±0.03 | 0.19±0.06 |
| AD00910 | 1 | 0.21±0.04 | 0.13±0.04 | 0.12±0.05 |
| AD00910 | 2 | 0.08±0.02 | 0.05±0.00 | 0.05±0.00 |
| AD01176 | 1 | 0.32±0.06 | 0.23±0.03 | 0.25±0.05 |
| AD01177 | 1 | 0.15±0.01 | 0.13±0.01 | 0.13±0.03 |
| AD01177 | 2 | 0.09±0.03 | 0.05±0.03 | 0.10±0.04 |
| AD01204 | 1 | 0.32±0.04 | 0.21±0.04 | 0.25±0.08 |
| AD01205 | 1 | 0.29±0.02 | 0.20±0.03 | 0.20±0.03 |
| AD01205 | 2 | 0.16±0.02 | 0.09±0.01 | 0.09±0.01 |
| AD01210 | 1 | 0.30±0.02 | 0.19±0.02 | 0.19±0.02 |

Example 4. *In Vivo* Evaluation of Oligonucleotides Containing 5'-Terminal Nucleotides of Present Disclosure

To evaluate the *in vivo* activity of FXII double-stranded ribonucleic acid (also known as dsRNA), specific-pathogen-free 6-week-old female C57BL/6 mice (purchased from Shanghai SLAC Laboratory Animal Co., Ltd.) were

used.

**a) Experimental protocol**

In the animal experiment, each group consisted of 4 female C57BL/6 mice; administration was performed at a dose of 0.5 or 1 (mg/kg); SC administration was performed once on day 1; blood samples were collected on days 8, 22, and 29, and the FXII protein level in plasma was determined.

b) Definition of experimental days: The day of the first administration to the mice was defined as day 1, the day before that day was defined as day -1, the day after that day was defined as day 2, and so on.

c) All test compounds were prepared into 5 mg/mL stock solutions with PBS before administration. A small amount of a 5 mg/mL stock solution was diluted 20-fold, and the OD value was determined using a Nanodrop instrument. Based on the OD value, the actual concentration of the stock solution was calculated, and an appropriate amount of the solution was diluted to a working concentration (0.2 mg/mL) for administration.

d) After a 6-day acclimation period, all mice (C57BL/6, female, 6 weeks old) were given subcutaneous injections as described above on day 1.

e) On days 8, 22, and 29, blood was collected from the submandibular vein of all mice to obtain plasma for FXII protein level determination.

f) Sample detection and analysis

An ELISA kit was used to determine the FXII protein level in mouse plasma.

An ELISA kit (Molecular Innovations, IMSFXIIKTT) was used to determine the FXII protein level in mouse plasma. Briefly, plasma samples collected with EDTA-K2 were diluted 30,000-fold and incubated in a detection plate coated with a capture antibody. A detection antibody and an HRP-conjugated secondary antibody were then sequentially added, and color development was finally performed with TMB. The absorbance at 450 nm was measured. A four-parameter method was used to fit a standard curve, and the OD values of the test samples were used to calculate the FXII protein content in each sample. The FXII protein concentration in the original plasma was obtained by multiplying the FXII protein content by the dilution factor. The data results are shown in Table 5.

Table 5. The relative FXII protein expression levels, with oligomeric compounds corresponding to the sequences, chemical modifications, and delivery shown in Tables 2 and 3 used.

| Compound ID | Dose (mg/kg) | Day 8 | Day 15 | Day 29 |
|---|---|---|---|---|
| | | Remaining percentage ± SD | Remaining percentage ± SD | Remaining percentage ± SD |
| AD00699 | 0.5 | 0.34±0.03 | 0.39±0.02 | 0.37±0.02 |
| AD00911 | 0.5 | 0.22±0.05 | 0.25±0.04 | 0.21±0.05 |
| AD01475 | 1.0 | 0.28±0.05 | 0.28±0.04 | 0.40±0.05 |
| AD01476 | 0.5 | 0.50±0.03 | 0.46±0.04 | 0.74±0.06 |
| AD01531 | 1.0 | 0.18±0.03 | 0.19±0.04 | 0.26±0.06 |
| AD01534 | 0.5 | 0.23±0.03 | 0.21+0.01 | 0.27±0.01 |
| AD01535 | 0.5 | 0.25±0.01 | 0.31±0.04 | 0.32±0.05 |

Example *5. In Vivo* Evaluation of Oligonucleotides Containing 5'-Terminal Nucleotides of Present Disclosure

The experimental method was the same as that in Example 4. Relative FXII protein expression levels were obtained using oligomeric compounds corresponding to the sequences, chemical modifications, and delivery shown in Tables 2 and 3.

Table 6. The remaining percentage of FXII protein expression in mouse plasma

| Compound ID | Dose (mg/kg) | Day 8 | Day 15 | Day 29 | Day 43 |
|---|---|---|---|---|---|
| | | Remaining percentage ± SD | Remaining percentage ± SD | Remaining percentage ± SD | Remaining percentage ± SD |
| AD01177 | 0.5 | 0.30±0.14 | 0.13±0.02 | 0.15±0.04 | 0.23±0.05 |
| AD00910 | 0.5 | 0.13±0.02 | 0.24±0.04 | 0.13±0.03 | 0.15±0.03 |

(continued)

| Compound ID | Dose (mg/kg) | Day 8 | Day 15 | Day 29 | Day 43 |
|---|---|---|---|---|---|
| | | Remaining percentage ± SD | Remaining percentage ± SD | Remaining percentage ± SD | Remaining percentage ± SD |
| AD01558 | 0.5 | 0.68±0.11 | 0.87±0.01 | 0.48±0.06 | 0.40±0.07 |

As can be seen from Table 6, the dsRNAs containing 5'-S phosphonate-modified nucleoside analogs (e.g., AD01177) had better gene silencing effects than the dsRNA containing a 5'-O phosphonate-modified nucleoside analog (AD01558).

Table 7. The remaining percentage of FXII protein expression in mouse plasma

| Compound ID | Dose (mg/kg) | Day 8 | Day 15 | Day 29 | Day 43 |
|---|---|---|---|---|---|
| | | Remaining percentage ± SD | Remaining percentage ± SD | Remaining percentage ± SD | Remaining percentage ± SD |
| AD00911 | 0.5 | 0.19±0.01 | 0.09±0.04 | 0.10±0.03 | 0.31±0.02 |
| AD01177 | 0.5 | 0.18±0.01 | 0.14±0.02 | 0.12±0.01 | 0.39±0.02 |
| AD02405 | 0.5 | 0.18±0.02 | 0.12±0.03 | 0.15±0.02 | 0.31±0.03 |

Table 8. The remaining percentage of FXII protein expression in mouse plasma

| Compound ID | Dose (mg/kg) | Day 15 | Day 29 |
|---|---|---|---|
| | | Remaining percentage ± SD | Remaining percentage ± SD |
| AD02405 | 0.5 | 0.17±0.02 | 0.21±0.02 |
| AD02430 | 0.5 | 0.31±0.03 | 0.26±0.04 |
| AD02431 | 0.5 | 0.19±0.04 | 0.22±0.05 |

Example 6. *In Vivo* Evaluation of Oligonucleotides Containing 5'-Terminal Nucleotides of Present Disclosure

The experimental method was the same as that in Example 4. Relative FXII protein expression levels were obtained using oligomeric compounds corresponding to the sequences, chemical modifications, and delivery shown in Tables 2 and 3.

Table 9. The remaining percentage of FXII protein expression in mouse plasma

| Compound ID | Dose (mg/kg) | Day 8 | Day 15 | Day 29 | Day 43 |
|---|---|---|---|---|---|
| | | Remaining percentage ± SD | Remaining percentage ± SD | Remaining percentage ± SD | Remaining percentage ± SD |
| AD02123 | 0.5 | 0.36±0.05 | 0.24±0.01 | 0.55±0.07 | 0.63±0.05 |
| AD01177 | 0.5 | 0.09±0.00 | 0.06±0.01 | 0.14±0.02 | 0.15±0.06 |

**Equivalents**

Although several examples of the present disclosure have been described and illustrated herein, those of ordinary skill in the art will readily appreciate that various other means and/or structures for performing the functions and/or obtaining the results and/or one or more advantages described herein, as well as each of such variations and/or modifications, are considered to be within the scope of the present disclosure. More generally, those skilled in the art will readily appreciate that all parameters, dimensions, materials, and configurations described herein are exemplary, and that the actual parameters, dimensions, materials, and/or configurations will depend on the specific application for which the teachings of the present disclosure are used. Those skilled in the art will recognize or be able to determine, by routine experimentation only, many equivalents to the specific examples of the present disclosure described herein. It is therefore

to be understood that the foregoing examples are presented by way of example only and that, within the scope of the appended claims and their equivalents, the present disclosure may be practiced otherwise than as specifically described and claimed. The present disclosure is directed to each individual feature, system, article, material, and/or method described herein. Additionally, any combination of two or more such features, systems, articles, materials, and/or methods, if such features, systems, articles, materials, and/or methods are not mutually inconsistent, is also included within the scope of the present disclosure.

All definitions, as defined and used herein, should be understood in light of dictionary definitions, definitions in documents incorporated by reference, and/or ordinary meanings of the defined terms.

In the specification and claims, cases without a numerical limitation shall be understood to mean "at least one", unless explicitly stated otherwise.

In the specification and claims, the use of the phrase "and/or" should be understood to mean "one or two" of the elements so combined, i.e., elements that appear in combination in certain cases and separately in other cases. Unless explicitly stated otherwise, other elements may optionally exist in addition to the elements specifically identified by the term "and/or", whether related or unrelated to those specifically identified elements.

All references, patents, patent applications, and publications cited or referenced in the present application are incorporated herein by reference in their entirety.

## Claims

1. An oligonucleotide, comprising a 5'-terminal nucleotide represented by one of formula (V), formula (VI), or formula (VII), and a stereoisomer thereof:

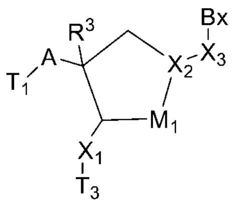 formula (V), 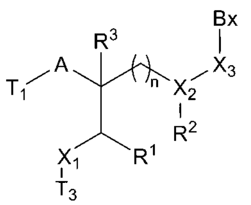 formula (VI), 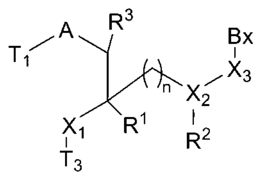 formula (VII)

wherein each $T_1$ is independently an optionally protected phosphine moiety;

each $T_3$ is independently an internucleoside linking group that links a 5'-terminal nucleotide to the oligonucleotide;

each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;

each $X_2$ is independently $CR^{15}$ or N;

each $X_3$ is independently a chemical bond, optionally substituted $C_1$-$C_3$ alkylene, SO, $SO_2$, C(=O), or P(=O)R; R is OH, SH, $C_1$-$C_6$ alkyl, $NH_2$, or $NHSO_2CH_3$;

each Bx is independently a heterocyclic base moiety;

each $R^1$ and each $R^2$ are each independently H, halogen, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, sulfinyl, sulfonyl, or acetyl;

each $R^3$ and each $R^{15}$ are each independently H, halogen, optionally substituted $C_1$-$C_6$ alkyl, optionally substituted $C_1$-$C_6$ alkoxy, optionally substituted $C_2$-$C_6$ alkenyl, or optionally substituted $C_2$-$C_6$ alkynyl;

each A independently has one of the following formulas:

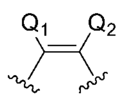, 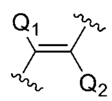, 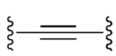, 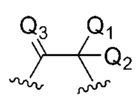, 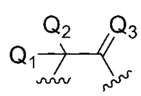, 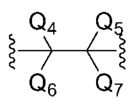, 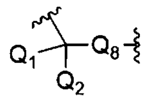,

and

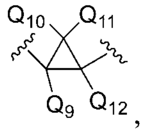

$Q_1$ and $Q_2$ are each independently H, halogen, -CN, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^4R^5$;
each $Q_3$ is independently O, S, $NR^6$, or $CR^7R^8$;
each $Q_4$, each $Q_5$, each $Q_6$, each $Q_7$, each $Q_9$, each $Q_{10}$, each $Q_{11}$, and each $Q_{12}$ are each independently H, halogen, optionally protected hydroxyl, acetoxy, azido, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^9R^{10}$;
each $Q_8$ is independently O, S, SO, $SO_2$, $PR^{16}R^{17}$, or $NR^{11}$;
each $R^{16}$ and each $R^{17}$ are independently (=O), (=S), OH, SH, $C_1$-$C_6$ alkyl, or $NR^{18}R^{19}$;
each $R^4$, each $R^5$, each $R^6$, each $R^7$, each $R^8$, each $R^9$, each $R^{10}$, each $R^{11}$, each $R^{18}$, and each $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$;
$M_1$ is C(Rd)(Re) or C(Rd)(Re)C(Rf)(Rg), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$;
each $J_3$, each $J_4$, each $J_5$, and each $J_6$ are independently H or $C_1$-$C_6$ alkyl;
n is 0, 1, or 2;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**2.** The oligonucleotide according to claim 1, wherein $T_1$ is an optionally protected phosphine moiety having the following formula:

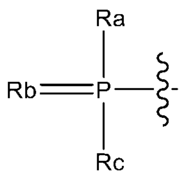

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group;
a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**3.** The oligonucleotide according to claim 2, wherein the protecting group for hydroxyl or sulfhydryl is independently selected from methyl, ethyl, benzyl (Bn), phenyl, isopropyl, tert-butyl, acetyl, chloroacetyl, trichloroacetyl, trifluoroacetyl, pivaloyl, tert-butoxymethyl, methoxymethyl, 1-ethoxyethyl, 1-(2-chloroethoxy)ethyl, 2-trimethylsilylethyl, allyl, cyclohexyl (cHex), 9-fluorenylmethoxycarbonyl, methanesulfonyl, p-toluenesulfonyl, trifluoromethanesulfonyl, benzoyl, benzoylformate, p-phenylbenzoyl, 4-methoxybenzyl, monomethoxytrityl, dimethoxytrityl, trimethoxytrityl, 4-chlorobenzyl, 4-nitrobenzyl, 2,4-dinitrophenyl, 4-acyloxybenzyl, 2-methylphenyl, 2,6-dimethylphenyl, 2-chlorophenyl, 2,6-dichlorobenzyl, diphenylmethyl, triphenylmethyl, 4-methylthio-1-butyl, 2-(S-acetylthio)ethyl (SATE), 2-cyanoethyl, 2-cyano-1,1-dimethylethyl (CDM), 4-cyano-2-butenyl, 2-(trimethylsilyl)ethyl (TSE), 2-(phenylthio)ethyl, 2-(triphenylsilyl)ethyl, 2-(benzylsulfonyl)ethyl, 2,2,2-trichloroethyl, 2,2,2-tribromoethyl, 2,3-dibromopropyl, 2,2,2-trifluoroethyl, phenylthio, 2-chloro-4-tritylphenyl, 2-bromophenyl, 2-[N-isopropyl-N-(4-methoxybenzoyl)amino]ethyl, 4-(N-trifluoroacetylamino)butyl, 4-oxopentyl, 4-tritylaminophenyl, **4-**benzylaminophenyl, tetrahydropyranyl, morpholino, trimethylsilyl, triethylsilyl, tert-butyldimethylsilyl, tert-butyldiphenylsilyl, triphenylsilyl, triisopropylsilyl, pivaloyloxymethyl (POM), and 9-phenylxanthine-9-yl;

the amino-protecting group is independently selected from 2-(trimethylsilyl)ethoxycarbonyl (Teoc), **1-**methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), benzyl, formyl, acetyl, pivaloyl, trihaloacetyl, benzoyl, nitrophenyl, acetyl, 2-nitrobenzenesulfonyl, phthalimido (Pht), p-toluenesulfonyl (Tos), trityl (Trt), 2,4-dimethoxybenzyl (PMB), and dithiosuccinyl.

**4.** The oligonucleotides according to claims 2-3, wherein $T_1$ is an optionally protected phosphine moiety having the following formula:

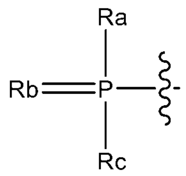

wherein $R_b$ is O or S, and Ra and Rc are each independently selected from protected hydroxyl, $C_1$-$C_6$ alkyl, and $C_1$-$C_6$ alkoxy.

**5.** The oligonucleotides according to claims 2-3, wherein Ra and Rc are each OH, SH, $OCH_3$, $OCH_2CH_3$, $OCH(CH_3)_2$, $OCH_2OC(=O)C(CH_3)_3$, $NH_2$, $OCH_2CH_2CN$, or $NHSO_2CH_3$.

**6.** The oligonucleotides according to claims 2-3, wherein Ra and Rc are each OH, and $R_b$ is O.

**7.** The oligonucleotides according to claims 1-6, wherein $T_3$ is an internucleoside linking group selected from a phosphorus-containing linking group and a phosphorus-free linking group.

**8.** The oligonucleotides according to claim 7, wherein the phosphorus-containing internucleoside linking group is independently a phosphodiester linking group, a phosphotriester linking group, a phosphorothioate linking group, a phosphorodithioate linking group, an alkylphosphonate linking group, an aminophosphonate linking group, a phosphonate linking group, a phosphinate linking group, a phosphoramidothioate linking group, or a phosphoramidate linking group.

**9.** The oligonucleotides according to claim 7, wherein the internucleoside linking group is independently an alkylphosphonate linking group, a phosphodiester internucleoside linking group, or a phosphorothioate internucleoside linking group.

**10.** The oligonucleotides according to claims 1-9, wherein the $B_X$ heterocyclic base moiety is selected from a natural nucleobase, a modified nucleobase, and a universal base.

**11.** The oligonucleotides according to claim 10, wherein the $B_X$ heterocyclic base moiety is selected from pyrimidine, substituted pyrimidine, pseudouracil, substituted pseudouracil, purine, hypoxanthine, and substituted purine.

**12.** The oligonucleotides according to claim 10, wherein the $B_X$ heterocyclic base moiety is selected from 2-thiouracil, 5-fluorouracil, dihydrouridine (D), 7-methylguanosine (m7G), uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, and guanine.

**13.** The oligonucleotides according to claims 1-12, wherein $R^{15}$ and $R^3$ are both H.

**14.** The oligonucleotides according to claims 1-13, wherein $X_1$ is O.

**15.** The oligonucleotides according to claims 1-14, wherein each $R^1$ and each $R^2$ are each independently H, methanesulfonyl, or acetyl.

**16.** The oligonucleotides according to claims 1-14, wherein each $X_2$ is independently N, and each $R^1$ and each $R^2$ are each independently H, methanesulfonyl, or acetyl.

**17.** The oligonucleotides according to claims 1-16, wherein A in formula (V), (VI), or (VII) has one of the following formulas:

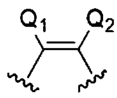, 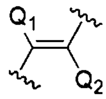, and 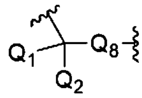,

wherein $Q_1$ and $Q_2$ are each independently H, halogen, -CN, or optionally substituted $C_1$-$C_6$ alkyl;
$Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$;
$R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$;
$J_3$ and $J_4$ are independently H or $C_1$-$C_6$ alkyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**18.** The oligonucleotides according to claims 1-17, wherein $Q_1$ and $Q_2$ are each independently H, F, - CN, or methyl.

**19.** The oligonucleotides according to claim 18, wherein $Q_1$ and $Q_2$ are each independently H.

**20.** The oligonucleotides according to claims 1-19, wherein a proviso is that when $M_1$ is C(Rd)(Re), $X_2$ is C, $X_3$ is a chemical bond, and A is

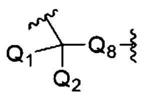,

$Q_8$ is S, SO, $SO_2$, $PR^{16}R^{17}$, or $NR^{11}$.

**21.** The oligonucleotides according to claims 1-20, wherein $Q_8$ is S, SO, $SO_2$, $PR^{16}R^{17}$, or $NR^{11}$.

**22.** The oligonucleotides according to claims 1-21, wherein $X_2$ is N, and $X_3$ is a chemical bond, $-CH_2-$, $-CH_2CH_2-$, SO, or $SO_2$.

**23.** The oligonucleotides according to claims 1-21, wherein $X_2$ is C, and $X_3$ is a chemical bond.

**24.** The oligonucleotides according to claims 1-23, wherein n is 0 or 1.

**25.** The oligonucleotide according to claim 1, comprising a 5'-terminal nucleotide represented by formula (V-1) or a stereoisomer thereof:

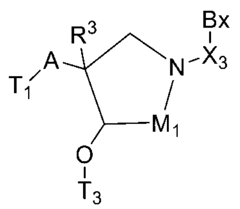

formula (V-1)

wherein $T_1$, $T_3$, A, $R^3$, and Bx are each as defined in claim 1; $X_3$ is a chemical bond, C(=O), P(=O)R, SO, or $SO_2$; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl; R is R is OH, SH, $C_1$-$C_6$ alkyl, $NH_2$, or $NHSO_2CH_3$.

**26.** The oligonucleotide according to claim 25, wherein $X_3$ is $SO_2$ or a chemical bond; $R^3$ is hydrogen.

**27.** The oligonucleotides according to claims 25-26, wherein Rd, Re, Rg, and Rf are each independently selected from the following substituent groups: hydrogen, fluorine, hydroxyl, $C_1$-$C_6$ alkoxy, and $O(CH_2)_2$-$OCH_3$.

**28.** The oligonucleotides according to claim 27, wherein Rd, Re, Rg, and Rf are each independently selected from the following substituent group: hydrogen.

**29.** The oligonucleotides according to claims 25-28, wherein A has one of the following formulas:

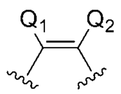 , 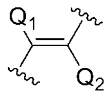 , and 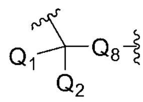 ,

wherein $Q_1$ and $Q_2$ are each independently H, halogen, or optionally substituted $C_1$-$C_6$ alkyl;
$Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$;
$R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl; each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**30.** The oligonucleotide according to claim 1, comprising a 5'-terminal nucleotide represented by formula (V-2) or a stereoisomer thereof:

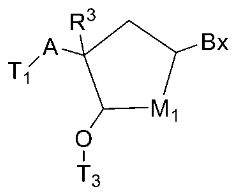

formula (V-2)

wherein $T_1$, $T_3$, A, $R^3$, and Bx are each as defined in claim 1; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl.

**31.** The oligonucleotide according to claim 30, wherein each Rd, each Re, each Rg, and each Rf are each independently selected from the following substituent groups: hydrogen, fluorine, hydroxyl, $C_1$-$C_6$ alkoxy, and $O(CH_2)_2$-$OCH_3$.

**32.** The oligonucleotide according to claim 30, wherein $M_1$ is $CH_2$ or $CH_2CH_2$.

**33.** The oligonucleotides according to claims 30-32, wherein $R^3$ is hydrogen.

**34.** The oligonucleotides according to claims 30-33, wherein A in formula (V-2) or the stereoisomer thereof has one of the following formulas:

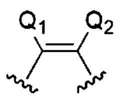 , 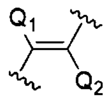 , and 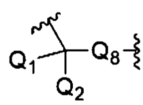 ,

$Q_1$ and $Q_2$ are each independently H, halogen, or optionally substituted $C_1$-$C_6$ alkyl; $Q_8$ is S, SO, $SO_2$ or $NR^{11}$, or $PR^{16}R^{17}$; $R^{16}$ and $R^{17}$ are independently (=O), (=S), OH, SH, $C_1$-$C_6$ alkyl, or $NR^{18}R^{19}$;
$R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl; each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**35.** The oligonucleotide according to claim 1, comprising a 5'-terminal nucleotide represented by formula (VI-1) or a stereoisomer thereof:

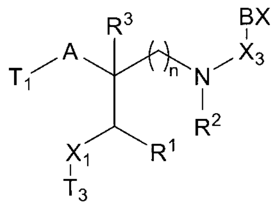

(VI-1)

wherein $T_1$, $T_3$, $X_1$, $X_3$, A, $R^3$, Bx, and n are each as defined in claim 1; $R^1$ and $R^2$ are each independently H, methanesulfonyl, or acetyl.

**36.** The oligonucleotide according to claim 35, wherein n is 0 or 1.

**37.** The oligonucleotides according to claims 35-36, wherein $X_3$ is $CH_2$ or $CH_2CH_2$; $R^3$ is H.

**38.** The oligonucleotide according to claim 1, comprising a 5'-terminal nucleotide represented by formula (VI-2) or a stereoisomer thereof:

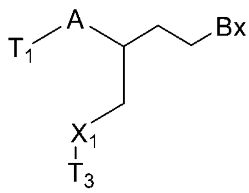

(VI-2)

wherein $T_1$, $T_3$, $X_1$, A, and Bx are each as defined in claim 1.

**39.** The oligonucleotide according to claim 1, comprising a 5'-terminal nucleotide represented by formula (VI-3) or a stereoisomer thereof:

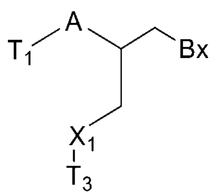

(VI-3)

wherein $T_1$, $T_3$, $X_1$, A, and Bx are each as defined in claim 1.

**40.** The oligonucleotide according to claim 1, comprising a 5'-terminal nucleotide represented by formula (VII-1) or a stereoisomer thereof:

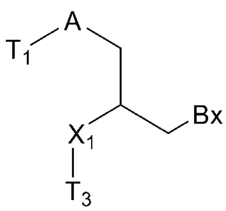

formula (VII-1)

wherein $T_1$, $T_3$, $X_1$, A, and Bx are each as defined in claim 1.

**41.** The oligonucleotide according to claim 1, comprising a 5'-terminal nucleotide represented by formula (VII-2) or a

stereoisomer thereof:

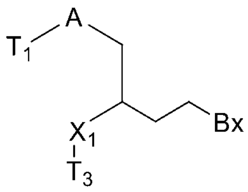

(VII-2)

wherein $T_1$, $T_3$, $X_1$, A, and Bx are each as defined in claim 1.

**42.** The oligonucleotides according to claims 38-41, wherein in the 5'-terminal nucleotide represented by formula (VI-1), formula (VI-2), formula (VI-3), formula (VII-1), formula (VII-2), or the stereoisomer thereof, A independently has one of the following formulas:

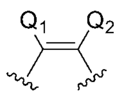 , 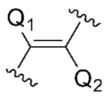 , and 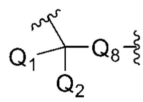 ,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**43.** The oligonucleotides according to claims 25-42, wherein each $Q_1$ and each $Q_2$ are each independently H, F, -CN, or methyl.

**44.** The oligonucleotides according to claim 43, wherein each $Q_1$ and each $Q_2$ are each independently H.

**45.** The oligonucleotides according to claims 25-42, wherein $Q_8$ is independently $NR^{11}$, and $R^{11}$ is independently methyl or methanesulfonyl.

**46.** The oligonucleotides according to claims 25-42, wherein $Q_8$ is S or $SO_2$.

**47.** The oligonucleotides according to claims 25-46, wherein $T_1$ is an optionally protected phosphine moiety having the following formula:

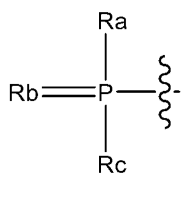

wherein:
Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group.

**48.** The oligonucleotides according to claim 47, wherein the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

**49.** The oligonucleotides according to claim 47, wherein $R_b$ is O or S.

**50.** The oligonucleotides according to claim 47, wherein Ra and Rc are each OH, SH, $NH_2$, $OCH_2CH_2CN$, or $NHSO_2CH_3$.

**51.** The oligonucleotides according to claim 47, wherein Ra and Rc are each OH, and $R_b$ is O.

**52.** The oligonucleotides according to claims 25-51, wherein $T_3$ is an internucleoside linking group selected from an alkylphosphonate linking group, a phosphodiester internucleoside linking group, and a phosphorothioate internucleoside linking group.

**53.** The oligonucleotides according to claims 25-52, wherein each $B_X$ heterocyclic base moiety is selected from a natural nucleobase, a modified nucleobase, and a universal base.

**54.** The oligonucleotides according to claim 53, wherein each $B_X$ heterocyclic base moiety is independently pyrimidine, substituted pyrimidine, pseudouracil, substituted pseudouracil, purine, hypoxanthine, or substituted purine.

**55.** The oligonucleotides according to claim 53, wherein each BX heterocyclic base moiety is 2-thiouracil, 5-fluorouracil, dihydrouridine (D), 7-methylguanosine (m7G), uracil, 5-thiazolouracil, thymine, cytosine, pseudouracil, N1-methylpseudouracil, hypoxanthine, 5-methylcytosine, 5-methyluracil, 3-benzoyluracil, 2,6-diaminopurine, adenine, or guanine.

**56.** An oligonucleotide, comprising a 5'-terminal nucleotide represented by formula (VIII) or a stereoisomer thereof:

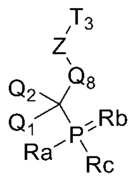
formula (VIII)

wherein $Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$;
Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, protected or optionally substituted amino, and a natural or modified nucleoside, and $R_b$ is O or S or $NR^{12}$, wherein $R^{12}$ is hydrogen, $C_1$-$C_6$ alkyl, or an amino-protecting group;
$Q_1$ and $Q_2$ are each independently H, halogen, -CN, or optionally substituted $C_1$-$C_6$ alkyl;
a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;
$R^{11}$, $R^{18}$, and $R^{19}$ are independently H, optionally substituted $C_1$-$C_6$ alkyl, optionally substituted $C_1$-$C_6$ alkoxy, methanesulfonyl, or sulfo;
Z is a nucleoside comprising a sugar or sugar surrogate moiety;
$T_3$ is an internucleoside linking group that links the 5'-terminal nucleotide of formula (VIII) or the stereoisomer thereof to the oligonucleotide;
each substituted group comprises one or more substituent groups optionally and independently selected from halogen, hydroxyl, $C_1$-$C_6$ alkyl, $C_1$-$C_6$ alkoxy, $C_1$-$C_6$ alkylsulfhydryl, and CN.

**57.** The oligonucleotide according to claim 56, wherein $Q_8$ is S, SO, or $SO_2$.

**58.** The oligonucleotides according to claim 57, wherein $Q_1$ and $Q_2$ are each independently H.

**59.** The oligonucleotides according to claims 56-58, wherein in the nucleoside comprising a sugar or sugar surrogate moiety, the sugar or sugar surrogate moiety includes a 5-membered furanose ring, a non-furanose ring, or a 5- to 6-membered carbocyclic system, or an open system.

**60.** The oligonucleotides according to claim 59, wherein the sugar or sugar surrogate moiety is selected from morpholinyl, cyclohexenyl, cyclohexyl, cyclopentyl, pyranyl, cyclohexanehexol group, furanose, unlocked nucleobase analog (UNA), glycerol nucleobase analog (GNA), locked nucleic acid (LNA), and bridged nucleic acid (BNA).

**61.** The oligonucleotides according to claims 59-60, wherein $Q_8$ is bonded to the 4'-carbon or 5'-carbon of the sugar or

sugar surrogate moiety.

**62.** The oligonucleotides according to claims 59-60, wherein the nucleoside comprising a sugar or sugar surrogate moiety has the following structural schematics:

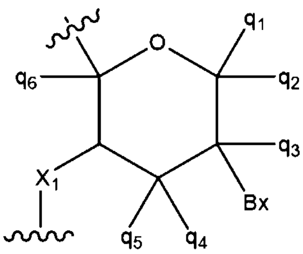 , 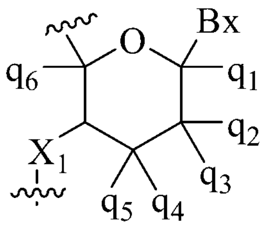 , 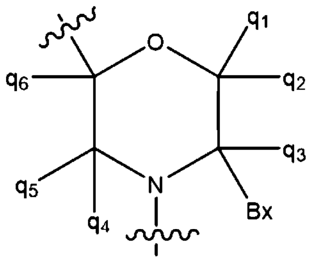 ,

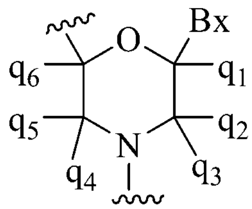 , 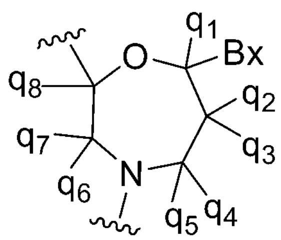 , 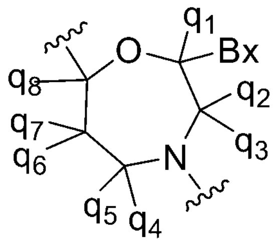 ,

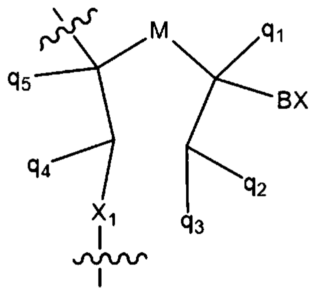 , 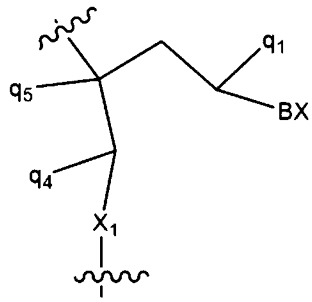 , 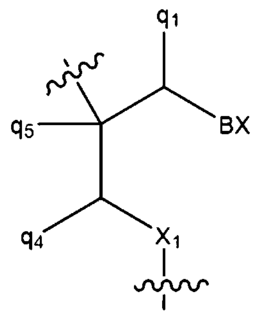 ,

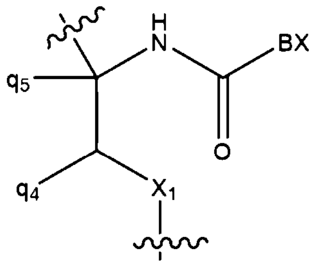 , 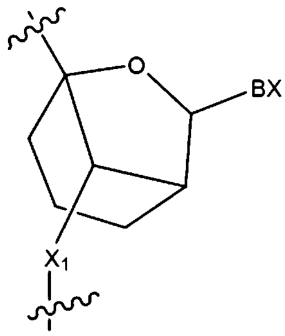 , 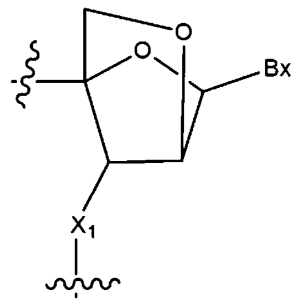 ,

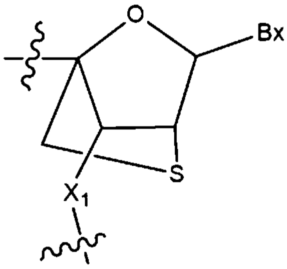 , 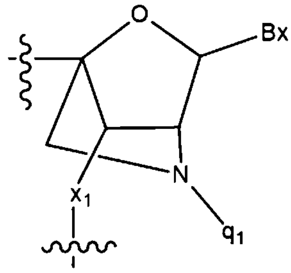 , 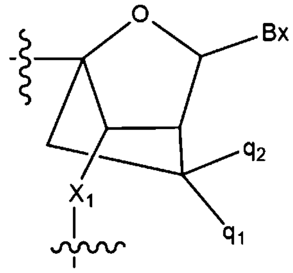 ,

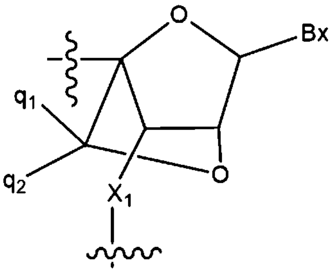, 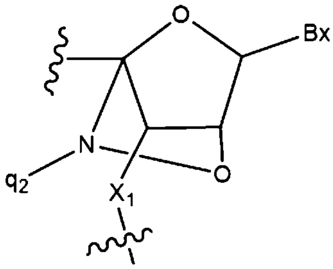, 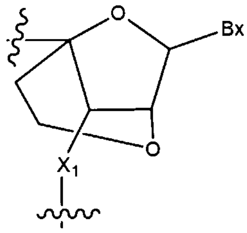,

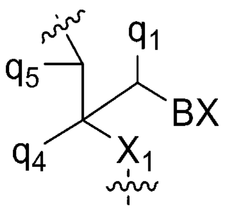, 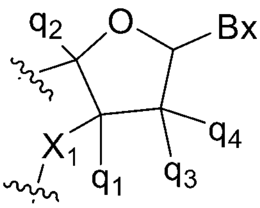, and 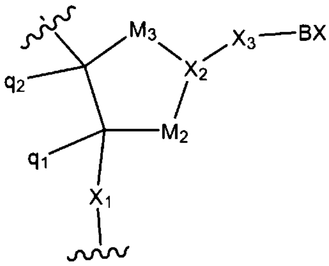,

wherein $M_2$ is $C(q_3)(q_4)$ or $C(q_3)(q_4)C(q_5)(q_6)$;
$M_3$ is O, S, $NR^{13}$, $C(q_7)(q_8)$, $C(q_7)(q_8)C(q_9)(q_{10})$, $C(q_7)=C(q_8)$, or $OC(q_7)(q_8)$;
each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
$X_2$ is $CR^{15}$ or N;
$X_3$ is a chemical bond, optionally substituted $C_1$-$C_3$ alkylene, SO, $SO_2$, C(=O), or P(=O)R;
R is OH, SH, $C_1$-$C_6$ alkyl, $NH_2$, or $NHSO_2CH_3$;
Bx is a heterocyclic base moiety;
$R^{15}$, $q_1$, $q_2$, $q_3$, $q_4$, $q_5$, $q_6$, $q_7$, $q_8$, $q_9$, and $q_{10}$ are each independently hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, CN, $OC(=O)J_5$, $OC(=O)N(J_5)(J_6)$, or $C(=O)N((J_5)(J_6)$, wherein $J_5$ and $J_6$ are independently H or $C_1$-$C_6$ alkyl;
$R^{13}$ is hydrogen or $C_1$-$C_6$ alkyl.

**63.** The oligonucleotides according to claim 62, wherein the nucleoside comprising a sugar or sugar surrogate moiety has the following structural schematic:

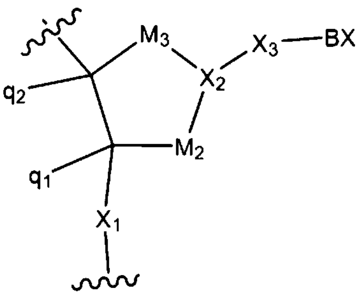.

**64.** The oligonucleotides according to claim 63, wherein $M_3$ is O, S, $C(q_7)(q_8)$, or $C(q_7)(q_8)C(q_9)(q_{10})$.

**65.** The oligonucleotide according to claim 56, comprising a 5'-terminal nucleotide represented by formula (VIII-1) or a stereoisomer thereof:

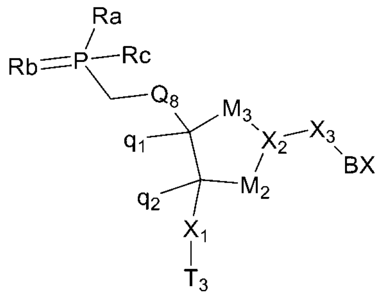

formula (VIII-1)

wherein $Q_8$, Ra, Rc, $R_b$, $M_2$, $M_3$, $X_1$, $X_2$, $X_3$, $q_1$, $q_2$, and Bx are each as defined in claim 56.

**66.** The oligonucleotides according to claims 62-65, wherein $R^{15}$, $q_1$, $q_2$, $q_3$, $q_4$, $q_5$, $q_6$, $q_7$, $q_8$, $q_9$, and $q_{10}$ are each independently selected from hydrogen, fluorine, hydroxyl, methyl, methoxy, and $O(CH_2)_2$-$OCH_3$.

**67.** The oligonucleotides according to claim 66, wherein $R^{15}$, $q_1$, $q_2$, $q_3$, $q_4$, $q_5$, $q_6$, $q_7$, $q_8$, $q_9$, and $q_{10}$ are each independently selected from hydrogen.

**68.** The oligonucleotides according to claims 65-67, wherein $M_3$ is O, S, $C(q_7)(q_8)$, $M_2$ is $C(q_3)(q_4)$, $X_2$ is $CR^{15}$ or N, and $X_3$ is a chemical bond, SO, or $SO_2$.

**69.** The oligonucleotides according to claim 68, wherein $M_3$ is $M_3$ is O, S, $CH_2$, $M_2$ is $CH_2$, $X_2$ is CH, and $X_3$ is a chemical bond.

**70.** The oligonucleotides according to claim 68, wherein $M_3$ is $CH_2$, $M_2$ is $CH_2$, $X_2$ is N, and $X_3$ is a chemical bond, SO, or $SO_2$.

**71.** The oligonucleotides according to claims 65-67, wherein $M_3$ is $C(q_7)(q_8)$, $M_2$ is $C(q_3)(q_4)C(q_5)(q_6)$, $X_2$ is $CR^{15}$ or N, and $X_3$ is a chemical bond, SO, or $SO_2$.

**72.** The oligonucleotides according to claim 71, wherein $M_3$ is $CH_2$, $M_2$ is $CH_2CH_2$, $X_2$ is CH, and $X_3$ is a chemical bond.

**73.** The oligonucleotides according to claim 71, wherein $M_3$ is $CH_2$, $M_2$ is $CH_2CH_2$, $X_2$ is N, and $X_3$ is a chemical bond, SO, or $SO_2$.

**74.** The oligonucleotides according to claims 56-73, wherein $Q_8$ is S, SO, or $SO_2$.

**75.** The oligonucleotides according to claims 56-73, wherein $R_b$ is oxygen.

**76.** The oligonucleotides according to claims 56-73, wherein $Q_8$ is SO or $SO_2$, and Ra and Rc are each independently selected from OH, SH, $NH_2$, and $NHSO_2CH_3$.

**77.** The oligonucleotides according to claims 56-73, wherein $Q_8$ is S, SO, or $SO_2$, $R_b$ is oxygen, and Ra and Rc are each independently selected from OH.

**78.** The oligonucleotides according to claims 56-77, wherein $X_1$ is oxygen.

**79.** The oligonucleotides according to claims 56-78, wherein the $B_X$ heterocyclic base moiety is selected from a natural nucleobase, a modified nucleobase, and a universal base.

**80.** The oligonucleotides according to claim 79, wherein the $B_X$ heterocyclic base moiety is pyrimidine, substituted pyrimidine, pseudouracil, substituted pseudouracil, purine, hypoxanthine, or substituted purine.

**81.** The oligonucleotides according to claim 79, wherein the $B_X$ heterocyclic base moiety is 2-thiouracil, 5-fluorouracil, dihydrouridine (D), 7-methylguanosine (m7G), uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine.

**82.** An oligonucleotide, having a compound fragment of the following formula at a 5'-terminal nucleotide:

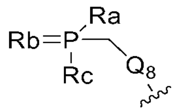

wherein Qs is S, SO, $SO_2$, $PR^{16}R^{17}$, or $NR_{11}$; $R^{16}$ and $R^{17}$ are independently (=O), (=S), OH, SH, $C_1$-$C_6$ alkyl, or $NR^{18}R^{19}$; Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, protected or optionally substituted amino, and a natural or modified nucleoside, and $R_b$ is O or S or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;

$R^{11}$, $R^{18}$, and $R^{19}$ are independently H, optionally substituted $C_1$-$C_6$ alkyl, optionally substituted $C_1$-$C_6$ alkoxy, methanesulfonyl, or sulfo;

each substituted group comprises one or more substituent groups optionally and independently selected from halogen, hydroxyl, $C_1$-$C_6$ alkyl, $C_1$-$C_6$ alkoxy, $C_1$-$C_6$ alkylsulfhydryl, and CN;

" " refers to the linkage to the remainder of the 5'-terminal nucleotide.

**83.** The oligonucleotides according to claims 1-82, comprising a 5'-terminal nucleoside represented by one of the following specific structures or a stereoisomer thereof:

| | |
|---|---|
| 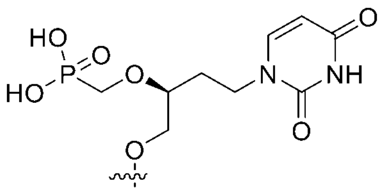 Phos-01 | 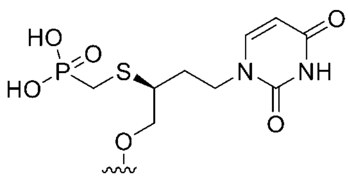 Phos-02 |
| 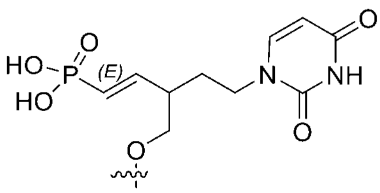 Phos-03 | 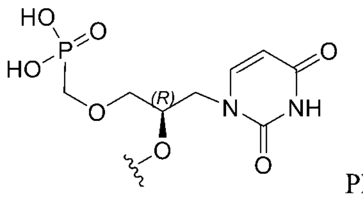 Phos-04 |
| 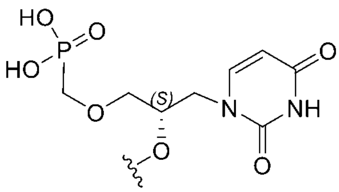 Phos-05 | 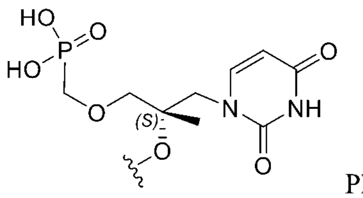 Phos-06 |
| 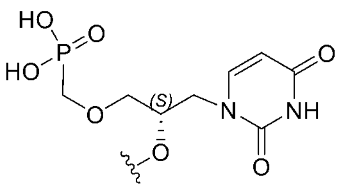 Phos-07 | 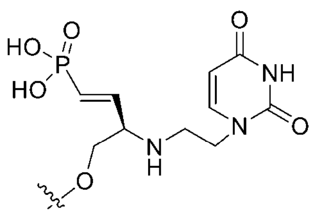 Phos-08 |

| | |
|---|---|
| 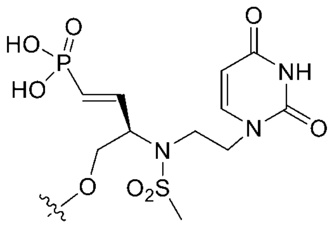 Phos-09 | 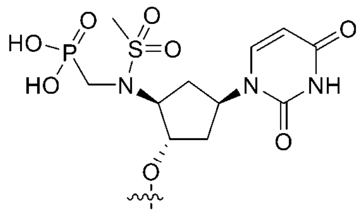 Phos-10 |
| 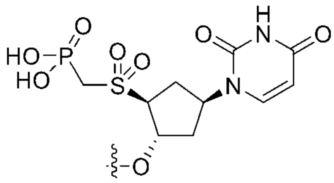 Phos-11-1 | 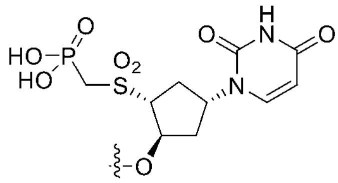 Phos-11-2 |
| 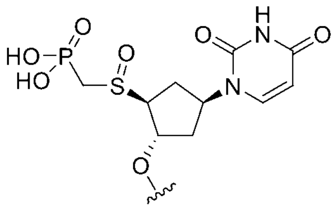 Phos-11-4 | 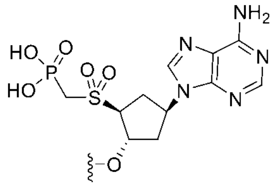 Phos-11-7 |
| 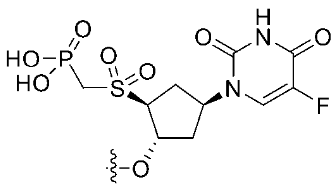 Phos-11-8 | 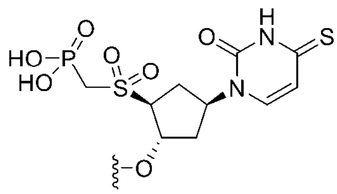 Phos-11-9 |
| 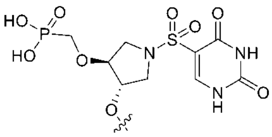 Phos-13 | 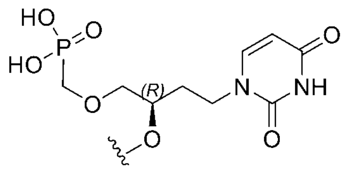 Phos-14 |
| 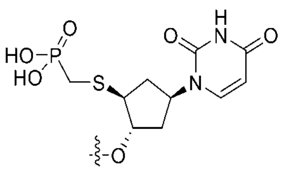 Phos-15-1 | 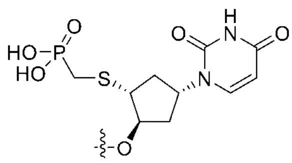 Phos-15-2 |
| 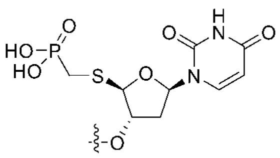 Phos-15-3 | 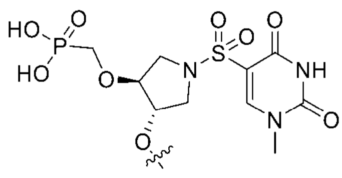 phos-18 |

| | |
|---|---|
| 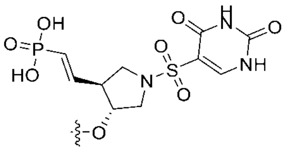 Phos-19-1 | 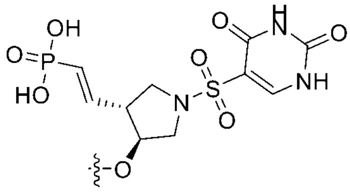 Phos-19-2 |
| 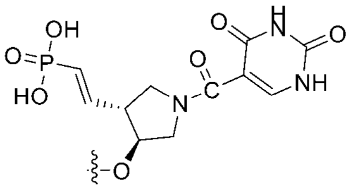 Phos-19-5 | 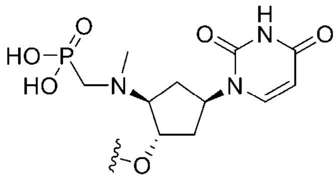 Phos-21 |
| 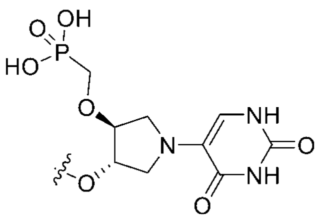 Phos-22 | 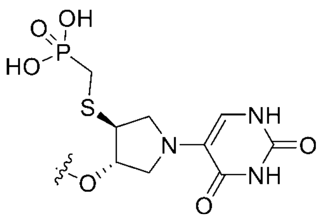 Phos-23 |
| 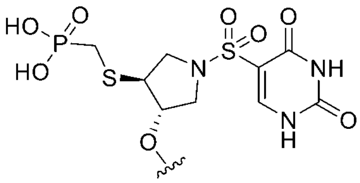 Phos-26 | 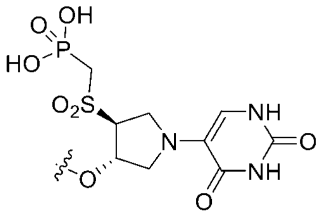 Phos-25 |
| 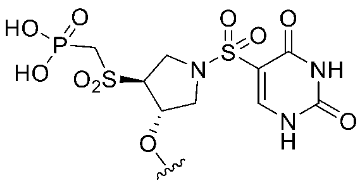 Phos-24 | 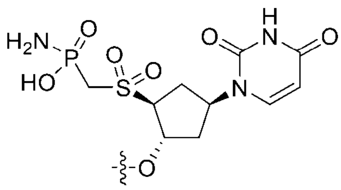 Phos-27 |
| 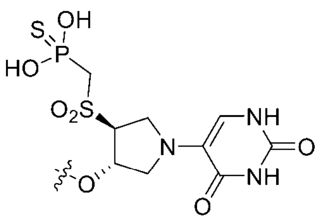 Phos-28 | 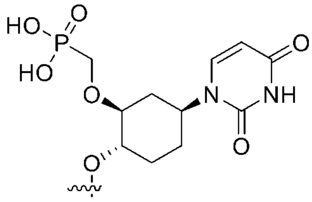 Phos-29 |
| 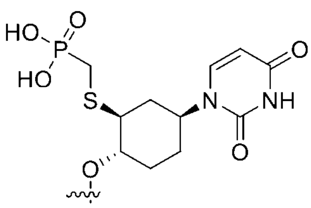 Phos-30 | 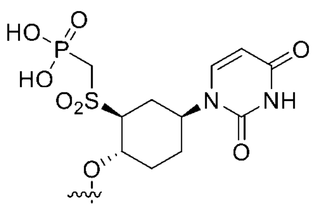 Phos-31 |

| | |
|---|---|
| 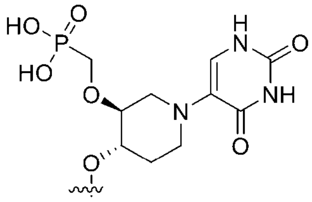 Phos-32 | 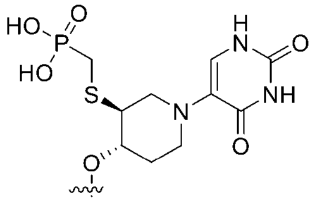 Phos-33 |
| 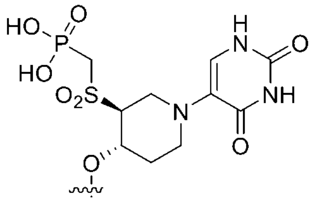 Phos-34 | 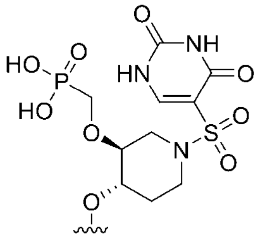 Phos-35 |
| 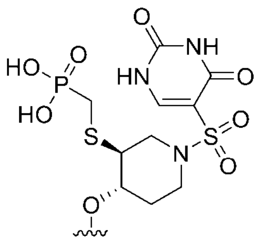 Phos-36-1 | 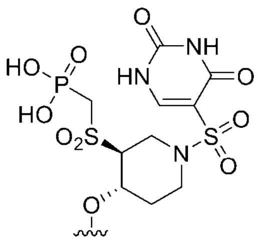 Phos-37 |
| 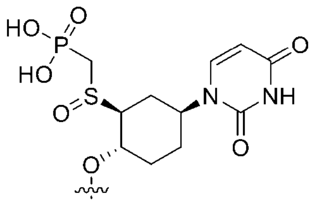 Phos-38 | 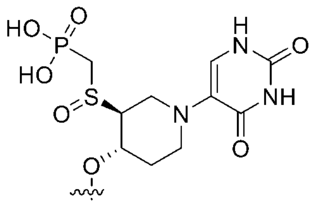 Phos-39 |
| 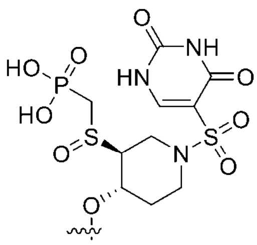 Phos-40 | 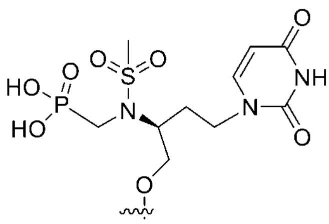 Phos-41 |
| 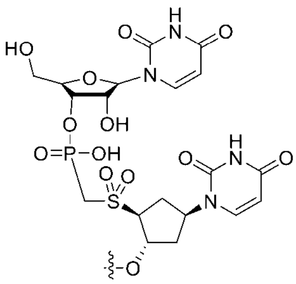 Phos-42-3 | 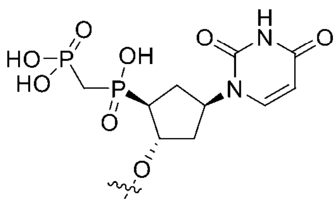 Phos-43-1 |

| | |
|---|---|
| 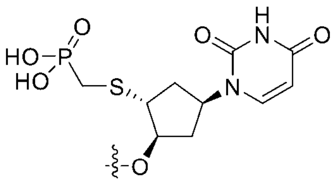 Phos-42-1 | 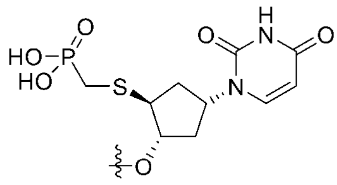 Phos-42-2 |
| 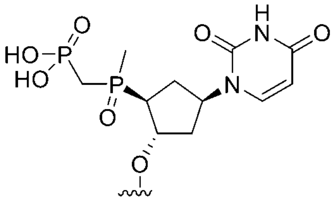 Phos-44-1 | 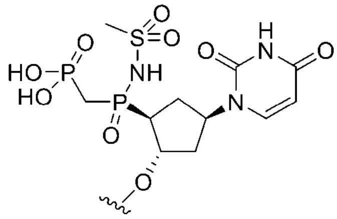 Phos-45-1 |
| 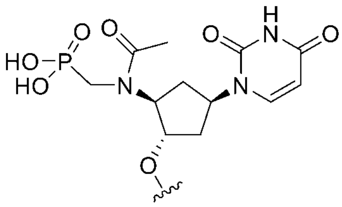 Phos-12-1 | 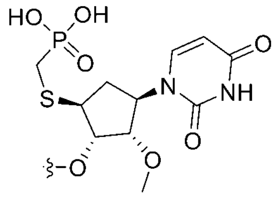 Phos-43 |
| 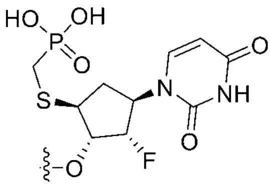 Phos-44 | 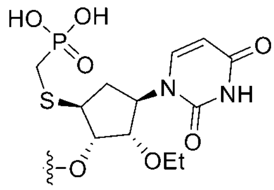 Phos-45 |
| 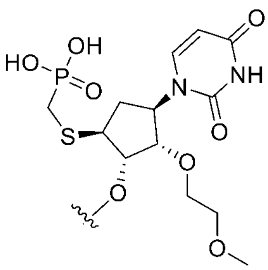 Phos-46 | 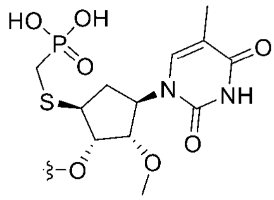 Phos-47 |
| 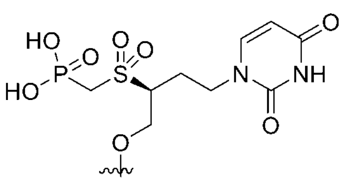 Phos-2-2 | 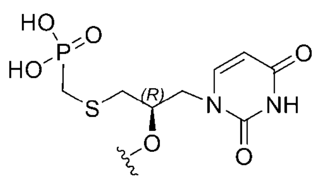 Phos-4-1 |
| 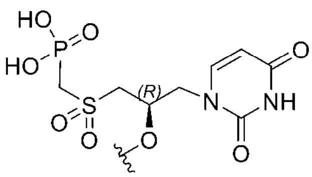 Phos-4-2 | 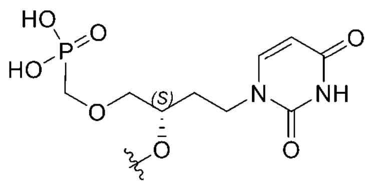 Phos-5-1 |

| | |
|---|---|
| 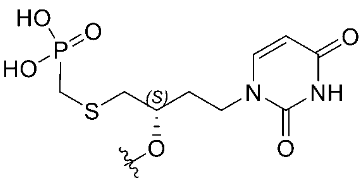 Phos-5-2 | 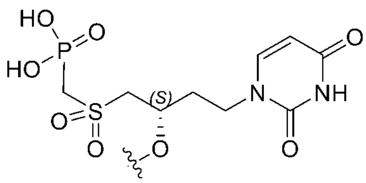 Phos-5-3 |
| 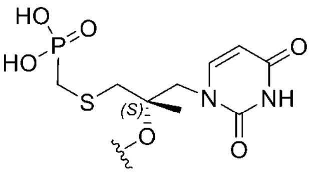 Phos-6-1 | 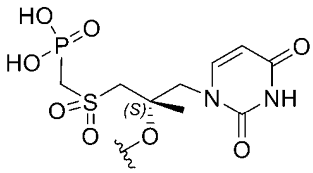 Phos-6-2 |
| 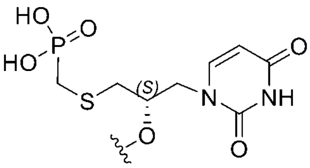 Phos-7-1 | 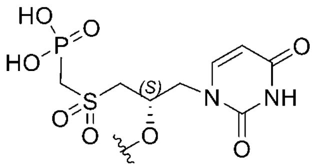 Phos-7-2 |
| 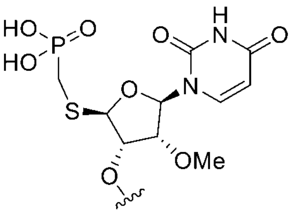 Phos-53 | . |

or

| | |
|---|---|
| 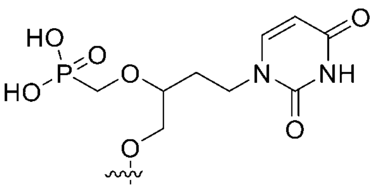 | 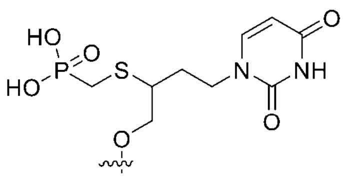 |
| 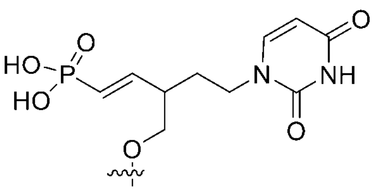 | 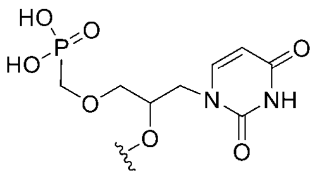 |
| 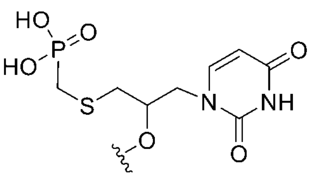 | 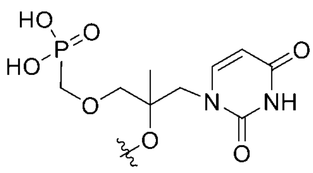 |

| | |
|---|---|
| 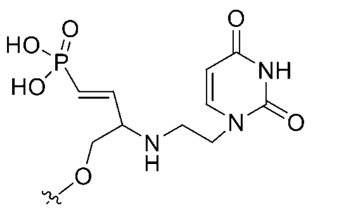 | 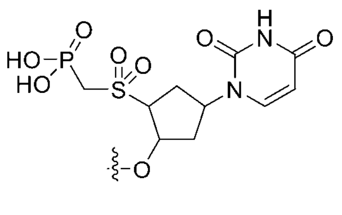 |
| 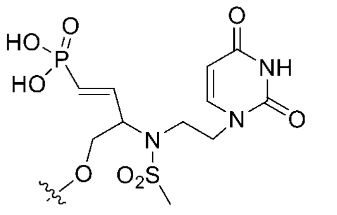 | 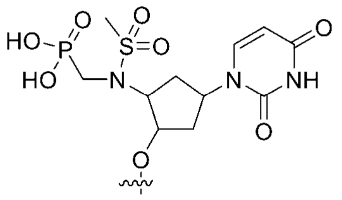 |
| 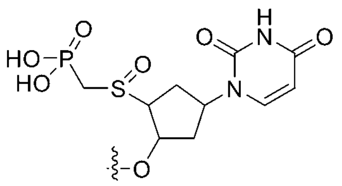 | 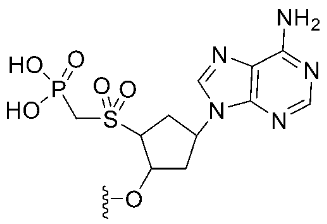 |
| 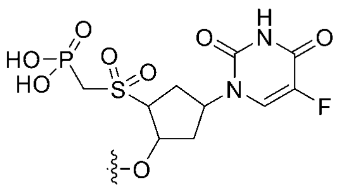 | 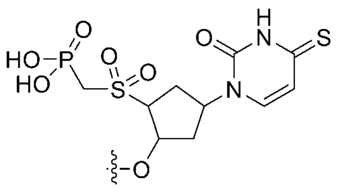 |
| 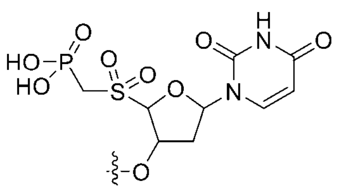 | 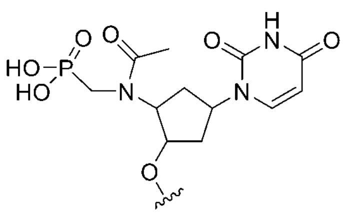 |
| 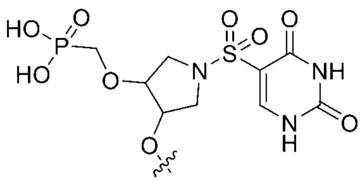 | 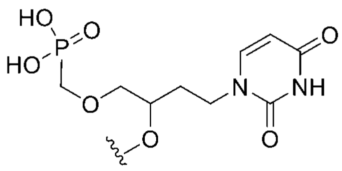 |
| 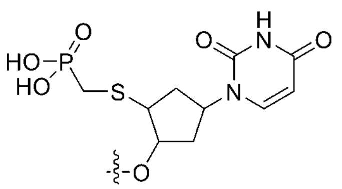 | 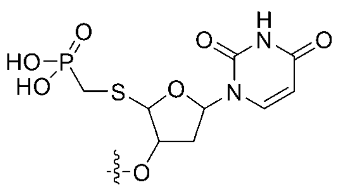 |

| | |
|---|---|
| 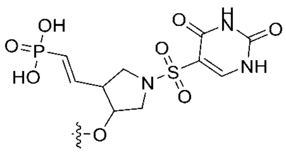 | 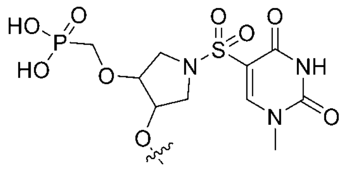 |
| 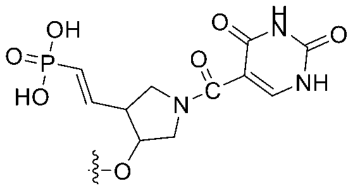 | 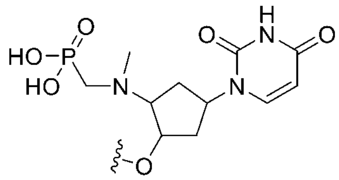 |
| 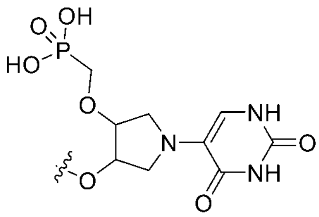 | 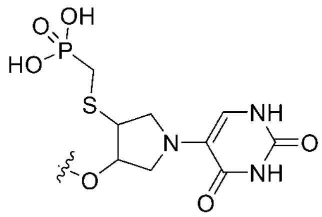 |
| 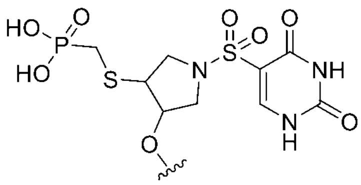 | 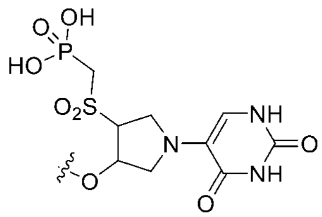 |
| 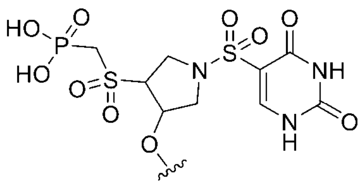 | 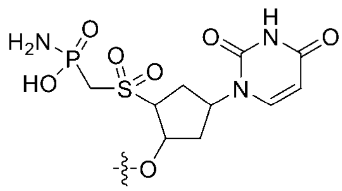 |
| 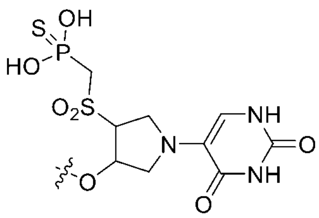 | 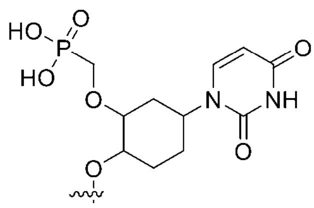 |
| 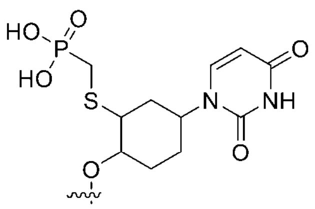 | 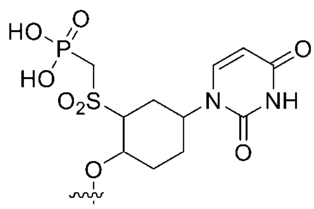 |

| | |
|---|---|
| 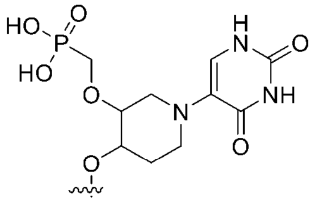 | 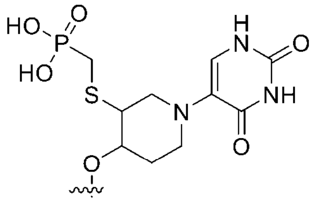 |
| 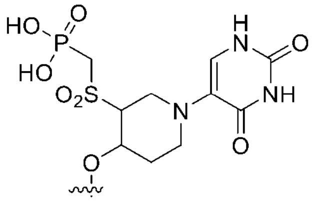 | 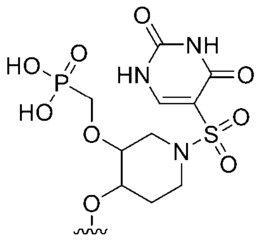 |
| 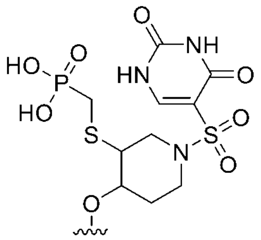 | 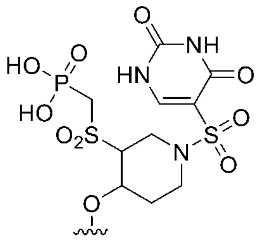 |
| 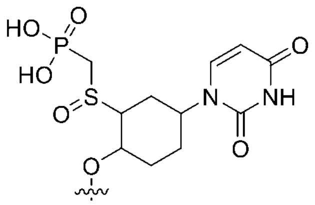 | 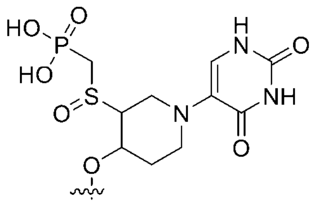 |
| 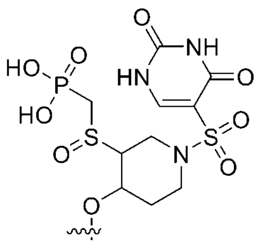 | 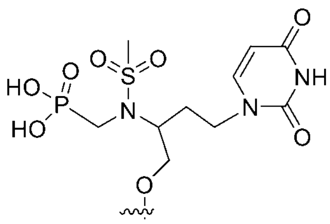 |
| 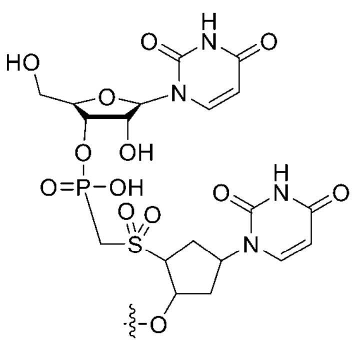 | 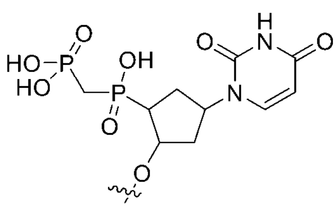 |

| | |
|---|---|
| 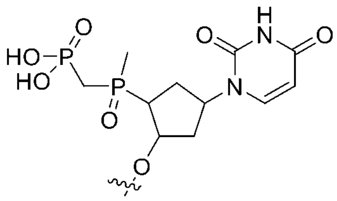 | 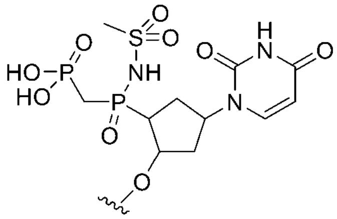 |
| 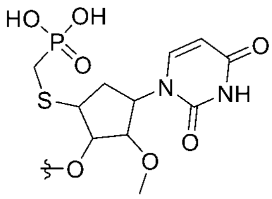 | 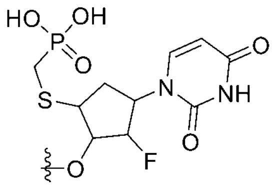 |
| 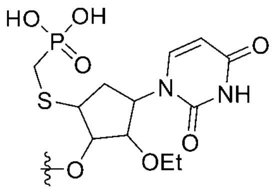 | 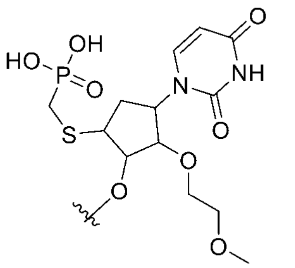 |
| 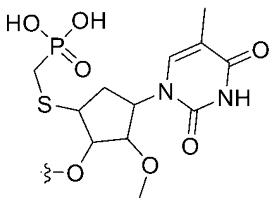 | 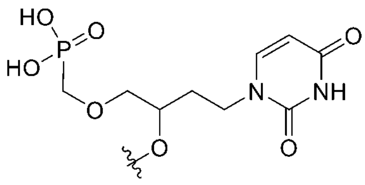 |
| 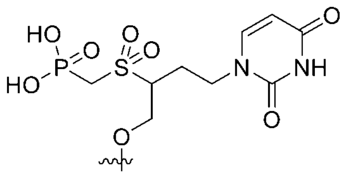 | 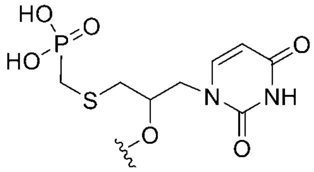 |
| 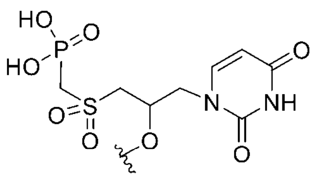 | 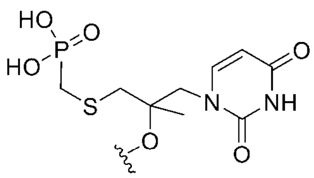 |
| 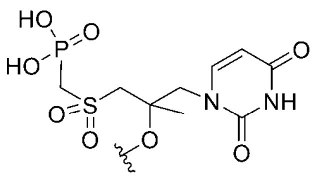 | 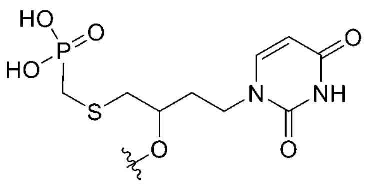 |

| | |
|---|---|
| 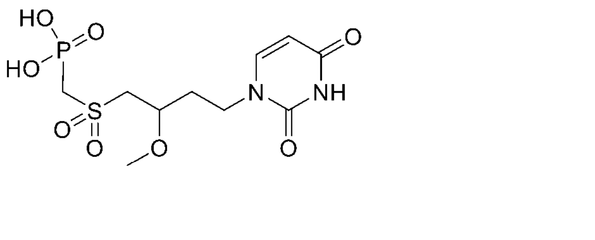 | 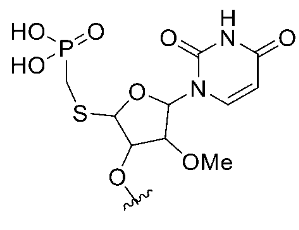 . |

**84.** The oligonucleotides according to claims 56-83, wherein the internucleoside linking group is independently an alkylphosphonate linking group, a phosphodiester internucleoside linking group, or a phosphorothioate nucleoside linking group.

**85.** The oligonucleotides according to claims 1-84, wherein the oligonucleotides are double-stranded ribonucleic acid (dsRNA) agents comprising: a sense strand and an antisense strand, wherein the sense strand and the antisense strand are completely or partially complementary, and the antisense strand is partially or completely complementary to a nucleic acid target gene; at least one of the sense strand and the antisense strand is the oligonucleotides of the represented 5'-terminal nucleotides according to claims 1-84; and the double-stranded ribonucleic acid (dsRNA) agents optionally further comprise an independent targeting group.

**86.** The oligonucleotides according to claim 85, wherein in the double-stranded ribonucleic acid (dsRNA) agents, the antisense strand comprises the oligonucleotides of the represented 5'-terminal nucleotides according to claims 1-84.

**87.** The oligonucleotides according to claims 1-86, wherein in the oligonucleotides or double-stranded ribonucleic acid (dsRNA), each strand comprises 8-40 nucleotides.

**88.** The oligonucleotides according to claims 1-87, wherein the oligonucleotides or double-stranded ribonucleic acid (dsRNA) agents are for use in the preparation of a medicament for inhibiting gene expression.

**89.** A compound represented by formula (I), formula (II), and formula (III), or stereoisomers thereof:

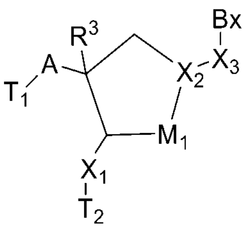
formula (I),
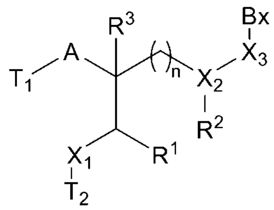
formula (II),
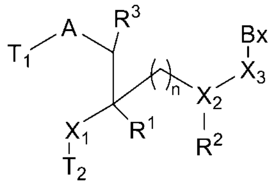
formula (III)

wherein each $T_1$ is independently an optionally protected phosphine moiety;
each $T_2$ is independently an active phosphorus group;
each $X_1$ is independently a chemical bond, O, S, $NJ_1$, or $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
each $X_2$ is independently $CR^{15}$ or N;
each $X_3$ is independently a chemical bond, optionally substituted $C_1$-$C_3$ alkylene, SO, $SO_2$, C(=O), or P(=O)R; R is OH, SH, $C_1$-$C_6$ alkyl, $NH_2$, or $NHSO_2CH_3$;
each Bx is independently a heterocyclic base moiety;
each $R^1$ and each $R^2$ are each independently H, halogen, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, sulfinyl, sulfonyl, or acetyl;
each $R^3$ and each $R^{15}$ are each independently H, halogen, optionally substituted $C_1$-$C_6$ alkyl, optionally substituted $C_1$-$C_6$ alkoxy, optionally substituted $C_2$-$C_6$ alkenyl, or optionally substituted $C_2$-$C_6$ alkynyl;
each A independently has one of the following formulas:

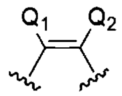, 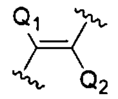, 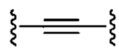, 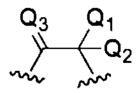, 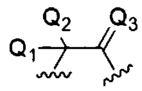, 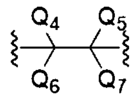, 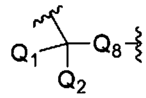,

and

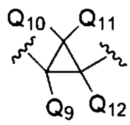,

wherein each $Q_1$ and each $Q_2$ are each independently H, halogen, -CN, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^4R^5$;

each $Q_3$ is independently O, S, $NR^6$, or $CR^7R^8$;

$Q_4$, $Q_5$, $Q_6$, $Q_7$, $Q_9$, $Q_{10}$, $Q_{11}$, and $Q_{12}$ are each independently H, halogen, optionally protected hydroxyl, acetoxy, azido, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^9R^{10}$;

$Q_8$ is O, S, SO, $SO_2$, $PR^{16}R^{17}$, or $NR^{11}$;

$R^{16}$ and $R^{17}$ are independently (=O), (=S), OH, SH, $C_1$-$C_6$ alkyl, or $NR^{18}R^{19}$;

each $R^4$, each $R^5$, each $R^6$, each $R^7$, each $R^8$, each $R^9$, each $R^{10}$, each $R^{11}$, each $R^{18}$, and each $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$;

$M_1$ is C(Rd)(Re) or C(Rd)(Re)C(Rf)(Rg), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$;

each $J_3$, each $J_4$, each $J_5$, and each $J_6$ are independently H or $C_1$-$C_6$ alkyl;

each n is independently 0, 1, or 2;

each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**90.** The compound according to claim 89, wherein each $T_1$ is independently an optionally protected phosphine moiety having the following formula:

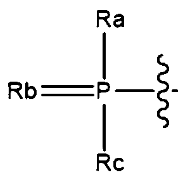

wherein:

Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group;

a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;

each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**91.** The compound according to claim 90, wherein a protecting group for the hydroxyl or sulfhydryl is independently selected from methyl, ethyl, benzyl (Bn), phenyl, isopropyl, tert-butyl, acetyl, chloroacetyl, trichloroacetyl, trifluoroacetyl, pivaloyl, tert-butoxymethyl, methoxymethyl, 1-ethoxyethyl, 1-(2-chloroethoxy)ethyl, 2-trimethylsilylethyl, allyl, cyclohexyl (cHex), 9-fluorenylmethoxycarbonyl, methanesulfonyl, p-toluenesulfonyl, trifluoromethanesulfonyl, benzoyl, benzoylformate, p-phenylbenzoyl, 4-methoxybenzyl, monomethoxytrityl, dimethoxytrityl, trimethoxytrityl, 4-chlorobenzyl, 4-nitrobenzyl, 2,4-dinitrophenyl, 4-acyloxybenzyl, 2-methylphenyl, 2,6-dimethylphenyl, 2-chlorophenyl, 2,6-dichlorobenzyl, diphenylmethyl, triphenylmethyl, 4-methylthio-1-butyl, 2-(S-acetylthio)ethyl (SATE), 2-cyanoethyl, 2-cyano-1,1-dimethylethyl (CDM), 4-cyano-2-butenyl, 2-(trimethylsilyl)ethyl (TSE), 2-(phenylthio)ethyl, 2-(triphenylsilyl)ethyl, 2-(benzylsulfonyl)ethyl, 2,2,2-trichloroethyl, 2,2,2-tribromoethyl, 2,3-dibromopropyl, 2,2,2-trifluoroethyl, phenylthio, 2-chloro-4-tritylphenyl, 2-bromophenyl, 2-[N-isopropyl-N-(4-methoxybenzoyl)amino]ethyl, 4-(N-trifluoroacetylamino)butyl, 4-oxopentyl, 4-tritylaminophenyl, 4-benzylaminophenyl, tetrahydropyranyl, morpholino, trimethylsilyl, triethylsilyl, tert-butyldimethylsilyl, tert-butyldiphenylsilyl, triphenylsilyl, triisopropylsilyl, pivaloyloxymethyl (POM), and 9-phenylxanthine-9-yl.
the amino-protecting group is independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), benzyl, formyl, acetyl, pivaloyl, trihaloacetyl, benzoyl, nitrophenyl, acetyl, 2-nitrobenzenesulfonyl, phthalimido (Pht), p-toluenesulfonyl (Tos), trityl (Trt), 2,4-dimethoxybenzyl (PMB), and dithiosuccinyl.

**92.** The compounds according to claims 90-91, wherein each $T_1$ is independently an optionally protected phosphine moiety having the following formula:

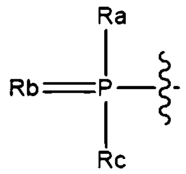

wherein:

Ra and Rc are each independently selected from protected hydroxyl and protected sulfhydryl, and $R_b$ is O or S;
protecting groups for the hydroxyl are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, methanesulfonyl, p-toluenesulfonyl, trifluoromethanesulfonyl, trityl, monomethoxytrityl, dimethoxytrityl, trimethoxytrityl, pivaloyloxymethyl (POM), and substituted 9-phenylxanthine-9-yl;
protecting groups for the sulfhydryl are each independently selected from methyl, ethyl, acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, methanesulfonyl, p-toluenesulfonyl, trifluoromethanesulfonyl, trityl, monomethoxytrityl, dimethoxytrityl, trimethoxytrityl, and substituted 9-phenylxanthine-9-yl.

**93.** The compounds according to claim 92, wherein the protecting groups for the hydroxyl are each independently selected from acetyl, benzyl, tert-butyldimethylsilyl, pivaloyloxymethyl (POM), tert-butyldiphenylsilyl, and 4,4'-dimethoxytrityl; the protecting groups for the sulfhydryl are each independently selected from benzyl, 4,4'-dimethoxytrityl, and trityl.

**94.** The compounds according to claims 90-93, wherein $R_b$ is O or S, and Ra and Rc are each independently selected from protected hydroxyl, $C_1$-$C_6$ alkyl, and $C_1$-$C_6$ alkoxy.

**95.** The compounds according to claims 90-94, wherein $R_b$ is O, and Ra and Rc are each independently selected from OH, SH, $OCH_3$, $OCH_2CH_3$, $OCH(CH_3)_2$, $OCH_2OC(=O)C(CH_3)_3$, $OCH_2CH_2CN$, and $NHSO_2CH_3$.

**96.** The compounds according to claims 89-95, wherein each $T_2$ is independently an active phosphorus group having the following structure:

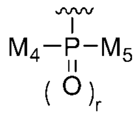
,

wherein $M_4$ is H, optionally substituted $C_1$-$C_6$ alkyl, OH, $OJ_7$, SH, $SJ_7$, or $NJ_7J_8$, and $M_5$ is optionally substituted $C_1$-$C_6$ alkyl, OH, $OJ_7$, SH, $SJ_7$, or $NJ_7J_8$, wherein each $J_7$ or $J_8$ is independently and optionally substituted $C_1$-$C_6$ alkyl or sulfonyl; r is 0 or 1;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**97.** The compounds according to claim 96, wherein each $J_7$ or $J_8$ is independently substituted $C_1$-$C_6$ alkyl, and a substituent is selected from cyano and halogen.

**98.** The compounds according to claim 96, wherein $M_4$ is selected from methyl, ethyl, propyl, and isopropyl.

**99.** The compounds according to claim 96, wherein $M_4$ is selected from methanesulfonamido.

**100.**
The compounds according to claim 96, wherein $M_4$ is $OJ_7$, wherein $J_7$ is substituted $C_1$-$C_6$ alkyl, and a substituent is selected from cyano and halogen.

**101.**
The compounds according to claim 96, wherein $M_4$ is $O(CH_2)_2CN$; $M_5$ is $N(CH(CH_3)_2)_2$; r is 0.

**102.**
The compounds according to claim 96, wherein each $T_2$ active phosphorus group is independently a phosphoramidite.

**103.**
The compounds according to claim 96, wherein each $T_2$ active phosphorus group is independently selected from diisopropylcyanoethoxy phosphoramidite, diisopropylethyl phosphoramidite, and H-phosphonate.

**104.**
The compounds according to claims 89-103, wherein each $X_1$ is independently O.

**105.**
The compounds according to claims 89-104, wherein each $B_X$ heterocyclic base moiety is independently selected from a natural nucleobase, a modified nucleobase, and a universal base.

**106.**
The compounds according to claim 105, wherein each $B_X$ heterocyclic base moiety is independently pyrimidine, substituted pyrimidine, pseudouracil, substituted pseudouracil, purine, hypoxanthine, or substituted purine.

**107.**
The compounds according to claim 105, wherein each $B_X$ heterocyclic base moiety is independently 2-thiouracil, 5-fluorouracil, dihydrouridine (D), 7-methylguanosine (m7G), uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine.

**108.**
The compounds according to claims 89-106, wherein each $R^{15}$ and each $R^3$ are each independently H.

**109.**
The compounds according to claims 89-108, wherein each $R^1$ and each $R^2$ are each independently H, methanesulfonyl, or acetyl.

**110.**
The compounds according to claims 89-109, wherein each $X_2$ is independently N, and each $R^1$ and each $R^2$ are each

independently H, methanesulfonyl, or acetyl.

**111.** The compounds according to claims 89-110, wherein A has one of the following formulas:

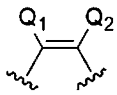, 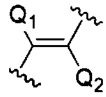, and 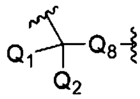,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**112.**
The compounds according to claims 89-111, wherein each $Q_1$ and each $Q_2$ are each independently H, F, -CN, or methyl.

**113.**
The compounds according to claim 112, wherein each $Q_1$ and each $Q_2$ are each independently H.

**114.**
The compounds according to claims 89-113, wherein a proviso is that when $M_1$ is C(Rd)(Re), $X_2$ is C, $X_3$ is a chemical bond, and A is

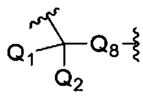,

$Q_8$ is S, SO, $SO_2$, $PR^{16}R^{17}$, or $NR^{11}$.

**115.**
The compounds according to claims 89-114, wherein a proviso is that when $M_1$ is C(Rd)(Re), $X_2$ is C, $X_3$ is a chemical bond, and A is

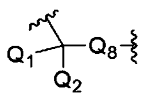,

$Q_8$ is S, SO, $SO_2$, or $NR^{11}$.

**116.**
The compounds according to claims 89-115, wherein $X_2$ is N, and $X_3$ is a chemical bond, $-CH_2-$, $-CH_2CH_2-$, SO, or $SO_2$.

**117.**
The compounds according to claims 89-116, wherein $X_2$ is CH, and $X_3$ is a chemical bond.

**118.**
The compounds according to claims 89-117, wherein n is 0 or 1.

**119.**
The compound according to claim 89, having a compound represented by formula (I-1) or a stereoisomer thereof:

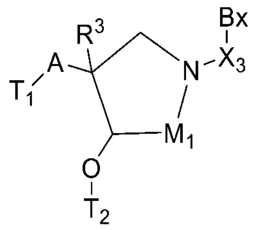

formula (I-1)

wherein $T_1$, $T_2$, A, $R^3$, and Bx are each as defined in claim 89; $X_3$ is a chemical bond, C(=O), P(=O)R, SO, or $SO_2$; $M_1$ is C(Rd)(Re) or C(Rd)(Re)C(Rg)(Rf), wherein each Rd, each Re, each Rg, and each Rf are each independently selected from the following substituent groups: hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl; R is OH, SH, $C_1$-$C_6$ alkyl, $NH_2$, or $NHSO_2CH_3$.

**120.**
The compound according to claim 119, wherein each Rd, each Re, each Rg, and each Rf are each independently selected from the following substituent groups: hydrogen, fluorine, hydroxyl, $C_1$-$C_6$ alkoxy, and $O(CH_2)_2$-$OCH_3$.

**121.**
The compound according to claim 120, wherein each Rd, each Re, each Rg, and each Rf are each independently hydrogen.

**122.**
The compounds according to claims 89-121, wherein $R^3$ is hydrogen.

**123.**
The compounds according to claims 89-122, wherein $X_3$ is $SO_2$ or a chemical bond.

**124.**
The compounds according to claims 89-123, wherein A has one of the following formulas:

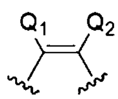 , 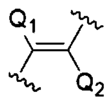 , and 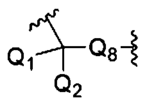 ,

wherein $Q_1$ and $Q_2$ are each independently H, halogen, or optionally substituted $C_1$-$C_6$ alkyl;
$Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**125.**
The compound according to claim 89, having a compound represented by formula (I-2) or a stereoisomer thereof:

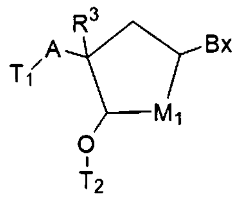

formula (I-2)

wherein $T_1$, $T_2$, A, $R^3$, and Bx are each as defined in claim 89; $M_1$ is C(Rd)(Re) or C(Rd)(Re)C(Rg)(Rf), wherein each Rd, each Re, each Rg, and each Rf are each independently selected from the following substituent groups: hydrogen,

halogen, hydroxyl, $C_1$-$C_6$ alkyl, $C_1$-$C_6$ alkoxy, $C_1$-$C_6$ alkylsulfhydryl, $O(CH_2)_2$-$OCH_3$, $NJ_5$, CN, $OC(=O)J_5$, $OC(=O)N(J_5)(J_6)$, and $C(=O)N((J_5)(J_6)$, wherein $J_5$ and $J_6$ are independently H or $C_1$-$C_6$ alkyl.

**126.**
The compound according to claim 125, wherein each Rd, each Re, each Rg, and each Rf are each independently selected from the following substituent groups: hydrogen, fluorine, hydroxyl, $C_1$-$C_6$ alkoxy, and $O(CH_2)_2$-$OCH_3$.

**127.**
The compound according to claim 125, wherein $M_1$ is $CH_2$ or $CH_2CH_2$.

**128.**
The compounds according to claims 125-127, wherein $R^3$ is hydrogen.

**129.**
The compounds according to claims 125-128, wherein A has one of the following formulas:

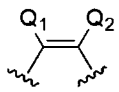 , 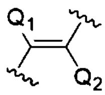 , and 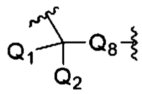 ,

wherein $Q_1$ and $Q_2$ are each independently H, halogen, or optionally substituted $C_1$-$C_6$ alkyl;
$Q_8$ is S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$;
$R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**130.**
The compound according to claim 89, having a compound represented by formula (II-1) or a stereoisomer thereof:

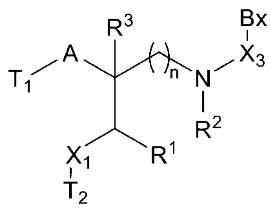
formula (II-1)

wherein $T_1$, $T_2$, $X_1$, $X_3$, A, $R^3$, Bx, and n are each as defined in claim 89; $R^1$ and $R^2$ are each independently H, methanesulfonyl, or acetyl.

**131.**
The compound according to claim 130, wherein n is 0 or 1.

**132.**
The compounds according to claims 130-131, wherein $X_3$ is $CH_2$ or $CH_2CH_2$.

**133.**
The compound according to claim 89, having a compound represented by formula (II-2) or formula (II-3) or a stereoisomer thereof:

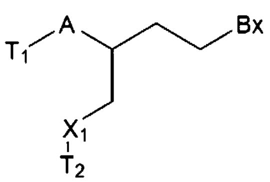
formula (II-2),

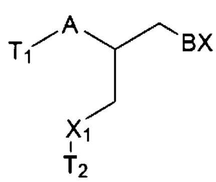
formula (II-3)

wherein $T_1$, $T_2$, $X_1$, A, and Bx are each as defined in claim 89.

**134.**
The compound according to claim 89, having a compound represented by formula (III-1) or formula (III-2) or a stereoisomer thereof:

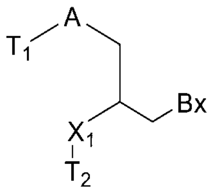 formula (III-1), 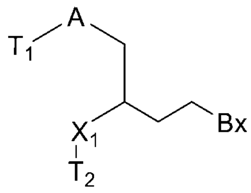 formula (III-2)

wherein $T_1$, $T_2$, $X_1$, A, and Bx are each as defined in claim 89.

**135.**
The compounds according to claims 130-134, wherein A has one of the following formulas:

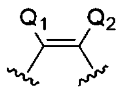 , 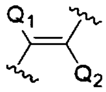 , and 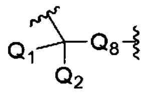 ,

wherein $Q_1$ and $Q_2$ are each independently H, halogen, -CN, or optionally substituted $C_1$-$C_6$ alkyl; $Q_8$ is O, S, SO, $SO_2$, $PR^{16}R^{17}$, or $NR^{11}$; $R^{16}$ and $R^{17}$ are independently (=O), (=S), OH, SH, $C_1$-$C_6$ alkyl, or $NR^{18}R^{19}$; $R^{11}$, $R^{18}$, and $R^{19}$ are independently H, optionally substituted $C_1$-$C_6$ alkyl, optionally substituted $C_1$-$C_6$ alkoxy, methanesulfonyl, sulfo, $C(=O)J_3$, $C(=O)OJ_3$, or $C(=O)N(J_3)(J_4)$; $J_3$ and $J_4$ are independently H or $C_1$-$C_6$ alkyl;
each optionally substituted group comprises one or more substituent groups independently selected from halogen, hydroxyl, $C_1$-$C_6$ alkyl, $C_1$-$C_6$ alkoxy, $C_1$-$C_6$ alkylsulfhydryl, and CN.

**136.**
The compounds according to claims 118-135, wherein each $Q_1$ and each $Q_2$ are each independently H, F, -CN, or methyl.

**137.**
The compounds according to claim 136, wherein each $Q_1$ and each $Q_2$ are each independently H.

**138.**
The compounds according to claims 118-137, wherein $T_1$ is an optionally protected phosphine moiety having the following formula:

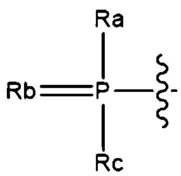

wherein:
Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, amino or protected/substituted amino, and a natural or modified nucleoside, and $R_b$ is O, S, or $NR^{12}$, wherein $R^{12}$ is hydrogen, $C_1$-$C_6$ alkyl, or an amino-protecting group.

**139.**
The compounds according to claim 138, wherein a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl.

**140.**

The compounds according to claim 138, wherein $R_b$ is O or S, and Ra and Rc are each independently selected from protected hydroxyl, $C_1$-$C_6$ alkyl, and $C_1$-$C_6$ alkoxy.

**141.**

The compounds according to claim 138, wherein $R_b$ is O, and Ra and Rc are each independently selected from OH, $OCH_3$, $OCH_2CH_3$, $OCH(CH_3)_2$, $OCH_2OC(=O)C(CH_3)_3$, $OCH_2CH_2CN$, and $NHSO_2CH_3$.

**142.**

The compounds according to claims 118-141, wherein $T_2$ is an active phosphorus group, and the active phosphorus group is a phosphoramidite.

**143.**

The compounds according to claim 142, wherein the $T_2$ active phosphorus group is selected from diisopropylcyanoethoxy phosphoramidite, diisopropylethyl phosphoramidite, and H-phosphonate.

**144.**

The compounds according to claims 118-142, wherein the $B_X$ heterocyclic base moiety is selected from a natural nucleobase, a modified nucleobase, and a universal base.

**145.**

The compounds according to claim 144, wherein the $B_X$ heterocyclic base moiety is pyrimidine, substituted pyrimidine, pseudouracil, substituted pseudouracil, purine, hypoxanthine, or substituted purine.

**146.**

The compounds according to claim 144, wherein the $B_X$ heterocyclic base moiety is 2-thiouracil, 5-fluorouracil, dihydrouridine (D), 7-methylguanosine (m7G), uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine.

**147.**

A compound represented by formula (IV) or a stereoisomer thereof:

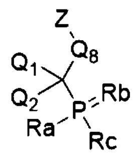

formula (IV)

wherein $Q_8$ is S, SO, $SO_2$, $PR^{16}R^{17}$, or $NR_{11}$; $R^{16}$ and $R^{17}$ are independently (=O), (=S), OH, SH, $C_1$-$C_6$ alkyl, or $NR^{18}R^{19}$; Ra and Rc are each independently selected from hydroxyl or protected hydroxyl, sulfhydryl or protected sulfhydryl, optionally substituted $C_1$-$C_6$ alkyl, optionally substituted $C_1$-$C_6$ alkoxy, protected or optionally substituted amino, and a natural or modified nucleoside, and $R_b$ is O, S, or $NR^{12}$, wherein $R^{12}$ is hydrogen, $C_1$-$C_6$ alkyl, or an amino-protecting group;
a substituent in the substituted amino is selected from optionally substituted $C_1$-$C_6$ alkyl, optionally substituted $C_2$-$C_6$ alkenyl, optionally substituted $C_2$-$C_6$ alkynyl, sulfinyl, sulfonyl, and acetyl;
$Q_1$ and $Q_2$ are each independently H, halogen, -CN, optionally substituted $C_1$-$C_6$ alkyl, optionally substituted $C_1$-$C_6$ alkoxy, optionally substituted $C_2$-$C_6$ alkenyl, optionally substituted $C_2$-$C_6$ alkynyl, or $NR^4R^5$;
each $R^4$, each $R^5$, each $R^{11}$, each $R^{18}$, and each $R^{19}$ are independently H, optionally substituted $C_1$-$C_6$ alkyl, optionally substituted $C_1$-$C_6$ alkoxy, methanesulfonyl, or sulfo;
Z is a nucleoside comprising a phosphoramidite or a sugar or sugar surrogate moiety;
each substituted group comprises one or more substituent groups optionally and independently selected from halogen, hydroxyl, $C_1$-$C_6$ alkyl, $C_1$-$C_6$ alkoxy, $C_1$-$C_6$ alkylsulfhydryl, and CN.

**148.**

The compound according to claim 147, wherein $Q_8$ is S, SO, or $SO_2$.

**149.**

The compounds according to claims 147-148, wherein $Q_1$ and $Q_2$ are each independently H.

**150.**

The compounds according to claims 147-149, wherein in the nucleoside comprising a sugar or sugar surrogate moiety, the sugar or sugar surrogate moiety comprises a 5-membered furanose ring, a non-furanose ring, or a 5- to 6-membered carbocyclic system, or an open system.

**151.**

The compounds according to claim 150, wherein the sugar or sugar surrogate moiety is selected from morpholinyl, cyclohexenyl, cyclohexyl, cyclopentyl, pyranyl, cyclohexanehexol group, furanose, unlocked nucleobase analog (UNA), glycerol nucleobase analog (GNA), locked nucleic acid (LNA), and bridged nucleic acid (BNA).

**152.**

The compounds according to claim 151, wherein $Q_8$ is bonded to the 4'-carbon or 5'-carbon of the sugar or sugar surrogate moiety.

**153.**

The compounds according to claims 150-151, wherein the nucleoside comprising a sugar or sugar surrogate moiety has the following structural schematics:

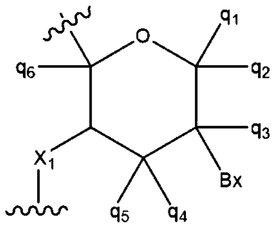 , 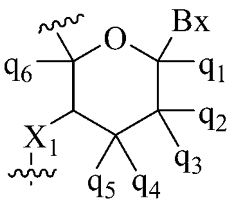 , 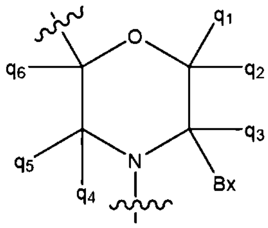 ,

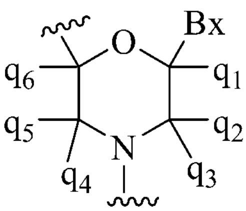 , 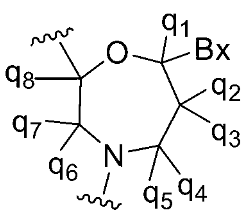 , 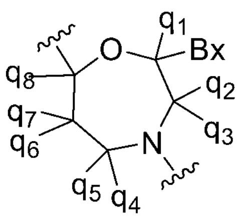 ,

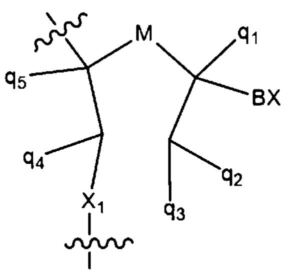 , 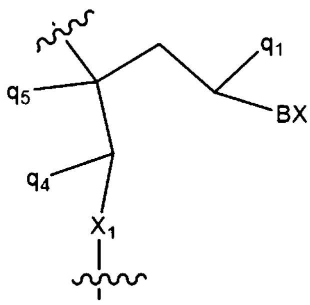 , 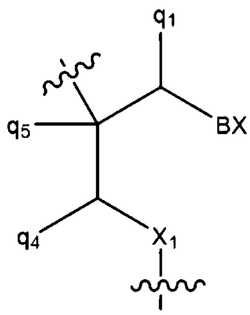 ,

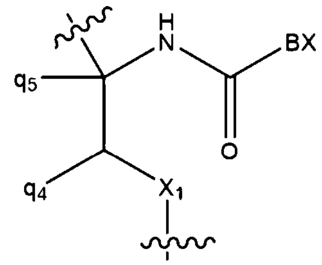, 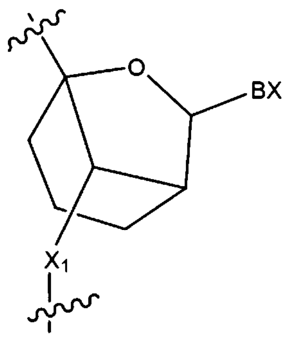, 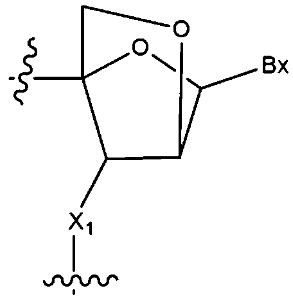,

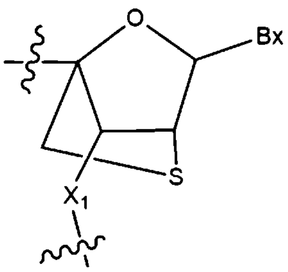, 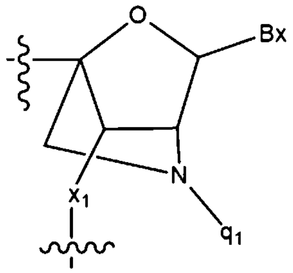, 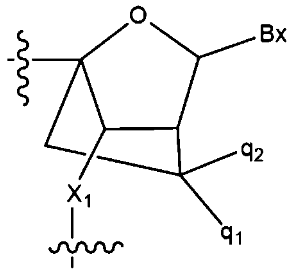,

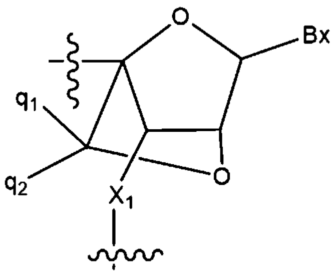, 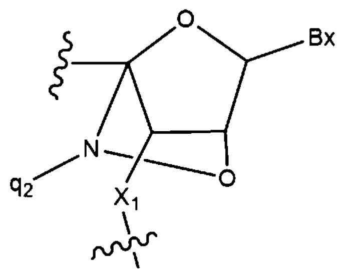, 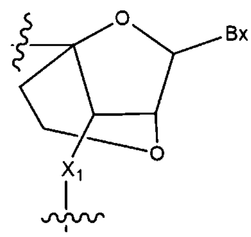,

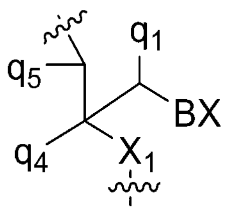, 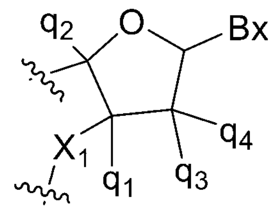, and 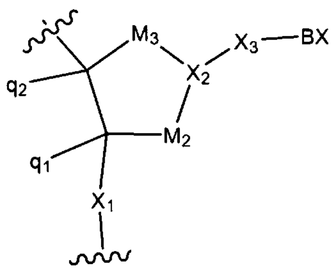,

wherein $M_2$ is $C(q_3)(q_4)$ or $C(q_3)(q_4)C(q_5)(q_6)$;
$M_3$ is O, S, $NR^{13}$, $C(q_7)(q_8)$, $C(q_7)(q_8)C(q_9)(q_{10})$, $C(q_7)=C(q_8)$, or $OC(q_7)(q_8)$;
$X_1$ is a chemical bond or is selected from O, S, $NJ_1$, and $CJ_1J_2$, wherein $J_1$ and $J_2$ are each independently hydrogen, halogen, sulfonyl, sulfinyl, optionally substituted $C_1$-$C_6$ alkyl, optionally substituted $C_3$-$C_6$ cycloalkyl, optionally substituted $C_2$-$C_6$ alkenyl, optionally substituted $C_2$-$C_6$ alkynyl, optionally substituted $C_5$-$C_{12}$ aryl, optionally substituted 5- to 12-membered heteroaryl, or optionally substituted 5- to 12-membered heterocycle;
$X_2$ is $CR^{15}$ or N;
$X_3$ is a chemical bond, optionally substituted $C_1$-$C_3$ alkylene, SO, $SO_2$, C(=O), or P(=O)R;
R is OH, SH, $C_1$-$C_6$ alkyl, $NH_2$, or $NHSO_2CH_3$;
Bx is a heterocyclic base moiety;
$R^{15}$, $q_1$, $q_2$, $q_3$, $q_4$, $q_5$, $q_6$, $q_7$, $q_8$, $q_9$, and $q_{10}$ are each independently hydrogen, halogen, hydroxyl, $C_1$-$C_6$ alkyl, $C_1$-$C_6$ alkoxy, $C_1$-$C_6$ alkylsulfhydryl, $O(CH_2)_2$-$OCH_3$, CN, $OC(=O)J_5$, $OC(=O)N(J_5)(J_6)$, or $C(=O)N((J_5)(J_6)$, wherein $J_5$ and $J_6$ are independently H or $C_1$-$C_6$ alkyl;
$R^{13}$ is hydrogen or $C_1$-$C_6$ alkyl.

**154.**

The compounds according to claim 153, wherein each $R^{15}$, each $q_1$, each $q_2$, each $q_3$, each $q_4$, each $q_5$, each $q_6$, each $q_7$, each $q_8$, each $q_9$, and each $q_{10}$ are each independently selected from the following groups: hydrogen, fluorine, hydroxyl, $C_1$-$C_6$ alkyl, $C_1$-$C_6$ alkoxy, and $O(CH_2)_2$-$OCH_3$.

**155.**

The compounds according to claim 154, wherein each $R^{15}$, each $q_1$, each $q_2$, each $q_3$, each $q_4$, each $q_5$, each $q_6$, each $q_7$, each $q_8$, each $q_9$, and each $q_{10}$ are each independently selected from the following group: hydrogen.

**156.**

The compounds according to claims 153-155, wherein the nucleoside comprising a sugar or sugar surrogate moiety has the following structural schematics:

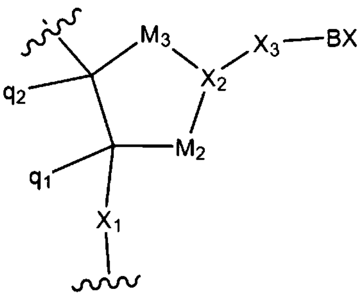

.

**157.**

The compounds according to claims 147-156,

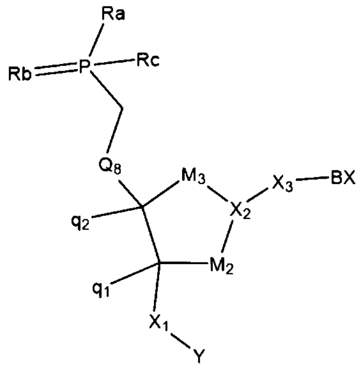

formula (IV-1)

wherein $Q_8$, Ra, Rc, $R_b$, $M_2$, $M_3$, $X_1$, $X_2$, $X_3$, $q_1$, $q_2$, and Bx are each as shown in claim 147.

**158.**

The compounds according to claim 157, wherein $R^{15}$, $q_1$, $q_2$, $q_3$, $q_4$, $q_5$, $q_6$, $q_7$, $q_8$, $q_9$, and $q_{10}$ are each independently selected from hydrogen, fluorine, hydroxyl, methyl, methoxy, and $O(CH_2)_2$-$OCH_3$.

**159.**

The compounds according to claim 158, wherein $R^{15}$, $q_1$, $q_2$, $q_3$, $q_4$, $q_5$, $q_6$, $q_7$, $q_8$, $q_9$, and $q_{10}$ are each independently selected from hydrogen.

**160.**

The compounds according to claims 157-159, wherein $M_3$ is O, S, $C(q_7)(q_8)$, $M_2$ is $C(q_3)(q_4)$, $X_2$ is $CR^{15}$ or N, and $X_3$ is a chemical bond, SO, or $SO_2$.

**161.**

The compounds according to claim 160, wherein $M_3$ is O, S, or $CH_2$, $M_2$ is $CH_2$, $X_2$ is CH, and $X_3$ is a chemical bond.

**162.**

The compounds according to claim 160, wherein $M_3$ is $CH_2$, $M_2$ is $CH_2$, $X_2$ is N, and $X_3$ is a chemical bond, SO, or $SO_2$.

**163.**
The compounds according to claims 157-159, wherein $M_3$ is $C(q_7)(q_8)$, $M_2$ is $C(q_3)(q_4)C(q_5)(q_6)$, $X_2$ is $CR^{15}$ or N, and $X_3$ is a chemical bond, SO, or $SO_2$.

**164.**
The compounds according to claim 163, wherein $M_3$ is $CH_2$, $M_2$ is $CH_2CH_2$, $X_2$ is CH, and $X_3$ is a chemical bond. 165. The compounds according to claim 163, wherein $M_3$ is $CH_2$, $M_2$ is $CH_2CH_2$, $X_2$ is N, and $X_3$ is a chemical bond, SO, or $SO_2$.

**165.**
The compounds according to claims 147-164, wherein $Q_8$ is S, SO, or $SO_2$.

**166.**
The compounds according to claims 147-165, wherein $R_b$ is O or S, and Ra and Rc are each independently selected from protected hydroxyl, $C_1$-$C_6$ alkyl, and $C_1$-$C_6$ alkoxy.

**167.**
The compounds according to claims 147-165, wherein Ra and Rc are each independently selected from hydroxyl, $OCH_3$, $OCH_2CH_3$, $OCH(CH_3)_2OCH_3$, $OCH_2CH_2CN$, and $NHSO_2CH_3$.

**168.**
The compounds according to claims 147-167, wherein the $B_X$ heterocyclic base moiety is selected from a natural nucleobase, a modified nucleobase, and a universal base.

**169.**
The compounds according to claim 168, wherein the $B_X$ heterocyclic base moiety is pyrimidine, substituted pyrimidine, pseudouracil, substituted pseudouracil, purine, hypoxanthine, or substituted purine.

**170.**
The compounds according to claim 168, wherein the $B_X$ heterocyclic base moiety is 2-thiouracil, 5-fluorouracil, dihydrouridine (D), 7-methylguanosine (m7G), uracil, 5-thiazolouracil, thymine, cytosine, pseudouracil, N1-methyl-pseudouracil, hypoxanthine, 5-methylcytosine, 5-methyluracil, 3-benzoyluracil, 2,6-diaminopurine, adenine, or guanine.

**171.**
The compounds according to claims 147-170, wherein the phosphoramidite is selected from diisopropylcyanoethoxy phosphoramidite, diisopropylethyl phosphoramidite, and H-phosphonate.

**172.**
The compounds according to claims 147-171, having the following specific structures:

| | |
|---|---|
| 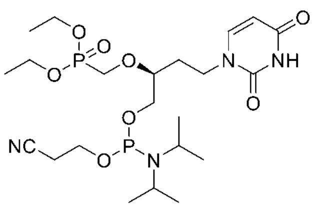 phosphoramidite-01 | 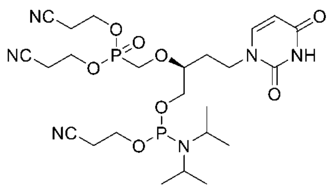 phosphoramidite-02 |

| | |
|---|---|
| 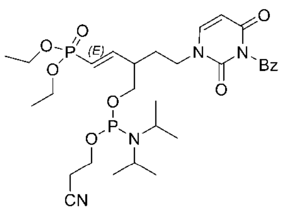 phosphoramidite-03 | 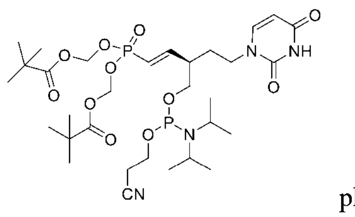 phosphoramidite-03-1 |
| 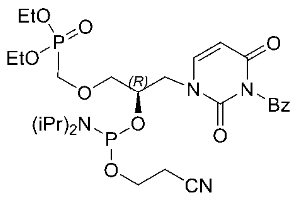 phosphoramidite-04 | 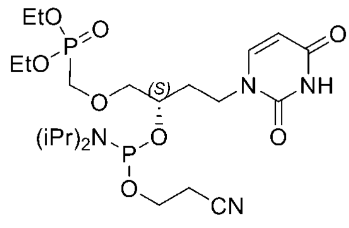 phosphoramidite-05 |
| 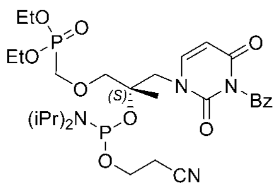 phosphoramidite-06 | 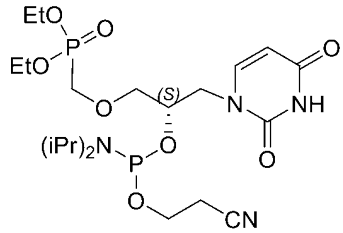 phosphoramidite-07 |
| 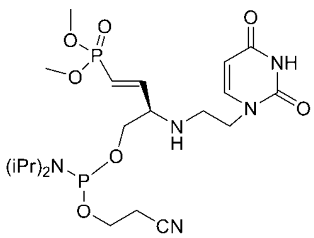 phosphoramidite-08 | 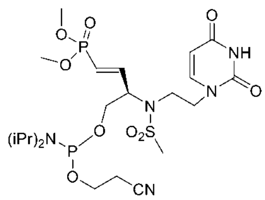 phosphoramidite-09 |
| 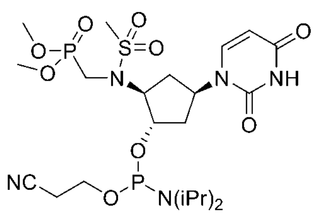 phosphoramidite-10 | 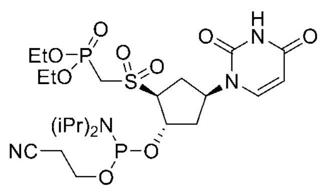 phosphoramidite-11-1 |
| 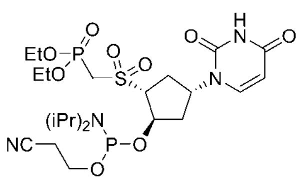 phosphoramidite-11-2 | 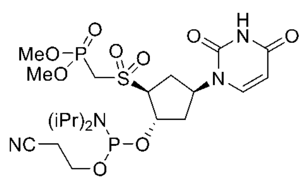 phosphoramidite-11-3 |
| 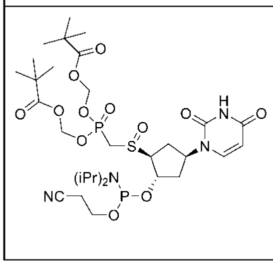 phosphoramidite-11-4 | 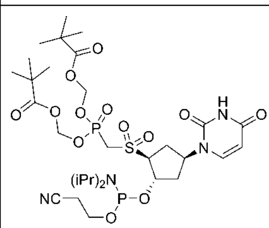 phosphoramidite-11-5 |

| | |
|---|---|
| 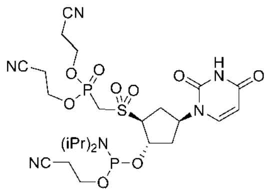 phosphoramidite-11-6 | 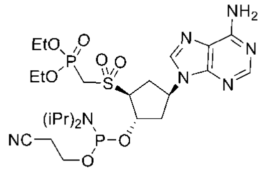 phosphoramidite-11-7 |
| 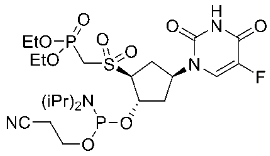 phosphoramidite-11-8 | 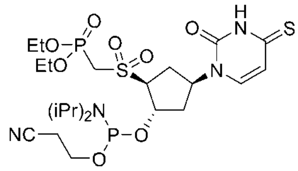 phosphoramidite-11-9 |
| 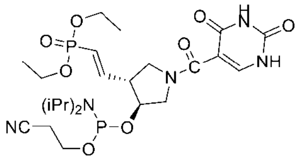 phosphoramidite-19-5 | 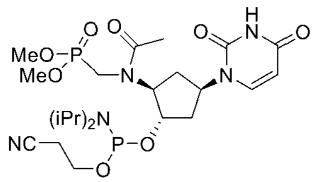 phosphoramidite-12-1 |
| 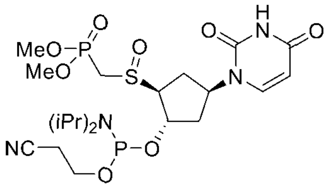 phosphoramidite-12 | 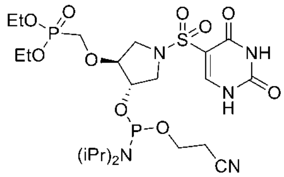 phosphoramidite-13 |
| 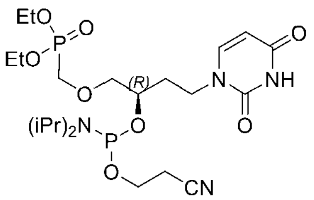 phosphoramidite-14 | 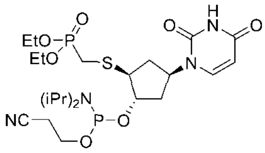 phosphoramidite-15-1 |
| 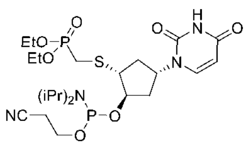 phosphoramidite-15-2 | 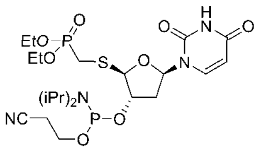 phosphoramidite-15-3 |
| 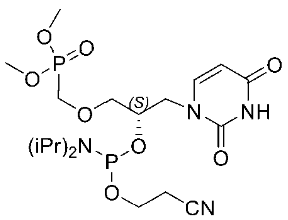 phosphoramidite-07-1 | 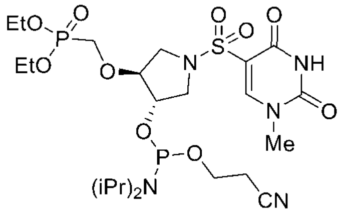 phosphoramidite-18 |
| 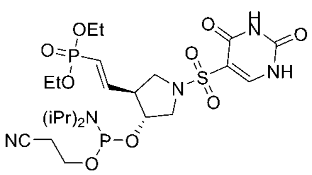 phosphoramidite-19- | 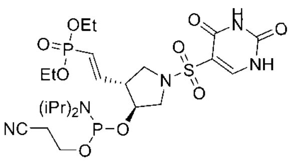 phosphoramidite-19-2 |

| 1 | |
|---|---|
| 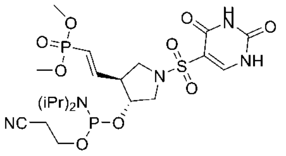 phosphoramidite-19-3 | 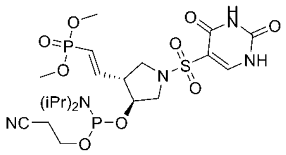 phosphoramidite-19-4 |
| 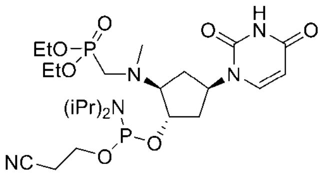 phosphoramidite-21 | 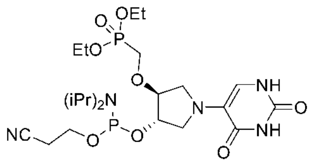 phosphoramidite-22 |
| 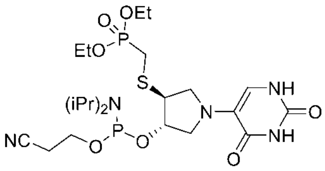 phosphoramidite-23 | 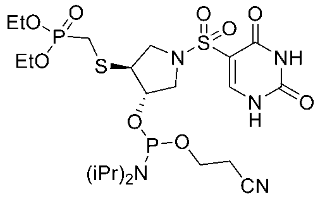 phosphoramidite-26 |
| 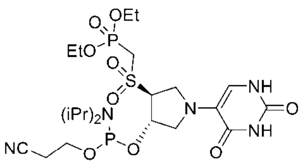 phosphoramidite-25 | 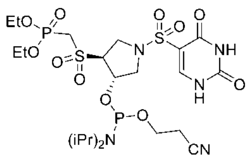 phosphoramidite-24 |
| 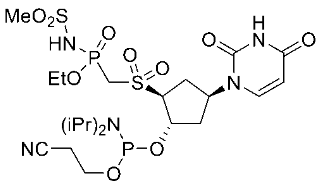 phosphoramidite-27 | 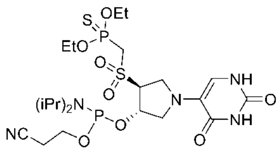 phosphoramidite-28 |
| 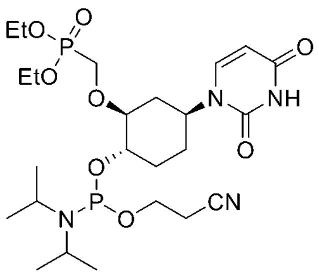 phosphoramidite-29 | 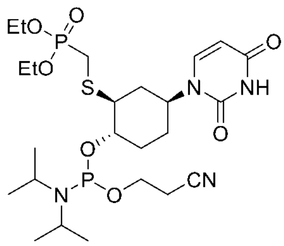 phosphoramidite-30 |
| 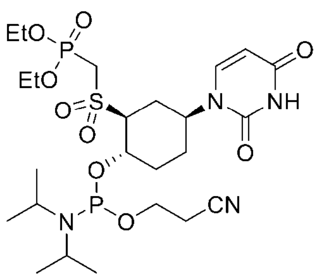 phosphoramidite-31 | 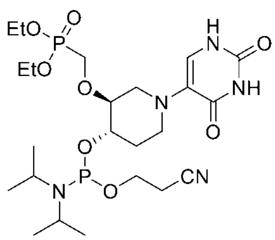 phosphoramidite-32 |

| | |
|---|---|
| 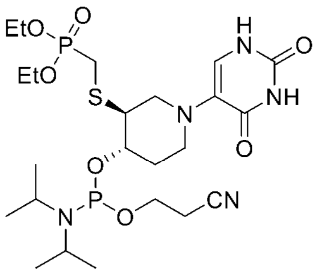 phosphoramidite-33 | 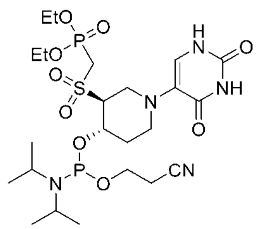 phosphoramidite-34 |
| 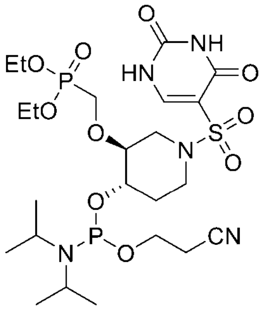 phosphoramidite-35 | 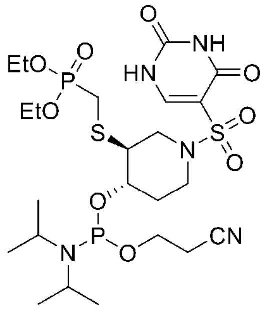 phosphoramidite-36-1 |
| 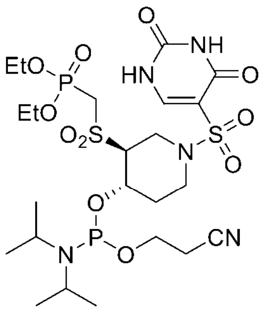 phosphoramidite-37 | 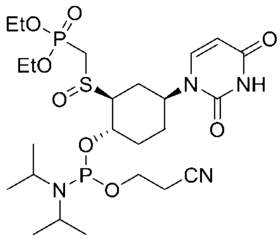 phosphoramidite-38 |
| 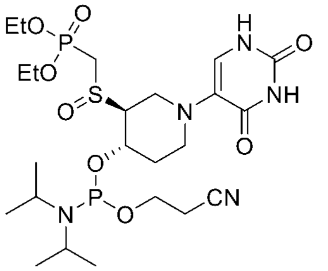 phosphoramidite-39 | 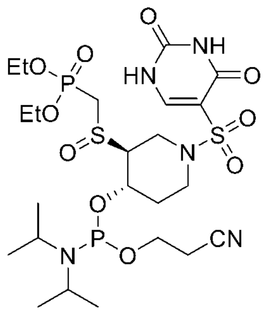 phosphoramidite-40 |
| 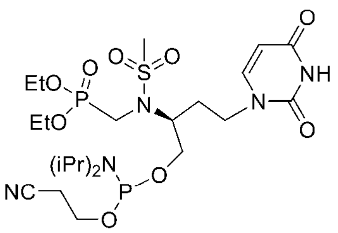 phosphoramidite-41 | 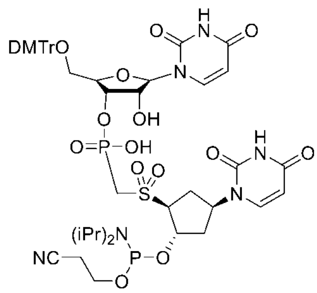 phosphoramidite-42-3 |

| | |
|---|---|
| 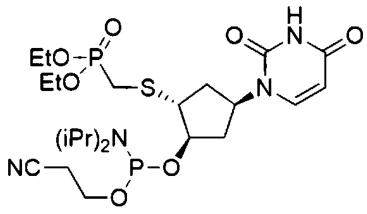<br>phosphoramidite-42-1 | 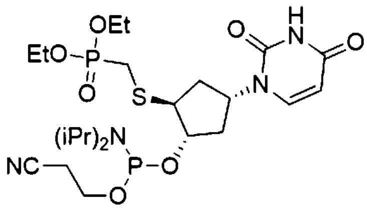<br>phosphoramidite-42-2 |
| 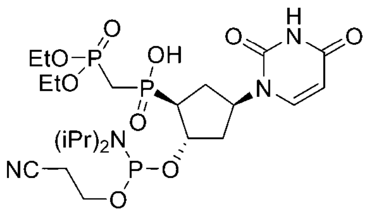<br>phosphoramidite-43-1 | 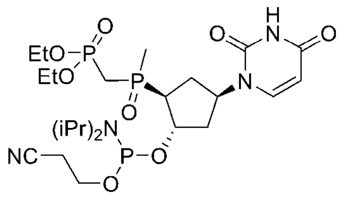<br>phosphoramidite-44-1 |
| 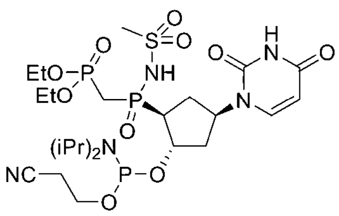<br>phosphoramidite-45-1 | 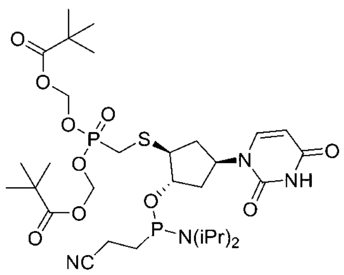<br>phosphoramidite-15-4 |
| 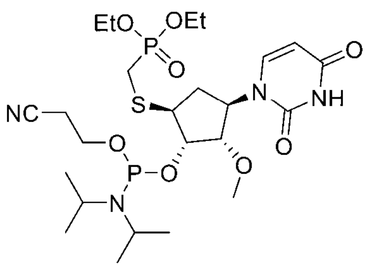<br>phosphoramidite-43 | 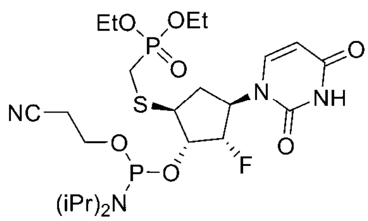<br>phosphoramidite-44 |
| 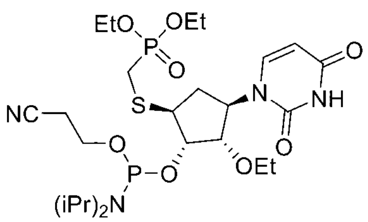<br>phosphoramidite-45 | 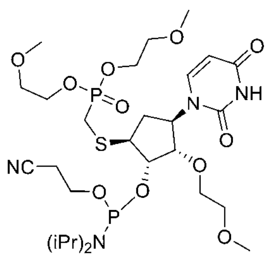<br>phosphoramidite-46 |

| | |
|---|---|
| 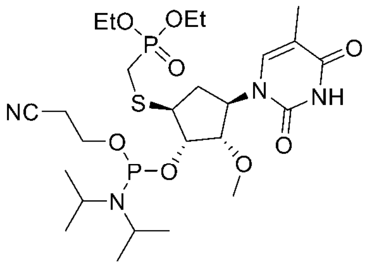 phosphoramidite-47 | 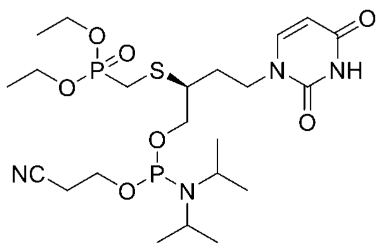 phosphoramidite-1-1 |
| 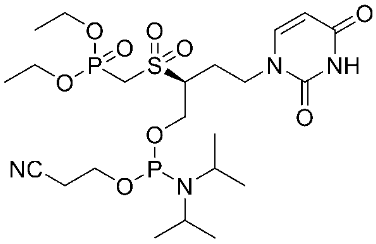 phosphoramidite-1-2 | 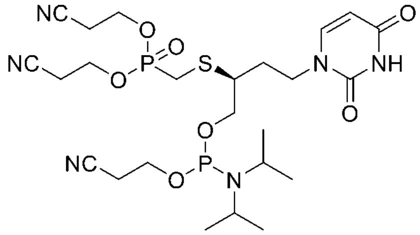 phosphoramidite-2-1 |
| 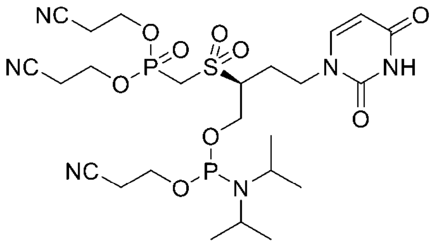 phosphoramidite-2-2 | 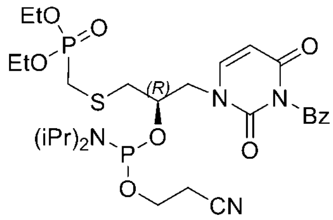 phosphoramidite-4-1 |
| 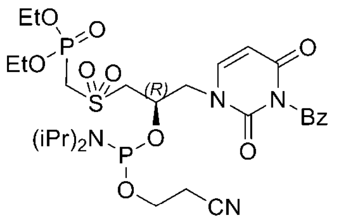 phosphoramidite-4-2 | 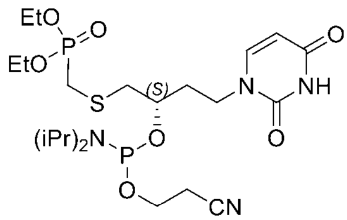 phosphoramidite-5-1 |
| 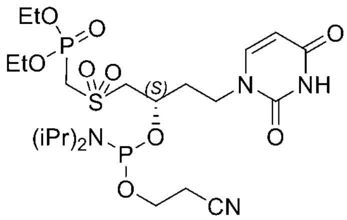 phosphoramidite-5-2 | 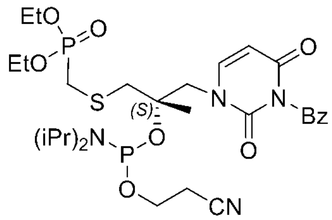 phosphoramidite-6-1 |

| | |
|---|---|
| 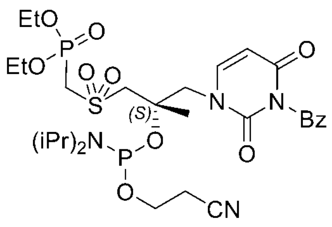 phosphoramidite-6-2 | 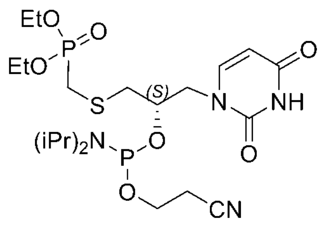 phosphoramidite-7-1 |
| 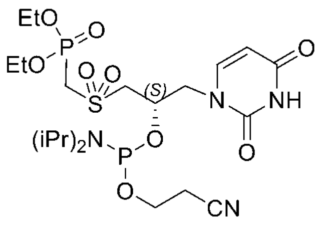 phosphoramidite-7-2 | 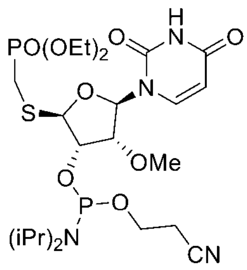 phosphoramidite-53 |
| 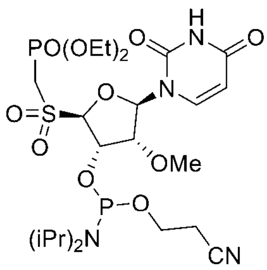 phosphoramidite-53-1. | |

or

| | |
|---|---|
| 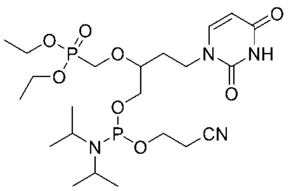 | 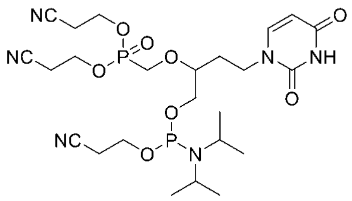 |
| 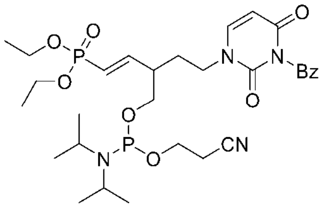 | 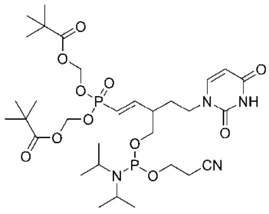 |
| 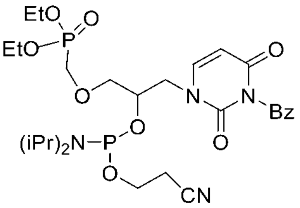 | 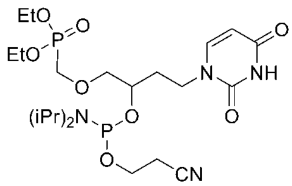 |
| | |

| | |
|---|---|
| 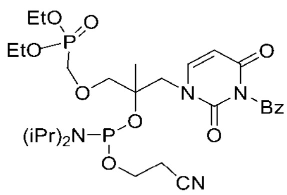 | 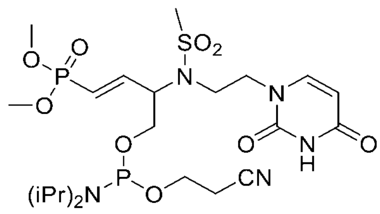 |
| 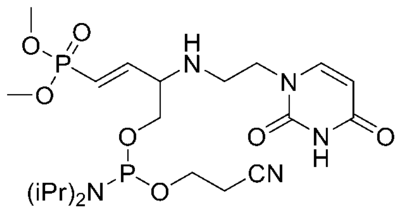 | 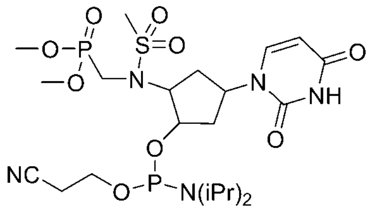 |
| 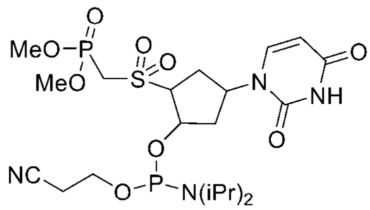 | 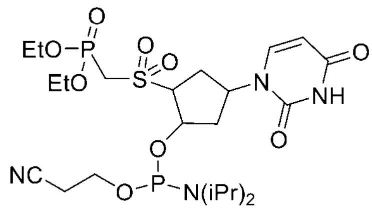 |
| 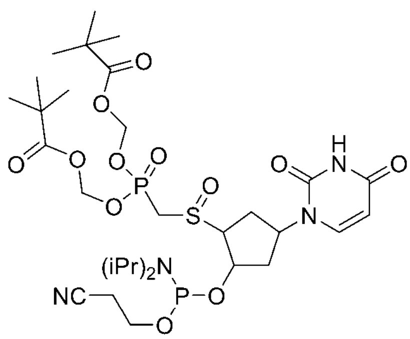 | 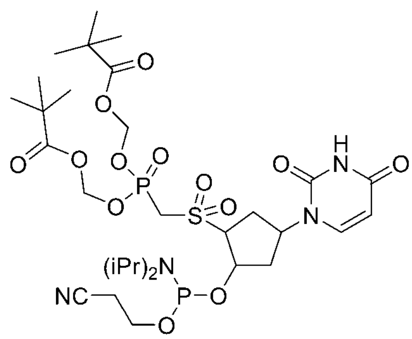 |
| 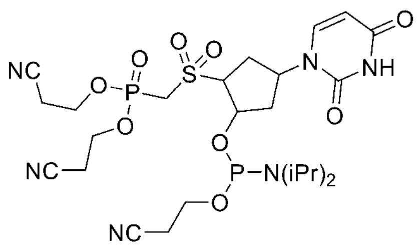 | 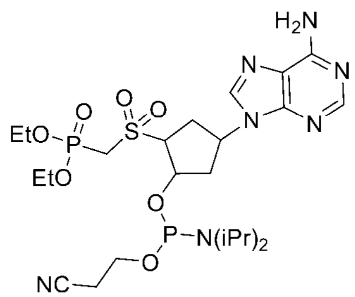 |
| 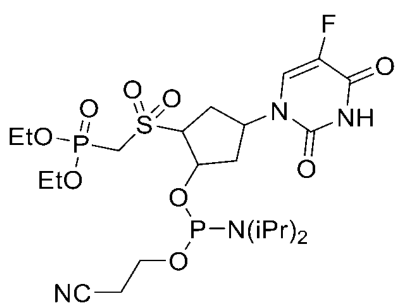 | 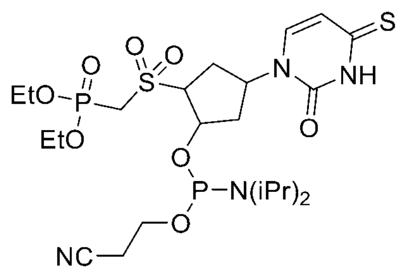 |

| | |
|---|---|
| 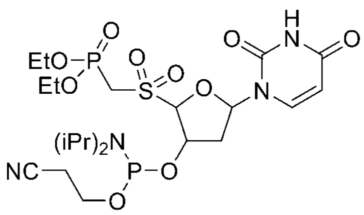 | 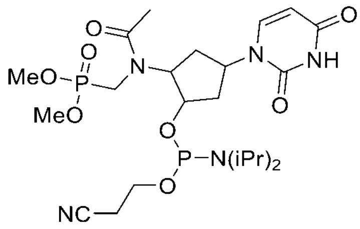 |
| 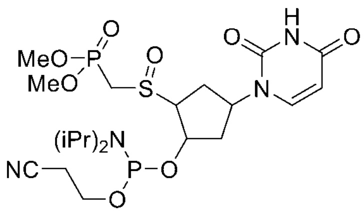 | 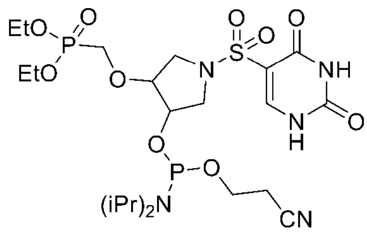 |
| 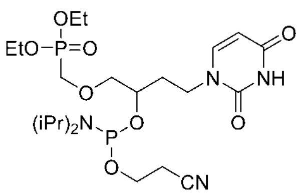 | 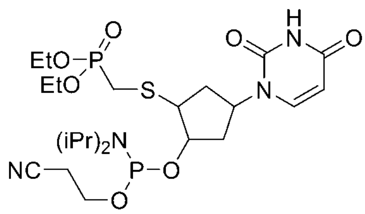 |
| 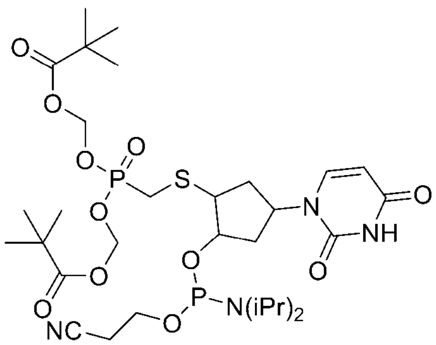 | 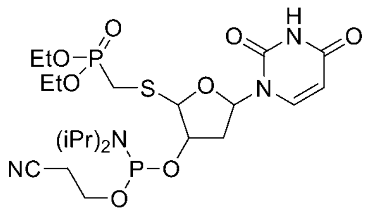 |
| 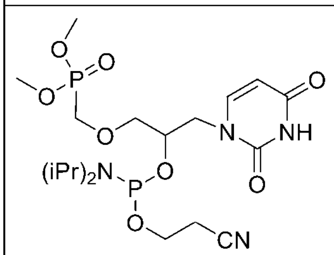 | 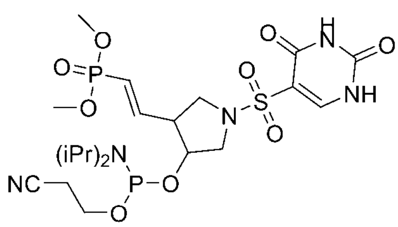 |
| 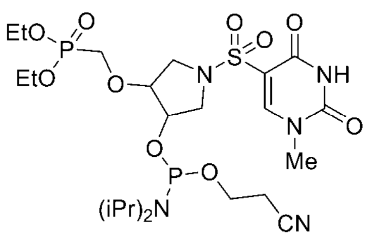 | 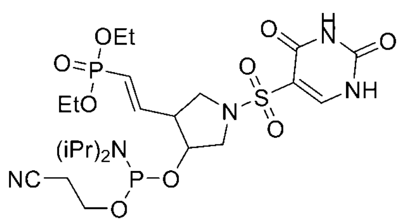 |

| | |
|---|---|
| 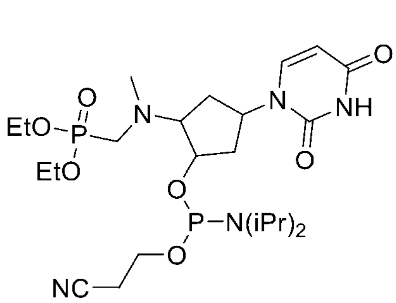 | 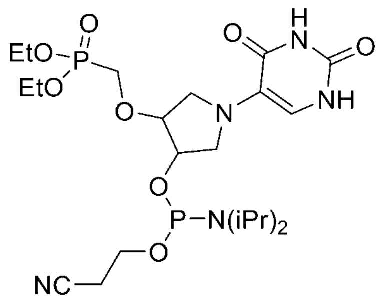 |
| 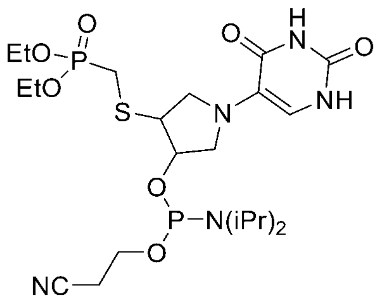 | 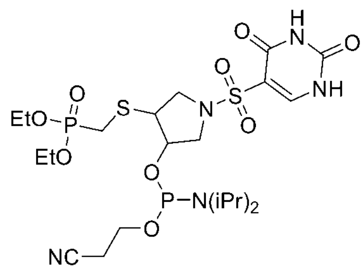 |
| 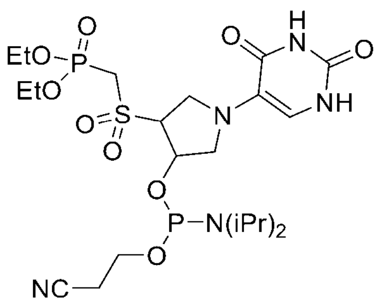 | 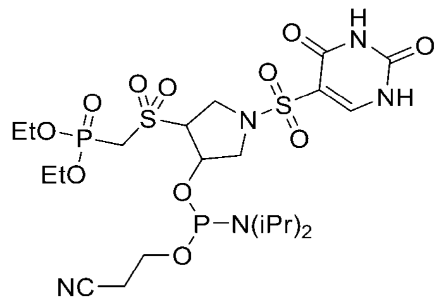 |
| 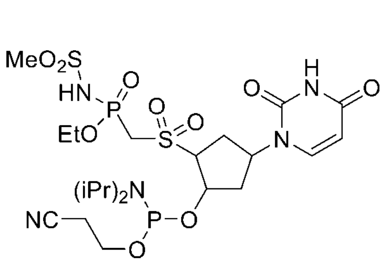 | 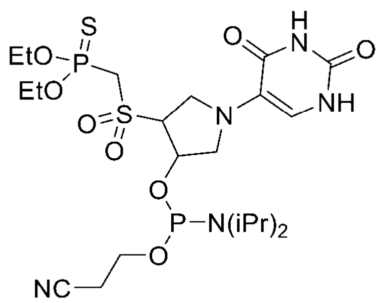 |
| 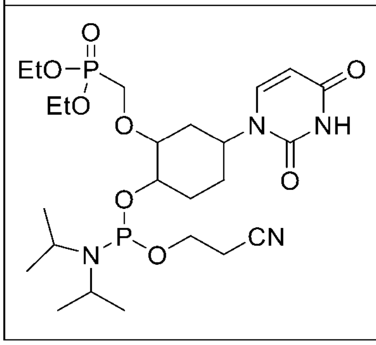 | 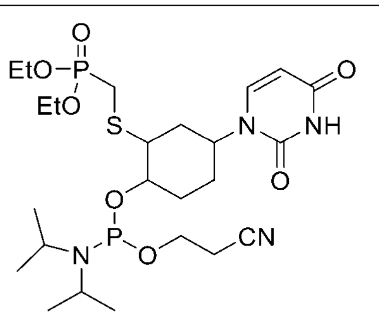 |

| | |
|---|---|
| 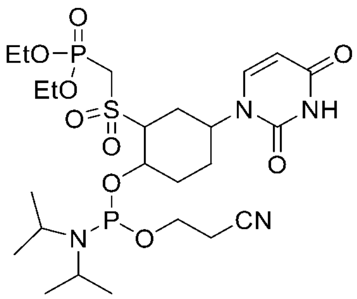 | 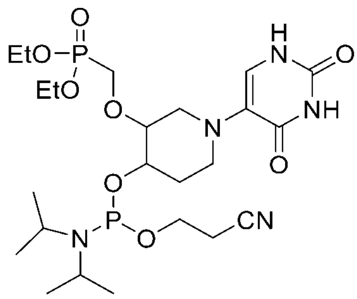 |
| 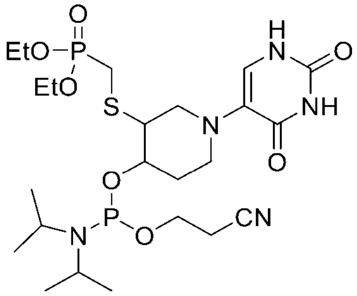 | 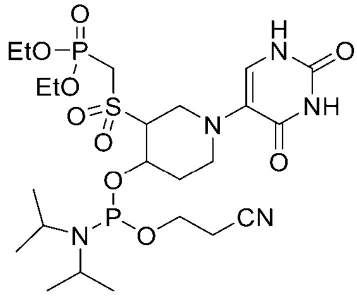 |
| 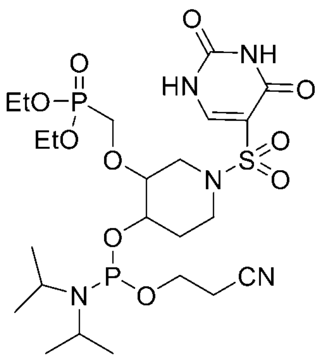 | 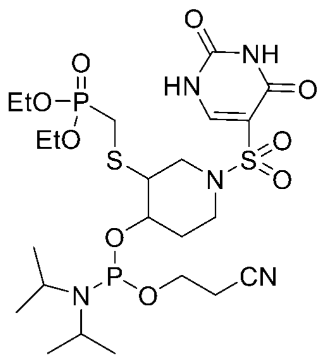 |
| 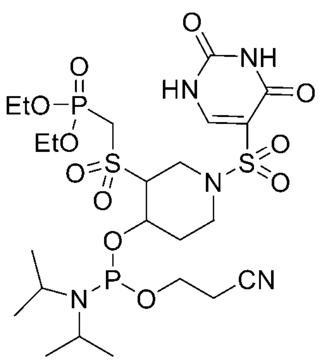 | 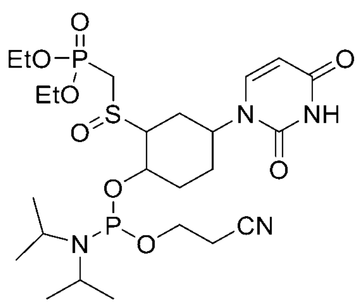 |

| | |
|---|---|
| 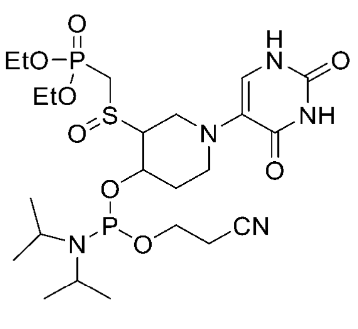 | 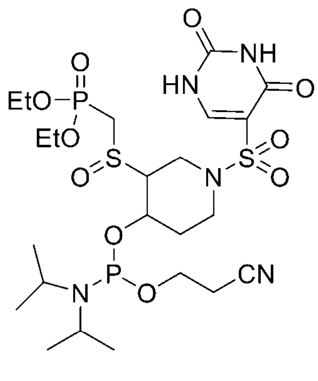 |
| 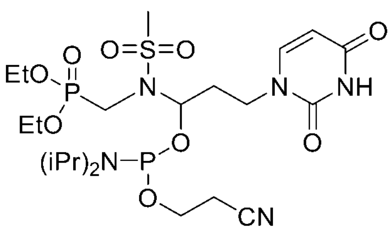 | 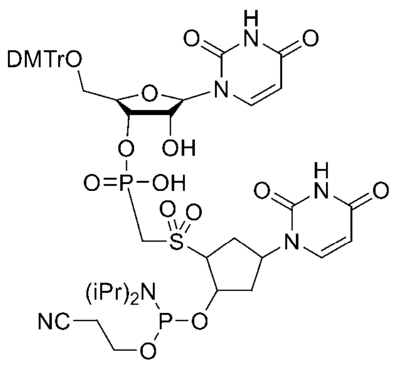 |
| 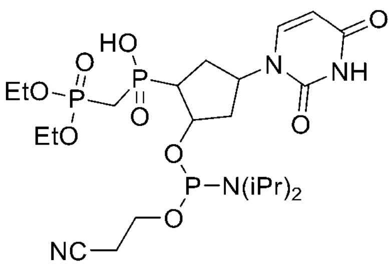 | 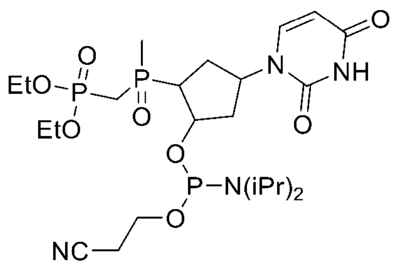 |
| 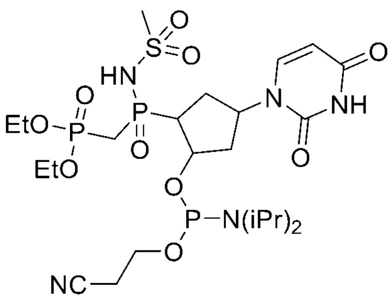 | 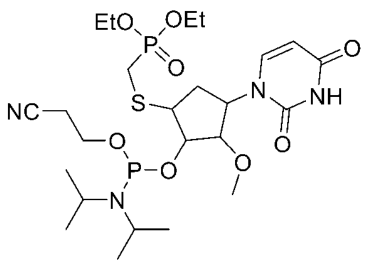 |
| 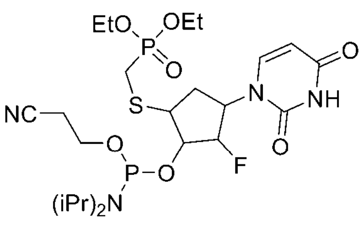 | 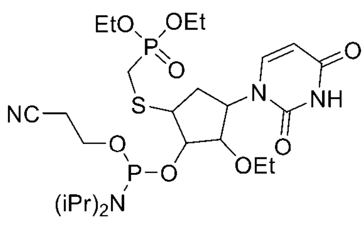 |

| | |
|---|---|
| 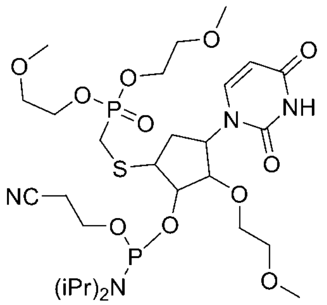 | 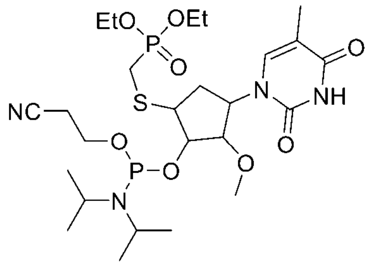 |
| 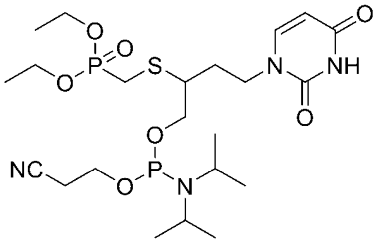 | 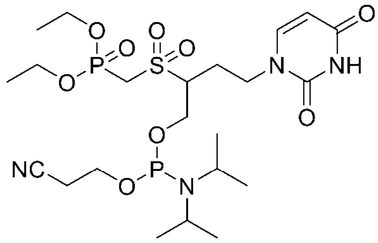 |
| 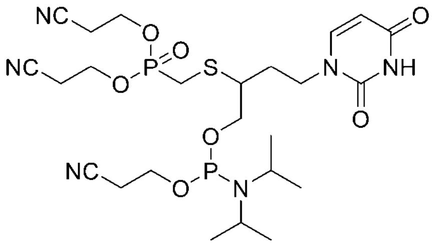 | 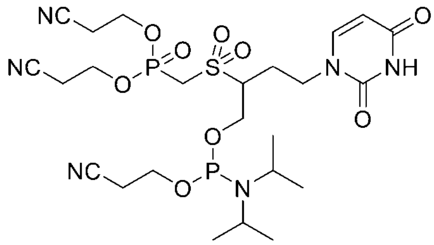 |
| 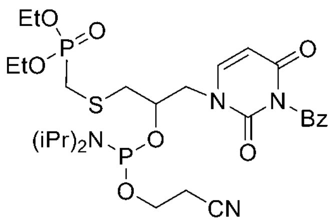 | 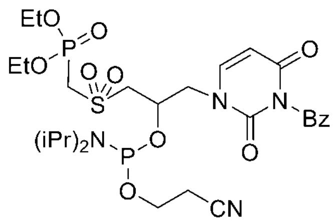 |
| 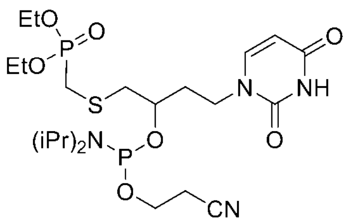 | 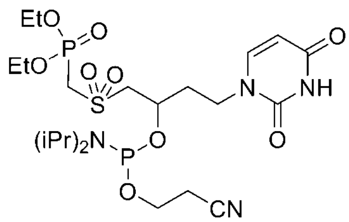 |
| 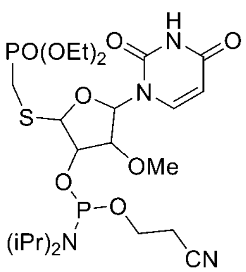 | 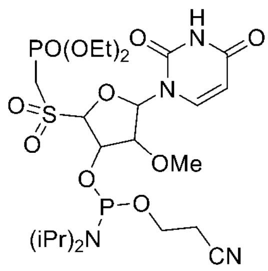 . |

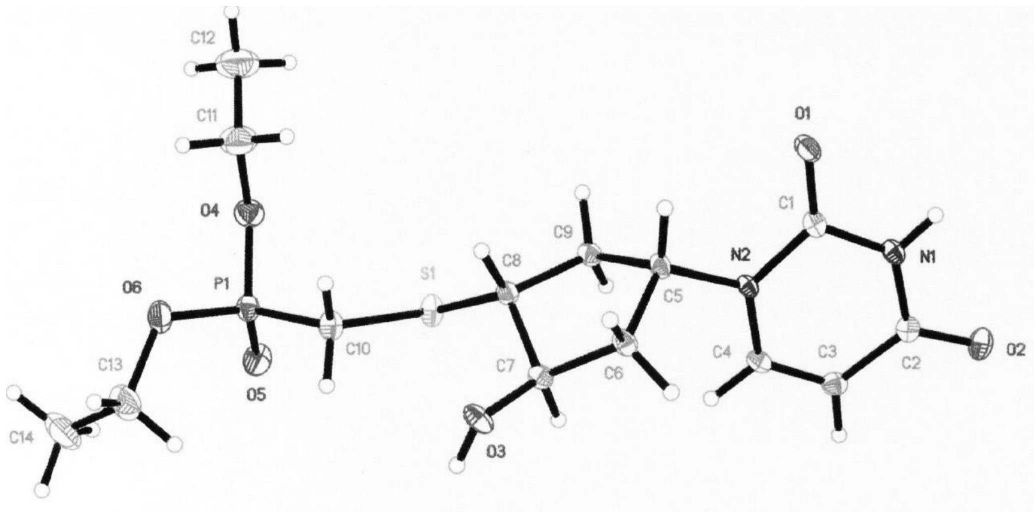
C12
C11
O1
O4
C1
C9
C5
N2
N1
S1
C8
O6
P1
C2
O2
C10
C4
C3
C6
C7
C13
O5
C14
O3

FIG. 1

# INTERNATIONAL SEARCH REPORT

International application No.
**PCT/CN2024/085752**

## A. CLASSIFICATION OF SUBJECT MATTER

C07H21/04(2006.01)i；C07D239/54(2006.01)i；C07F9/141(2006.01)i；C07F9/22(2006.01)i；C07F9/32(2006.01)i；A61P43/00(2006.01)i

According to International Patent Classification (IPC) or to both national classification and IPC

## B. FIELDS SEARCHED

Minimum documentation searched (classification system followed by classification symbols)

C07H, C07D, C07F, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 上海舶望制药有限公司, 邵鹏程, 舒东旭, 顾开春, 寡核苷酸, 核苷, 修饰, 开环, 膦酸酯, Oligonucleotides, Nucleosides, Modifi+, Ring Open, Phosphonate, structural formula search

## C. DOCUMENTS CONSIDERED TO BE RELEVANT

| Category* | Citation of document, with indication, where appropriate, of the relevant passages | Relevant to claim No. |
|---|---|---|
| PX | WO 2023164464 A1 (SANEGENE BIO USA INC.) 31 August 2023 (2023-08-31) abstract, and claims 1-57 | 1-34, 43-129, 138-172 |
| X | CN 103154014 A (ISIS PHARMACEUTICALS INC.) 12 June 2013 (2013-06-12) abstract, claims 1-58, and embodiments 13 and 15-16 | 1-34, 43-129, 138-172 |
| Y | CN 103154014 A (ISIS PHARMACEUTICALS INC.) 12 June 2013 (2013-06-12) abstract, claims 1-58, and embodiments 13 and 15-16 | 1-24, 35-55, 83-118, 130-146, 172 |
| Y | CN 109526222 A (ARROWHEAD PHARMACEUTICALS, INC.) 26 March 2019 (2019-03-26) abstract, and claims 1-30 | 1-24, 35-55, 83-118, 130-146, 172 |
| A | CN 110072530 A (DICERNA PHARMACEUTICALS, INC.) 30 July 2019 (2019-07-30) abstract, and claims 1-49 | 1-172 |
| A | CN 114375296 A (AVIDITY BIOSCIENCES, INC.) 19 April 2022 (2022-04-19) abstract, claims 1-58, embodiment 7, and description, paragraphs 68-69 | 1-172 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2024** | **10 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/085752**

**C. DOCUMENTS CONSIDERED TO BE RELEVANT**

| Category* | Citation of document, with indication, where appropriate, of the relevant passages | Relevant to claim No. |
|---|---|---|
| A | NAVE, J. F. et al. "Enzymatic phosphorylation and pyrophosphorylation of 2',3'-dideoxyadenosine-5'-monophosphate, a key metabolite in the pathway for activation of the anti-HIV (human immunodeficiency virus) agent 2',3'-dideoxyinosine" *Biochemical Pharmacology,* Vol. 48, No. 6, 31 December 1994 (1994-12-31), pp. 1105-1112 | 1-172 |
| A | WOLFF-KUGEL, D. et al. "Studies Towards the Synthesis of the Saturated and Unsaturated Carbocyclic Methylene Phosphonate Analogs of Dideoxyadenosine" *Nucleosides and Nucleotides,* Vol. 12, 31 December 1993 (1993-12-31), pp. 279-294 | 1-172 |

Form PCT/ISA/210 (second sheet) (July 2022)

**INTERNATIONAL SEARCH REPORT**

International application No.
**PCT/CN2024/085752**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/085752**

| Patent document cited in search report | Publication date (day/month/year) | Patent family member(s) | Publication date (day/month/year) |
|---|---|---|---|
| WO 2023164464 A1 | 31 August 2023 | US 2023346819 A1 | 02 November 2023 |
| CN 103154014 A | 12 June 2013 | WO 2011139702 A2 | 10 November 2011 |
| | | WO 2011139702 A3 | 02 May 2013 |
| | | US 2018371005 A1 | 27 December 2018 |
| | | US 11084844 B2 | 10 August 2021 |
| | | US 2015159163 A1 | 11 June 2015 |
| | | US 9321799 B2 | 26 April 2016 |
| | | EP 2601204 A2 | 12 June 2013 |
| | | EP 2601204 B1 | 07 September 2016 |
| | | EP 3173419 A1 | 31 May 2017 |
| | | JP 2013532122 A | 15 August 2013 |
| | | JP 6005628 B2 | 12 October 2016 |
| | | KR 20130105294 A | 25 September 2013 |
| | | KR 101869570 B1 | 20 June 2018 |
| | | US 2013084576 A1 | 04 April 2013 |
| | | US 8993738 B2 | 31 March 2015 |
| | | JP 2016166222 A | 15 September 2016 |
| | | US 2016186185 A1 | 30 June 2016 |
| | | US 10087210 B2 | 02 October 2018 |
| CN 109526222 A | 26 March 2019 | JP 2022059052 A | 12 April 2022 |
| | | CA 3023764 A1 | 14 December 2017 |
| | | KR 20220108204 A | 02 August 2022 |
| | | KR 102639586 B1 | 23 February 2024 |
| | | JP 2019517588 A | 24 June 2019 |
| | | JP 7028865 B2 | 02 March 2022 |
| | | AU 2017279512 A1 | 06 December 2018 |
| | | AU 2017279512 B2 | 19 August 2021 |
| | | TW 201801748 A | 16 January 2018 |
| | | TWI 815794 B | 21 September 2023 |
| | | EP 3464313 A1 | 10 April 2019 |
| | | EP 3464313 A4 | 26 February 2020 |
| | | KR 20190015277 A | 13 February 2019 |
| | | KR 102426487 B1 | 27 July 2022 |
| | | IL 263437 A | 31 January 2019 |
| | | IL 263437 B | 01 March 2022 |
| | | WO 2017214112 A1 | 14 December 2017 |
| | | MX 2018015109 A | 22 April 2019 |
| | | MX 2023008478 A | 27 July 2023 |
| | | AU 2023275805 A1 | 22 February 2024 |
| | | AU 2021266265 A1 | 09 December 2021 |
| | | IL 290633 A | 01 April 2022 |
| | | EA 201892285 A1 | 31 July 2019 |
| | | US 2019085012 A1 | 21 March 2019 |
| | | US 11078227 B2 | 03 August 2021 |
| | | US 2022119439 A1 | 21 April 2022 |
| CN 110072530 A | 30 July 2019 | EP 3506909 A1 | 10 July 2019 |
| | | EP 3506909 A4 | 26 February 2020 |
| | | EP 3506909 B1 | 29 June 2022 |
| | | JP 2019530656 A | 24 October 2019 |
| | | JP 6978099 B2 | 08 December 2021 |

Form PCT/ISA/210 (patent family annex) (July 2022)

**INTERNATIONAL SEARCH REPORT**
**Information on patent family members**

International application No.
**PCT/CN2024/085752**

| Patent document cited in search report | Publication date (day/month/year) | Patent family member(s) | Publication date (day/month/year) |
|---|---|---|---|
| | | EP 4101859 A1 | 14 December 2022 |
| | | AU 2017321892 A1 | 28 February 2019 |
| | | IL 288418 A | 01 January 2022 |
| | | HUE 059718 T2 | 28 December 2022 |
| | | DK 3506909 T3 | 22 August 2022 |
| | | PL 3506909 T3 | 12 September 2022 |
| | | IL 264887 B | 01 January 2022 |
| | | KR 20190044653 A | 30 April 2019 |
| | | KR 102350647 B1 | 14 January 2022 |
| | | PT 3506909 T | 16 August 2022 |
| | | KR 20220010579 A | 25 January 2022 |
| | | KR 102493872 B1 | 30 January 2023 |
| | | WO 2018045317 A1 | 08 March 2018 |
| | | US 2023064031 A1 | 02 March 2023 |
| | | CA 3033756 A1 | 08 March 2018 |
| | | US 2019177729 A1 | 13 June 2019 |
| | | US 11414659 B2 | 16 August 2022 |
| | | MX 2019002339 A | 16 May 2019 |
| | | JP 2022017481 A | 25 January 2022 |
| | | ES 2924806 T3 | 11 October 2022 |
| CN 114375296 A | 19 April 2022 | WO 2020247782 A1 | 10 December 2020 |
| | | KR 20220024419 A | 03 March 2022 |
| | | AU 2020289464 A1 | 20 January 2022 |
| | | US 2020385414 A1 | 10 December 2020 |
| | | US 11578090 B2 | 14 February 2023 |
| | | CA 3142283 A1 | 10 December 2020 |
| | | US 2023234978 A1 | 27 July 2023 |
| | | JP 2022535588 A | 09 August 2022 |
| | | EP 3980436 A1 | 13 April 2022 |
| | | EP 3980436 A4 | 20 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011139702 A
- WO 2017214112 A
- WO 200836127 A
- WO 2011150408 A
- US 6001840 A
- WO 2010036696 A
- US 7399845 B
- WO 2009006478 A
- WO 2008150150729 A
- US 7427672 B
- US 8314227 B
- WO 2016028649 A
- WO 2022007986 A
- US 3687808 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5645985 A
- US 5681941 A
- US 5750692 A
- US 5763588 A
- US 5830653 A
- WO 6005096 A
- US 20070254362, Quay
- WO 9524929 A
- US 5075109 A
- US 4452775 A
- US 4675189 A
- US 5736152 A
- US 3854480 A
- US 5133974 A
- US 5407686 A
- US 5508270 A
- US 4469863 A
- US 5610289 A
- US 5625050 A
- US 5256775 A
- US 5366878 A
- WO 9417093 A
- WO 9402499 A
- US 5476925 A
- US 5023243 A
- US 5378825 A
- US 5386023 A
- US 5489677 A
- US 5602240 A
- CN 110072530 A
- CN 103154014 A


### Non-patent literature cited in the description


- **BEAUCAGE** ; **IYER**. *Tetrahedron*, 1992, vol. 48 (12), 2223-2311
- Carbohydrate Modifications in Antisense Research. ACS Symposium Series, vol. 580, 40-65
- **LEUMANN, CJ.** *Bioorg. & Med. Chem.*, 2002, vol. 10, 841-854
- **ROBEYNS et al.** *J. Am. Chem. Soc.*, 2008, vol. 130 (6), 1979-1984
- **GU et al.** *Nucleosides, Nucleotides & Nucleic Acids*, 2005, vol. 24 (5-7), 993-998
- **NAUWELAERTS et al.** *Nucleic Acids Research*, 2005, vol. 33 (8), 2452-2463
- **GU et al.** *Oligonucleotides*, 2003, vol. 13 (6), 479-489
- **LEUMANN, J.C**. *Bioorganic & Medicinal Chemistry*, 2002, vol. 10, 841-854
- **KOSHKIN et al.** *Tetrahedron*, 1998, vol. 54, 3607-3630
- **MITSUOKA et al.** *Nucleic Acids Res.*, 2009, vol. 37 (4), 1225-38
- **MEGHAN A. et al.** Locked vs. unlocked nucleic acids (LNA vs. UNA): contrasting structures work towards common therapeutic goals. *Chem. Soc. Rev.*, 2011, vol. 40, 5680-5689
- **VAN AERSCHOT et al.** An acyclic 5-nitroindazole nucleoside analogue as ambiguous nucleoside. *NUCLEIC ACIDS RES.*, 11 November 1995, vol. 23 (21), 4363-70

- **LOAKES et al.** 3-Nitropyrrole and 5-nitroindole as universal bases in primers for DNA sequencing and PCR. *NUCLEIC ACIDS RES.*, 11 July 1995, vol. 23 (13), 2361-2366
- **DU**. A systematic analysis of the silencing effects of an active siRNA at all single-nucleotide mismatched target sites.. *Nucleic Acids Res.*, 21 March 2005, vol. 33 (5), 1671-7
- *Nucleic Acids Res.*, 2005, vol. 33 (11), 3698
- **H. S. SAWHNEY** ; **C. P. PATHAK** ; **J. A. HUBELL**. *Macromolecules*, 1993, vol. 26, 581-587
- Current protocols in Nucleic Acid Chemistry. John Wiley & Sons, Inc.
- **BEILSTEIN**. *J Org Chem.*, 2017, vol. 13, 1368-1387